# EUROPEAN PATENT SPECIFICATION

(11) **EP 4 480 479 B1**
(45) Date of publication and mention of the grant of the patent: **09.12.2026**
(21) Application number: 23755867.1
(22) Date of filing: 17.02.2023
(51) Int. Cl.: C07D 471/04, A61K 31/395, A61K 31/435, A61K 31/437, A61K 31/438, A61K 31/4353, A61P 35/00, C07D 401/02, C07D 401/14, C07D 471/02, C07D 471/12, C07D 471/22, C07D 487/04, C07D 487/12, C07D 487/22

(54) **PYRAZOLOPYRIDINE DERIVATIVE AND APPLICATION THEREOF IN MEDICINE**
PYRAZOLOPYRIDINDERIVAT UND ANWENDUNG DAVON IN DER MEDIZIN
DÉRIVÉ DE PYRAZOLOPYRIDINE ET SON APPLICATION EN MÉDECINE

(30) Priority: 18.02.2022 CN 202210137155; 25.03.2022 CN 202210284775; 09.11.2022 CN 202211396761
(43) Date of publication of application: 25.12.2024
(73) Proprietor: Xizang Haisco Pharmaceutical Co., Ltd., Lhoka, Tibet 856099 (CN)
(72) Inventor: ZHANG, Chen, Chengdu, Sichuan 611130 (CN); WANG, Jianmin, Chengdu, Sichuan 611130 (CN); QIAN, Guofei, Chengdu, Sichuan 611130 (CN); YU, Yan, Chengdu, Sichuan 611130 (CN); TANG, Pingming, Chengdu, Sichuan 611130 (CN); CHEN, Tengfei, Chengdu, Sichuan 611130 (CN); LI, Yao, Chengdu, Sichuan 611130 (CN); YAN, Pangke, Chengdu, Sichuan 611130 (CN)
(74) Representative: IP TRUST SERVICES
(86) International application number: PCT/CN2023/076700
(87) International publication number: WO 2023/155866

(56) References cited:
- EP-A1- 2 298 731
- WO-A1-2013/095761
- WO-A1-2015/051713
- WO-A1-2020/132269
- WO-A1-2023/083285
- CN-A- 108 948 002
- CN-A- 109 232 358
- CN-A- 109 311 843
- CN-A- 113 164 485
- CN-A- 113 387 931
- US-A1- 2012 165 305
- XIAOJING WANG ET AL: "Discovery of 5-Azaindazole (GNE-955) as a Potent Pan-Pim Inhibitor with Optimized Bioavailability", JOURNAL OF MEDICINAL CHEMISTRY, vol. 60, no. 10, 5 May 2017 (2017-05-05), US, pages 4458 - 4473, XP055595633, ISSN: 0022-2623, DOI: 10.1021/acs.jmedchem.7b00418

## Description

### Technical Field

The present invention relates to a compound shown in general formula (I) or to a stereoisomer, tautomer, deuterated substance, solvate, pharmaceutically acceptable salt or eutectic crystal thereof, and to an intermediate and a preparation method thereof, and also to an application thereof in the preparation of a medicine for treating diseases related to USP1 activity or expression.

### Background Art

Protein ubiquitination in cells is a key protein modification that can regulate multiple cellular processes. Protein ubiquitination is controlled by E3 ubiquitin ligase and deubiquitinating enzymes (DUBs) in a synergistic manner. DUBs can cleave an isopeptide bond between ubiquitin and a modified protein, and is responsible for removing ubiquitin from the target protein and rescuing it from the degradation pathway; they are also involved in editing, maturation and recycling of ubiquitin molecules after degradation. At present, more than 100 deubiquitinating enzymes are known, and these proteins are subdivided into six subfamilies. Ubiquitin-specific protease (USP) subfamily is the largest subfamily among them and is currently known to have 58 members. USP is a cysteine protease having a highly conserved catalytic domain, and USP1 is one of USP subfamilies among DUBs (Cancers, 2020, 12, 1579; Molecular Cell, 2018, 72, 925-941).

Fanconi Anemia (FA) and DNA Translesion Synthesis (TLS) pathways are the first DNA damage tolerance and repair pathways found to be regulated by reversible ubiquitination. USP1 can participate in the regulation of DNA damage-repair pathway by regulating deubiquitination of specific proteins in FA and TLS pathways (Nature Chemical Biology, 2014, 10, 298-304). USP1 plays an important role in DNA repair in tumor cells. It has been reported that the deletion of USP1 leads to the decrease of survival rate of BRCA1-deficient cells and replication fork degradation (Molecular Cell, 2018, 72, 925-941). UAF1 (USP1-associated factor 1), as an auxiliary factor for USP1, USP12 and USP46, can enhance their activity as deubiquitinating enzymes by forming a stable USP/UAF1 protein complex. The USP1/UAF1 complex deubiquitinates various substrates and is related to DNA repair process, tumor pathogenesis and regulation of antiviral innate immunity (Nat Commun, 2020, 11, 6042). At present, there are no drugs targeting USP1 protein on the market, and the research on USP1 inhibitors has broad application prospects.

WO 2020/132269 A1 provides compounds having Formula I: and the pharmaceutically acceptable salts and solvates thereof, wherein X1, X2, X11, X12, R1, R3, R5, R5', R6, and R7 are defined as set forth in the specification. The present disclosure is also directed to the use of compounds of Formula I to inhibit a USP1 protein and/or to treat a disorder responsive to the inhibition of USP1 proteins and USP1 activity. Compounds of the present disclosure are especially useful for treating cancer.

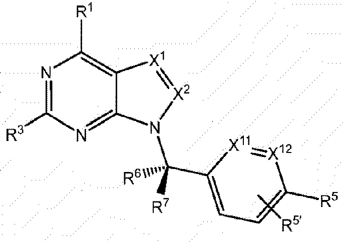

XIAOJING WANG("Discovery of 5-Azaindazole (GNE-955) as a Potent Pan-Pim Inhibitor with Optimized Bioavailability", JOURNAL OF MEDICINAL CHEMISTRY, vol. 60, no. 10, 5 May 2017 (2017-05-05), pages 4458-4473) provides that Pim kinases have been identified as promising therapeutic targets for hematologic-oncology indications, including multiple myeloma and certain leukemia. Here, we describe our continued efforts in optimizing a lead series by improving bioavailability while maintaining high inhibitory potency against all three Pim kinase isoforms. The discovery of extensive intestinal metabolism and major metabolites helped refine our design strategy, and we observed that optimizing the pharmacokinetic properties first and potency second was a more successful approach than the reverse. In the resulting work, novel analogs such as 20 (GNE-955) were discovered bearing 5-azaindazole core with noncanonical hydrogen bonding to the hinge.

WO2023/083285A1 provides small molecules inhibitory compounds of ubiquitin specific protease 1 (USP1) and compositions comprising the same. Further provided herein are methods for targeting ubiquitin specific protease 1 (USP1) and methods of treating diseases or disorders related to USP1, such as cancer.

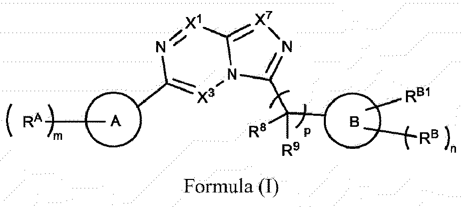

### Summary of the Invention

An object of the present invention is to provide a compound capable of inhibiting USP1 or a stereoisomer, tautomer, deuterated substance, solvate, pharmaceutically acceptable salt or eutectic crystal thereof, an intermediate and a preparation method thereof, and an application thereof in the preparation of a medicine for treating a disease related to USP1 activity or expression.

The present invention provides a compound shown in general formula (I) or a stereoisomer, tautomer, deuterated substance, solvate, pharmaceutically acceptable salt or eutectic crystal thereof, wherein 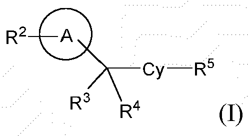
in some embodiments, ring A is selected from 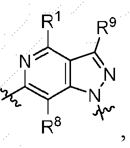 wherein the left side is connected to R²;
in some embodiments, ring A is selected from 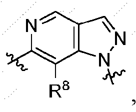 wherein the left side is connected to R²;
in some embodiments, ring A is selected from 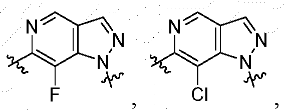 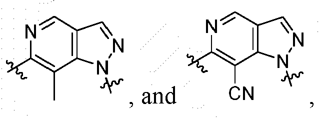 wherein the left side is connected to R²;
in some embodiments, R¹ and R⁹ are each independently selected from H, halogen, cyano, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, or cyano-substituted C₁₋₆ alkyl;
in some embodiments, R¹ and R⁹ are each independently selected from H, halogen, cyano, C₁₋₄ alkyl, C₂₋₄ alkenyl, C₂₋₄ alkynyl, or C₁₋₄ alkoxy, wherein the alkyl, alkenyl, alkynyl, or alkoxy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, or cyano-substituted C₁₋₄ alkyl;
in some embodiments, R¹ and R⁹ are each independently selected from H, F, Cl, Br, I, cyano, methyl, or ethyl;
in some embodiments, R¹ is H;
in some embodiments, R⁹ is H;
in some embodiments, R⁸ is selected from halogen, cyano, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, or cyano-substituted C₁₋₆ alkyl;
in some embodiments, R⁸ is selected from halogen, cyano, C₁₋₄ alkyl, C₂₋₄ alkynyl, and C₁₋₄ alkoxy, wherein the alkyl, alkynyl, or alkoxy is optionally further substituted with 0 to 4 substituents selected from H, halogen, OH, cyano, NH₂, C₁₋₄ alkyl, C₂₋₄ alkynyl, and C₁₋₄ alkoxy;
in some embodiments, R⁸ is selected from F, Cl, Br, I, CN, methyl, ethyl, ethynyl, methoxy, or ethoxy;
in some embodiments, R⁸ is selected from F, Cl, Br, CN, methyl, ethynyl, or methoxy;
in some embodiments, R⁸ is selected from F or Cl;
in some embodiments, R² is selected from C₆₋₁₀ carbocycle or 5- to 10-membered heterocycle, wherein the carbocycle or heterocycle is optionally further substituted with 0 to 4 R^{2a};
in some embodiments, R² is selected from phenyl, 6-membered heteroaryl, benzo C₄₋₆ carbocycle, benzo 4- to 6-membered heterocycle, pyridino C₄₋₆ carbocycle, and pyridino 4- to 6-membered heterocycle;
in some embodiments, R² is selected from phenyl, pyridine, pyrimidine, pyrrole, pyrazole, imidazole, furan, thiophene, thiazole, or oxazole, wherein the phenyl, pyridine, pyrimidine, pyrrole, pyrazole, imidazole, furan, thiophene, thiazole, or oxazole is optionally further substituted with 0 to 4 R^{2a};
in some embodiments, R² is selected from 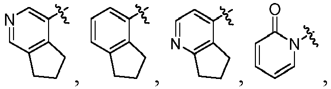 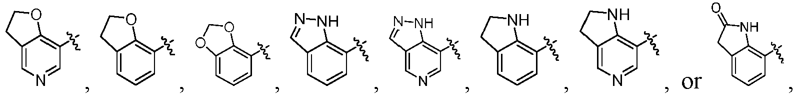 and R² is optionally further substituted with 0 to 4 R^{2a};
in some embodiments, R² is selected from pyrrole, pyrazole, imidazole, pyrimidine, 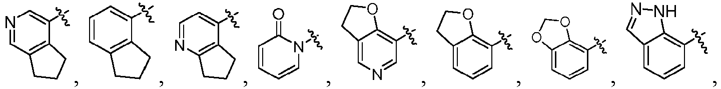 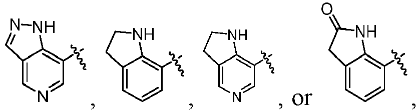 wherein the pyrrole, pyrazole, imidazole, pyrimidine, 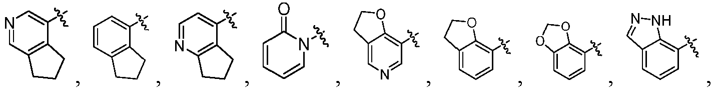 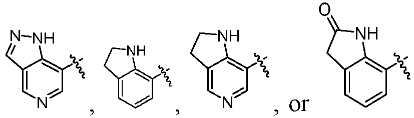 is optionally further substituted with 0 to 4 substituents selected from H, F, Cl, Br, I, cyano, -SO₂-methyl, -SO₂-ethyl, -SO₂-propyl, -SO₂-isopropyl, -SO₂-cyclopropyl, CH₂F, CHF₂, CF₃, -OCH₂F, -OCHF₂, - OCF₃, methyl, ethyl, propyl, isopropyl, methoxy, ethoxy, propoxy, -OCD₃, methylthio, ethylthio, propylthio, cyclopropyl, cyclobutyl, cyclopentyl, cyclohexyl, azetidinyl, azacyclopentyl, azacyclohexyl, piperazine, oxetanyl, oxacyclopentyl, oxacyclohexyl, morpholine, -O-azetidinyl, -O-azacyclopentyl, -O-azacyclohexyl, - O-oxetanyl, -O-oxacyclopentyl, -O-oxacyclohexyl, -OCH₂-phenyl, -OCH₂-cyclopropyl, -OCH₂-cyclobutyl, -OCH₂-cyclopentyl, -OCH₂-cyclohexyl, 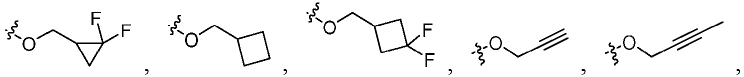 pyrrole, pyrazole, imidazole, thiazole, thiophene, furan, oxazole, 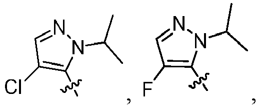 or 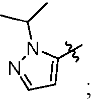
in some embodiments, R² is selected from 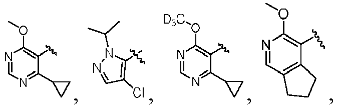 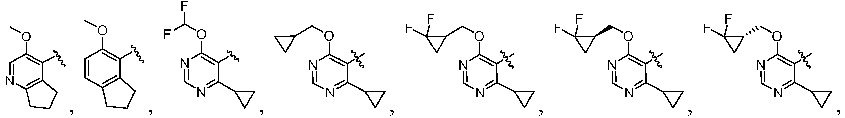 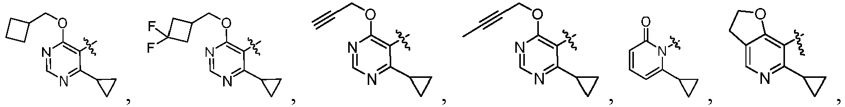 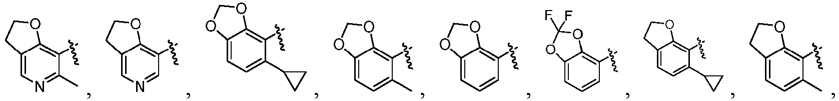 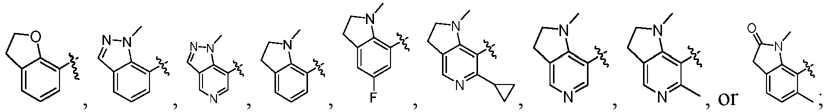
in some embodiments, R² is selected from 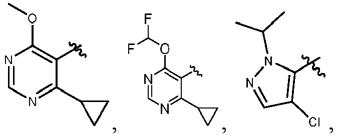 or 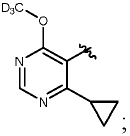
in some embodiments, each R^{2a} is independently selected from H, halogen, OH, =O, cyano, NH₂, NH(C₁₋₆ alkyl), N(C₁₋₆ alkyl)₂, -SO₂-C₁₋₆ alkyl, -SO₂-C₃₋₆ cycloalkyl, C₁₋₆ alkyl, C₁₋₆ alkoxy, C₁₋₆ alkylthio, -Z-C₃₋₆ cycloalkyl, or -Z-3- to 8-membered heterocycle, wherein the alkyl, cycloalkyl, alkoxy, alkylthio, or heterocycle is optionally further substituted with 0 to 4 substituents selected from H, D, halogen, OH, =O, cyano, NH₂, C₁₋₆ alkyl, C₂₋₆ alkynyl, or C₁₋₆ alkoxy, and the heterocycle contains 1 to 4 heteroatoms selected from O, S, and N;
in some embodiments, each R^{2a} is independently selected from H, halogen, OH, =O, cyano, NH₂, NH(C₁₋₄ alkyl), N(C₁₋₄ alkyl)₂, -SO₂-C₁₋₄ alkyl, -SO₂-C₃₋₆ cycloalkyl, C₁₋₄ alkyl, C₁₋₄ alkoxy, C₁₋₄ alkylthio, -Z-C₃₋₆ cycloalkyl, or -Z-3- to 6-membered heterocycle, wherein the alkyl, cycloalkyl, alkoxy, alkylthio, or heterocycle is optionally further substituted with 0 to 4 substituents selected from H, D, halogen, OH, =O, cyano, NH₂, C₁₋₄ alkyl, C₂₋₄ alkynyl, C₁₋₄ alkoxy, and the heterocycle contains 1 to 4 heteroatoms selected from O, S, and N;
in some embodiments, each R^{2a} is independently selected from H, F, Cl, Br, I, or cyano, or each R^{2a} is independently selected from one of the following substituted or unsubstituted groups: -SO₂-methyl, -SO₂-ethyl, -SO₂-propyl, -SO₂-isopropyl, -SO₂-cyclopropyl, methyl, ethyl, propyl, isopropyl, methoxy, ethoxy, propoxy, methylthio, ethylthio, propylthio, cyclopropyl, cyclobutyl, cyclopentyl, cyclohexyl, azetidinyl, azacyclopentyl, azacyclohexyl, piperazine, oxetanyl, oxacyclopentyl, oxacyclohexyl, morpholine, -O-azetidinyl, -O-azacyclopentyl, -O-azacyclohexyl, -O-oxetanyl, -O-oxacyclopentyl, -O-oxacyclohexyl, -OCH₂-phenyl, pyrrole, pyrazole, imidazole, thiazole, thiophene, furan, and oxazole, which, when substituted, are optionally further substituted with 0 to 4 substituents selected from H, D, halogen, OH, =O, cyano, NH₂, C₁₋₄ alkyl, C₂₋₄ alkynyl, and C₁₋₄ alkoxy;
in some embodiments, Z is selected from a bond, O, S, NH, N(C₁₋₄ alkyl), C(=O)NH, NHC(=O), -OCH₂-, -CH₂O-, or C₁₋₃ alkylene;
in some embodiments, R³ and R⁴ are each independently selected from H, halogen, OH, cyano, NH₂, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, or 3- to 8-membered heterocyclyl, and the heterocyclyl contains 1 to 4 heteroatoms selected from O, S, and N;
in some embodiments, R³ and R⁴ are each independently selected from H, halogen, OH, cyano, C₁₋₄ alkyl;
in some embodiments, R³ and R⁴ are each independently selected from H, F, Cl, Br, I, methyl, and ethyl;
in some embodiments, R³ and R⁴ are H;
in some embodiments, Cy is selected from C₃₋₁₂ carbocycle or 4- to 12-membered heterocycle, and the Cy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, or 3- to 8-membered heterocyclyl, wherein the heterocycle contains 1 to 4 heteroatoms selected from O, S, and N;
in some embodiments, Cy is selected from C₃₋₆ monocyclic cycloalkyl, C₅₋₁₁ fused cycloalkyl, C₅₋₁₁ spirocycloalkyl, C₅₋₁₁ bridged cycloalkyl, 4- to 7-membered monocyclic heterocycloalkyl, 6- to 11-membered fused heterocycloalkyl, 6- to 11-membered spiroheterocycloalkyl, 6- to 11-membered bridged heterocycloalkyl, benzene ring, benzo C₄₋₆ carbocycle, benzo 4- to 6-membered heterocycle, 5- to 6-membered heteroaromatic ring, or 8- to 10-membered fused heteroaromatic ring, and the Cy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, or 3- to 8-membered heterocycle, wherein the heterocycle contains 1 to 4 heteroatoms selected from O, S, and N;
in some embodiments, Cy is selected from cyclopropyl, cyclobutyl, cyclopentyl, cyclohexyl, bicyclo[2.2.2]octyl, bicyclo[1.1.1]pentyl, cyclopentyl-fused cyclopentyl, cyclobutylspirocyclohexyl, cyclobutylspirocyclopentyl, azetidinyl, azacyclopentyl, azacyclohexyl, azetidinylspirocyclobutyl, azetidinylspirocyclopentyl, azetidinylspirocyclohexyl, azetidinylspiroazetidinyl, azetidinylspiroazacyclopentyl, azetidinylspiroazacyclohexyl, 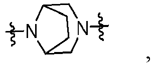 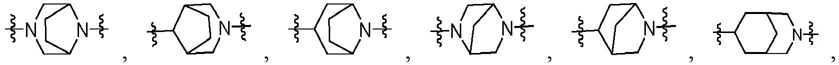 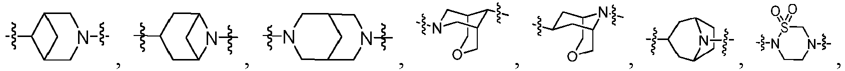 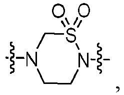 benzene ring, thiophene, furan, pyrrole, thiazole, oxazole, pyrazole, pyrazine, pyrimidine, 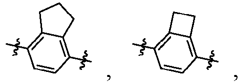 benzothiazole, benzothiophene, benzofuran, benzoxazole, benzopyrrole, benzopyrazole, benzimidazole, indolizine, pyridinopyrrole, pyridinoimidazole, pyridinopyrazole, pyrimidoimidazole, pyrimidopyrazole, pyridinotriazole, or 2-pyridone, and the Cy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, or 3- to 8-membered heterocycle, wherein the heterocycle contains 1 to 4 heteroatoms selected from O, S, and N;
in some embodiments, Cy is selected from 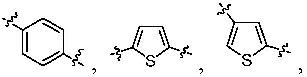 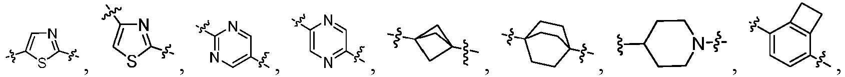 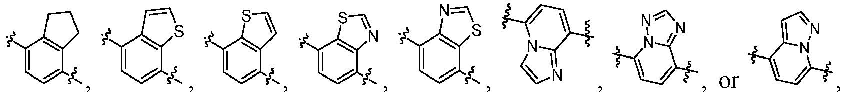 and the Cy is optionally further substituted with 0 to 4 substituents selected from H, F, Cl, Br, I, cyano, CH₂F, CHF₂, CF₃, methyl, ethyl, propyl, isopropyl, methoxy, ethoxy, or cyclopropyl;
in some embodiments, Cy is selected from 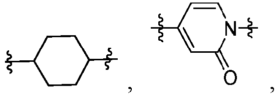 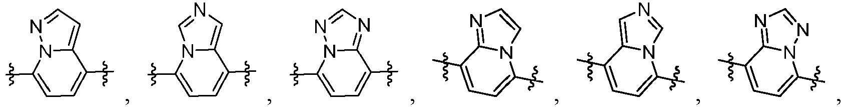 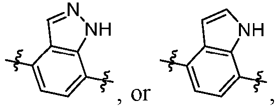 and the Cy is optionally further substituted with 0 to 4 substituents selected from H, F, Cl, Br, I, cyano, CH₂F, CHF₂, CF₃, methyl, ethyl, propyl, isopropyl, methoxy, ethoxy, or cyclopropyl;
in some embodiments, R⁵ is selected from -Z-C₃₋₁₂ carbocycle or -Z-4- to 12-membered heterocycle, and R⁵ is optionally further substituted with 0 to 4 R^{5a};
in some embodiments, R⁵ is selected from -Z-C₆₋₁₂ carbocycle or -Z-5- to 12-membered heterocycle, and R⁵ is optionally further substituted with 0 to 4 R^{5a};
in some embodiments, R⁵ is selected from -Z-C₃₋₁₀ carbocycle or -Z-4- to 10-membered heterocycle, and R⁵ is optionally further substituted with 0 to 4 R^{5a};
in some embodiments, R⁵ is selected from -Z-C₆₋₁₀ carbocycle or -Z-5- to 10-membered heterocycle, and R⁵ is optionally further substituted with 0 to 4 R^{5a};
in some embodiments, Z is selected from a bond, O, S, NH, N(C₁₋₄ alkyl), C(=O)NH, NHC(=O), -OCH₂-, -CH₂O-, or C₁₋₃ alkylene;
in some embodiments, R⁵ is selected from imidazole or imidazo 4- to 9-membered heterocycle, and R⁵ is optionally further substituted with 0 to 4 R^{5a};
in some embodiments, R⁵ is selected from benzene ring, pyrazole, pyrrole, imidazole, triazole, pyridine, pyrimidine, pyrazine, or pyridazine, and R⁵ is optionally further substituted with 0 to 4 R^{5a};
in some embodiments, R⁵ is selected from pyrrole, pyrazole, or imidazole, and the pyrrole, pyrazole, or imidazole is optionally further substituted with 0 to 4 substituents selected from H, F, Cl, Br, I, cyano, CH₂F, CHF₂, CF₃, CD₃, methyl, ethyl, propyl, isopropyl, -CH₂CH₂F, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, azacyclohexyl, 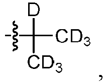 -CH₂-cyclopropyl, - CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, - CH₂-azacyclohexyl, 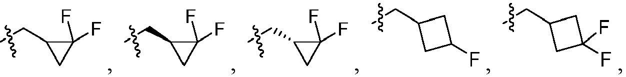 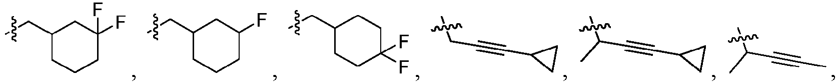 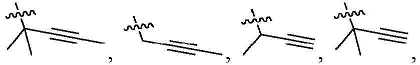 or propargyl;
in some embodiments, R⁵ is selected from 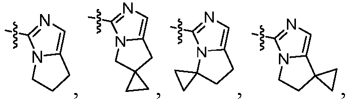 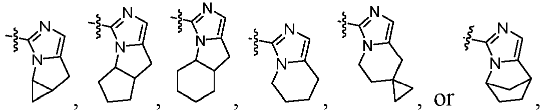 and R⁵ is optionally further substituted with 0 to 4 substituents selected from H, F, Cl, Br, I, cyano, CH₂F, CHF₂, CF₃, CD₃, methyl, ethyl, propyl, isopropyl, -CH₂CH₂F, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, azacyclohexyl, 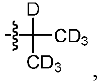 -CH₂-cyclopropyl, -CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, -CH₂-azacyclohexyl, 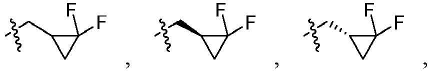 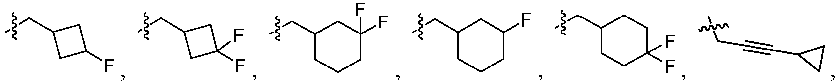 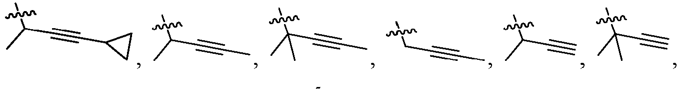 or propargyl;
in some embodiments, R⁵ is selected from pyrrole, pyrazole, or imidazole, wherein the pyrrole, pyrazole, or imidazole is optionally further substituted with 0 to 4 substituents selected from H, F, Cl, Br, I, cyano, CH₂F, CHF₂, CF₃, CD₃, methyl, ethyl, propyl, isopropyl, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, or azacyclohexyl;
in some embodiments, R⁵ is selected from pyrrole, pyrazole, or imidazole, wherein the pyrrole, pyrazole, or imidazole is optionally further substituted with 0 to 4 substituents selected from H, F, Cl, Br, I, cyano, CH₂F, CHF₂, CF₃, methyl, ethyl, propyl, isopropyl, methoxy, ethoxy, cyclopropyl, cyclobutyl, cyclopentyl, or cyclohexyl;
in some embodiments, R⁵ is selected from 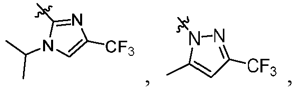 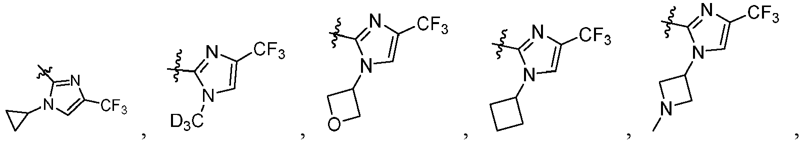 or 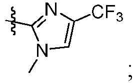
in some embodiments, R⁵ is selected from 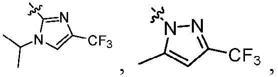 or 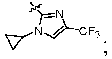
in some embodiments, R⁵ is selected from 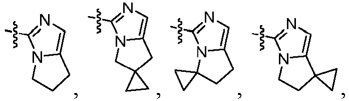 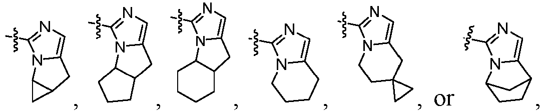 and R⁵ is optionally further substituted with 0 to 4 R^{5a};
in some embodiments, R⁵ is selected from 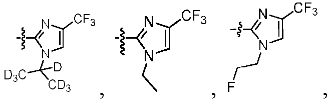 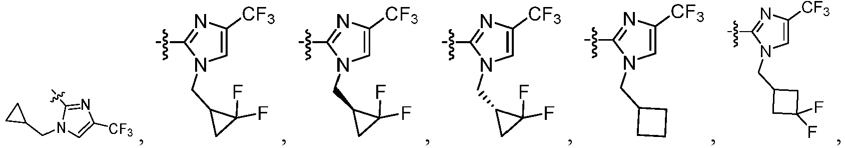 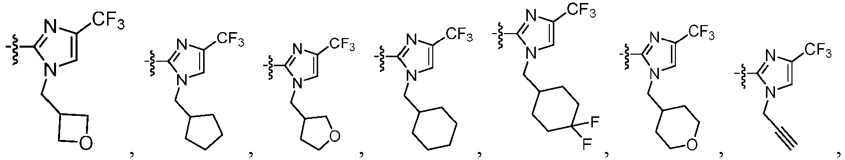 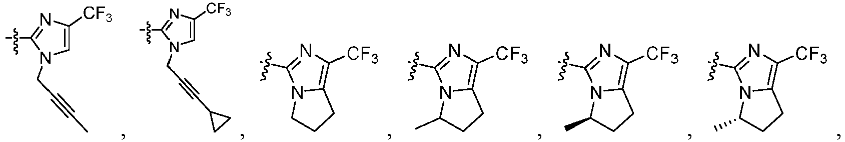 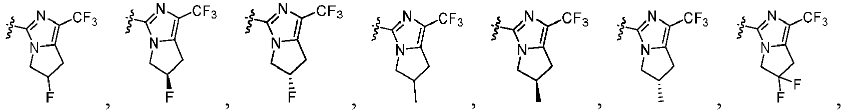 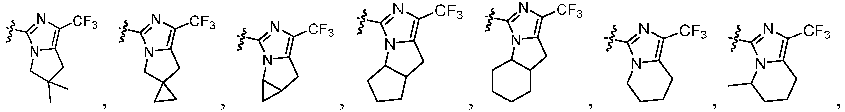 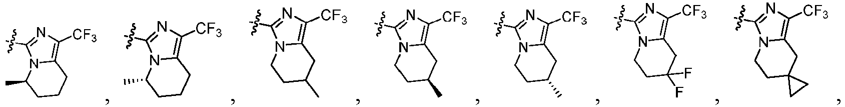 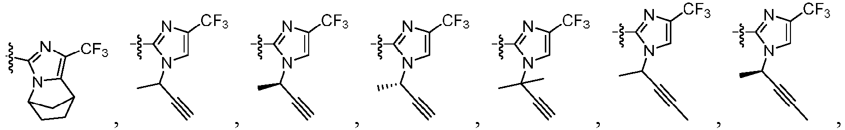 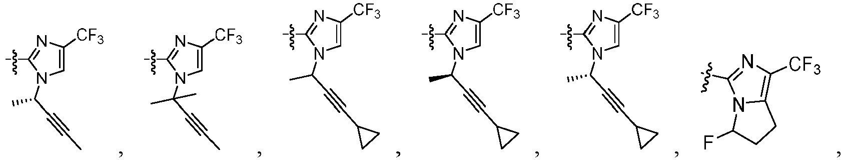 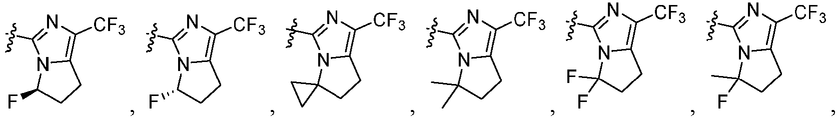 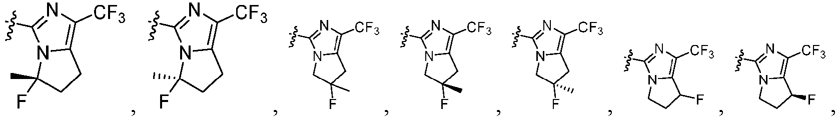 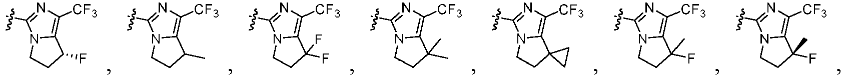 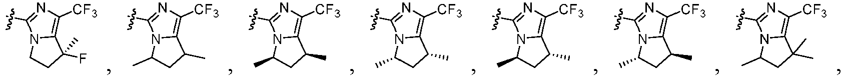 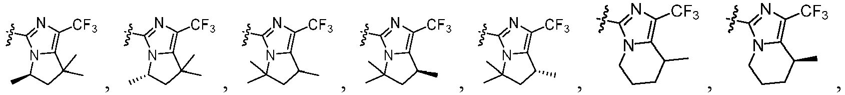 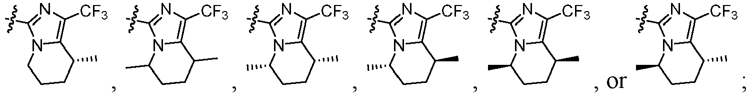
in some embodiments, each R^{5a} is independently selected from H, halogen, OH, cyano, NH₂, NHC₁₋₆ alkyl, N(C₁₋₆ alkyl)₂, C₁₋₆ alkyl, C₂₋₆ alkynyl, C₁₋₆ alkoxy, -C₁₋₄ alkylene-C₃₋₆ cycloalkyl, -C₁₋₄ alkylene-3- to 6-membered heterocycle, C₃₋₆ cycloalkyl, 3- to 8-membered heterocycle, -CONHC₁₋₆ alkyl, -CONHC₁₋₆ alkoxy, - CONHC₃₋₆ cycloalkyl, -CONH 3- to 8-membered heterocycle, -NHCO-C₁₋₆ alkyl, - NHCO-C₁₋₆ alkoxy, -NHCO-C₃₋₆ cycloalkyl, or -NHCO-3- to 8-membered heterocycle, wherein the alkyl, alkylene, alkynyl, alkoxy, cycloalkyl, or heterocycle is optionally further substituted with 0 to 4 substituents selected from H, D, halogen, OH, =O, cyano, NH₂, -C(=O)NH₂, -C(=O)NHC₁₋₆ alkyl, - C(=O)N(C₁₋₆ alkyl)₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, halogen-substituted C₃₋₆ cycloalkyl, or 3- to 8-membered heterocycle, and the heterocycle contains 1 to 4 heteroatoms selected from O, S, and N;
in some embodiments, each R^{5a} is independently selected from H, halogen, OH, cyano, NH₂, C₁₋₄ alkyl, C₂₋₄ alkynyl, C₁₋₄ alkoxy, -C₁₋₂ alkylene-C₃₋₆ cycloalkyl, -C₁₋₂ alkylene-3- to 6-membered heterocycle, C₃₋₆ cycloalkyl, or 3- to 6-membered heterocycle, wherein the alkyl, alkylene, alkynyl, alkoxy, cycloalkyl, or heterocycle is optionally further substituted with 0 to 4 substituents selected from H, D, halogen, OH, =O, cyano, NH₂, -C(=O)NH₂, -C(=O)NHC₁₋₄ alkyl, - C(=O)N(C₁₋₄ alkyl)₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, halogen-substituted C₃₋₆ cycloalkyl, or 3- to 8-membered heterocycle, and the heterocycle contains 1 to 4 heteroatoms selected from O, S, and N;
in some embodiments, each R^{5a} is independently selected from H, F, Cl, Br, I, cyano, methyl, ethyl, propyl, isopropyl, propargyl, butargyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, -CH₂-cyclopropyl, - CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, or - CH₂-azacyclohexyl, wherein the methyl, ethyl, propyl, isopropyl, propargyl, butargyl, methoxy, ethoxy, cyclopropyl, cyclobutyl, cyclopentyl, cyclohexyl, oxetanyl, oxacyclopentyl, oxacyclohexyl, azetidinyl, azacyclopentyl, or azacyclohexyl is optionally further substituted with 0 to 4 substituents selected from H, halogen, OH, =O, cyano, NH₂, C₁₋₄ alkyl, C₂₋₄ alkynyl, C₁₋₄ alkoxy, C₃₋₆ cycloalkyl, or halogen-substituted C₃₋₆ cycloalkyl;
in some embodiments, the compound shown in general formula (I) is selected from a compound shown in general formula (Ia), and the definitions of the groups in the compound shown in general formula (Ia) are the same as the definitions of those in the compound shown in general formula (I).

A first embodiment of the present invention is a compound shown in the above general formula (I) or a stereoisomer, tautomer, deuterated substance, solvate, pharmaceutically acceptable salt or eutectic crystal thereof,
wherein ring A is selected from 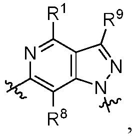 and the left side is connected to R²;
R¹ and R⁹ are each independently selected from H, halogen, cyano, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, or cyano-substituted C₁₋₆ alkyl;
R⁸ is selected from halogen, cyano, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, or cyano-substituted C₁₋₆ alkyl;
R² is selected from C₆₋₁₀ carbocycle or 5- to 10-membered heterocycle, wherein the carbocycle or heterocycle is optionally further substituted with 0 to 4 R^{2a};
each R^{2a} is independently selected from H, halogen, OH, =O, cyano, NH₂, NH(C₁₋₆ alkyl), N(C₁₋₆ alkyl)₂, -SO₂-C₁₋₆ alkyl, -SO₂-C₃₋₆ cycloalkyl, C₁₋₆ alkyl, C₁₋₆ alkoxy, C₁₋₆ alkylthio, -Z-C₃₋₆ cycloalkyl, or -Z-3- to 8-membered heterocycle, wherein the alkyl, cycloalkyl, alkoxy, alkylthio, or heterocycle is optionally further substituted with 0 to 4 substituents selected from H, D, halogen, OH, =O, cyano, NH₂, C₁₋₆ alkyl, C₂₋₆ alkynyl, C₁₋₆ alkoxy, or C₃₋₆ cycloalkyl, and the heterocycle contains 1 to 4 heteroatoms selected from O, S, and N;
R³ and R⁴ are each independently selected from H, halogen, OH, cyano, NH₂, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, or 3- to 8-membered heterocyclyl, and the heterocyclyl contains 1 to 4 heteroatoms selected from O, S, and N;
Cy is selected from C₃₋₁₂ carbocycle or 4- to 12-membered heterocycle, and the Cy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, or 3- to 8-membered heterocyclyl, wherein the heterocycle contains 1 to 4 heteroatoms selected from O, S, and N;
R⁵ is selected from -Z-C₃₋₁₂ carbocycle or -Z-4- to 12-membered heterocycle, and the R⁵ is optionally further substituted with 0 to 4 R^{5a};
each R^{5a} is independently selected from H, halogen, OH, cyano, NH₂, NHC₁₋₆ alkyl, N(C₁₋₆ alkyl)₂, C₁₋₆ alkyl, C₂₋₆ alkynyl, C₁₋₆ alkoxy, -C₁₋₄ alkylene-C₃₋₆ cycloalkyl, -C₁₋₄ alkylene-3- to 6-membered heterocycle, C₃₋₆ cycloalkyl, 3- to 8-membered heterocycle, -CONHC₁₋₆ alkyl, -CONHC₁₋₆ alkoxy, -CONHC₃₋₆ cycloalkyl, -CONH 3- to 8-membered heterocycle, -NHCO-C₁₋₆ alkyl, -NHCO-C₁₋₆ alkoxy, -NHCO-C₃₋₆ cycloalkyl, or -NHCO-3- to 8-membered heterocycle, wherein the alkyl, alkylene, alkynyl, alkoxy, cycloalkyl, or heterocycle is optionally further substituted with 0 to 4 substituents selected from H, D, halogen, OH, =O, cyano, NH₂, -C(=O)NH₂, -C(=O)NHC₁₋₆ alkyl, -C(=O)N(C₁₋₆ alkyl)₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, halogen-substituted C₃₋₆ cycloalkyl, or 3- to 8-membered heterocycle, and the heterocycle contains 1 to 4 heteroatoms selected from O, S, and N;
Z is selected from a bond, O, S, NH, N(C₁₋₄ alkyl), C(=O)NH, NHC(=O), - OCH₂-, -CH₂O-, or C₁₋₃ alkylene.

A second embodiment of the present invention is a compound shown in general formula (Ia) below or a stereoisomer, tautomer, deuterated substance, solvate, pharmaceutically acceptable salt or eutectic crystal thereof, 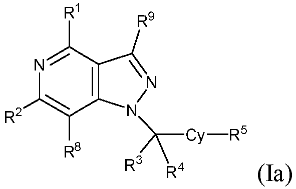
wherein R¹ and R⁹ are each independently selected from H, halogen, cyano, C₁₋₄ alkyl, C₂₋₄ alkenyl, C₂₋₄ alkynyl, or C₁₋₄ alkoxy, wherein the alkyl, alkenyl, alkynyl, or alkoxy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, or cyano-substituted C₁₋₄ alkyl;
R⁸ is selected from halogen, cyano, C₁₋₄ alkyl, C₂₋₄ alkynyl, and C₁₋₄ alkoxy, wherein the alkyl, alkynyl, or alkoxy is optionally further substituted with 0 to 4 substituents selected from H, halogen, OH, cyano, NH₂, C₁₋₄ alkyl, C₂₋₄ alkynyl, and C₁₋₄ alkoxy;
each R^{2a} is independently selected from H, halogen, OH, =O, cyano, NH₂, NH(C₁₋₄ alkyl), N(C₁₋₄ alkyl)₂, -SO₂-C₁₋₄ alkyl, -SO₂-C₃₋₆ cycloalkyl, C₁₋₄ alkyl, C₁₋₄ alkoxy, C₁₋₄ alkylthio, -Z-C₃₋₆ cycloalkyl, or -Z-3- to 6-membered heterocycle, wherein the alkyl, cycloalkyl, alkoxy, alkylthio, or heterocycle is optionally further substituted with 0 to 4 substituents selected from H, D, halogen, OH, =O, cyano, NH₂, C₁₋₄ alkyl, C₂₋₄ alkynyl, C₁₋₄ alkoxy, or C₃₋₆ cycloalkyl, and the heterocycle contains 1 to 4 heteroatoms selected from O, S, and N;
R³ and R⁴ are each independently selected from H, halogen, OH, cyano, and C₁₋₄ alkyl;
Cy is selected from C₃₋₆ monocyclic cycloalkyl, C₅₋₁₁ fused cycloalkyl, C₅₋₁₁ spirocycloalkyl, C₅₋₁₁ bridged cycloalkyl, 4- to 7-membered monocyclic heterocycloalkyl, 6- to 11-membered fused heterocycloalkyl, 6- to 11-membered spiroheterocycloalkyl, 6- to 11-membered bridged heterocycloalkyl, benzene ring, benzo C₄₋₆ carbocycle, benzo 4- to 6-membered heterocycle, 5- to 6-membered heteroaromatic ring, or 8- to 10-membered fused heteroaromatic ring, and the Cy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, or 3- to 8-membered heterocycle, wherein the heterocycle contains 1 to 4 heteroatoms selected from O, S, and N;
R⁵ is selected from -Z-C₆₋₁₂ carbocycle or -Z-5- to 12-membered heterocycle, and the R⁵ is optionally further substituted with 0 to 4 R^{5a};
each R^{5a} is independently selected from H, halogen, OH, cyano, NH₂, C₁₋₄ alkyl, C₂₋₄ alkynyl, C₁₋₄ alkoxy, -C₁₋₂ alkylene-C₃₋₆ cycloalkyl, -C₁₋₂ alkylene-3- to 6-membered heterocycle, C₃₋₆ cycloalkyl, or 3- to 6-membered heterocycle, wherein the alkyl, alkylene, alkynyl, alkoxy, cycloalkyl, or heterocycle is optionally further substituted with 0 to 4 substituents selected from H, D, halogen, OH, =O, cyano, NH₂, -C(=O)NH₂, -C(=O)NHC₁₋₄ alkyl, -C(=O)N(C₁₋₄ alkyl)₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, halogen-substituted C₃₋₆ cycloalkyl, or 3- to 8-membered heterocycle, and the heterocycle contains 1 to 4 heteroatoms selected from O, S, and N;
the definitions of the remaining substituents are the same as those in the first solution of the present invention.

A third embodiment of the present invention is a compound shown in general formula (Ia) above or a stereoisomer, tautomer, deuterated substance, solvate, pharmaceutically acceptable salt or eutectic crystal thereof, wherein
R¹ and R⁹ are each independently selected from H, F, Cl, Br, I, cyano, methyl, or ethyl;
R⁸ is selected from F, Cl, Br, I, CN, methyl, ethyl, ethynyl, methoxy, or ethoxy;
R² is selected from phenyl, pyridine, pyrimidine, pyrrole, pyrazole, imidazole, furan, thiophene, thiazole, or oxazole, wherein the phenyl, pyridine, pyrimidine, pyrrole, pyrazole, imidazole, furan, thiophene, thiazole, or oxazole is optionally further substituted with 0 to 4 R^{2a};
or R² is selected from 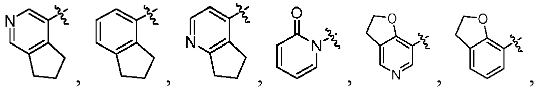 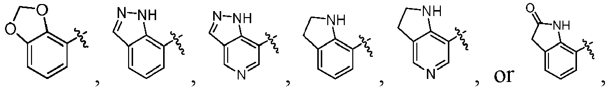 and R² is optionally further substituted with 0 to 4 R^{2a};
each R^{2a} is independently selected from H, F, Cl, Br, I, or cyano, or each R^{2a} is independently selected from one of the following substituted or unsubstituted groups: -SO₂-methyl, -SO₂-ethyl, -SO₂-propyl, -SO₂-isopropyl, -SO₂-cyclopropyl, methyl, ethyl, propyl, isopropyl, methoxy, ethoxy, propoxy, methylthio, ethylthio, propylthio, cyclopropyl, cyclobutyl, cyclopentyl, cyclohexyl, azetidinyl, azacyclopentyl, azacyclohexyl, piperazine, oxetanyl, oxacyclopentyl, oxacyclohexyl, morpholine, -O-azetidinyl, -O-azacyclopentyl, -O-azacyclohexyl, - O-oxetanyl, -O-oxacyclopentyl, -O-oxacyclohexyl, -OCH₂-phenyl, -OCH₂-cyclopropyl, -OCH₂-cyclobutyl, -OCH₂-cyclopentyl, -OCH₂-cyclohexyl, pyrrole, pyrazole, imidazole, thiazole, thiophene, furan, and oxazole, which, when substituted, are optionally further substituted with 0 to 4 substituents selected from H, D, halogen, OH, =O, cyano, NH₂, C₁₋₄ alkyl, C₂₋₄ alkynyl, C₁₋₄ alkoxy, and C₃₋₆ cycloalkyl;
R³ and R⁴ are each independently selected from H, F, Cl, Br, I, methyl, and ethyl;
Cy is selected from cyclopropyl, cyclobutyl, cyclopentyl, cyclohexyl, bicyclo[2.2.2]octyl, bicyclo[1.1.1]pentyl, cyclopentyl-fused cyclopentyl, cyclobutylspirocyclohexyl, cyclobutylspirocyclopentyl, azetidinyl, azacyclopentyl, azacyclohexyl, azetidinylspirocyclobutyl, azetidinylspirocyclopentyl, azetidinylspirocyclohexyl, azetidinylspiroazetidinyl, azetidinylspiroazacyclopentyl, azetidinylspiroazacyclohexyl, 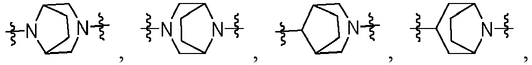 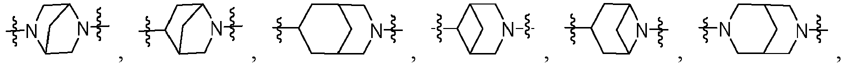 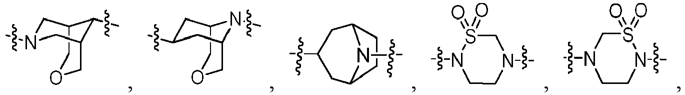 benzene ring, thiophene, furan, pyrrole, thiazole, oxazole, pyrazole, pyrazine, pyrimidine, 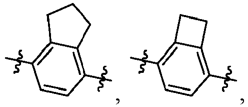 benzothiazole, benzothiophene, benzofuran, benzoxazole, benzopyrrole, benzopyrazole, benzimidazole, indolizine, pyridinopyrrole, pyridinoimidazole, pyridinopyrazole, pyrimidoimidazole, pyrimidopyrazole, pyridinotriazole, or 2-pyridone, and the Cy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, or 3- to 8-membered heterocycle, wherein the heterocycle contains 1 to 4 heteroatoms selected from O, S, and N;
R⁵ is selected from benzene ring, pyrazole, pyrrole, imidazole, triazole, pyridine, pyrimidine, pyrazine, or pyridazine, and the R⁵ is optionally further substituted with 0 to 4 R^{5a};
or R⁵ is selected from 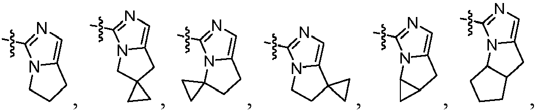 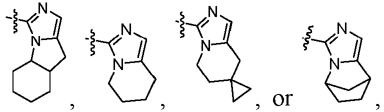 and the R⁵ is optionally further substituted with 0 to 4 R^{5a};
each R^{5a} is independently selected from F, Cl, Br, I, cyano, methyl, ethyl, propyl, isopropyl, propargyl, butargyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, -CH₂-cyclopropyl, -CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, or -CH₂-azacyclohexyl, wherein the methyl, ethyl, propyl, isopropyl, propargyl, butargyl, methoxy, ethoxy, cyclopropyl, cyclobutyl, cyclopentyl, cyclohexyl, oxetanyl, oxacyclopentyl, oxacyclohexyl, azetidinyl, azacyclopentyl, or azacyclohexyl is optionally further substituted with 0 to 4 substituents selected from H, halogen, OH, =O, cyano, NH₂, C₁₋₄ alkyl, C₂₋₄ alkynyl, C₁₋₄ alkoxy, C₃₋₆ cycloalkyl, or halogen-substituted C₃₋₆ cycloalkyl;
the definitions of the remaining substituents are the same as those in the first or second solution of the present invention.

A fourth embodiment of the present invention is a compound shown in general formula (Ia) above or a stereoisomer, tautomer, deuterated substance, solvate, pharmaceutically acceptable salt or eutectic crystal thereof, wherein
R¹ and R⁹ are H;
R⁸ is selected from F, Cl, Br, CN, methyl, ethynyl, or methoxy;
R² is selected from pyrrole, pyrazole, imidazole, pyrimidine, 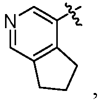 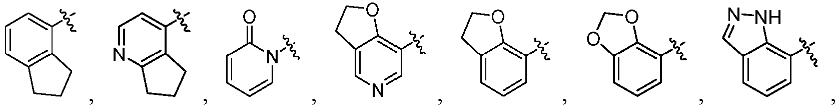 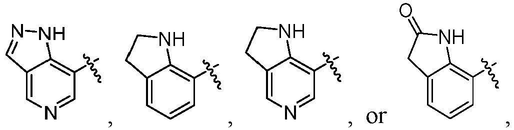 wherein the pyrrole, pyrazole, imidazole, pyrimidine, 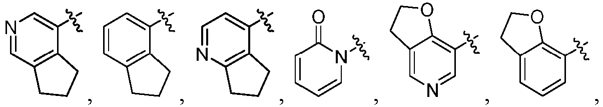 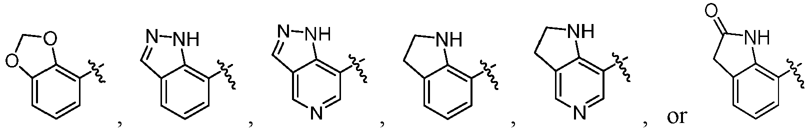 is optionally further substituted with 0 to 4 substituents selected from H, F, Cl, Br, I, cyano, -SO₂-methyl, -SO₂-ethyl, -SO₂-propyl, -SO₂-isopropyl, -SO₂-cyclopropyl, CH₂F, CHF₂, CF₃, -OCH₂F, -OCHF₂, -OCF₃, methyl, ethyl, propyl, isopropyl, methoxy, ethoxy, propoxy, -OCD₃, methylthio, ethylthio, propylthio, cyclopropyl, cyclobutyl, cyclopentyl, cyclohexyl, azetidinyl, azacyclopentyl, azacyclohexyl, piperazine, oxetanyl, oxacyclopentyl, oxacyclohexyl, morpholine, -O-azetidinyl, - O-azacyclopentyl, -O-azacyclohexyl, -O-oxetanyl, -O-oxacyclopentyl, -O-oxacyclohexyl, -OCH₂-phenyl, -OCH₂-cyclopropyl, -OCH₂-cyclobutyl, -OCH₂-cyclopentyl, -OCH₂-cyclohexyl, 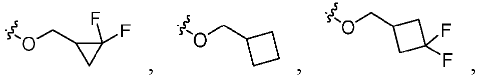 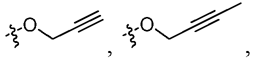 pyrrole, pyrazole, imidazole, thiazole, thiophene, furan, oxazole, 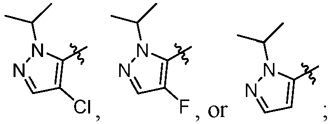
Cy is selected from 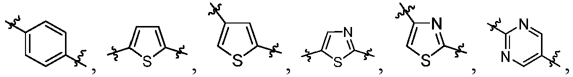 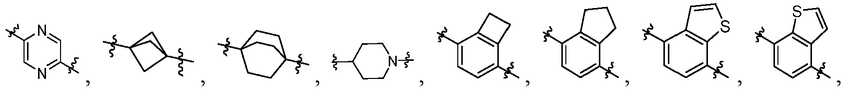 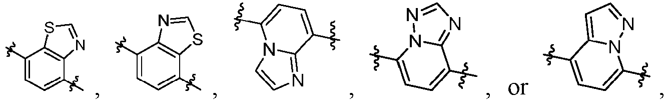 and the Cy is optionally further substituted with 0 to 4 substituents selected from H, F, Cl, Br, I, cyano, CH₂F, CHF₂, CF₃, methyl, ethyl, propyl, isopropyl, methoxy, ethoxy, or cyclopropyl;
alternatively, Cy is selected from 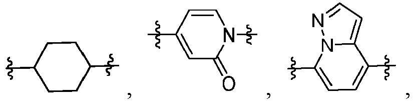 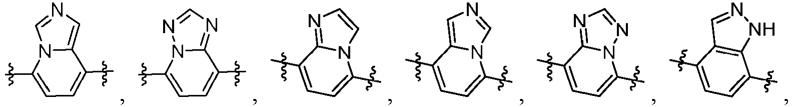 and 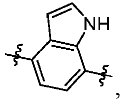 and the Cy is optionally further substituted with 0 to 4 substituents selected from H, F, Cl, Br, I, cyano, CH₂F, CHF₂, CF₃, methyl, ethyl, propyl, isopropyl, methoxy, ethoxy, or cyclopropyl;
R⁵ is selected from pyrrole, pyrazole, or imidazole, and the pyrrole, pyrazole, or imidazole is optionally further substituted with 0 to 4 substituents selected from H, F, Cl, Br, I, cyano, CH₂F, CHF₂, CF₃, CD₃, methyl, ethyl, propyl, isopropyl, - CH₂CH₂F, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, azacyclohexyl, 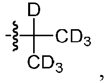 -CH₂-cyclopropyl, -CH₂-cyclobutyl, - CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, -CH₂-azacyclohexyl, 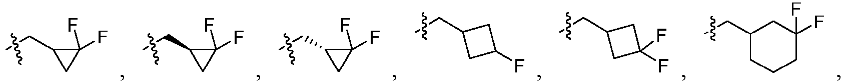 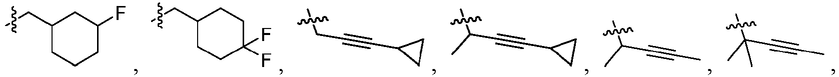 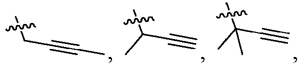 or propargyl;
alternatively, R⁵ is selected from 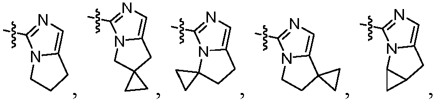 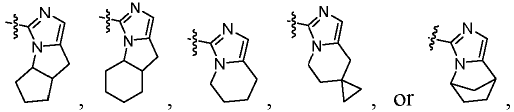 and R⁵ is optionally further substituted with 0 to 4 substituents selected from H, F, Cl, Br, I, cyano, CH₂F, CHF₂, CF₃, CD₃, methyl, ethyl, propyl, isopropyl, -CH₂CH₂F, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, azacyclohexyl, 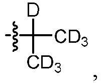 -CH₂-cyclopropyl, -CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, -CH₂-azacyclohexyl, 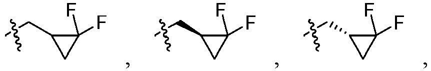 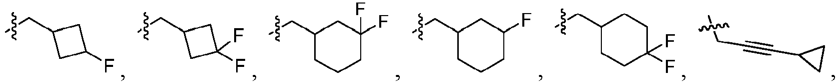 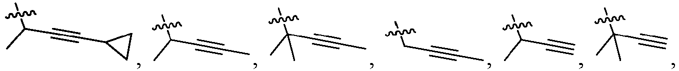 or propargyl;
the definitions of the remaining substituents are the same as those in the first, second, or third solution of the present invention.

A fifth embodiment of the present invention is a compound shown in general formula (Ia) above or a stereoisomer, tautomer, deuterated substance, solvate, pharmaceutically acceptable salt or eutectic crystal thereof, wherein
R² is selected from 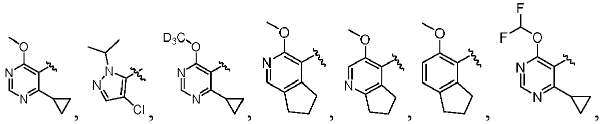 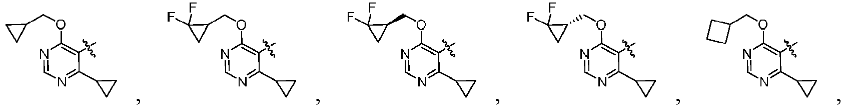 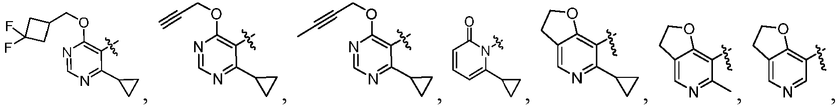 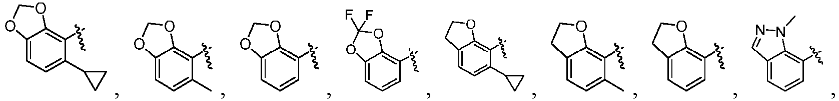 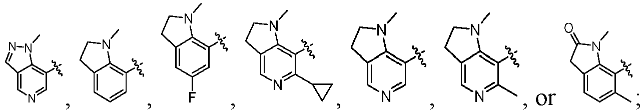
R⁵ is selected from 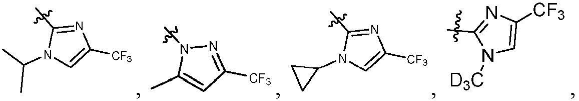 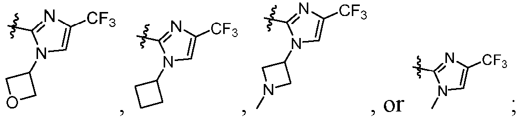
R⁵ is selected from 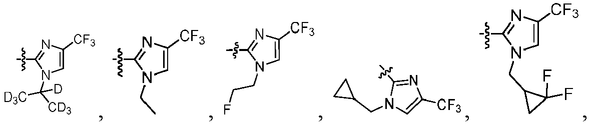 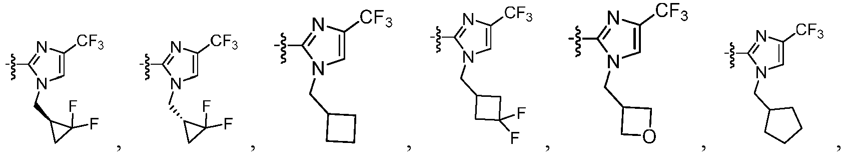 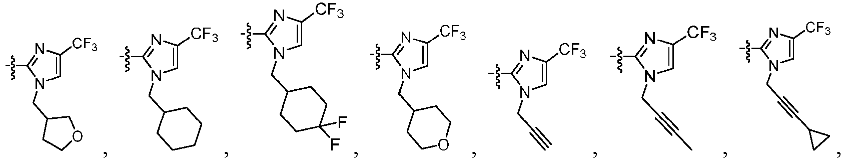 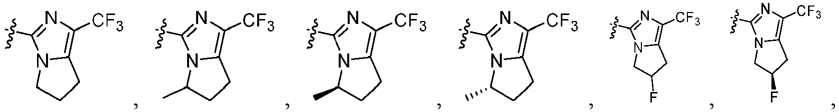 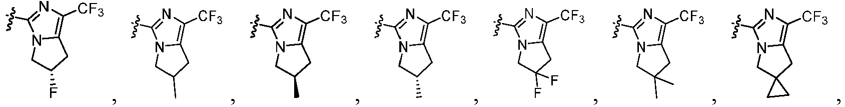 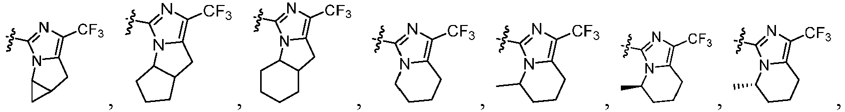 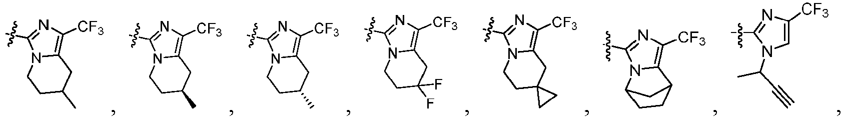 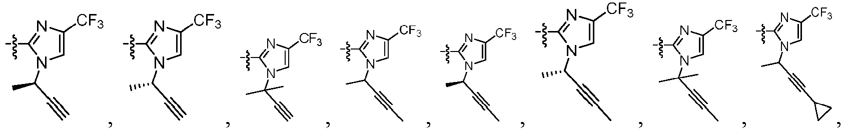 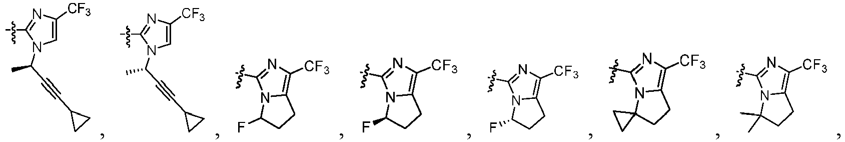 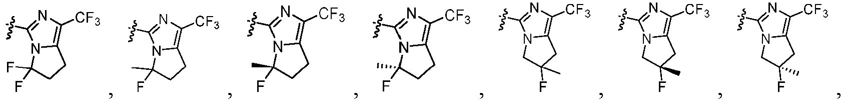 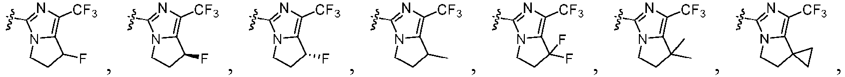 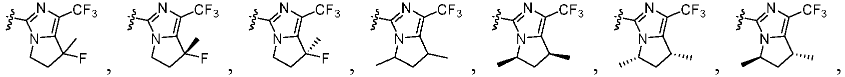 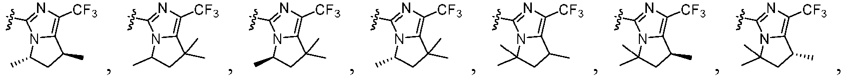 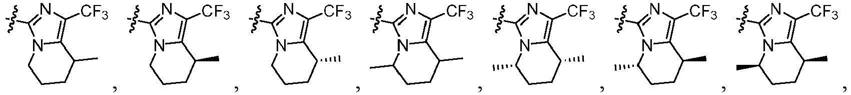 or 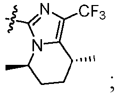
R⁸ is selected from F or Cl;
the definitions of the remaining substituents are the same as those in the first, second, third, or fourth solution of the present invention.

The present invention relates to the following compound or a stereoisomer, tautomer, deuterated substance, solvate, pharmaceutically acceptable salt or eutectic crystal thereof, wherein the compound is selected from one of the structures shown in Table E.

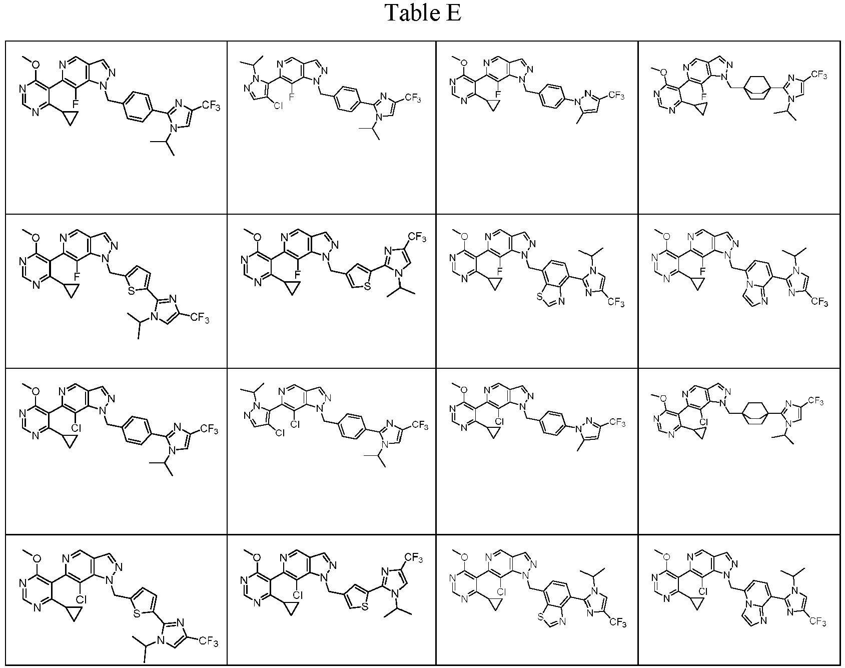

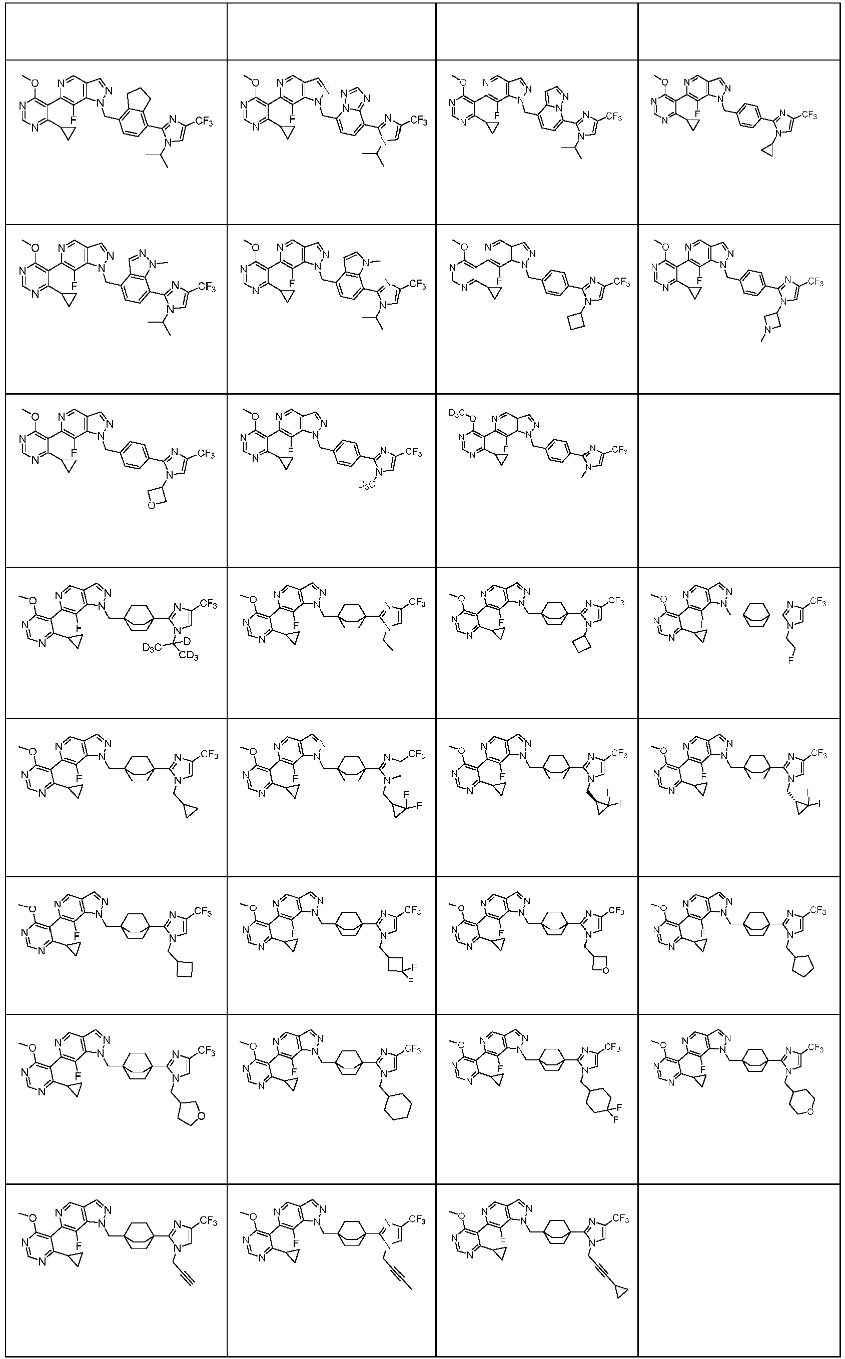

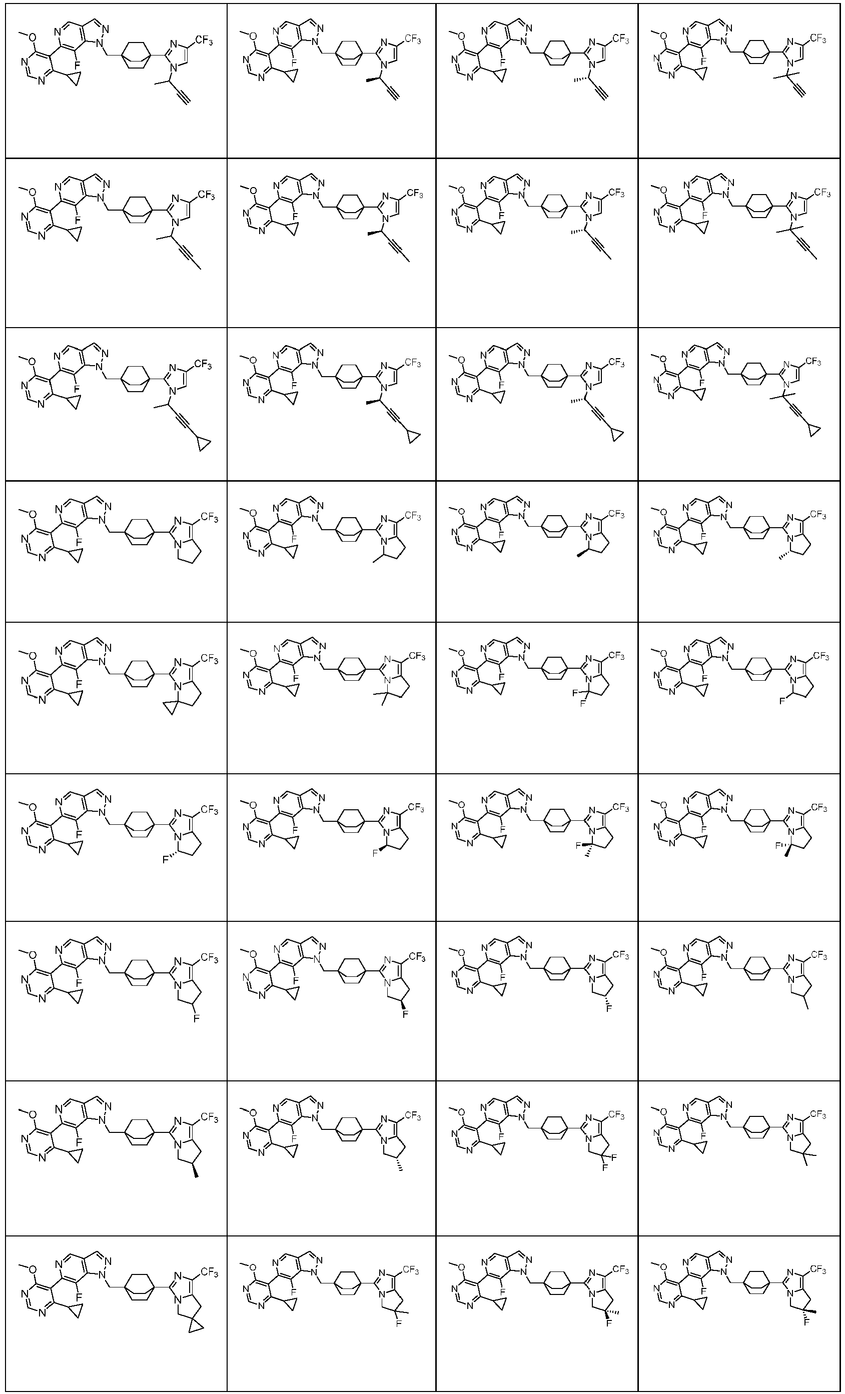

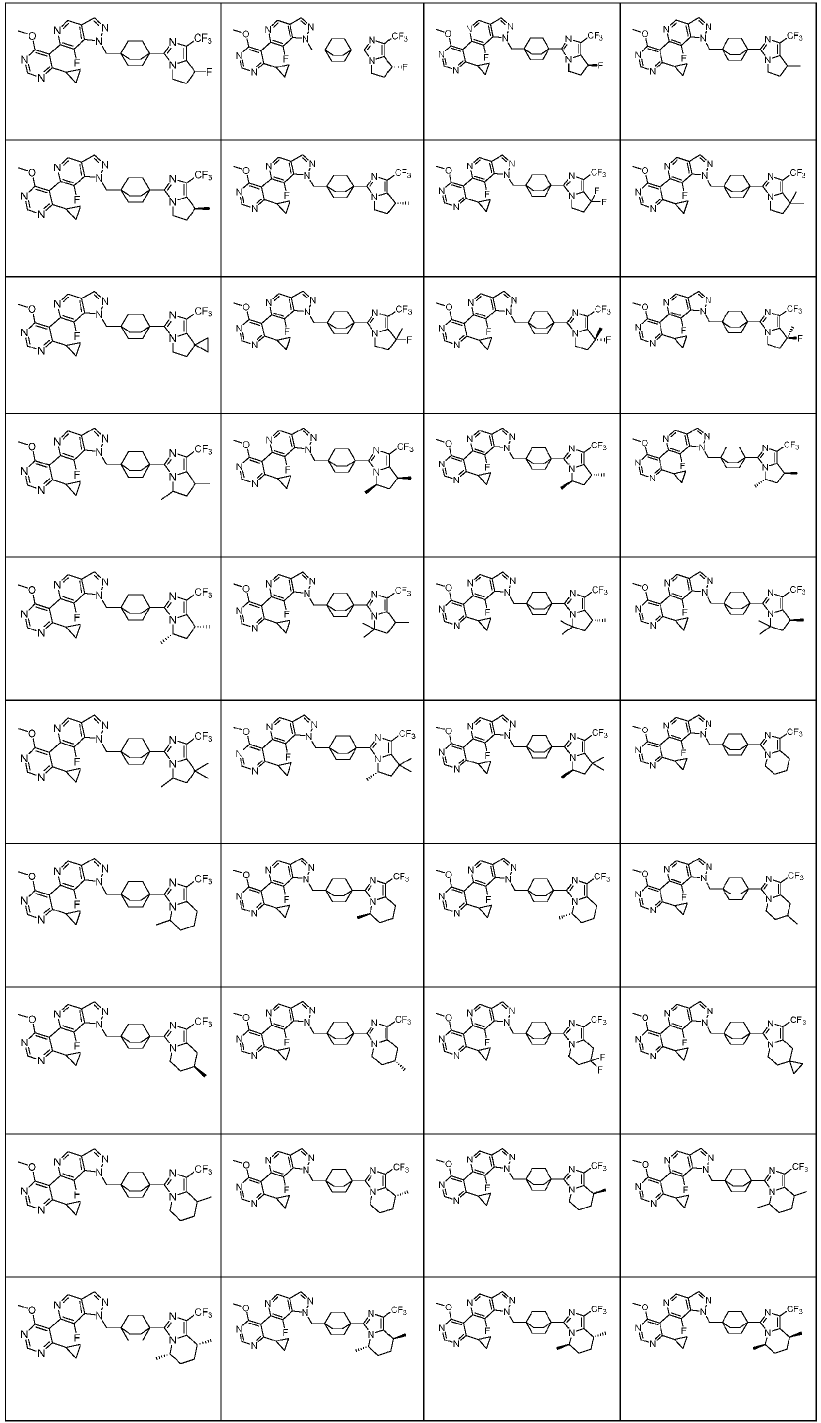

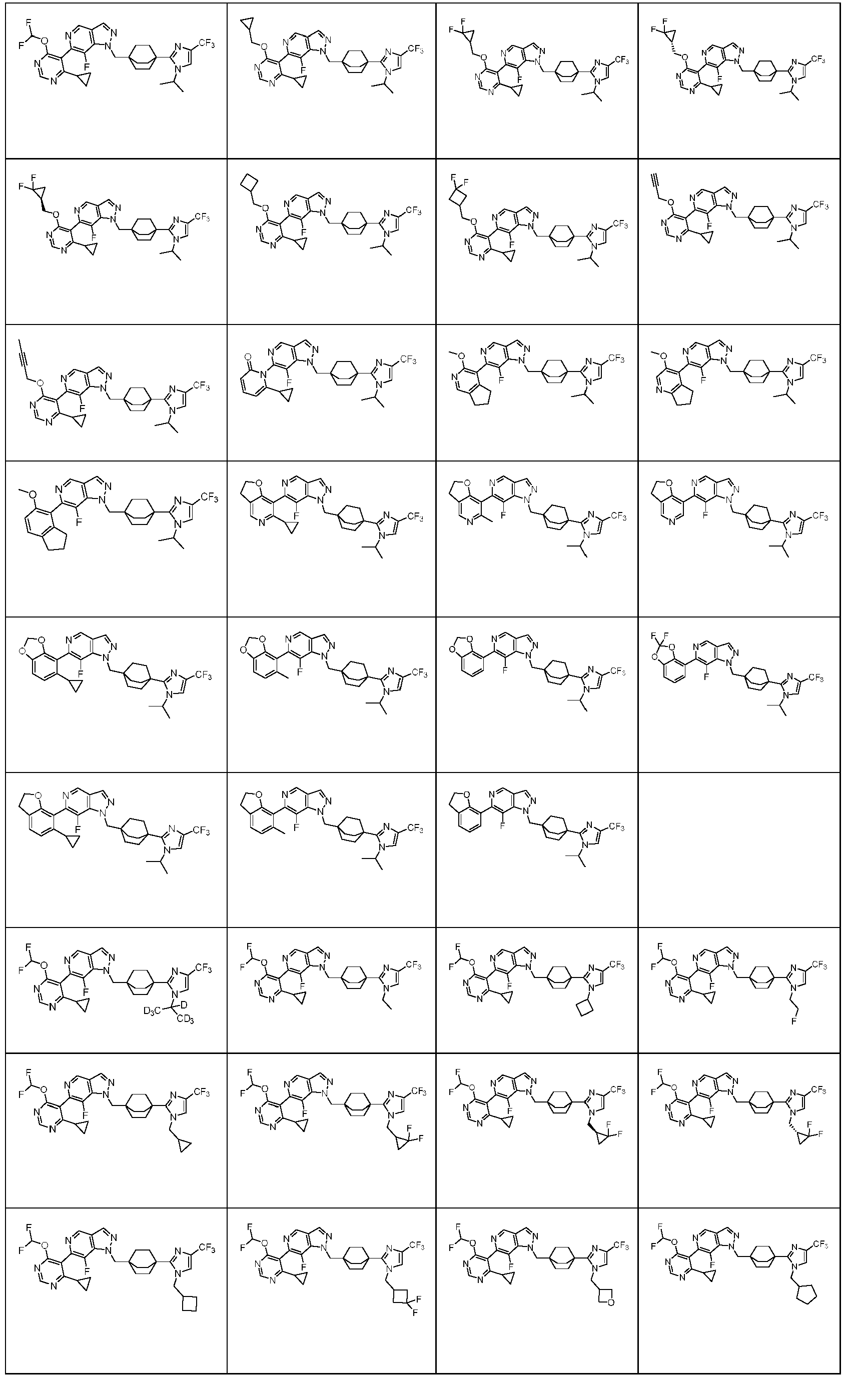

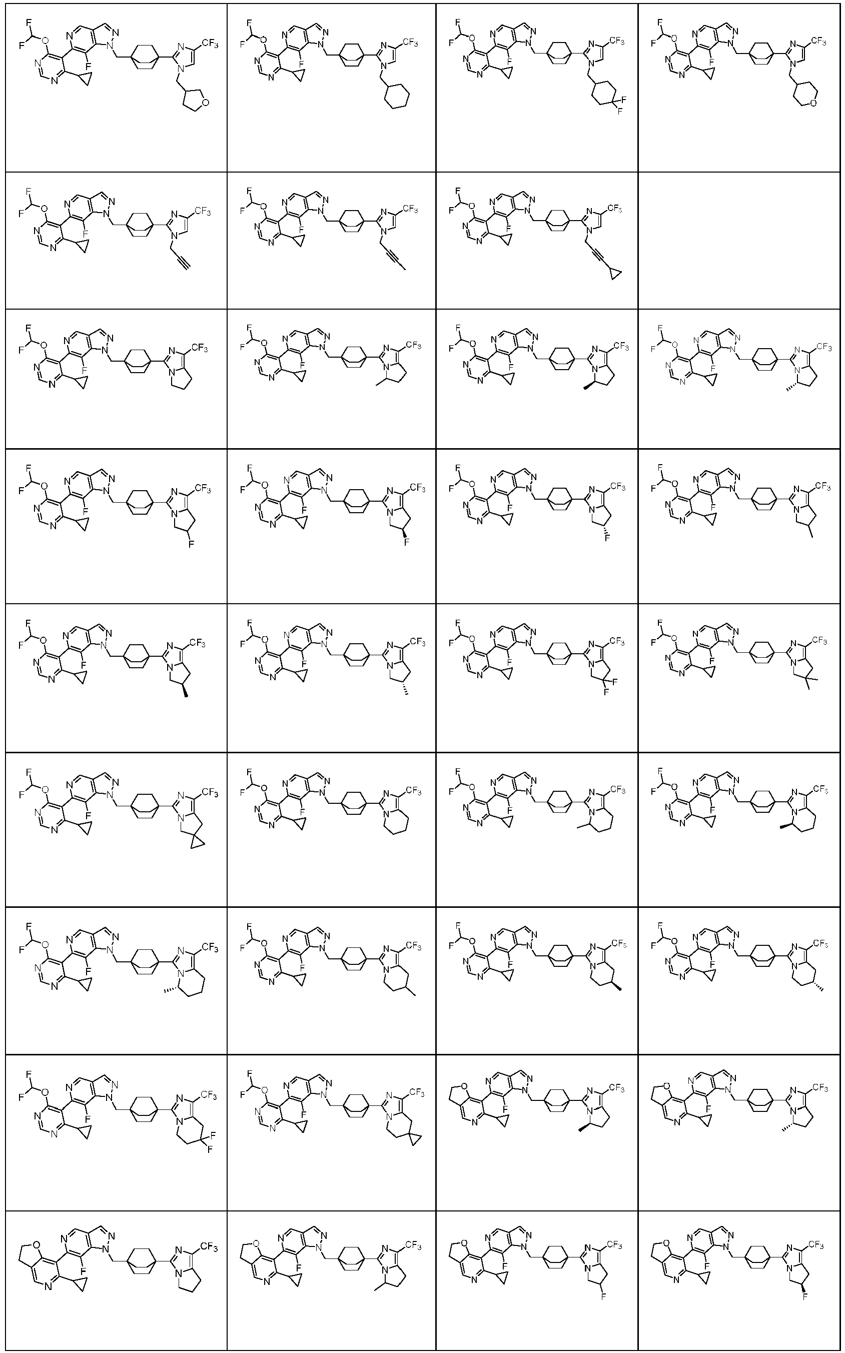

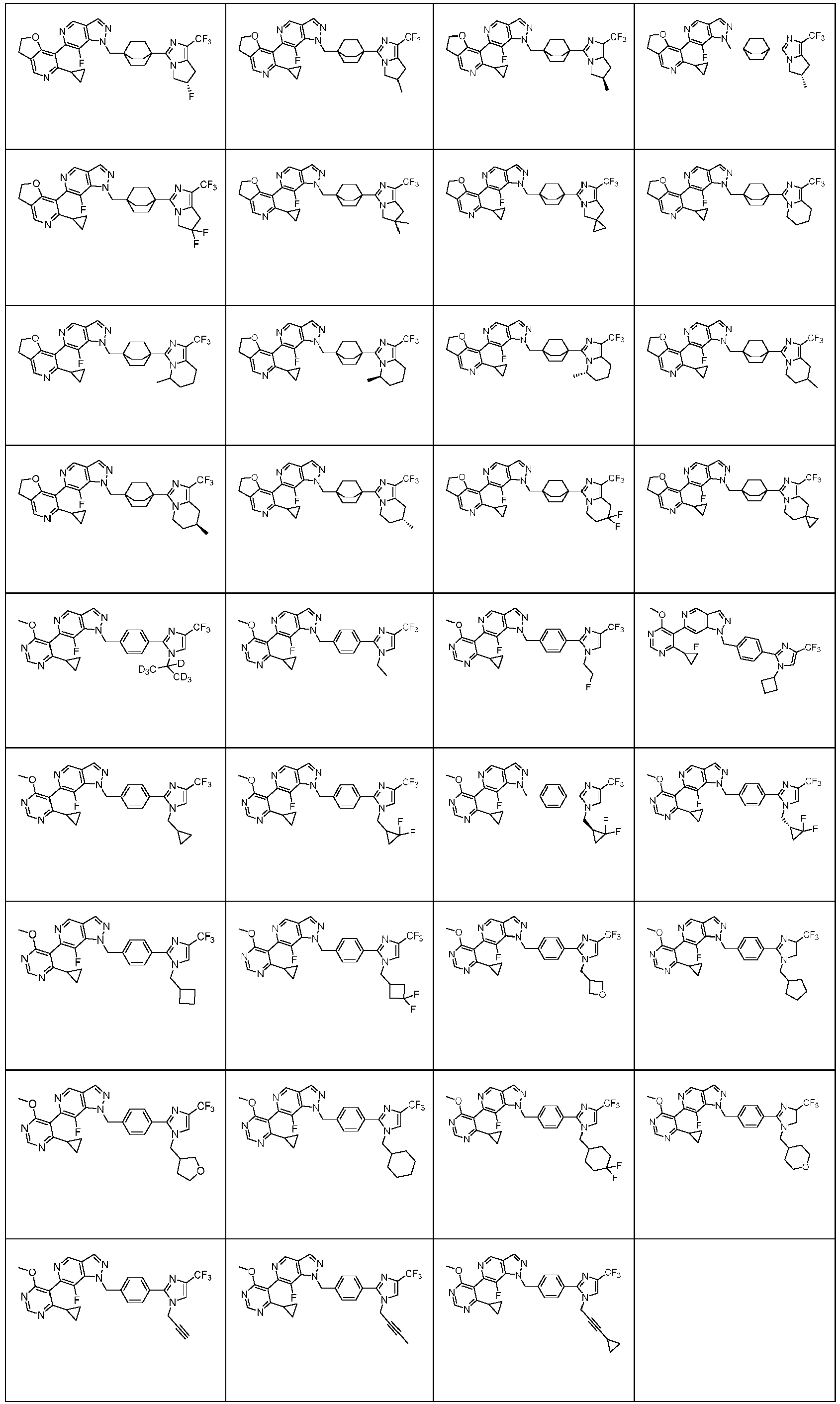

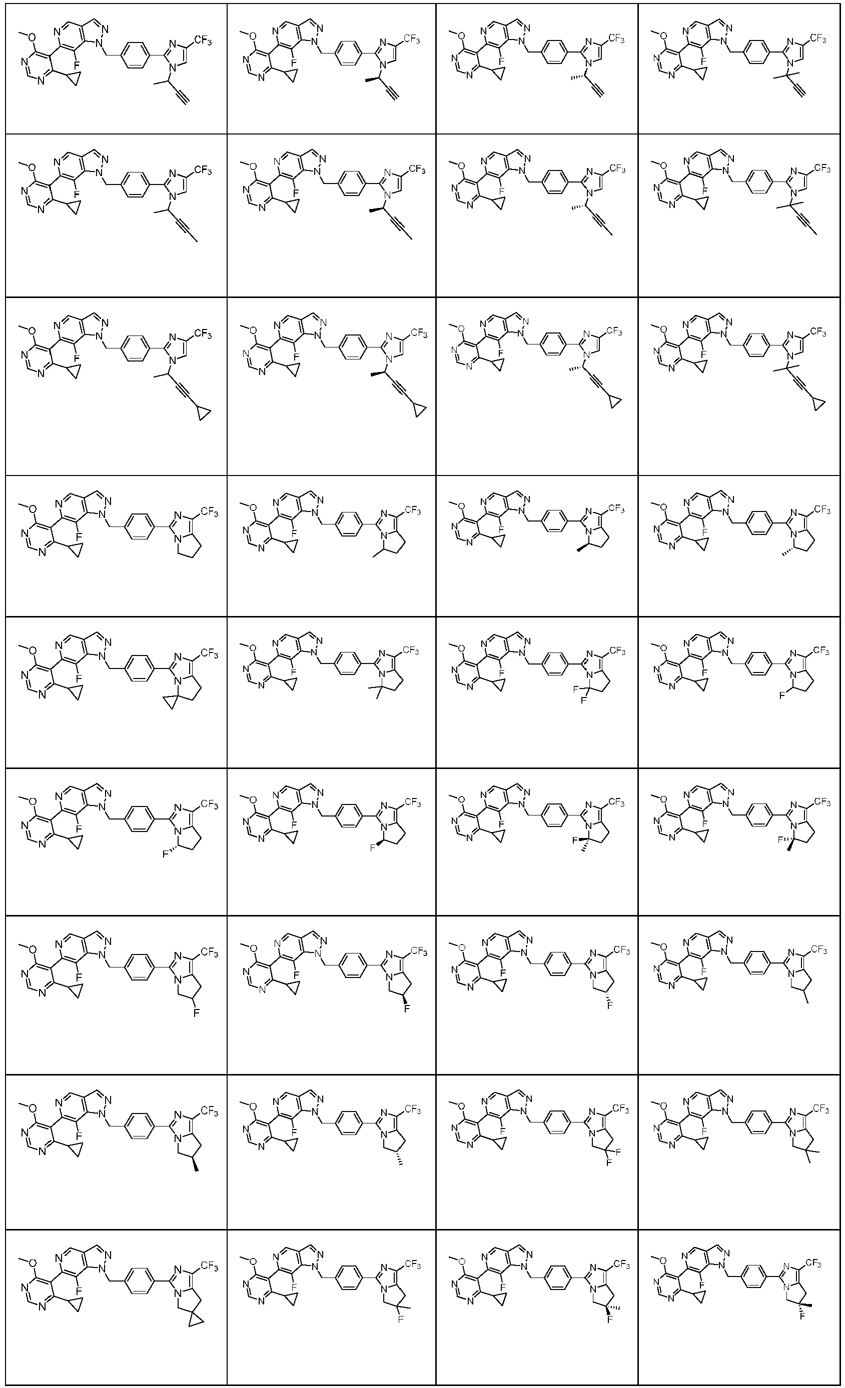

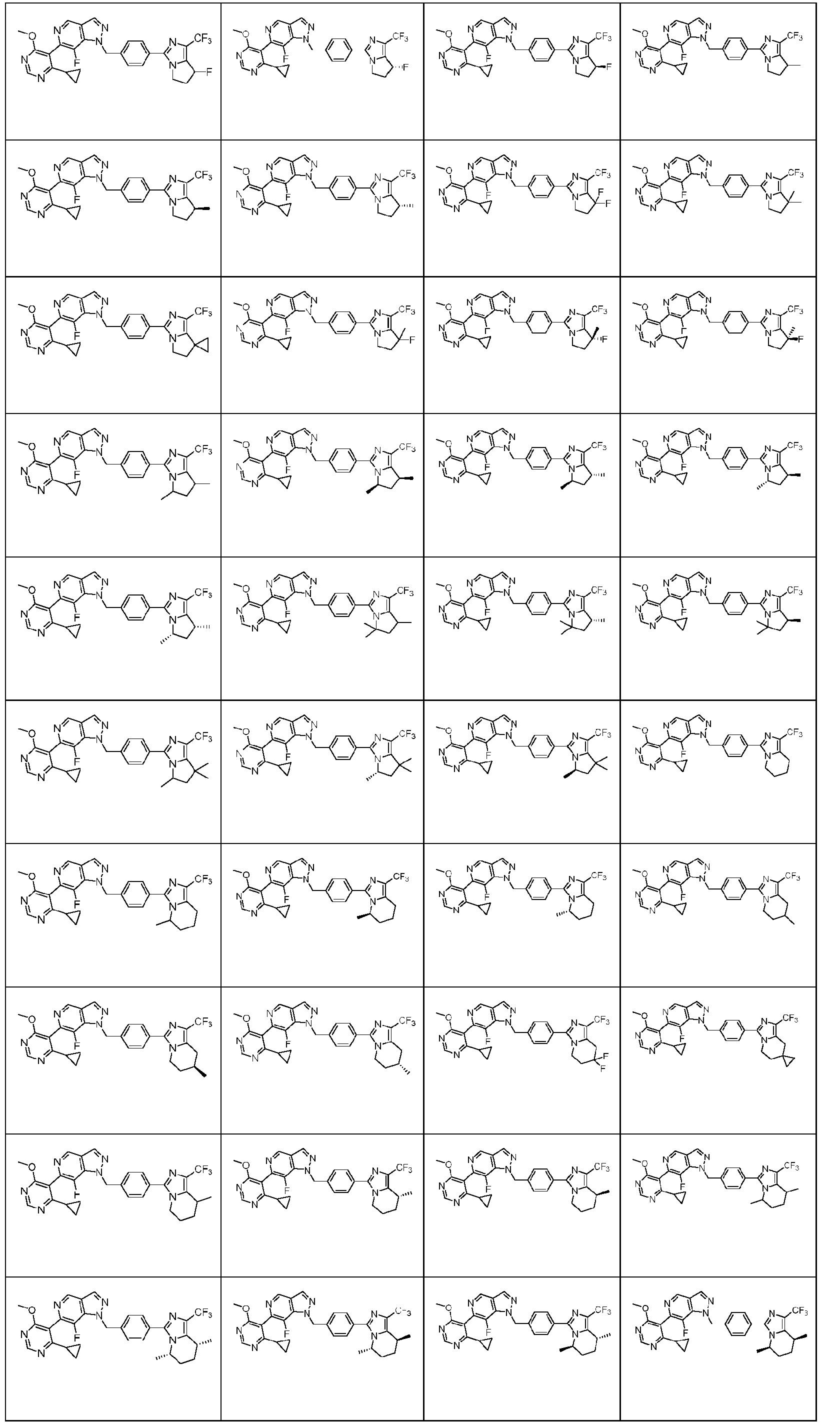

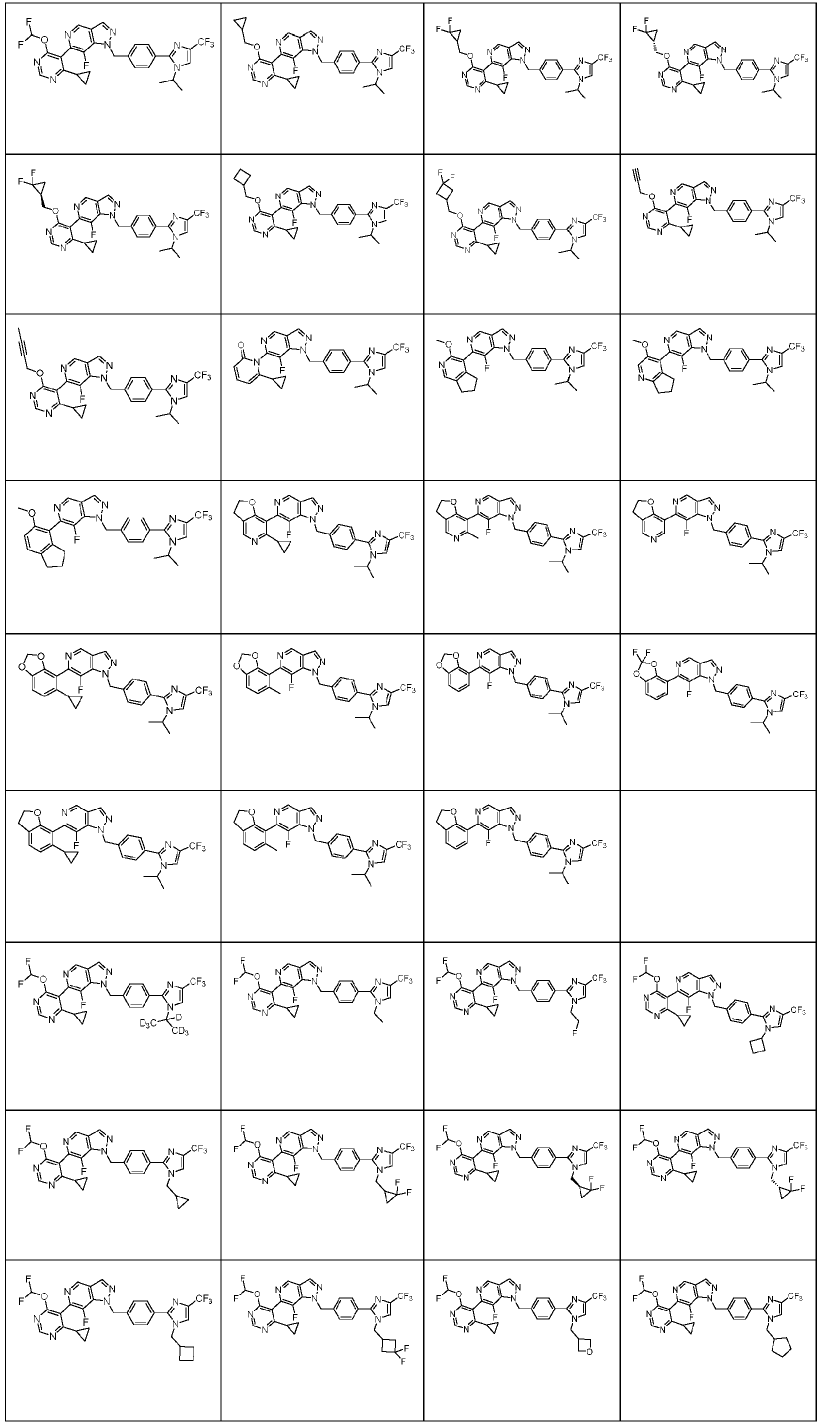

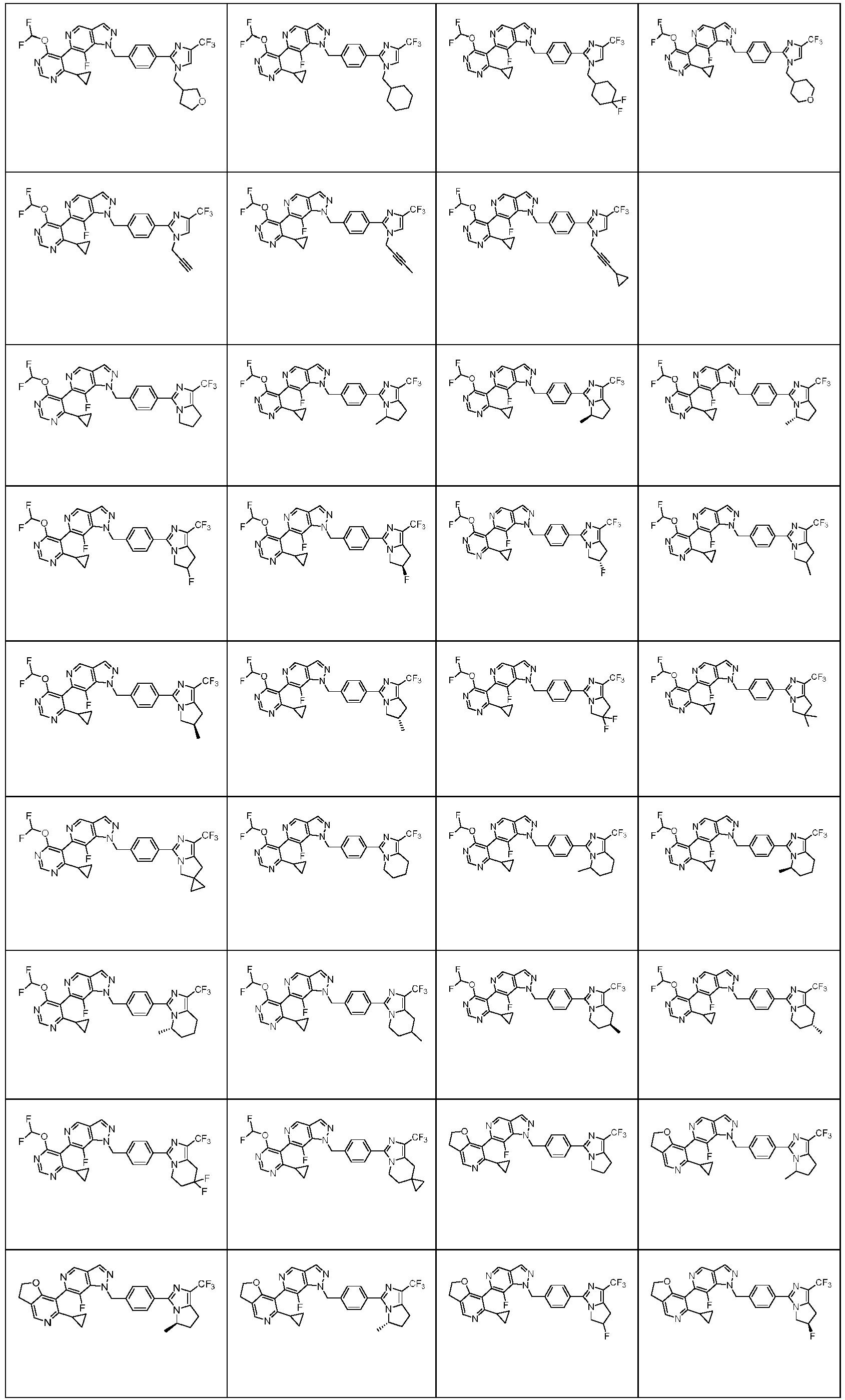

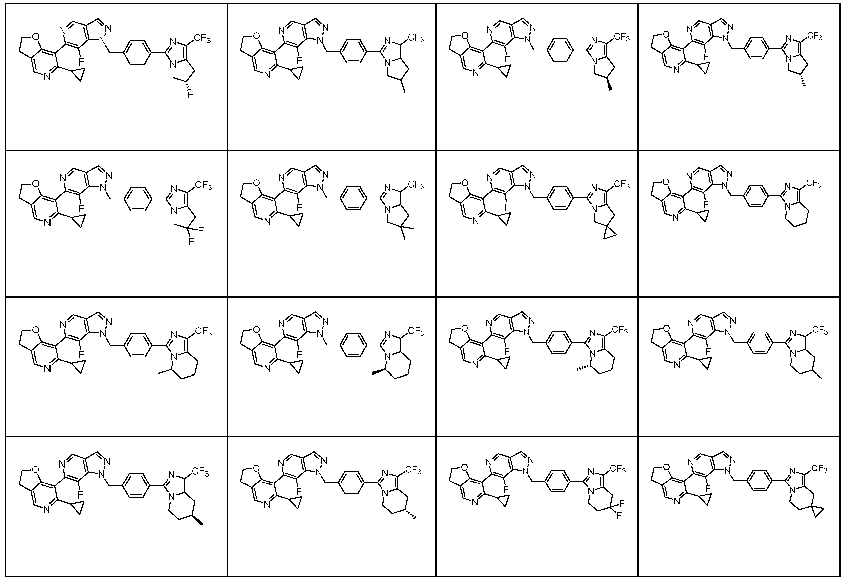

The present invention relates to a pharmaceutical composition comprising the compound or a stereoisomer, tautomer, deuterated substance, solvate, pharmaceutically acceptable salt or eutectic crystal thereof according to the present invention, and a pharmaceutically acceptable carrier.

The present invention relates to an application of the compound or a stereoisomer, tautomer, deuterated substance, solvate, pharmaceutically acceptable salt or eutectic crystal thereof according to the present invention in the preparation of a medicine for treating a disease related to USP1 activity or expression, preferably in the preparation of a medicine for treating a tumor disease.

The present invention relates to an application of any of the above-mentioned compounds or stereoisomers, deuterated substances, solvates, pharmaceutically acceptable salts or eutectic crystals thereof, or the above pharmaceutical composition in the preparation of a medicine for treating a disease related to USP1 activity or expression, wherein the disease is preferably selected from tumors.

The present invention relates to a pharmaceutical composition or pharmaceutical preparation comprising a therapeutically effective amount of the compound or a stereoisomer, deuterated substance, solvate, pharmaceutically acceptable salt or eutectic crystal thereof according to the present invention, and a pharmaceutical excipient. The pharmaceutical composition can be in a unit preparation form (the amount of the active drug in the unit preparation is also referred to as the "preparation specification").

The present invention further provides compounds for use in a method for treating a disease in a mammal, the method comprising administering to the mammal a therapeutically effective amount of the compound, or the stereoisomer, deuterated substance, solvate, pharmaceutically acceptable salt or eutectic crystal thereof or the pharmaceutical composition according to the present invention. In some embodiments, the mammal mentioned in the present invention includes human.

The term "effective amount" or "therapeutically effective amount" in the present application refers to a sufficient amount of the compound disclosed in the present application that is administered to alleviate, to some extent, one or more symptoms of a disease or condition being treated (for example, a disease related to USP1 activity or expression, such as tumor). In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of therapeutically effective amounts include, but are not limited to 1-1500 mg, 1-1400 mg, 1-1200 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 100-1000 mg, 200-1000 mg, 400-1000 mg, 100-800 mg, 200-800 mg, and 400-800 mg.

In some embodiments, the pharmaceutical composition comprises the compound or a stereoisomer, deuterated substance, solvate, pharmaceutically acceptable salt or eutectic crystal thereof according to the present invention in an amount including but not limited to 1-1500 mg, 1-1400 mg, 1-1200 mg, 1-600 mg, 20-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 400 mg, 480 mg, and 500 mg.

There is provided a method for treating a disease in a mammal, the method comprising administering to a subject a therapeutically effective amount of the compound, or the stereoisomer, deuterated substance, solvate, pharmaceutically acceptable salt, or eutectic crystal thereof according to the present invention, wherein the therapeutically effective amount is preferably 1-1500 mg, and the disease is preferably tumors.

There is provided a method for treating a disease in a mammal, comprising administering the compound or a stereoisomer, deuterated substance, solvate, pharmaceutically acceptable salt or eutectic crystal thereof according to the present invention, as a drug, to a subject at a daily dose of 1-1500 mg/day, wherein the daily dose may be a single dose or divided doses. In some embodiments, the daily dose includes, but is not limited to, 10-1500 mg/day, 10-800 mg/day, 25-800 mg/day, 50-800 mg/day, 100-800 mg/day, 200-800 mg/day, 25-400 mg/day, 50-400 mg/day, 100-400 mg/day, and 200-400 mg/day, and in some embodiments, the daily dose includes, but is not limited to, 10 mg/day, 20 mg/day, 25 mg/day, 50 mg/day, 100 mg/day, 125 mg/day, 150 mg/day, 200 mg/day, 400 mg/day, 600 mg/day, 800 mg/day, 1000 mg/day, and 1200 mg/day.

The present invention relates to a kit, which can comprise a composition in a single dose or a multi-dose form. The kit comprises the compound or a stereoisomer, deuterated substance, solvate, pharmaceutically acceptable salt or eutectic crystal thereof according to the present invention, wherein the amount of the compound or a stereoisomer, deuterated substance, solvate, pharmaceutically acceptable salt or eutectic crystal thereof according to the present invention is the same as the amount thereof in the above-mentioned pharmaceutical composition.

In the present invention, the amount of the compound or a stereoisomer, deuterated substance, solvate, pharmaceutically acceptable salt or eutectic crystal thereof according to the present invention is calculated in the form of free base in each case.

### Synthesis Method I:

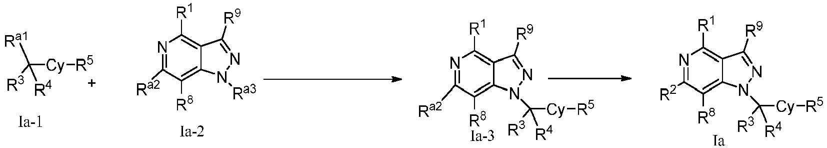

R^{a1} is selected from halogen or OH;
R^{a2} is selected from halogen, preferably from Br or Cl;
R^{a3} is selected from an amino protecting group or H;
the definitions of the remaining groups are the same as in the description;
the compound shown in general formula (Ia-1) and the compound shown in general formula (Ia-2) are subjected to a substitution reaction to obtain a compound shown in general formula (Ia-3), or the compound shown in general formula (Ia-3) is obtained by removing the amino protecting group from the compound shown in general formula (Ia-2) and then performing a substitution reaction by reacting same with the compound shown in general formula (Ia-1);
the compound shown in general formula (Ia-3) is subjected to a coupling reaction to obtain a compound shown in general formula (Ia).

Unless stated to the contrary, the terms used in the description and the claims have the following meanings.

The carbon, hydrogen, oxygen, sulfur, nitrogen or F, Cl, Br, I involved in the groups and compounds of the present invention all comprise their isotopes, and the carbon, hydrogen, oxygen, sulfur or nitrogen involved in the groups and compounds of the present invention is optionally further substituted with one or more of their corresponding isotopes, wherein the isotopes of carbon comprise ¹²C, ¹³C and ¹⁴C, the isotopes of hydrogen comprise protium (H), deuterium (D, also known as heavy hydrogen), tritium (T, also known as superheavy hydrogen), the isotopes of oxygen comprise ¹⁶O, ¹⁷O and ¹⁸O, the isotopes of sulfur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"Halogen" refers to F, Cl, Br, or I.

"Halogen-substituted" refers to a substitution with F, Cl, Br, or I, including but not limited to a substitution with 1 to 10 substituents selected from F, Cl, Br, or I, or a substitution with 1 to 6 substituents selected from F, Cl, Br, or I, or a substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbyl group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Alkylene" refers to a substituted or unsubstituted linear or branched divalent saturated hydrocarbyl group, including -(CH₂)ᵥ- (v is an integer from 1 to 10), and examples of alkylene include, but are not limited to, methylene, ethylene, propylene, butylene, *etc.*

"Cycloalkyl" refers to a substituted or unsubstituted saturated carbocyclic hydrocarbyl group, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Heterocycloalkyl" refers to a substituted or unsubstituted saturated heteroatom-containing cyclic hydrocarbyl group, including but not limited to 3 to 10 atoms, 3 to 8 atoms, or 1 to 3 heteroatoms selected from N, O or S. N and S selectively substituted in the heterocycloalkyl ring can be oxidized to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxacyclobutyl, azetidinyl, tetrahydrofuryl, tetrahydro-2*H*-pyranyl, dioxolanyl, dioxanyl, pyrrolidyl, piperidyl, imidazolidinyl, oxazolidinyl, oxazinanyl, morpholinyl, hexahydropyrimidyl or piperazinyl. Heterocycloalkyl can be monovalent, divalent, trivalent or tetravalent.

"Alkenyl" refers to a substituted or unsubstituted linear or branched unsaturated hydrocarbyl group, having at least 1, usually 1, 2 or 3 carbon-carbon double bonds, with a main chain including but not limited to 2 to 10, 2 to 6, or 2 to 4 carbon atoms. Examples of alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, *etc.* The definition of the "alkenyl" herein is consistent with this definition. Alkenyl can be monovalent, divalent, trivalent or tetravalent.

"Alkynyl" refers to a substituted or unsubstituted linear or branched monovalent unsaturated hydrocarbyl group, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, *etc.* Alkynyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, 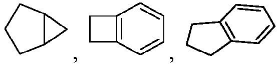 or 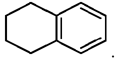 "Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S, and the selectively substituted N and S in the heterocyclyl ring can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxetanyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl, 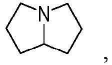 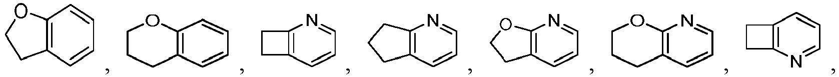 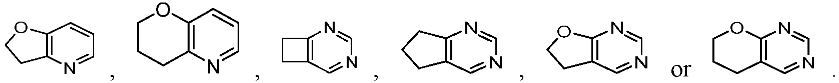 "Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or S(=O)ₙ. 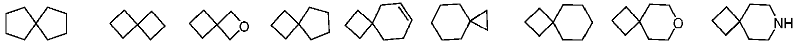 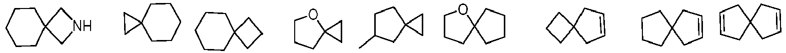 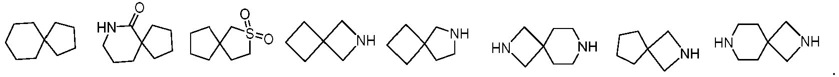 "Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, S(=O)ₙ or O, wherein n is 0, 1 or 2). The number of ring atoms in the fused ring system includes but is not limited to 5 to 20, 5 to 14, 5 to 12, or 5 to 10. Non-limiting examples include: 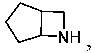 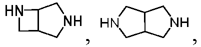 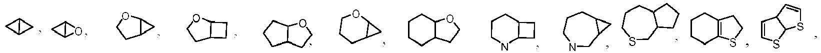 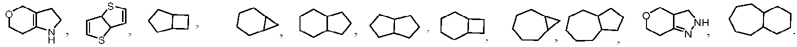 "Fused ring" or "fused ring group" can be monovalent, divalent, trivalent or tetravalent.

"Bridged ring" or "bridged ring group" refers to a substituted or unsubstituted polycyclic group containing any two atoms that are not directly connected, and may contain 0 or more double bonds. Any ring in the fused ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or 5 to 10. Non-limiting examples include 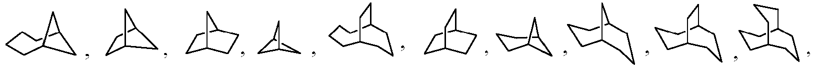 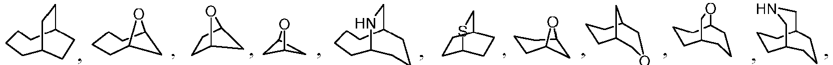 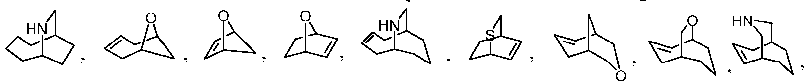 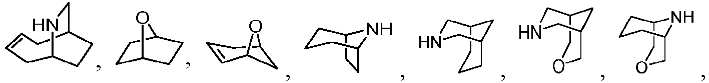 cubane and adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl" or "heterocycle" with a monocyclic system. The definition of the "heterocyclyl", "monocyclic heterocyclyl" or "mono-heterocyclic ring group" herein is consistent with that of heterocycle.

"Fused-heterocyclic ring", "fused-heterocyclic ring group", "fused ring heterocyclyl" or "fused-heterocyclic ring group" refers to a "fused ring" containing a heteroatom. The definition of the "fused-heterocyclic ring", "fused-heterocyclic ring group", "fused ring heterocyclyl" or "fused-heterocyclic ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl group with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or 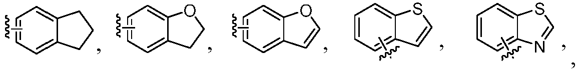 and "aryl" or "aromatic ring" may be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl group containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5-15, 5-10 or 5-6. Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring may be fused to a saturated or unsaturated carbocycle or heterocycle, wherein the ring connected to the parent structure is a heteroaryl ring. Non-limiting examples include 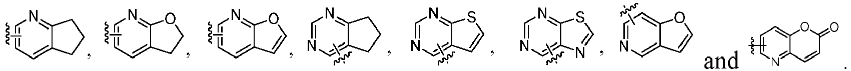 The definition of the "heteroaryl" herein is consistent with this definition. Heteroaryl can be monovalent, divalent, trivalent or tetravalent. When divalent, trivalent or tetravalent, the point of connection is on the heteroaryl ring.

"Substitution" or "substituted" refers to a substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, -(CH₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d} or -(CH₂)ₘ-alkynyl-R^{a} (wherein m and n are 0, 1 or 2), arylthio, thiocarbonyl, silyl or -NR^{b}R^{c} and the like, wherein R^{b} and R^{c} are independently selected from H, hydroxyl, amino, carbonyl, alkyl, alkoxy, cycloalkyl, heterocyclyl, aryl, heteroaryl, sulfonyl, or trifluoromethylsulfonyl. Alternatively, R^{d} and R^{c} may form a five- or six-membered cycloalkyl or heterocyclyl; each R^{a} or R^{d} is independently selected from aryl, heteroaryl, alkyl, alkoxy, cycloalkyl, heterocyclyl, carbonyl, ester group, bridged ring group, spiro ring group or fused ring group.

"Containing 1 to 4 heteroatoms selected from O, S, and N" refers to containing 1, 2, 3, or 4 heteroatoms selected from O, S, and N.

"Substituted with 0 to X substituents" refers to substituted with 0, 1, 2, 3 ... X substituents, wherein X is selected from any integer between 1 and 10. For example, "substituted with 0 to 4 substituents" refers to substituted with 0, 1, 2, 3 or 4 substituents. For example, "substituted with 0 to 5 substituents" refers to substituted with 0, 1, 2, 3, 4 or 5 substituents. For example, "bridged-heterocyclic ring is optionally further substituted with 0 to 4 substituents selected from H or F" means that the bridged-heterocyclic ring is optionally further substituted with 0, 1, 2, 3 or 4 substituents selected from H or F.

An X- to Y-membered ring (X is selected from an integer less than Y and greater than or equal to 3, and Y is selected from any integer between 4 and 12) includes X-, X+1-, X+2-, X+3-, X+4-, ..., to Y-membered rings. Rings include heterocycle, carbocycle, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "stereoisomer" refers to an isomer produced as a result of different spatial arrangement of atoms in molecules, including cis-trans isomers, enantiomers and conformational isomers.

The term "tautomer" refers to a functional group isomer produced by the rapid movement of an atom in two positions in a molecule, such as keto-enol isomerization and amide-imino alcohol isomerization.

The term "preparation specification" refers to the weight of the active drug contained in each vial, tablet or other unit preparation.

The term "subject" refers to a human or another mammal.

### Detailed Description of Embodiments

The technical solutions of the present invention will be described in detail by the following examples, but the scope of protection of the present invention includes but is not limited thereto.
The structures of the compounds are determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvent for determination is deuterated dimethyl sulfoxide (DMSO-d6), deuterated chloroform (CDCl3) and deuterated methanol (CD3OD); and the internal standard is tetramethylsilane (TMS);
MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 µM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

The known starting materials of the present invention can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies.
Tf: trifluoromethylsulfonyl; Ts: p-toluenesulfonyl; TMS: trimethylsilyl; SEM: 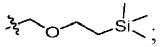 THP: 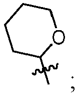 Boc: tert-butoxycarbonyl; Ms: 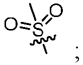 TBS: 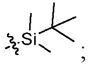 MTBE: methyl tert-butyl ether; Bn: benzyl; DIPEA: N,N-diisopropylethylamine; DMAc: N,N-dimethylacetamide; DMF: N,N-dimethylformamide; DMSO: dimethyl sulfoxide; DCM: dichloromethane; Cbz: 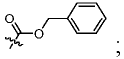 LDA: lithium diisopropylamide;
Pd(dppf)Cl₂·CH₂Cl₂ : CAS 95464-05-4; XPhos Pd G2: CAS 1310584-14-5

### Example 1:

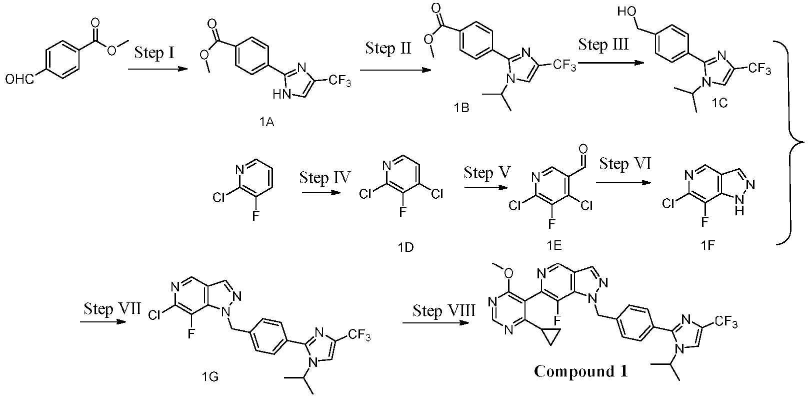

### Step I: Preparation of 1A

Sodium acetate (4.15 g, 50.55 mmol) was added to a solution of 1,1-dibromo-3,3,3-trifluoroacetone (12.4 g, 45.95 mmol) in water (24 mL), heated at 100°C for 1 h, and cooled to room temperature, a solution of methyl 4-formylbenzoate (8.3 g, 50.55 mmol) in methanol (180 mL) and aqueous ammonia (44 mL) was added, and the mixture was stirred at room temperature for 40 minutes, reacted at 100°C for 2 h, cooled to room temperature, quenched by adding water (50 mL), and extracted with ethyl acetate (60 mL × 3). The organic phases were combined, dried over anhydrous sodium sulfate and then concentrated under reduced pressure, and the residue was purified by silica gel column chromatography (ethyl acetate/petroleum ether (V/V) = 1/10-1/1) to obtain **1A** (8 g, yield: 64.43%).

LCMS m/z = 271.1 [M+H]⁺.

### Step II: Preparation of 1B

**1A** (17 g, 62.91 mmol) was dissolved in 200 mL of DMF, isopropyl iodide (32.08 g, 188.73 mmol) and cesium carbonate (61.49 g, 188.75 mmol) were added in sequence, and the mixture was reacted at 90°C overnight. The reaction product was cooled to room temperature and extracted by adding water and ethyl acetate, the organic layer was washed with an aqueous saturated sodium chloride solution, dried over anhydrous sodium sulfate, and concentrated under reduced pressure, and the residue was purified by silica gel column chromatography (ethyl acetate/petroleum ether (V/V) = 1/20-1/1) to obtain **1B** (11.8 g, yield: 60%).

LCMS m/z = 313.1 [M+H]⁺.

### Step III: Preparation of 1C

**1B** (11.8 g, 37.79 mmol) was dissolved in 150 mL of THF, lithium borohydride (4.12 g, 188.95 mmol) was added, and the mixture was heated to 50°C and stirred for 2 h. After cooling to room temperature, an aqueous saturated ammonium chloride solution was added in an ice-water bath to quench the reaction, the reaction product was extracted by adding water and ethyl acetate, and the organic layer was dried over anhydrous sodium sulfate, concentrated under reduced pressure to obtain **1C** (10 g).

LCMS m/z = 285.1 [M+H]⁺.

### Step IV: Preparation of 1D

2-Chloro-3-fluoropyridine (5 g, 38.03 mmol) was dissolved in THF (25 mL), the mixture was cooled to -70°C under nitrogen protection, and a THF solution of lithium bis(trimethylsilyl)amide (57 mL, 1*N,* 57 mmol) was dropwise added slowly; after the addition was complete, the mixture was heated to -30°C and stirred for 1 h, hexachloroethane (10.80 g, 45.64 mmol) was added at -30°C, and after the addition was complete, the mixture was naturally heated to room temperature and stirred overnight. Sequentially, 20 mL of an aqueous saturated ammonium chloride solution was added to quench the reaction, 100 mL of water and 100 mL of ethyl acetate were added, and after layering, the organic layer was washed with 100 mL of an aqueous saturated sodium chloride solution, dried over anhydrous sodium sulfate, and concentrated under reduced pressure. The residue was purified by silica gel column chromatography (dichloromethane/petroleum ether (V/V) = 0/1-1/4) to obtain **1D** (2 g, yield 32%).

¹H NMR (400 MHz, CDCl₃) δ 8.13 (d, 1H), 7.34 (t, 1H).

### Step V: Preparation of 1E

**1D** (2 g, 12.02 mmol) was dissolved in THF (10 mL), the mixture was cooled to -70°C under nitrogen protection, and a THF solution of LDA (14.4 mL, 1*N*, 14.42 mmol) was dropwise added slowly at -70°C. The mixture was stirred at - 70°C for 1 h, DMF (1.76 g, 24.04 mmol) was then dropwise added slowly, and the mixture was stirred for 30 min. 10 mL of an aqueous saturated ammonium chloride solution was added to quench the reaction, the mixture was heated to room temperature, 20 mL of water and 20 mL of ethyl acetate were added, and after layering, the organic layer was washed with 20 mL of an aqueous saturated sodium chloride solution, dried over anhydrous sodium sulfate, and concentrated under reduced pressure. The residue was purified by silica gel column chromatography (ethyl acetate/petroleum ether (V/V) = 0/1-1/4) to obtain **1E** (0.56 g, yield: 24%).

¹H NMR (400 MHz, CDCl₃) δ 10.40 (s, 1H), 8.69 (s, 1H).

### Step VI: Preparation of 1F

**1E** (0.55 g, 2.84 mmol) was dissolved in n-butanol (10 mL), and hydrazine hydrate (0.89 g, 14.2 mmol, wt% = 80%) was added and stirred at 100°C for 8 h. After cooling to room temperature, 20 mL of water and 20 mL of ethyl acetate were added, and after layering, the organic phase was dried over anhydrous sodium sulfate, and concentrated under reduced pressure. The residue was purified by silica gel column chromatography (ethyl acetate/petroleum ether (V/V) = 1/10-1/2) to obtain **1F** (0.28 g, yield: 57%)

LCMS m/z = 172.1 [M+H]⁺.

### Step VII: Preparation of 1G

**1F** (0.09 g, 0.52 mmol), **1C** (0.15 g, 0.52 mmol) and tri-n-butylphosphine (0.21 g, 1.04 mmol) were added to THF (5 mL) in sequence, and diethyl azodicarboxylate (0.18 g, 1.03 mmol) was added dropwise under nitrogen protection at room temperature and stirred at room temperature for 1 h. The solvent was removed by concentration under reduced pressure, and the residue was purified by silica gel column chromatography (ethyl acetate/petroleum ether (V/V) = 1/10-1/2) to obtain **1G** (0.15 g, yield: 66%).

LCMS m/z = 438.1 [M+H]⁺.

### Step VIII: Preparation of Compound 1

**1G** (0.13 g, 0.30 mmol) was dissolved in a mixed solvent of 1,4-dioxane (4 mL) and water (1 mL), and (4-cyclopropyl-6-methoxypyrimidin-5-yl)boronic acid (0.12 g, 0.62 mmol), potassium phosphate (0.13 g, 0.60 mmol), and XPhos G3 (CAS: 1445085-55-1, 0.025 g, 0.030 mmol) were added in sequence and heated to 90°C under nitrogen protection and stirred overnight. After cooling to room temperature, 10 mL of ethyl acetate and 10 mL of water were added, and after layering, the organic layer was dried over anhydrous sodium sulfate and concentrated under reduced pressure. The residue was purified by silica gel column chromatography (ethyl acetate/petroleum ether (V/V) = 1/5-1/1) to obtain **Compound 1** (80 mg, yield: 48%).

LCMS m/z = 552.2 [M+H]⁺.

¹H NMR (400 MHz, DMSO-*d₆*) δ 9.08 (d, 1H), 8.71 (s, 1H), 8.54 (d, 1H), 8.18 - 8.13 (m, 1H), 7.60 - 7.50 (m, 2H), 7.41 - 7.33 (m, 2H), 5.84 (s, 2H), 4.50 - 4.32 (m, 1H), 3.83 (s, 3H), 1.74 - 1.64 (m, 1H), 1.38 (d, 6H), 1.15 - 1.06 (m, 1H), 1.04 - 0.96 (m, 1H), 0.94 - 0.79 (m, 2H).

### Example 2:

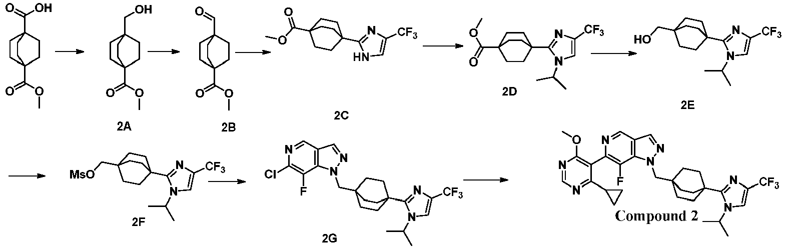

### Step I: Preparation of 2A

4-(Methoxycarbonyl)bicyclo[2.2.2]octane-1-carboxylic acid (5 g, 23.58 mmol) was dissolved in THF (60 mL), and a borane tetrahydrofuran solution (47 mL, 1M) was dropwise added slowly at 0°C and stirred at room temperature overnight. Methanol was added at 0°C for quenching, the resulting product was concentrated under reduced pressure, and the residue was purified by silica gel column chromatography (ethyl acetate/petroleum ether (V/V) = 1/20-1/2) to obtain **2A** (3.8 g, yield: 81%).

LCMS m/z = 199.2 [M+H]⁺.

### Step II: Preparation of 2B

**2A** (3.8 g, 19.16 mmol) was dissolved in DCM (100 mL), and Dess-Martin periodinane (16.25 g, 38.32 mmol) was added at 0°C in portions, and reacted at room temperature for 3 h. TLC monitoring showed that the reaction was complete. The resulting product was concentrated under reduced pressure, and the residue was purified by silica gel column chromatography (ethyl acetate/petroleum ether (V/V) = 1/20-1/2) to obtain **2B** (3.02 g, yield: 80%).

LCMS m/z = 197.1 [M+H]⁺.

### Step III: Preparation of 2C

1,1-Dibromo-3,3,3-trifluoroacetone (4.98 g, 18.49 mmol, CAS: 431-67-4) was placed in a 250 mL single-mouth flask, and 30 mL of water and sodium acetate (1.89 g, 23.09 mmol) were added and reacted at 90°C for 1 h. The resulting product was cooled to room temperature, and 60 mL of methanol, **2B** (3.02 g, 15.39 mmol), and concentrated aqueous ammonia (15 mL) were added in sequence and reacted at 90°C for 2 h. The resulting product was cooled to room temperature, the reaction liquid was poured into 200 mL of water and filtered, the filter cake was washed with water (2 x 10 mL), and the filter cake was dried under reduced pressure to obtain **2C** (2.9 g, yield: 62%).

LCMS m/z = 303.1 [M+H]⁺.

¹H NMR (400 MHz, DMSO-*d₆*) δ 12.36 - 12.16 (m, 1H), 7.68 - 7.55 (m, 1H), 3.59 (s, 3H), 1.90-1.72 (m, 12H).

### Step IV: Preparation of 2D

**2C** (2.0 g, 6.62 mmol) was dissolved in 30 mL of DMF at room temperature, isopropyl iodide (4.50 g, 26.48 mmol) and cesium carbonate (6.47 g, 19.86 mmol) were added in sequence, and the mixture was reacted at 90°C overnight. The reaction product was cooled to room temperature and extracted by adding water and ethyl acetate, the organic layer was washed with an aqueous saturated sodium chloride solution, dried over anhydrous sodium sulfate, and concentrated under reduced pressure, and the residue was purified by silica gel column chromatography (ethyl acetate/petroleum ether (V/V) = 1/20-1/1) to obtain **2D** (1.2 g, yield: 52%).

LCMS m/z = 345.2 [M+H]⁺.

### Step V: Preparation of 2E

**2D** (1.2 g, 3.48 mmol) was dissolved in 20 mL of THF, lithium borohydride (0.38 g, 17.40 mmol) was added, and the mixture was stirred at 60°C for 2 h. After cooling to room temperature, an aqueous saturated ammonium chloride solution was added to quench the reaction, the reaction product was extracted by adding water and ethyl acetate, and the organic layer was dried over anhydrous sodium sulfate, concentrated under reduced pressure to obtain **2E** (0.9 g, yield: 81%).

LCMS m/z = 317.2 [M+H]⁺.

### Step VI: Preparation of 2F

At 0°C, methylsufonyl chloride (64 mg, 0.56 mmol) was added to a solution of **2E** (0.12 g, 0.37 mmol) and triethylamine (0.11 g, 1.09 mmol) in dichloromethane (15 mL), which was stirred at room temperature for 1 h. 20 mL of an aqueous saturated sodium bicarbonate solution was added for quenching, and dichloromethane (20 mL × 3) was used for extraction. The organic phases were combined, dried over anhydrous sodium sulfate, concentrated under reduced pressure, and purified by silica gel column chromatography (ethyl acetate/petroleum ether (V/V) = 1/100-1/20) to obtain **2F** (0.11 g, yield 74%).

LCMS m/z = 395.1 [M+H]⁺.

### Step VII: Preparation of 2G

**1F** (0.1 g, 0.58 mmol) and **2F** (0.23 g, 0.58 mmol) were dissolved in DMF (5 mL), and cesium carbonate (0.38 g, 1.17 mmol) and sodium iodide (0.087 g, 0.58 mmol) were added and reacted at 140°C for 2 h. The reaction product was cooled to room temperature, 20 mL of water and 20 mL of ethyl acetate were added for quenching, the organic layer was washed with an aqueous saturated sodium chloride solution (10 mL x 3), dried over anhydrous sodium sulfate, and concentrated under reduced pressure, and the residue was purified by silica gel column chromatography (ethyl acetate/petroleum ether (V/V) = 1/10-1/2) to obtain **2G** (0.060 g, yield: 22%).

LCMS m/z = 470.2 [M+H]⁺.

### Step VIII: Preparation of Compound 2

**2G** (0.06 g, 0.13 mmol), (4-cyclopropyl-6-methoxypyrimidin-5-yl)boronic acid (0.050 g, 0.26 mmol), XPhos G3 (0.011 g, 0.013 mmol, CAS: 1445085-55-1), and potassium phosphate (0.056 g, 0.27 mmol) were placed in a 50 mL single-mouth flask, 1,4-dioxane (4 mL) and water (1 mL) were added, and after displacement with nitrogen three times, the mixture was heated to 90°C and stirred overnight. After cooling to room temperature, 20 mL of water and 20 mL of ethyl acetate were added for quenching, the organic layer was dried over anhydrous sodium sulfate and then concentrated under reduced pressure, the residue was purified by silica gel column chromatography (ethyl acetate/petroleum ether (V/V) = 1/10-1/1), and after concentration, the residue was purified by secondary silica gel column chromatography (dichloromethane/methanol (V/V) = 100/0-100/3) to obtain **Compound 2** (30 mg, yield: 39%).

LCMS m/z = 584.2 [M+H]⁺.

¹H NMR (400 MHz, CD₃OD) δ 8.97 (d, 1H), 8.64 (s, 1H), 8.38 (d, 1H), 7.65 - 7.58 (m, 1H), 4.93 - 4.84 (m, 1H), 4.39 (s, 2H), 3.93 (s, 3H), 2.06 - 1.94 (m, 6H), 1.74 - 1.59 (m, 7H), 1.41 (d, 6H), 1.27 - 1.20 (m, 1H), 1.16 - 1.09 (m, 1H), 0.99 - 0.86 (m, 2H).

### Example 3:

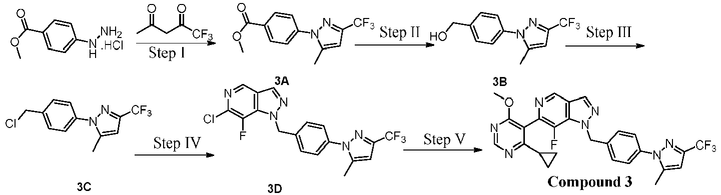

### Step I: Preparation of 3A

Methyl 4-hydrazinobenzoate hydrochloride (2.02 g, 10.0 mmol) was dissolved in hexafluoroisopropanol (10 mL), 1,1,1-trifluoro-2,4-pentanedione (1.54 g, 10.0 mmol) was added, triethylamine (2.23 g, 22.00 mmol) was dropwise added slowly under nitrogen protection in an ice bath, and the mixture was reacted at room temperature overnight. The reaction was quenched by adding water (50 mL) and extracted by adding ethyl acetate (60 mL × 3), the organic phases were combined, dried over anhydrous sodium sulfate and then concentrated under reduced pressure, and the residue was purified by silica gel column chromatography (ethyl acetate/petroleum ether (V/V) = 1/10-1/5) to obtain **3A** (2.4 g, yield: 84 %).

LCMS m/z = 285.1 [M+H]⁺.

### Step II: Preparation of 3B

**3A** (2.4 g, 8.44 mmol) was dissolved in 50 mL of THF, and lithium borohydride (1.84 g, 84.4 mmol) was added slowly in an ice-water bath and stirred at 50°C for 4 h. After the reaction was complete, the reaction product was cooled to room temperature, the reaction flask was placed in an ice-water bath, the reaction was quenched by slowly adding an aqueous saturated ammonium chloride solution, 20 mL of water was added, ethyl acetate (60 mL × 3) was added for extraction, the organic layers were collected, dried over anhydrous sodium sulfate, and filtered, and the filtrate was concentrated under reduced pressure to obtain **3B** (2.0 g, yield: 92%).

LCMS m/z = 257.2 [M+H]⁺.

### Step III: Preparation of 3C

Thionyl dichloride (8 mL, 110.28 mmol) was added to a solution of **3B** (1.0 g, 3.9 mmol) in dichloromethane (10 mL) and stirred at room temperature for 5 h, the mixture was then concentrated under reduced pressure, and an aqueous saturated sodium bicarbonate solution and ethyl acetate were added to the resulting crude product; after layering, the organic phase was collected, dried over anhydrous sodium sulfate, and filtered, and the filtrate was concentrated under reduced pressure to obtain **3C** (1.0 g, yield: 93%).

LCMS m/z = 275.0 [M+H]⁺.

### Step IV: Preparation of 3D

**3C** (106 mg, 0.39 mmol) and **1F** (60 mg, 0.35 mmol) were dissolved in a solution of N,N-dimethylformamide (2 mL), and cesium carbonate (342 mg, 1.05 mmol) was added and stirred at room temperature overnight. After the reaction was complete, the reaction product was filtered by diatomaceous earth to remove the residue, the filtrate was diluted by adding 10 mL of ethyl acetate and washed by adding water (10 mL × 3), the organic phases were collected and concentrated under reduced pressure, the residue was purified by silica gel column chromatography (ethyl acetate/petroleum ether (V/V) = 0/1-1/1) to obtain **3D** (65 mg, yield 45%).

LCMS m/z = 410.1 [M+H]⁺.

### Step V: Preparation of Compound 3 trifluoroacetate

**3D** (65 mg, 0.16mmol) was dissolved in a mixed solvent of 1,4-dioxane (10 mL) and water (1 mL), and (4-cyclopropyl-6-methoxypyrimidin-5-yl)boronic acid (46.6 mg, 0.24 mmol), potassium phosphate (101.9 mg, 0.48 mmol), and XPhos G3 (CAS: 1445085-55-1, 13.5 mg, 0.016 mmol) were added in sequence and heated to 90°C under nitrogen protection and stirred overnight. After the reaction was complete, a large amount of solvent was removed by concentration, 10 mL of water was added thereto, and ethyl acetate (10 mL × 3) was added for extraction. The organic phases were combined, dried over anhydrous sodium sulfate, and concentrated under reduced pressure, the crude product was purified by preparative HPLC (instrument: waters 2767 preparative liquid chromatographic instrument; chromatographic column: XBridge @ Prep C18 (30 mm × 150 mm); composition of mobile phase: mobile phase A: acetonitrile, mobile phase B: water (containing 0.1% trifluoroacetic acid)), and the preparation liquid was collected and freeze-dried to obtain **Compound 3** trifluoroacetate (10 mg, yield: 12%).

LCMS m/z = 524.2 [M+H]⁺.

¹H NMR (400 MHz, DMSO-*d*₆) δ 9.08 (d, 1H), 8.71 (s, 1H), 8.55 (d, 1H), 7.60-7.53 (m, 2H), 7.44-7.37 (m, 2H), 6.74 (s, 1H), 5.85 (s, 2H), 3.83 (s, 3H), 2.31 (s, 3H), 1.73 - 1.63 (m, 1H), 1.15-1.06 (m, 1H), 1.04-0.96 (m, 1H), 0.95 - 0.80 (m, 2H).

### Example 4:

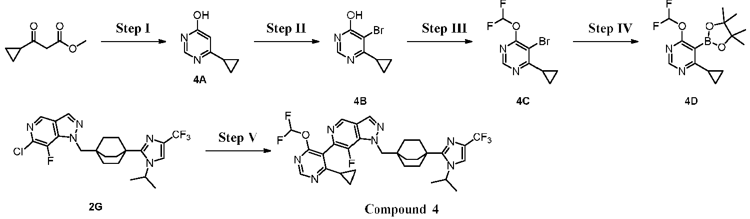

### Step I: Preparation of 4A

Methyl 3-cyclopropyl-3-oxopropionate (20 g, 140.66 mmol) and formamidine acetate (29.29 g, 281.32 mmol) were dissolved in methanol (400 mL), and sodium methoxide (53.19 g, 984.62 mmol) was added and reacted at room temperature for 2 days. The pH was adjusted to 7 with acetic acid, suction filtration was performed by diatomaceous earth, the filtrate was concentrated under reduced pressure, and the residue was purified by silica gel column chromatography (ethyl acetate/petroleum ether (V/V) = 0/1-1/3); (methanol/dichloromethane (V/V) = 0/1-1/10) to obtain **4A** (9 g, yield: 47%).

LCMS m/z = 137.1 [M+H]⁺.

### Step II: Preparation of 4B

**4A** (8.5 g, 62.43 mmol) was dissolved in acetonitrile (100 mL), and NBS (11.67 g, 65.60 mmol) was added and reacted at room temperature for 1 h. After extraction by adding water and ethyl acetate, the organic layer was washed with an aqueous saturated sodium chloride solution, dried over anhydrous sodium sulfate, and concentrated under reduced pressure, and the residue was purified by silica gel column chromatography (ethyl acetate/petroleum ether (V/V) = 0/1-1/1) to obtain **4B** (9.1 g, yield: 67%).

LCMS m/z = 215.0 [M+H]⁺.

### Step III: Preparation of 4C

**4B** (6 g, 27.90 mmol) was dissolved in acetonitrile (200 mL), sodium hydride (2.2 g, 54.90 mmol) was added, stirred at room temperature and reacted for 45 min, and 2-fluorosulfonyl difluoroacetic acid (8.94 g, 50.22 mmol) was added and reacted at room temperature for 2 h. After extraction by adding water and ethyl acetate, the organic layer was washed with an aqueous saturated sodium chloride solution, dried over anhydrous sodium sulfate, and concentrated under reduced pressure, and the residue was purified by silica gel column chromatography (ethyl acetate/petroleum ether (V/V) = 0/1-1/2) to obtain **4C** (4.0 g, yield: 54%).

LCMS m/z = 265.0 [M+H]⁺.

### Step IV: Preparation of 4D

**4C** (1.0 g, 3.77 mmol) was dissolved in 1,4-dioxane (15 mL), bis(pinacolato)diboron (1.05 g, 4.16 mmol), Pd(dppf)Cl₂·CH₂Cl₂ (0.31 g, 0.38 mmol), and potassium acetate (0.74 g, 7.54 mmol) were added in sequence, and the mixture was reacted under nitrogen protection at 90°C overnight. The reaction product was cooled to room temperature and extracted by adding water and ethyl acetate, the organic layer was washed with an aqueous saturated sodium chloride solution, dried over anhydrous sodium sulfate, and concentrated under reduced pressure, and the residue was purified by silica gel column chromatography (ethyl acetate/petroleum ether (V/V) = 1/1-1/20) to obtain **4D** (420 mg, yield: 35%).

LCMS m/z = 313.0 [M+H]⁺.

### Step V: Preparation of Compound 4

**2G** (100 mg, 0.21 mmol), **4D** (85.21 mg, 0.27 mmol), Pd(dppf)Cl₂·CH₂Cl₂ (17.15 mg, 0.021 mmol), and potassium carbonate (87.07 mg, 0.63 mmol) were placed in a 25 mL single-mouth flask, 1,4-dioxane (4 mL) and water (1 mL) were added, and after displacement with nitrogen three times, the mixture was stirred at 90°C for 3 h. After cooling to room temperature, 20 mL of water and 20 mL of ethyl acetate were added for extraction, the organic layer was dried over anhydrous sodium sulfate and then concentrated under reduced pressure, the residue was purified by silica gel column chromatography (ethyl acetate/petroleum ether (V/V) = 1/10-1/1), and the resulting crude product was further subjected to separation and purification by preparative HPLC (instrument: waters 2767 preparative liquid chromatographic instrument; chromatographic column: SunFire @ Prep C18 (19 mm × 150 mm); composition of mobile phase: mobile phase A: acetonitrile, mobile phase B: water (containing 5 mM ammonium acetate)) to obtain **Compound 4** (20 mg, yield: 15%).

LCMS m/z = 620.3 [M+H]⁺.

¹H NMR (400 MHz, DMSO-*d₆*) δ 9.10 (d, 1H), 8.85 (s, 1H), 8.52 (d, 1H), 8.03 - 7.63 (m, 2H), 4.88 - 4.74 (m, 1H), 4.39 - 4.26 (m, 2H), 1.95 - 1.79 (m, 7H), 1.61 - 1.46 (m, 6H), 1.32 (d, 6H), 1.23 - 1.18 (m, 1H), 1.13 - 0.92 (m, 3H).

### Example 5:

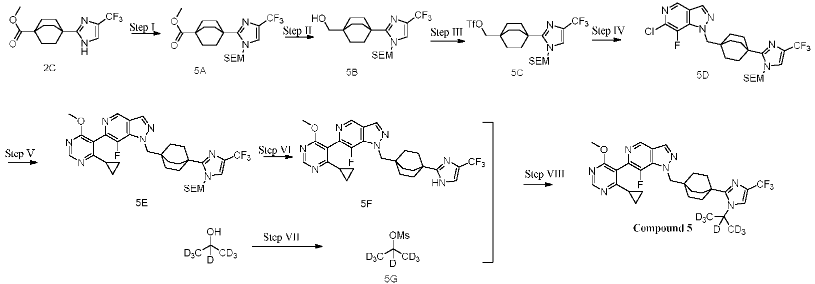

### Step I: Preparation of 5A

**2C** (4 g, 13.23 mmol) was dissolved in DMF (20 mL), NaH (0.79 g, 19.84 mmol, wt% = 60%) was slowly added in an ice bath and stirred in the ice bath for 10 min, 2-(trimethylsilyl)ethoxymethyl chloride (3.31 g, 19.85 mmol) was then added, and the mixture was naturally heated to room temperature and stirred for 30 min. 10 mL of an aqueous saturated ammonium chloride solution was added to quench the reaction, 20 mL of ethyl acetate was added for extraction, the organic layer was washed with an aqueous saturated sodium chloride solution (10 mL x 3), dried over anhydrous sodium sulfate, and concentrated under reduced pressure, and the residue was purified by silica gel column chromatography (ethyl acetate/petroleum ether (V/V) = 1/10-1/3) to obtain **5A** (5.2 g, yield: 90%).

### Step II: Preparation of 5B

**5A** (5.2 g, 12.02 mmol) was dissolved in THF (25 mL), lithium borohydride (1.31 g, 59.86 mmol) was added at room temperature, and after the addition was complete, the mixture was stirred at 60°C for 2 h. After cooling to room temperature, 10 mL of an aqueous saturated ammonium chloride solution was dropwise added slowly to quench the reaction, the reaction product was extracted by adding 20 ml of ethyl acetate, and the organic layer was dried over anhydrous sodium sulfate and concentrated under reduced pressure to obtain **5B** (5 g, yield: 100%).

LCMS m/z = 405.2 [M+H]⁺.

### Step III: Preparation of 5C

**5B** (4.81 g, 11.89 mmol) was dissolved in DCM (30 mL), pyridine (1.41 g, 17.84 mmol) was added, trifluoromethanesulfonic anhydride (4.03 g, 14.29 mmol) was dropwise added slowly in an ice bath, and the mixture was naturally heated to room temperature and stirred for 1 h. 30 mL of dichloromethane and 50 mL of water were added for extraction, and the organic layer was washed once with 30 ml of an aqueous saturated sodium chloride solution, dried over anhydrous sodium sulfate, and concentrated under reduced pressure to obtain 5C.

### Step IV: Preparation of 5D

**1F** (1.7 g, 9.91 mmol) and **5C** from the previous step were dissolved in DMF (30 mL), and in a water bath at room temperature, cesium carbonate (9.69 g, 29.73 mmol) was added. After the addition was complete, the mixture was stirred for 1 h, 50 mL of ethyl acetate and 50 mL of water were added for extraction, the organic layer was washed with an aqueous saturated sodium chloride solution (30 mL x 3), dried over anhydrous sodium sulfate, and concentrated under reduced pressure, and the residue was purified by silica gel column chromatography (ethyl acetate/petroleum ether (V/V) = 1/10-1/2) to obtain **5D** (2.3 g, yield: 41%).

LCMS m/z = 558.2 [M+H]⁺.

### Step V: Preparation of 5E

**5D** (1 g, 1.79 mmol) and (4-cyclopropyl-6-methoxypyrimidin-5-yl)boronic acid (0.69 g, 3.56 mmol) were dissolved in a mixed solvent of 1,4-dioxane (10 mL) and water (2 mL), and XPhos G3 (0.15 g, 0.18 mmol) and potassium phosphate (0.76 g, 3.58 mmol) were added in sequence; and after the addition was complete, displacement with nitrogen was performed three times, and the mixture was heated to 90°C and stirred overnight. After cooling to room temperature, 20 ml of ethyl acetate and 10 ml of water were added for extraction, the organic layer was dried over anhydrous sodium sulfate and concentrated under reduced pressure, and the residue was purified by column chromatography (ethyl acetate/petroleum ether (V/V) = 1/10-1/1) to obtain **5E** (1.1 g, yield: 91%).

### Step VI: Preparation of 5F

**5E** (1.1 g, 1.64 mmol) was dissolved in THF (15 mL), tetrabutylammonium fluoride (4.29 g, 16.4 mmol) was added, and the mixture was heated to 70°C and stirred for 6 h. After cooling to room temperature, 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/3-1/0) to obtain 5F (0.8 g, yield 90%).

LCMS m/z = 542.2 [M+H]⁺.

### Step VII: Preparation of 5G

Isopropanol-D7 (2 g, 29.79 mmol) was dissolved in DCM (20 mL), triethylamine (6.03 g, 59.59 mmol) was added in an ice bath, methylsufonyl chloride (4.10 g, 35.79 mmol) was dropwise added slowly, and after the addition was complete, the mixture was naturally heated to room temperature and stirred for 20 min. 30 mL of water and 30 mL of dichloromethane were added for extraction, and the organic layer was then washed with 20 mL of a saturated sodium bicarbonate solution and 20 mL of an aqueous sodium chloride solution in sequence, dried over anhydrous sodium sulfate, and concentrated under reduced pressure to obtain **5G** (4 g).

### Step VIII: Preparation of Compound 5

**5F** (0.1 g, 0.18 mmol) and **5G** (0.13 g, 0.90 mmol) were dissolved in DMF (5 mL), and cesium carbonate (0.29 g, 0.90 mmol) and sodium iodide (0.027 g, 0.18 mmol) were added in sequence and stirred at 80°C overnight. After cooling to room temperature, 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 5** (60 mg, yield 56%).

LCMS m/z = 591.3 [M+H]⁺.

¹H NMR (400 MHz, DMSO-*d*₆) δ 9.05 (d, 1H), 8.72 (s, 1H), 8.48 (d, 1H), 7.93 - 7.85 (m, 1H), 4.38 - 4.25 (m, 2H), 3.86 (s, 3H), 1.96 - 1.82 (m, 6H), 1.78 - 1.68 (m, 1H), 1.62 - 1.45 (m, 6H), 1.18 - 0.98 (m, 2H), 0.97 - 0.81 (m, 2H).

### Example 6:

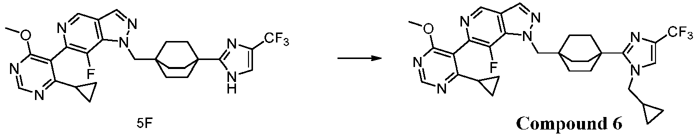

**5F** (0.1 g, 0.18 mmol) and bromomethylcyclopropane (0.049 g, 0.36 mmol) were dissolved in DMF (5 mL), and cesium carbonate (0.12 g, 0.36 mmol) was added and stirred at 60°C for 3 h. After cooling to room temperature, 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 6** (60 mg, yield 56%).

LCMS m/z = 596.3 [M+H]⁺.

¹H NMR (400 MHz, DMSO-*d*₆) δ 9.04 (d, 1H), 8.72 (s, 1H), 8.48 (d, 1H), 7.79 - 7.71 (m, 1H), 4.39 - 4.24 (m, 2H), 3.94 (d, 2H), 3.86 (s, 3H), 1.96 - 1.82 (m, 6H), 1.79 - 1.68 (m, 1H), 1.61 - 1.45 (m, 6H), 1.22 - 1.08 (m, 2H), 1.05 - 0.98 (m, 1H), 0.97 - 0.80 (m, 2H), 0.59 - 0.49 (m, 2H), 0.46 - 0.37 (m, 2H).

### Example 7:

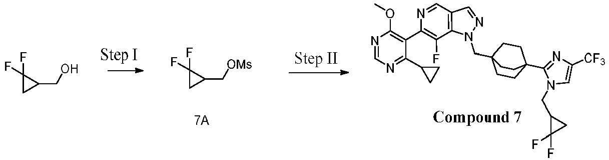

### Step I: Preparation of 7A

2,2-Difluorocyclopropyl methanol (1 g, 9.28 mmol) was dissolved in DCM (20 mL), triethylamine (1.88 g, 18.54 mmol) was added in an ice bath, methylsufonyl chloride (1.28 g, 11.11 mmol) was dropwise added slowly, and the mixture was naturally heated to room temperature and stirred for 20 min. 30 mL of water and 30 mL of dichloromethane were added for extraction, and the organic layer was then washed with 20 mL of an aqueous saturated sodium bicarbonate solution and 20 mL of an aqueous sodium chloride solution in sequence, dried over anhydrous sodium sulfate, and concentrated under reduced pressure to obtain 7A (1.6 g).

### Step II: Preparation of Compound 7

**5F** (0.1 g, 0.18 mmol) and **7A** (0.067 g, 0.36 mmol) were dissolved in DMF (5 mL), and cesium carbonate (0.12 g, 0.36 mmol) and sodium iodide (0.027 g, 0.18 mmol) were added in sequence and stirred at 60°C for 3 h. After cooling to room temperature, 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 7** (65 mg, yield 57%).

LCMS m/z = 632.7 [M+H]⁺.

¹H NMR (400 MHz, DMSO-*d*₆) δ 9.04 (d, 1H), 8.72 (s, 1H), 8.48 (d, 1H), 7.73 - 7.67 (m, 1H), 4.39 - 4.26 (m, 3H), 4.19 - 4.08 (m, 1H), 3.86 (s, 3H), 2.28 - 2.15 (m, 1H), 1.96 - 1.83 (m, 6H), 1.77 - 1.62 (m, 3H), 1.59 - 1.45 (m, 6H), 1.18 - 1.09 (m, 1H), 1.06 - 0.97 (m, 1H), 0.97 - 0.83 (m, 2H).

### Example 8:

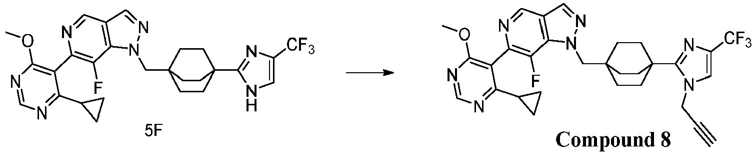

**5F** (0.1 g, 0.18 mmol) and 3-bromopropyne (0.043 g, 0.36 mmol) were dissolved in DMF (5 mL), and cesium carbonate (0.12 g, 0.36 mmol) was added and stirred at room temperature for 3 h. 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 8** (90 mg, yield 86%).

LCMS m/z = 580.2 [M+H]⁺.

¹H NMR (400 MHz, DMSO-*d*₆) δ 9.05 (d, 1H), 8.72 (s, 1H), 8.48 (d, 1H), 7.76 - 7.69 (m, 1H), 5.03 (d, 2H), 4.41 - 4.24 (m, 2H), 3.86 (s, 3H), 3.53 (t, 1H), 1.96 - 1.83 (m, 6H), 1.79 - 1.69 (m, 1H), 1.60 - 1.43 (m, 6H), 1.18 - 1.09 (m, 1H), 1.06 - 0.98 (m, 1H), 0.98 - 0.82 (m, 2H).

### Example 9:

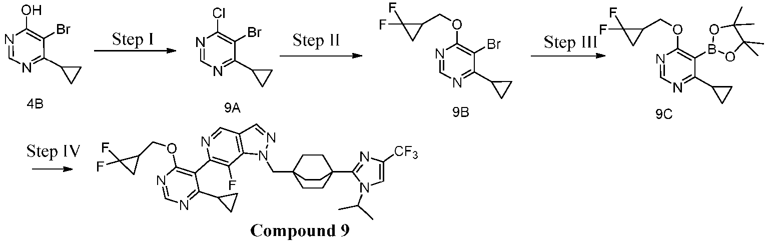

### Step I: Preparation of 9A

4B (1 g, 4.65 mmol) was dissolved in acetonitrile (10 mL), phosphorus oxychloride (7.13 g, 46.50 mmol) was added slowly at room temperature, and after the addition was complete, the mixture was stirred at 80°C for 4 h. After cooling to room temperature, the reaction liquid was poured into ice-water, the pH was adjusted to alkalinity with an aqueous saturated sodium bicarbonate solution, 30 mL of ethyl acetate was added for extraction, and the organic layer was dried over anhydrous sodium sulfate and then concentrated under reduced pressure to obtain 9A.

### Step II: Preparation of 9B

2,2-Difluorocyclopropyl methanol (0.93 g, 8.60 mmol) was dissolved in THF (10 mL), and sodium hydride (0.25 g, 6.3 mmol, wt% = 60%) was added slowly in an ice bath; and after the addition was complete, the mixture was stirred in an ice bath for 20 min, **9A** (1 g, 4.28 mmol) was slowly added, and the mixture was naturally heated to room temperature and stirred for 20 min. 10 mL of an aqueous saturated ammonium chloride solution was added to quench the reaction, 20 mL of ethyl acetate and 20 mL of water were added for extraction, and the organic layer was dried over anhydrous sodium sulfate and concentrated under reduced pressure to obtain 9B (1.4 g).

LCMS m/z = 305.1 [M+H]⁺.

### Step III: Preparation of 9C

**9B** (1.3 g, 4.26 mmol) was dissolved in THF (10 mL), and a solution of isopropylmagnesium chloride in tetrahydrofuran (3.2 mL, 6.39 mmol, 2N) was dropwise added slowly in an ice bath and stirred at room temperature for 1.5 h. At 0°C, 2 mL of a solution of isopropyl pinacol borate (1.19 g, 6.39 mmol) in THF was dropwise added slowly and stirred at room temperature for 1 h. 20 mL of water was added to quench the reaction, 20 mL of ethyl acetate was added for extraction, the organic layer was dried over anhydrous sodium sulfate and concentrated under reduced pressure, and the residue was purified by silica gel column chromatography (ethyl acetate/petroleum ether (V/V) = 0/1-1/10) to obtain **9C** (0.23 g, yield: 15%).

LCMS m/z = 353.2 [M+H]⁺.

### Step IV: Preparation of Compound 9

**2G** (0.11 g, 0.23 mmol) and **9C** (0.12 g, 0.34 mmol) were dissolved in a mixed solvent of 1,4-dioxane (4 mL) and water (1 mL), XPhos G3 (0.019 g, 0.023 mmol) and potassium phosphate (0.098 g, 0.46 mmol) were added in sequence; and after the addition was complete, displacement with nitrogen was performed three times, and the mixture was stirred at 90°C overnight. After cooling to room temperature, 20 mL of ethyl acetate and 10 mL of water were added for extraction, the organic layer was dried over anhydrous sodium sulfate and concentrated under reduced pressure, the residue was purified by silica gel column chromatography (ethyl acetate/petroleum ether (V/V) = 1/10-1/1), and the resulting crude product was further subjected to separation and purification by preparative HPLC (instrument: waters 2767 preparative liquid chromatographic instrument; chromatographic column: SunFire @ Prep C18 (19 mm × 150 mm); composition of mobile phase: mobile phase A: acetonitrile, mobile phase B: water (containing 5 mM ammonium acetate)) to obtain **Compound 9** (30 mg, yield: 20%).

LCMS m/z = 660.90 [M+H]⁺.

¹H NMR (400 MHz, DMSO-*d*₆) δ 9.06 (d, 1H), 8.71 (d, 1H), 8.49 (d, 1H), 7.91 - 7.84 (m, 1H), 4.88 - 4.73 (m, 1H), 4.68 - 4.14 (m, 4H), 2.17 - 2.00 (m, 1H), 1.94 - 1.80 (m, 6H), 1.78 - 1.69 (m, 1H), 1.65 - 1.45 (m, 7H), 1.43 - 1.28 (m, 7H), 1.18 - 1.10 (m, 1H), 1.05 - 0.91 (m, 2H), 0.90 - 0.80 (m, 1H).

### Example 10:

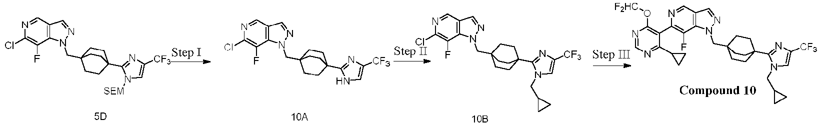

### Step I: Preparation of 10A

**5D** (0.7 g, 1.25 mmol) was dissolved in DCM (4 mL), and trifluoroacetic acid (4 mL) was added and stirred at room temperature overnight. The mixture was concentrated under reduced pressure, 20 mL of dichloromethane was added to the residue, 10 mL of an aqueous saturated sodium bicarbonate solution was added and stirred for 10 min, and after liquid separation, the organic layer was dried over anhydrous sodium sulfate and concentrated under reduced pressure to obtain **10A.**

### Step II: Preparation of 10B

**10A** (0.1 g, 0.23 mmol) was dissolved in DMF (5 mL), and bromomethylcyclopropane (0.062 g, 0.46 mmol) was added and stirred at 60°C for 3 h. After cooling to room temperature, 20 mL of ethyl acetate and 20 mL of water were added and stirred; and after layering, the organic layer was washed with an aqueous saturated sodium chloride solution (10 mL x 3), dried over anhydrous sodium sulfate, and concentrated under reduced pressure, and the residue was purified by silica gel column chromatography (ethyl acetate/petroleum ether (V/V) = 1/5-1/2) to obtain 10B (0.1 g, yield: 90%).

### Step III: Preparation of Compound 10

**10B** (0.1 g, 0.21 mmol) and 4D (0.098 g, 0.32 mmol) were dissolved in a mixed solvent of 1,4-dioxane (4 mL) and water (1 mL), XPhos Pd G2 (0.017 g, 0.021 mmol) and potassium phosphate (0.089 g, 0.42 mmol) were added in sequence, and after displacement with nitrogen three times, the mixture was stirred at 90°C for 3 h. After cooling to room temperature, 20 ml of ethyl acetate and 10 ml of water were added for extraction, the organic layer was dried over anhydrous sodium sulfate and concentrated under reduced pressure, and the residue was purified by silica gel column chromatography (ethyl acetate/petroleum ether (V/V) = 1/10-1/1) to obtain **Compound 10** (90 mg, yield: 68%).

LCMS m/z = 632.90 [M+H]⁺.

¹H NMR (400 MHz, DMSO-*d*₆) δ 9.10 (d, 1H), 8.85 (s, 1H), 8.52 (d, 1H), 8.05 - 7.62 (m, 2H), 4.42 - 4.24 (m, 2H), 3.93 (d, 2H), 1.94 - 1.81 (m, 7H), 1.64 - 1.46 (m, 6H), 1.27 - 1.18 (m, 2H), 1.18 - 1.13 (m, 1H), 1.06 - 0.93 (m, 2H), 0.58 - 0.50 (m, 2H), 0.45 - 0.37 (m, 2H).

### Example 11:

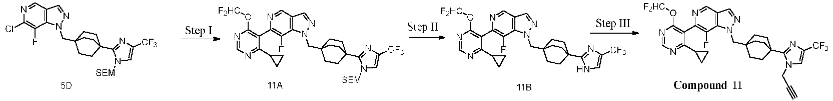

### Step I: Preparation of 11A

**5D** (0.100 g, 0.18 mmol) and **4D** (0.084 g, 0.27 mmol) were dissolved in a mixed solvent of 1,4-dioxane (4 mL) and water(1 mL), potassium phosphate (0.076 g, 0.36 mmol) and XPhos Pd G2 (0.014 g, 0.018 mmol) were added in sequence, and after displacement with nitrogen three times, the mixture was stirred at 90°C overnight. After cooling to room temperature, 20 mL of ethyl acetate and 10 mL of water were added for extraction, the organic layer was dried over anhydrous sodium sulfate and concentrated under reduced pressure, and the residue was purified by silica gel column chromatography (ethyl acetate/petroleum ether (V/V) = 1/10-1/2) to obtain **11A** (0.13 g, yield: 99%).

### Step II: Preparation of 11B

**11A** (0.13 g, 0.18 mmol) was dissolved in DCM (4 mL), and trifluoroacetic acid (4 mL) was added at room temperature and stirred at room temperature overnight. The mixture was concentrated under reduced pressure, 20 mL of dichloromethane was added to the residue, 10 mL of an aqueous saturated sodium bicarbonate solution was added and stirred for 10 min, and after liquid separation, the organic layer was dried over anhydrous sodium sulfate and concentrated under reduced pressure to obtain 11B.

### Step III: Preparation of Compound 11

**11B** (0.11 g, 0.19 mmol) and 3-bromopropyne (0.045 g, 0.38 mmol) were dissolved in DMF (5 mL), and cesium carbonate (0.12 g, 0.38 mmol) was added and stirred at room temperature for 3 h. 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by silica gel column chromatography (ethyl acetate/petroleum ether (V/V) = 1/5-1/1) to obtain **Compound 11** (70 mg, yield 60%).

LCMS m/z = 616.80 [M+H]⁺.

¹H NMR (400 MHz, DMSO-*d*₆) δ 9.10 (d, 1H), 8.85 (s, 1H), 8.52 (d, 1H), 8.03 - 7.62 (m, 2H), 5.02 (d, 2H), 4.40 - 4.26 (m, 2H), 3.51 (t, 1H), 1.95 - 1.81 (m, 7H), 1.63 - 1.41 (m, 6H), 1.30 - 1.20 (m, 1H), 1.12 - 1.01 (m, 2H), 1.01 - 0.92 (m, 1H).

### Example 12:

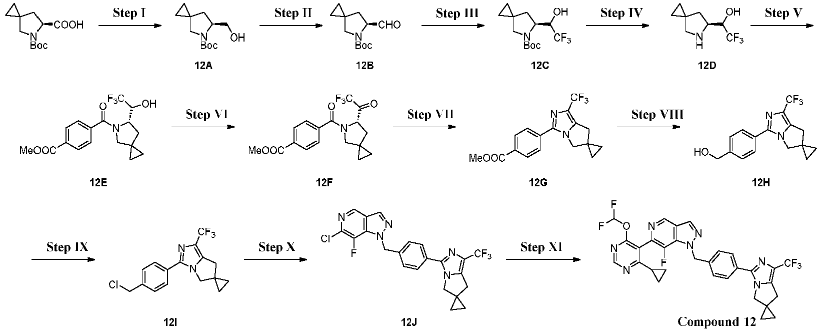

### Step I: Preparation of 12A

(S)-5-(tert-butyloxycarboryl)-5-azaspiro[2.4]heptane -6- carboxylic acid (10 g, 41.49 mmol) was dissolved in THF (100 mL), and a borane tetrahydrofuran solution (62 mL, 1M) was dropwise added slowly at 0°C and stirred at room temperature for 2 h. Methanol was added at 0°C for quenching, the mixture was concentrated under reduced pressure, ethyl acetate and water were added to the residue for extraction, and the organic phase was washed with an aqueous saturated sodium chloride solution, dried over anhydrous sodium sulfate, and concentrated under reduced pressure to obtain **12A** (9.42 g).

### Step II: Preparation of 12B

**12A** (9.42 g, 41.44 mmol) was dissolved in DMSO (60 mL), and 2-iodoxybenzoic acid (12.76 g, 45.58 mmol) was added and stirred at room temperature for 2 h. 2-Iodoxybenzoic acid (6 g, 21.41 mmol) was added and reacted at 40°C for 1 h. 100 mL of ethyl acetate was added and stirred at room temperature for 10 min. After filtration, the filter cake was washed with ethyl acetate (3 × 30 mL), 100 mL of an aqueous saturated sodium thiosulfate solution was added to the filtrate and then stirred for 10 min, 50 mL of a saturated sodium bicarbonate solution was added for extraction, the organic phase was washed with an aqueous saturated sodium chloride solution three times, dried over anhydrous sodium sulfate, dried over anhydrous sodium sulfate, and concentrated under reduced pressure, and the residue was purified by silica gel column chromatography (ethyl acetate/petroleum ether (V/V) = 0/1-1/5) to obtain **12B** (8 g, yield: 85%).

### Step III: Preparation of 12C

At 0°C, (trifluoromethyl)trimethylsilane (4.73 g, 33.29 mmol) was added to a solution of **12B** (5 g, 22.19 mmol) in tetrahydrofuran (50 mL). After stirring at 0°C for 10 min, tetrabutylammonium fluoride (4.66 mL, 4.66 mmol, 1M solution in THF) was added and stirred at 0°C for 1 h, tetrabutylammonium fluoride (5 mL, 5 mmol, 1M solution in THF) was then added, the mixture was stirred at room temperature for 2 h, an aqueous saturated ammonium chloride solution (100 mL) was then added for quenching, and the reaction product was extracted with ethyl acetate (100 mL × 3). The organic phases were combined, dried over anhydrous sodium sulfate and then concentrated under reduced pressure, and the residue was purified by silica gel column chromatography (ethyl acetate/petroleum ether (V/V) = 1/100-1/5) to obtain **12C** (5.9 g, yield: 90%).

LCMS m/z = 240.2 [M-55]⁺.

### Step IV: Preparation of 12D hydrochloride

**12C** (1.73 g, 5.86 mmol) was dissolved in a hydrochloric acid-dioxane solution (30 mL, 4 M) and stirred at room temperature for 2 h. After the reaction was complete, **12D hydrochloride** (1.34 g) was obtained by concentration under reduced pressure.

### Step V: Preparation of 12E

2-(7-Azobenzotriazole)-N,N,N',N'-tetramethylurea hexafluorophosphate (4.16 g, 10.95 mmol) was added to **12D hydrochloride** (1.69 g, 7.30 mmol) and 4-(methoxycarbonyl)benzoic acid (1.45 g, 8.03 mmol) in N,N-dimethylformamide (30 mL) and stirred at room temperature for 5 min. N,N-diisopropylethylamine (2.83 g, 21.9 mmol) was then added, 200 mL of ethyl acetate and 100 mL of water were added. After layering, the organic layer was dried under reduced pressure, and the residue was purified by silica gel column chromatography (ethyl acetate/petroleum ether (V/V) = 1/100-1/5) to obtain **12E** (2.5 g, yield 95%).

LCMS m/z = 358.2 [M+H]⁺.

### Step VI: Preparation of 12F

**12E** (2.5 g, 7.00 mmol) was dissolved in dichloromethane (30 mL), and Dess-Martin periodinane (4.45 g, 10.5 mmol) was added at 0°C in portions, and reacted at room temperature for 3 h. After direct concentration under reduced pressure, the residue was purified by silica gel column chromatography (ethyl acetate/petroleum ether (V/V) = 1/100-1/5) to obtain **12F** (2 g, yield: 80%).

### Step VII: Preparation of 12G

Ammonium trifluoroacetate (33.12 g, 252.73 mmol) was added to **12F** (2 g, 5.63 mmol) and reacted at 130°C for 2 h. After cooling to room temperature, 100 mL of water and ethyl acetate (100 mL × 3) were added for extraction, the organic phases were combined, dried over anhydrous sodium sulfate and then concentrated under reduced pressure, and the residue was purified by silica gel column chromatography (ethyl acetate/petroleum ether (V/V) = 1/100-1/2) to obtain **12G** (1.4 g, yield: 73%).

LCMS m/z = 337.1 [M+H]⁺.

### Step VIII: Preparation of 12H

At 0°C, diisobutylaluminium hydride (10.4 mL, 10.4 mmol, 1.0 M solution in hexanes) was dropwise added to **12G** (1.4 g, 4.16 mmol) in tetrahydrofuran solution (20 mL), and the mixture was naturally heated to room temperature and reacted for 3 h. 60 mL of water and ethyl acetate (60 mL × 3) were added for extraction, the organic phases were combined, dried over anhydrous sodium sulfate, and concentrated under reduced pressure, and the residue was purified by silica gel column chromatography (ethyl acetate/petroleum ether (V/V) = 1/100-1/2) to obtain **12H** (0.95 g, yield: 74%).

LCMS m/z = 309.2 [M+H]⁺.

### Step IX: Preparation of 12I

**12H** (0.95 g, 3.08 mmol) was dissolved in 10 mL of dichloromethane, and thionyl chloride (1.83 g, 15.40 mmol) was dropwise added slowly in an ice-water bath and stirred in the ice-water bath for 2 h. Saturated sodium bicarbonate (20 mL) was added for quenching, dichloromethane (30 mL × 3) was added for extraction, and the organic phases were combined, dried over anhydrous sodium sulfate, and concentrated under reduced pressure to obtain **12I** (0.95 g).

LCMS m/z = 327.3 [M+H]⁺.

### Step X: Preparation of 12J

Cesium carbonate (1495.51 mg, 4.59 mmol) was added to a solution of **121** (500 mg, 1.53 mmol) and **1F** (275.61 mg, 1.61 mmol) in N,N-dimethylformamide (10 mL) and reacted at room temperature for 3 h. After extraction by adding water and ethyl acetate, the organic phase was washed with an aqueous saturated sodium chloride solution, dried over anhydrous sodium sulfate, and concentrated under reduced pressure, and the residue was purified by silica gel column chromatography (ethyl acetate/petroleum ether (V/V) = 0/1-2/1) to obtain **12J** (330 mg, yield: 46%).

LCMS m/z = 462.1 [M+H]⁺.

### Step XI: Preparation of 12

**12J** (100 mg, 0.22 mmol), **4D** (103 mg, 0.33 mmol), XPhos Pd G2 (17.31 mg, 0.022 mmol) and potassium phosphate (140.10 mg, 0.66 mmol) were placed in a 25 mL single-mouth flask, 1,4-dioxane (4 mL) and water (1 mL) were radded, and after displacement with nitrogen three times, the mixture was stirred at 90°C for 3 h. After cooling to room temperature, 20 mL of water and 20 mL of ethyl acetate were added for extraction, the organic layer was dried over anhydrous sodium sulfate and then concentrated under reduced pressure, and the residue was purified by silica gel column chromatography (ethyl acetate/petroleum ether (V/V) = 0/1-2/1) to obtain **Compound 12** (50 mg, yield: 37%).

LCMS m/z = 612.7 [M+H]⁺.

¹H NMR (400 MHz, DMSO-*d*₆) δ 9.12 (d, 1H), 8.84 (s, 1H), 8.56 (d, 1H), 8.03 - 7.59 (m, 3H), 7.34 (d, 2H), 5.90 - 5.72 (m, 2H), 4.23 (s, 2H), 2.95 (s, 2H), 1.89 - 1.76 (m, 1H), 1.21 - 1.14 (m, 1H), 1.12 - 0.98 (m, 2H), 0.98 - 0.89 (m, 1H), 0.78 (s, 4H).

### Example 13:

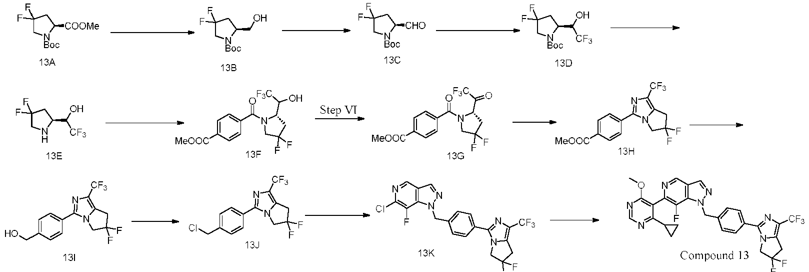

### Step I: Preparation of 13B

**13A** (7 g, 26.4 mmol) was dissolved in tetrahydrofuran (100 mL), and lithium borohydride (1.72 g, 79.2 mmol) was added at 0°C in portions and reacted at room temperature for 3 h. Water (50 mL) was added for quenching, ethyl acetate (40 mL × 3) was added for extraction, the organic phases were combined, dried over anhydrous sodium sulfate and then concentrated under reduced pressure, and the residue was purified by silica gel column chromatography (ethyl acetate/petroleum ether (V/V) = 1/100-1/10) to obtain **13B** (6 g, yield: 96%).

LCMS m/z = 182.1 [M-55]⁺.

### Step II: Preparation of 13C

**13B** (6 g, 25.3 mmol) was dissolved in dichloromethane (100 mL), and Dess-Martin periodinane (16.1 g, 37.95 mmol) was added at 0°C in portions, and reacted at room temperature for 3 h. A saturated sodium thiosulfate solution (50 mL) was added for quenching, dichloromethane (30 mL × 3) was added for extraction, the organic phases were combined, dried over anhydrous sodium sulfate and then concentrated under reduced pressure, and the residue was purified by silica gel column chromatography (ethyl acetate/petroleum ether (V/V) = 1/100-1/10) to obtain **13C** (3.2 g, yield 54%).

### Step III: Preparation of 13D

At 0°C, (trifluoromethyl)trimethylsilane (2.9 g, 20.4 mmol) was added to a solution of **13C** (3.2 g, 13.6 mmol) in tetrahydrofuran (50 mL). After stirring at 0°C for 10 min, tetrabutylammonium fluoride (0.86 mL, 0.86 mmol, 1M solution in THF) was added and stirred at 0°C for 1 h, tetrabutylammonium fluoride (2 mL, 2 mmol, 1M solution in THF) was then added, the mixture was stirred at room temperature for 1 h, an aqueous saturated ammonium chloride solution (50 mL) was then added for quenching, and the reaction product was extracted with ethyl acetate (30 mL × 3). The organic phases were combined, dried over anhydrous sodium sulfate and then concentrated under reduced pressure, and the residue was purified by silica gel column chromatography (ethyl acetate/petroleum ether (V/V) = 1/100-1/10) to obtain **13D** (4 g, yield: 96%).

### Step IV: Preparation of 13E hydrochloride

**13D** (4 g, 13.1 mmol) was dissolved in a hydrochloric acid-dioxane solution (50 mL, 4 M) and stirred at room temperature for 2 h. After the reaction was complete, **13E hydrochloride** was obtained by concentration under reduced pressure.

### Step V: Preparation of 13F

2-(7-azobenzotriazole)-N,N,N',N'-tetramethylurea hexafluorophosphate (7.23 g, 19 mmol) was added to **13E** (2.6 g, 12.67 mmol) and 4-(methoxycarbonyl)benzoic acid (2.05 g, 11.4 mmol) in N,N-dimethylformamide (20 mL) and stirred at room temperature for 5 min. N,N-diisopropylethylamine (3.27 g, 25.34 mmol) was then added, 100 mL of ethyl acetate and 50 mL of water were added. After layering, the organic layer was dried under reduced pressure, and the residue was purified by silica gel column chromatography (ethyl acetate/petroleum ether (V/V) = 1/100-1/5) to obtain **13F** (3.3 g, yield 71%).

LCMS m/z = 368.4 [M+H]⁺.

### Step VI: Preparation of 13G

**13F** (2.5 g, 6.81 mmol) was dissolved in dichloromethane (50 mL), and Dess-Martin periodinane (4.33 g, 10.21 mmol) was added at 0°C in portions, and the mixture was heated to room temperature and reacted for 3 h. A aqueous saturated sodium thiosulfate solution (50 mL) was added for quenching, dichloromethane (30 mL × 3) was added for extraction, the organic phases were combined, dried over anhydrous sodium sulfate and then concentrated under reduced pressure, and the residue was purified by silica gel column chromatography (ethyl acetate/petroleum ether (V/V) = 1/100-1/10) to obtain **13G** (2.4 g, yield 96%).

### Step VII: Preparation of 13H

Ammonium trifluoroacetate (20 g, 76.31 mmol) was added to **13G** (2.4 g, 6.57 mmol) in 1,4-dioxane solution (10 mL) and reacted at 130°C for 2 h. After cooling to room temperature, 50 mL of water and ethyl acetate (30 mL × 3) were added for extraction, the organic phases were combined, dried over anhydrous sodium sulfate and then concentrated under reduced pressure, and the residue was purified by silica gel column chromatography (ethyl acetate/petroleum ether (V/V) = 1/100-1/10) to obtain **13H** (1.1 g, yield: 48%).

LCMS m/z = 347.1 [M+H]⁺.

### Step VIII: Preparation of 13I

At 0°C, diisobutylaluminium hydride (9.54 mL, 9.54 mmol, 1.0 M solution in hexanes) was dropwise added to **13H** (1.1 g, 3.18 mmol) in tetrahydrofuran solution (55 mL) and reacted at 0°C for 2 h. 50 mL of water and ethyl acetate (30 mL × 3) were added for extraction, the organic phases were combined, dried over anhydrous sodium sulfate, and concentrated under reduced pressure, and the residue was purified by silica gel column chromatography (ethyl acetate/petroleum ether (V/V) = 1/100-1/10) to obtain **13I** (0.76 g, yield: 75%).

LCMS m/z = 319.3 [M+H]⁺.

### Step IX: Preparation of 13J

**13I** (760 mg, 2.39 mmol) was dissolved in 20 mL of dichloromethane, and thionyl chloride (0.85 g, 7.17 mmol) was dropwise added slowly in an ice-water bath and stirred in the ice-water bath for 2 h. Saturated sodium bicarbonate (20 mL) was added for quenching, dichloromethane (30 mL × 3) was added for extraction, and the organic phases were combined, dried over anhydrous sodium sulfate, and concentrated under reduced pressure to obtain **13J** (800 mg).

LCMS m/z = 337.3 [M+H]⁺.

### Step X: Preparation of 13K

Cesium carbonate (1.45 g, 4.47 mmol) was added to a solution of **13J** (500 mg, 1.49 mol) and **1F** (268 mg, 1.56 mmol) in N,N-dimethylformamide (10 mL) and stirred at room temperature for 2 h. After extraction by adding water and ethyl acetate, the organic phase was washed with an aqueous saturated sodium chloride solution, dried over anhydrous sodium sulfate, and concentrated under reduced pressure, and the residue was purified by silica gel column chromatography (ethyl acetate/petroleum ether (V/V) = 0/1-2/1) to obtain **13K** (300 mg, yield: 43%).

LCMS m/z = 472.1 [M+H]⁺.

### Step XI: Preparation of Compound 13

**13K** (0.1 g, 0.21 mmol) was dissolved in a mixed solvent of 1,4-dioxane (10 mL) and water (3 mL), (4-cyclopropyl-6-methoxypyrimidin-5-yl)boronic acid (61 mg, 0.32 mmol), potassium phosphate (0.13 g, 0.60 mmol) and XPhos G2 (CAS: 1310584-14-5, 16.5 mg, 0.021 mmol) were added in sequence, and the mixture was stirred under nitrogen protection at 90°C for 3 h. After cooling to room temperature, 10 mL of ethyl acetate and 10 mL of water were added, and after layering, the organic layer was dried over anhydrous sodium sulfate, and concentrated under reduced pressure. The residue was purified by silica gel column chromatography (ethyl acetate/petroleum ether (V/V) = 1/5-1/1) to obtain **13** (60 mg, yield: 48.8%).

LCMS m/z = 586.2 [M+H]⁺.

¹H NMR (400 MHz, DMSO-*d*₆) δ 9.07 (d, 1H), 8.71 (s, 1H), 8.54 (d, 1H), 7.82 (d, 2H), 7.37 (d, 2H), 5.82 (s, 2H), 4.93 (t, 2H), 3.84 (s, 3H), 3.72 (t, 2H), 1.73 - 1.64 (m, 1H), 1.16 - 1.06 (m, 1H), 1.05 - 0.96 (m, 1H), 0.95 - 0.78 (m, 2H).

### Example 14:

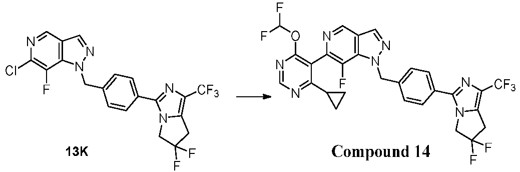

**13K** (0.1 g, 0.21 mmol) was dissolved in a mixed solvent of 1,4-dioxane (10 mL) and water (3 mL), **4D** (98 mg, 0.32 mmol), potassium phosphate (0.13 g, 0.6 mmol) and XPhos G2 (CAS: 1310584-14-5, 17 mg, 0.021 mmol) were added in sequence, and the mixture was stirred under nitrogen protection at 90°C for 3 h. After cooling to room temperature, 10 mL of ethyl acetate and 10 mL of water were added, and after layering, the organic layer was dried over anhydrous sodium sulfate, and concentrated under reduced pressure. The residue was purified by silica gel column chromatography (ethyl acetate/petroleum ether (V/V) = 1/5-1/1) to obtain **Compound 14** (66 mg, yield: 51%).

LCMS m/z = 622.7 [M+H]⁺.

¹H NMR (400 MHz, DMSO-*d*₆) δ 9.12 (d, 1H), 8.84 (s, 1H), 8.57 (d, 1H), 8.00 - 7.60 (m, 3H), 7.37 (d, 2H), 5.89 - 5.77 (m, 2H), 4.91 (t, 2H), 3.71 (t, 2H), 1.88 - 1.78 (m, 1H), 1.21 - 1.12 (m, 1H), 1.11 - 0.90 (m, 3H).

### Example 15:

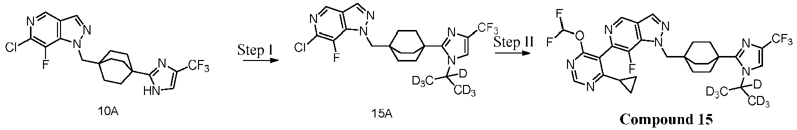

### Step I: Preparation of 15A

**10A** (0.15 g, 0.35 mmol) and **5G** (0.25 g, 1.72 mmol) were dissolved in DMF (5 mL), cesium carbonate (0.57 g, 1.75 mmol) and sodium iodide (0.052 g, 0.35 mmol) were added in sequence, and the mixture was stirred at 80°C overnight. After cooling to room temperature, 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/2) to obtain Compound 15A (0.1 g, yield 60%).

### Step II: Preparation of Compound 15

**15A** (0.1 g, 0.21 mmol) and **4D** (0.098 g, 0.32 mmol) were dissolved in 1,4-dioxane (4 mL) and water (1 mL), XPhos Pd G2 (0.017 g, 0.021 mmol) and potassium phosphate (0.089 g, 0.42 mmol) were added in sequence, and after displacement with nitrogen three times, the mixture was stirred at 90°C for 3 h. After cooling to room temperature, 20 ml of ethyl acetate and 10 ml of water were added for extraction, the organic layer was dried over anhydrous sodium sulfate and concentrated under reduced pressure, and the residue was purified by column chromatography (ethyl acetate/petroleum ether (V/V) = 1/10-1/1) to obtain **Compound 15** (70 mg, yield: 53%).

LCMS m/z = 627.80 [M+H]⁺.

¹H NMR (400 MHz, DMSO-*d*₆) δ 9.10 (d, 1H), 8.85 (s, 1H), 8.52 (d, 1H), 8.04-7.62 (m, 2H), 4.40-4.25 (m, 2H), 1.93-1.81 (m, 7H), 1.63 - 1.43 (m, 6H), 1.24-1.16 (m, 1H), 1.12 - 1.02 (m, 2H), 1.01-0.93 (m, 1H).

### Example 16:

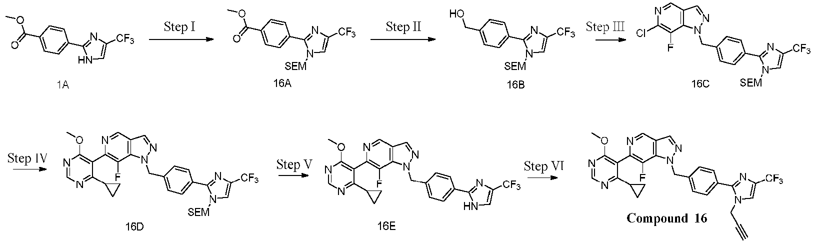

### Step I: Preparation of 16A

**1A** (3 g, 11.10 mmol) was dissolved in 30 mL of DMF, sodium hydride (0.66 g, 16.5 mmol, wt% = 60%) was added in an ice-water bath and reacted at 0°C for 30 min, and 2-(trimethylsilyl)ethoxymethyl chloride (2.77 g, 16.63 mmol) was added and reacted at room temperature for 1 h. After quenching by adding water, water and ethyl acetate were added for extraction, the organic phase was washed with an aqueous saturated sodium chloride solution, dried over anhydrous sodium sulfate, and concentrated under reduced pressure, and the residue was purified by silica gel column chromatography (ethyl acetate/petroleum ether (V/V) = 0/1-1/5) to obtain **16A** (3.9 g, yield: 87%).

LCMS m/z = 401.3 [M+H]⁺.

### Step II: Preparation of 16B

**16A** (3.9 g, 9.74 mmol) was dissolved in 30 mL of THF, and lithium borohydride (1.06 g, 48.7 mmol) was added slowly in portions and stirred at 50°C for 3 h. After cooling to room temperature, an aqueous saturated ammonium chloride solution was added in an ice-water bath to quench the reaction, the reaction product was extracted by adding water and ethyl acetate, and the organic layer was dried over anhydrous sodium sulfate, concentrated under reduced pressure to obtain **16B** (3.5 g, yield: 96%).

LCMS m/z = 373.4 [M+H]⁺.

### Step III: Preparation of 16C

**1F** (0.5 g, 2.91 mmol), **16B** (1.19 g, 3.20 mmol) and tri-n-butyl phosphorus (1.18 g, 5.82 mmol) were added to THF (10 mL), diethyl azodicarboxylate (1.01 g, 5.82 mmol) was dropwise added at room temperature under nitrogen protection and stirred at room temperature for 1 h. After concentration under reduced pressure, the residue was purified by silica gel column chromatography (ethyl acetate/petroleum ether (V/V) =1/10-1/2) to obtain **16C** (0.52 g, yield: 34%).

LCMS m/z = 526.60 [M+H]⁺.

### Step IV: Preparation of 16D

**16C** (0.2 g, 0.38 mmol) and (4-cyclopropyl-6-methoxypyrimidin-5-yl)boronic acid (0.13 g, 0.67 mmol) were dissolved in a mixed solvent of 1,4-dioxane (4 mL) and water (1 mL), potassium phosphate (0.16 g, 0.76 mmol) and XPhos G3 (0.032 g, 0.038 mmol) were added in sequence, and after displacement with nitrogen three times, the mixture was stirred at 90°C for 3 h. After cooling to room temperature, 20 ml of ethyl acetate and 10 ml of water were added for extraction, the organic layer was dried over anhydrous sodium sulfate and concentrated under reduced pressure, and the residue was purified by column chromatography (ethyl acetate/petroleum ether (V/V) = 1/10-1/2) to obtain **16D** (0.22 g, yield: 90%).

### Step V: Preparation of 16E

**16D** (0.22 g, 0.34 mmol) was dissolved in DCM (4 mL), and TFA (4 mL) was added and stirred at room temperature overnight. After concentration under reduced pressure, 20 mL of dichloromethane was added, 10 mL of an aqueous saturated sodium bicarbonate solution was added for alkalization, and after liquid separation, the organic layer was dried over anhydrous sodium sulfate and concentrated under reduced pressure to obtain 16E.

### Step VI: Preparation of Compound 16

**16E** (0.090 g, 0.18 mmol) and 3-bromopropyne (0.043 g, 0.36 mmol) were dissolved in DMF (5 mL), and cesium carbonate (0.12 g, 0.36 mmol) was added and stirred at room temperature for 3 h. 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 16** (70 mg, yield 71%).

LCMS m/z = 548.2 [M+H]⁺.

¹H NMR (400 MHz, DMSO-*d*₆) δ 9.07 (d, 1H), 8.71 (s, 1H), 8.54 (d, 1H), 8.04-8.01 (m, 1H), 7.75-7.70 (m, 2H), 7.42-7.37 (m, 2H), 5.84 (s, 2H), 4.99 (d, 2H), 3.84 (s, 3H), 3.56 (t, 1H), 1.74 - 1.65 (m, 1H), 1.14-1.07 (m, 1H), 1.04 - 0.96 (m, 1H), 0.94 - 0.78 (m, 2H).

### Example 17:

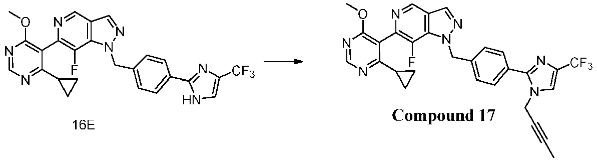

**16E** (0.090 g, 0.18 mmol) and 1-bromo-2-butyne (0.047 g, 0.35 mmol) were dissolved in DMF (5 mL), and cesium carbonate (0.12 g, 0.36 mmol) was added and stirred at room temperature for 3 h. 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 17** (70 mg, yield 69%).

LCMS m/z = 562.2 [M+H]⁺.

¹H NMR (400 MHz, DMSO-*d*₆) δ 9.08 (d, 1H), 8.71 (s, 1H), 8.54 (d, 1H), 8.03-7.99 (m, 1H), 7.75-7.69 (m, 2H), 7.43-7.37 (m, 2H), 5.84 (s, 2H), 4.93-4.88 (m, 2H), 3.84 (s, 3H), 1.75 (t, 3H), 1.73 -1.66 (m, 1H), 1.15-1.07 (m, 1H), 1.05-0.97 (m, 1H), 0.94 - 0.82 (m, 2H).

### Example 18:

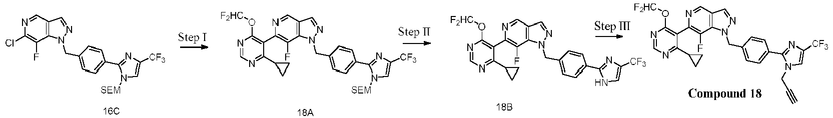

### Step I: Preparation of 18A

**16C** (0.3 g, 0.57 mmol) and **4D** (0.44 g, 1.41 mmol) were dissolved in 1,4-dioxane (4 mL) and water (1 mL), XPhos Pd G2 (0.045 g, 0.057 mmol) and potassium phosphate (0.36 g, 1.70 mmol) were added in sequence, and after displacement with nitrogen three times, the mixture was stirred at 90°C for 3 h. After cooling to room temperature, 20 ml of ethyl acetate and 10 ml of water were added for extraction, the organic layer was dried over anhydrous sodium sulfate and concentrated under reduced pressure, and the residue was purified by column chromatography (ethyl acetate/petroleum ether (V/V) = 1/10-1/1) to obtain **18A** (0.35 g, yield: 91%).

LCMS m/z = 676.80 [M+H]⁺.

### Step II: Preparation of 18B

**18A** (0.35 g, 0.52 mmol) was dissolved in DCM (4 mL), and TFA (4 mL) was added and stirred at room temperature overnight. After concentration under reduced pressure, 20 mL of dichloromethane was added, 10 mL of an aqueous saturated sodium bicarbonate solution was added for alkalization, and after liquid separation, the organic layer was dried over anhydrous sodium sulfate and concentrated under reduced pressure to obtain 18B.

### Step III: Preparation of Compound 18

**18B** (0.070 g, 0.13 mmol) and 3-bromopropyne (0.031 g, 0.26 mmol) were dissolved in DMF (5 mL), and cesium carbonate (0.085 g, 0.26 mmol) was added and stirred at room temperature for 3 h. 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 18** (30 mg, yield 40%).

LCMS m/z = 584.6 [M+H]⁺.

¹H NMR (400 MHz, DMSO-*d*₆) δ 9.13 (d, 1H), 8.84 (s, 1H), 8.57 (d, 1H), 8.04-8.01 (m, 1H), 7.99-7.62 (m, 3H), 7.44-7.38 (m, 2H), 5.91 - 5.80 (m, 2H), 4.97 (d, 2H), 3.55 (t, 1H), 1.88-1.78 (m, 1H), 1.21-1.13 (m, 1H), 1.10 - 0.89 (m, 3H).

### Example 19:

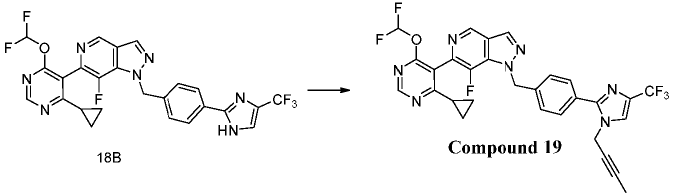

**18B** (0.076 g, 0.14 mmol) and 1-bromo-2-butyne (0.037 g, 0.28 mmol) were dissolved in DMF (5 mL), and cesium carbonate (0.091 g, 0.28 mmol) was added and stirred at room temperature for 3 h. 20 mL of ethyl acetate and 20 mL of water were added for extraction, the organic layer was washed with an aqueous saturated sodium chloride solution (10 mL x 3), dried over anhydrous sodium sulfate and concentrated under reduced pressure, and the residue was purified by column chromatography (ethyl acetate/petroleum ether (V/V) = 1/5-1/1) to obtain **Compound 19** (50 mg, yield 60%).

LCMS m/z = 598.80 [M+H]⁺.

¹H NMR (400 MHz, DMSO-*d*₆) δ 9.13 (d, 1H), 8.85 (s, 1H), 8.57 (d, 1H), 8.02-8.00 (m, 1H), 8.00-7.63 (m, 3H), 7.44-7.38 (m, 2H), 5.91-5.80 (m, 2H), 4.92-4.86 (m, 2H), 1.88 - 1.80 (m, 1H), 1.76 (t, 3H), 1.23 - 1.14 (m, 1H), 1.12 - 0.90 (m, 3H).

### Example 20:

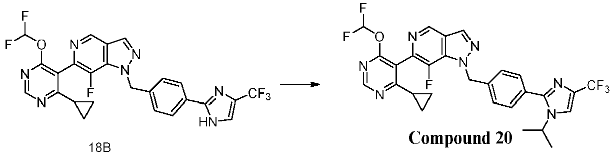

**18B** (0.1 g, 0.18 mmol) and isopropyl iodide (0.15 g, 0.90 mmol) were dissolved in acetonitrile (5 mL), and cesium carbonate (0.29 g, 0.90 mmol) was added and stirred at 60°C for 16 h. After cooling to room temperature, 20 mL of ethyl acetate and 20 mL of water were added for extraction, the organic layer was washed with an aqueous saturated sodium chloride solution (10 mL), dried over anhydrous sodium sulfate and concentrated under reduced pressure, and the residue was purified by column chromatography (ethyl acetate/petroleum ether (V/V) = 1/5-1/1) to obtain **Compound 20** (70 mg, yield 66%).

LCMS m/z = 588.7 [M+H]⁺.

¹H NMR (400 MHz, DMSO-*d*₆) δ 9.13 (d, 1H), 8.84 (s, 1H), 8.58 (d, 1H), 8.17-8.14 (m, 1H), 8.00-7.61 (m, 1H), 7.56-7.50 (m, 2H), 7.41-7.35 (m, 2H), 5.92-8.81 (m, 2H), 4.48-4.36 (m, 1H), 1.88 - 1.80 (m, 1H), 1.40-1.34 (m, 6H), 1.22 - 1.13 (m, 1H), 1.11 - 0.87 (m, 3H).

### Example 21:

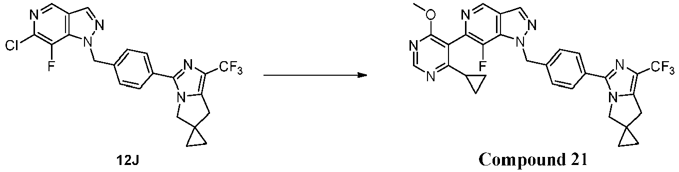

**12J** (100 mg, 0.22 mmol), (4-cyclopropyl-6-methoxypyrimidin-5-yl)boronic acid (64.02 mg, 0.33 mmol), XPhos Pd G2 (17.31 mg, 0.022 mmol) and potassium phosphate (140.10 mg, 0.66 mmol) were placed in a 25 mL single-mouth flask, 1,4-dioxane (4 mL) and water (1 mL) were added, and after displacement with nitrogen three times, the mixture was stirred at 90°C for 3 hours. After cooling to room temperature, 20 mL of water and 20 mL of ethyl acetate were added for extraction, the organic layer was dried over anhydrous sodium sulfate and then concentrated under reduced pressure, the residue was purified by silica gel column chromatography (ethyl acetate/petroleum ether (V/V) = 0/1-2/1), and the resulting crude product was further subjected to separation and purification by preparative HPLC (instrument: waters 2767 preparative liquid chromatographic instrument; chromatographic column: SunFire @ Prep C18 (19 mm × 150mm); composition of mobile phase: mobile phase A: acetonitrile, mobile phase B: water (containing 5 mM ammonium acetate)) to obtain **Compound 21** (35 mg, yield: 27%).

LCMS m/z = 576.3 [M+H]⁺.

¹H NMR (400 MHz, DMSO-*d*₆) δ 9.06 (d, 1H), 8.71 (s, 1H), 8.53 (d, 1H), 7.81-7.75 (m, 2H), 7.37-7.31 (m, 2H), 5.80 (s, 2H), 4.25 (s, 2H), 3.83 (s, 3H), 2.97-2.93 (m, 2H), 1.74 - 1.62 (m, 1H), 1.14-1.06 (m, 1H), 1.04-0.96 (m, 1H), 0.94 - 0.80 (m, 2H), 0.78 (s, 4H).

### Example 22:

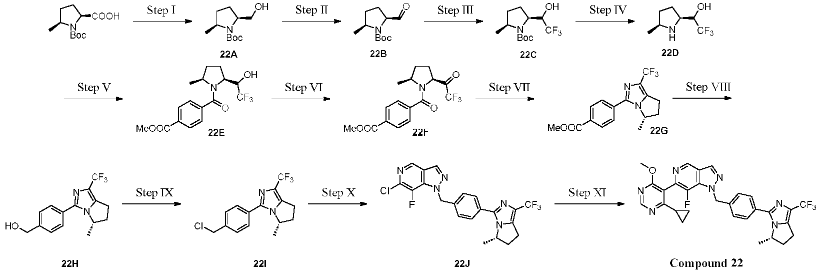

### Step I: Preparation of22A

(2S,5S)-N-Boc-5-methylpyrrolidine-2-carboxylic acid (3 g, 13.09 mmol) was dissolved in THF (30 mL), and a borane tetrahydrofuran solution (19.63 mL, 1M) was dropwise added slowly at 0°C and stirred at room temperature for 2 h. Methanol was added at 0°C for quenching, the mixture was concentrated under reduced pressure, ethyl acetate and water were added to the residue for extraction, and the organic phase was washed with an aqueous saturated sodium chloride solution, dried over anhydrous sodium sulfate, and concentrated under reduced pressure to obtain **22A** (2.8 g).

### Step II: Preparation of 22B

**22A** (2.8 g, 13.01 mmol) was dissolved in DMSO (15 mL), and 2-iodoxybenzoic acid (4.01 g, 14.31 mmol) was added and stirred at 40°C for 2 h. 60 mL of ethyl acetate was added and stirred at room temperature for 10 min. After filtration, the filter cake was washed with ethyl acetate (3 × 10 ml), 60 mL of an aqueous saturated sodium thiosulfate solution was added to the filtrate and then stirred for 10 min, 30 mL of a saturated sodium bicarbonate solution was added for extraction, the organic phase was washed with an aqueous saturated sodium chloride solution three times, dried over anhydrous sodium sulfate, dried over anhydrous sodium sulfate, and concentrated under reduced pressure, and the residue was purified by silica gel column chromatography (ethyl acetate/petroleum ether (V/V) = 0/1-1/5) to obtain **22B** (2 g, yield: 72%).

### Step III: Preparation of 22C

At 0°C, (trifluoromethyl)trimethylsilane (2 g, 14.07 mmol) was added to **22B** (2 g, 9.38 mmol) in tetrahydrofuran (20 mL) and stirred at 0°C for 10 min, tetrabutylammonium fluoride (1.97 mL, 1.97 mmol, 1M solution in THF) was added and stirred at room temperature for 2 h, an aqueous saturated ammonium chloride solution(30 mL) was added for quenching, ethyl acetate (50 mL × 3) was added for extraction, the organic phases were combined, dried over anhydrous sodium sulfate and then concentrated under reduced pressure, and the residue was purified by silica gel column chromatography (ethyl acetate/petroleum ether (V/V) = 1/100-1/5) to obtain **22C** (2.46 g, yield: 92%).

LCMS m/z = 228.1 [M-55]⁺.

### Step IV: Preparation of 22D hydrochloride

**22C** (2.46 g, 8.68 mmol) was dissolved in a hydrochloric acid-dioxane solution (20 mL, 4 M) and stirred at room temperature for 2 hours. Concentration under reduced pressure afforded Compound **22D** hydrochloride (1.8 g).

### Step V: Preparation of 22E

HATU (5.61 g, 14.75 mmol) was added to **22D hydrochloride** (1.8 g) and 4-(methoxycarbonyl)benzoic acid (1.86 g, 10.32 mmol) in DMF (20 mL) and stirred at room temperature for 5 min, DIPEA (3.81 g, 29.49 mmol) was then added, and 100 mL of ethyl acetate and 100 mL of water were added. After layering, the organic layer was dried under reduced pressure, and the residue was purified by silica gel column chromatography (ethyl acetate/petroleum ether (V/V) = 1/100-1/5) to obtain **22E** (3 g, yield 88%).

LCMS m/z = 346.1 [M+H]⁺.

### Step VI: Preparation of 22F

**22E** (2 g, 5.79 mmol) was dissolved in dichloromethane (30 mL), and Dess-Martin periodinane (3.68 g, 8.69 mmol) was added at 0°C in portions, and reacted at room temperature for 3 h. After direct concentration under reduced pressure, the residue was purified by silica gel column chromatography (ethyl acetate/petroleum ether (V/V) = 1/100-1/5) to obtain **22F** (1.59 g, yield: 80%).

### Step VII: Preparation of 22G

Ammonium trifluoroacetate (11 g, 83.94 mmol) was added to **22F** (1.59 g, 4.63 mmol) and reacted at 130°C for 2 h. After cooling to room temperature, 50 mL of water and ethyl acetate (50 mL × 3) were added for extraction, the organic phases were combined, dried over anhydrous sodium sulfate and then concentrated under reduced pressure, and the residue was purified by silica gel column chromatography (ethyl acetate/petroleum ether (V/V) = 1/100-1/2) to obtain **22G** (1.3 g, yield: 86%).

LCMS m/z = 325.1 [M+H]⁺.

### Step VIII: Preparation of 22H

At 0°C, diisobutylaluminium hydride (10.02 mL, 10.02 mmol, 1.0 M solution in hexanes) was dropwise added to **22G** (1.3 g, 4.01mmol) in tetrahydrofuran solution (20 mL), and the mixture was naturally heated to room temperature and reacted for 3 hours. 60 mL of water was added, ethyl acetate (60 mL × 3) was added for extraction, the organic phases were combined, dried over anhydrous sodium sulfate, and concentrated under reduced pressure, and the residue was purified by silica gel column chromatography (ethyl acetate/petroleum ether (V/V) = 1/100-1/2) to obtain **22H** (1.1 g, yield: 92%).

LCMS m/z = 297.2 [M+H]⁺.

### Step IX: Preparation of 22I

**22H** (1.1 g, 3.71 mmol) was dissolved in 20 mL of dichloromethane, and thionyl chloride (2.21 g, 18.55 mmol) was dropwise added slowly in an ice-water bath and reacted at room temperature for 2 h. A aqueous saturated sodium bicarbonate solution (30 mL) was added for quenching, dichloromethane (30 mL × 3) was added for extraction, and the organic phases were combined, dried over anhydrous sodium sulfate, and concentrated under reduced pressure to obtain **22I** (1 g).

LCMS m/z = 315.1 [M+H]⁺.

### Step X: Preparation of 22J

Cesium carbonate (1554.16 mg, 4.77 mmol) was added to a solution of **22I** (500 mg, 1.59 mmol) and **1F** (286.42 mg, 1.67 mmol) in DMF (10 mL) and reacted at room temperature for 3 h. After extraction by adding water and ethyl acetate, the organic phase was washed with aqueous saturated sodium chloride solution, dried over anhydrous sodium sulfate, and concentrated under reduced pressure, and the residue was purified by silica gel column chromatography (ethyl acetate/petroleum ether (V/V) = 0/1-2/1) to obtain Compound **22J** (310 mg, yield: 43%).

LCMS m/z = 450.1 [M+H]⁺.

### Step XI: Preparation of Compound 22

**22J** (150 mg, 0.33 mmol) was dissolved in a mixed solvent of 1,4-dioxane (4 mL) and water (1 mL), (4-cyclopropyl-6-methoxypyrimidin-5-yl)boronic acid (96.03 mg, 0.49 mmol), potassium phosphate (210.15 mg, 0.99 mmol) and XPhos G2 (CAS: 1310584-14-5, 25.96 mg, 0.033 mmol) were added in sequence, and the mixture was heated to 90°C under nitrogen protection and reacted for 3 h. After cooling to room temperature, 20 mL of ethyl acetate and 20 mL of water were added, and after layering, the organic layer was dried over anhydrous sodium sulfate, and concentrated under reduced pressure. The residue was purified by silica gel column chromatography (ethyl acetate/petroleum ether (V/V) = 1/5-2/1) to obtain **Compound 22** (100 mg, yield: 53%).

LCMS m/z = 564.60 [M+H]⁺.

¹H NMR (400 MHz, DMSO-*d*₆) δ 9.07 (d, 1H), 8.71 (s, 1H), 8.54 (d, 1H), 7.80 (d, 2H), 7.34 (d, 2H), 5.81 (s, 2H), 5.07 - 4.93 (m, 1H), 3.86 - 3.79 (m, 3H), 3.09 - 2.95 (m, 1H), 2.94 - 2.79 (m, 2H), 2.31 - 2.20 (m, 1H), 1.73 - 1.62 (m, 1H), 1.19 - 1.14 (m, 3H), 1.14 - 1.06 (m, 1H), 1.03 - 0.95 (m, 1H), 0.95-0.78 (m, 2H).

### Example 23:

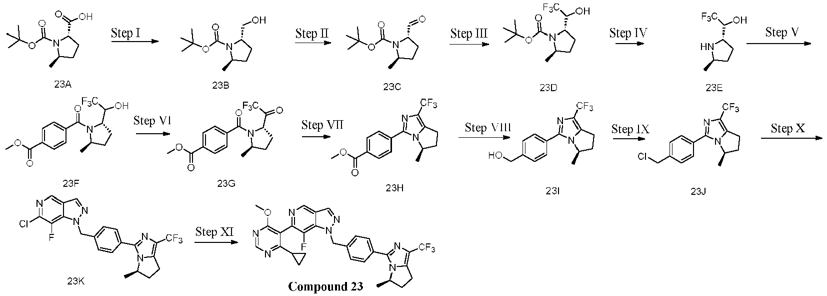

### Step I: Preparation of 23B

**23A** (CAS No.: 160033-52-3, 2 g, 8.73 mmol) was dissolved in THF (10 mL), and a borane tetrahydrofuran solution (17 mL, 17 mmol, 1*N*) was dropwise added in an ice bath and stirred at room temperature for 120 min. 10 mL of methanol was dropwise added to quench the reaction, the reaction product was concentrated under reduced pressure, the residue was dissolved by adding 50 mL of ethyl acetate and washed with 50 mL of a saturated sodium bicarbonate solution and 50 mL of an aqueous saturated sodium chloride solution in sequence, and the organic layer was dried over anhydrous sodium sulfate and concentrated under reduced pressure to obtain **23B.**

LCMS m/z = 160.1 [M-55]⁺.

### Step II: Preparation of 23C

**23B** (1.88 g, 8.73 mmol) was dissolved in DMSO (10 mL), and 2-iodoxybenzoic acid (2.93 g, 10.48 mmol) was added and stirred at room temperature for 30 min and at 40°C for 30 min. After cooling to room temperature, 20 mL of ethyl acetate was added. After filtration, 20 mL of an aqueous saturated sodium thiosulfate solution was added to the filtrate and stirred for 20 min, 10 mL of a saturated sodium bicarbonate solution was then added and stirred for 10 min, and after liquid separation, the organic layer was dried over anhydrous sodium sulfate and then concentrated under reduced pressure to obtain **23C.**

### Step III: Preparation of 23D

**23C** (1.88 g, 8.82 mmol) was dissolved in THF (20 mL), (trifluoromethyl)trimethylsilane (1.88 g, 13.23 mmol) was added, and tetrabutylammonium fluoride (0.69 g, 2.65 mmol, 1*N*) in tetrahydrofuran solution (2.65 mL) was dropwise added slowly in an ice bath and stirred at room temperature for 2 h. A aqueous saturated ammonium chloride solution (10 mL) was added for quenching, ethyl acetate (20 mL × 2) was added for extraction, the organic phases were combined, dried over anhydrous sodium sulfate and then concentrated under reduced pressure, and the residue was purified by silica gel column chromatography (ethyl acetate/petroleum ether (V/V) = 1/10-1/2) to obtain **23D** (2 g, yield: 80%).

LCMS m/z = 228.1 [M-55]⁺.

### Step IV: Preparation of 23E hydrochloride

**23D** (2 g, 7.06 mmol) was dissolved in DCM (5 mL), and a hydrochloric acid-dioxane solution (10 mL, 4 M) was added and stirred at room temperature for 2 h. After concentration under reduced pressure, 20 mL of methyl tert-butyl ether was added and stirred for 10 min, and the mixture was then filtered to obtain **23E** hydrochloride (1.16 g, yield: 75%).

### Step V: Preparation of 23F

HATU (2.42 g, 6.36 mmol) was added to a solution of **23E** hydrochloride (1.16 g, 5.30 mmol) and monomethyl terephthalate (0.95 g, 5.30 mmol) in DMF (15 mL) and stirred at room temperature for 5 min, and DIPEA (1.37 g, 10.60 mmol) was added and stirred for 20 min. 30 mL of ethyl acetate was added, the mixture was washed with an aqueous saturated sodium chloride solution (20 mL × 3), the organic layers were concentrated under reduced pressure, and the residue was purified by silica gel column chromatography (ethyl acetate/petroleum ether (V/V) = 1/10-1/2) to obtain **23F** (1.8 g, yield: 98%).

LCMS m/z = 346.1 [M+H]⁺.

### Step VI: Preparation of 23G

23F (1.8 g, 5.21 mmol) was dissolved in DCM (20 mL), and Dess-Martin periodinane (2.65 g, 6.25 mmol) was added at 0°C in portions and reacted at room temperature for 3 hours. 20 mL of an aqueous saturated sodium thiosulfate solution was added and stirred for 20 min, and 20 mL of a saturated sodium bicarbonate solution was then added and stirred for 10 min. After liquid separation, the organic layer was dried over anhydrous sodium sulfate and concentrated under reduced pressure, and the residue was purified by silica gel column chromatography (ethyl acetate/petroleum ether (V/V) = 1/100-1/2) to obtain 23G (1.5 g, yield: 84%).

### Step VII: Preparation of 23H

Ammonium trifluoroacetate (8.59 g, 65.55 mmol) was added to **23G** (1.5 g, 4.37 mmol) and reacted at 130°C for 1.5 h. After cooling to room temperature, 30 mL of water and ethyl acetate (20 mL × 2) were added for extraction, the organic phases were combined, dried over anhydrous sodium sulfate and then concentrated under reduced pressure, and the residue was purified by silica gel column chromatography (ethyl acetate/petroleum ether (V/V) = 1/100-1/2) to obtain **23H** (1 g, yield: 70%).

LCMS m/z = 325.3 [M+H]⁺.

### Step VIII: Preparation of 23I

At 0°C, diisobutylaluminium hydride (7.7 mL, 7.7 mmol, 1N) in THF solution was dropwise added to a solution of **23H** (1 g, 3.08 mmol) in THF (10 mL) and reacted at room temperature for 3 hours. 10 mL of an aqueous saturated ammonium chloride solution was added to quench the reaction, and 30 mL of water and 60 mL of ethyl acetate were added for extraction. After leaving to stand for 20 min, a solid precipitated out, and after filtration, the filtrate was subjected to liquid separation. The organic phase was washed once with 20 mL of an aqueous saturated sodium chloride solution, then dried over anhydrous sodium sulfate, and concentrated under reduced pressure, and the residue was purified by silica gel column chromatography (ethyl acetate/petroleum ether (V/V) = 1/100-1/2) to obtain **23I** (0.54 g, yield: 59%).

LCMS m/z = 297.3 [M+H]⁺.

### Step IX: Preparation of 23J

**23I** (0.54 g, 1.82 mmol) was dissolved in DCM (4 mL), and thionyl chloride (1.08 g, 9.1 mmol) was dropwise added slowly in an ice-water bath and stirred at room temperature for 2 h. After concentration under reduced pressure, an aqueous saturated sodium bicarbonate solution (10 mL) was added to the residue for alkalization, dichloromethane (30 mL) was added for extraction, and the organic phase was dried over anhydrous sodium sulfate and concentrated under reduced pressure to obtain **23J.**

### Step X: Preparation of 23K

Compound **1F** (0.11 g, 0.64 mmol) and **23J** (0.24 g, 0.76 mmol) were dissolved in DMF (5 mL), and cesium carbonate (0.42 g, 1.28 mmol) was added at room temperature and stirred at 40°C for 1 h. After cooling to room temperature, 20 mL of ethyl acetate and 20 mL of water were added for extraction, the organic layer was washed with an aqueous saturated sodium chloride solution (10 mL x 3), dried over anhydrous sodium sulfate, concentrated under reduced pressure, and purified by column chromatography (ethyl acetate/petroleum ether (V/V) = 1/5-1/2) to obtain Compound **23K** (0.14 g, yield: 48%).

LCMS m/z = 450.3 [M+H]⁺.

### Step XI: Preparation of Compound 23

Compound **23K** and (4-cyclopropyl-6-methoxypyrimidin-5-yl)boronic acid (0.12 g, 0.62 mmol) were dissolved in 1,4-dioxane (4 mL) and water (1 mL), and XPhos Pd G2 (0.024 g, 0.031 mmol) and potassium phosphate (0.20 g, 0.93 mmol) were added in sequence. After the addition was complete, displacement with nitrogen was performed three times, and the mixture was heated to 95°C and stirred for 3 h. After cooling to room temperature, 20 ml of ethyl acetate and 10 ml of water were added for extraction, the organic layer was dried over anhydrous sodium sulfate and concentrated under reduced pressure, and the residue was purified by column chromatography (ethyl acetate/petroleum ether (V/V) = 1/10-1/1) to obtain **Compound 23** (0.14 g, yield: 80%).

LCMS m/z = 564.5 [M+H]⁺.

¹H NMR (400 MHz, DMSO-*d*₆) δ 9.07 (d, 1H), 8.71 (s, 1H), 8.54 (d, 1H), 7.83-7.76 (m, 2H), 7.37-7.29 (m, 2H), 5.81 (s, 2H), 5.07 - 4.95 (m, 1H), 3.83 (d, 3H), 3.07-2.94 (m, 1H), 2.92-2.80 (m, 2H), 2.31 - 2.21 (m, 1H), 1.72-1.62 (m, 1H), 1.18 - 1.13 (m, 3H), 1.13-1.04 (m, 1H), 1.03 - 0.95 (m, 1H), 0.94 - 0.77 (m, 2H).

### Biological test examples

### Test example 1: Inhibitory activity on USP1/UAF1 enzymology

Ubiquitin rhodamine 110 was used as a substrate to detect the activity of the deubiquitinating enzyme USP1/UAF1 after drug treatment. The total test system was 20 µl, and the test buffer comprises 50 mM Tris-HCl (pH 7.8), 0.5 mM EDTA, 0.01% Bovine Serum Albumin, 1 mM DTT, and 0.01% Tween-20. At the beginning of the experiment, 0.5 nM USP1/UAF1 (Bio-Techne, E-568-050) protein was added to a 384-well plate (PerkinElmer, 6008269), and the test compound diluted in a gradient was then added. After incubation at room temperature for 10 min, 500 nM ubiquitin rhodamine 110 (Bio-Techne, U-555-050) was added, and the mixture was incubated at room temperature for 20 min. Enspire^{™} Multilabel Reader microplate reader (PerkinElmer) was used for detection at excitation light of 485 nm/emission light of 535 nm. The IC₅₀ value was calculated using GraphPad Prism software.

**Table 1. Results of inhibitory activity of test compounds on USP 1/UAF 1 enzymology**

| Compound No. | IC₅₀ (nM) | Compound No. | IC₅₀ (nM) |
|---|---|---|---|
| Compound 1 | A | Compound 14 | A |
| Compound 2 | A | Compound 15 | A |
| Compound 3 trifluoroacetate | A | Compound 16 | A |
| Compound 4 | A | Compound 18 | A |
| Compound 8 | A | Compound 19 | A |
| Compound 10 | A | Compound 20 | A |
| Compound 12 | A | Compound 21 | A |

| | | | |
|---|---|---|---|
| Note: A < 20 nM | | | |

Conclusion: The compounds of the present invention have a good inhibitory effect on the enzymatic activity of USP1/UAF1.

### Test example 2: Pharmacokinetic test in beagle dogs

**Experimental objective:** a single dose of test compounds was administered to beagle dogs intravenously and intragastrically, and the concentrations of the test compounds in plasma of the beagle dogs were measured to evaluate pharmacokinetic characteristics of the test compounds in the beagle dogs.

**Experimental animals:** male beagle dogs, about 8-11 kg, were purchased from Beijing Marshall Biotechnology Co., Ltd.

**Experimental method:** On the day of the experiment, the beagle dogs were randomly grouped according to their body weights. The animals were fasted but with water available for 14 to 18 hours one day before administration, and were fed 4 hours after administration. The administration was performed based on the information in the following table.

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound of the present invention | 1 | 1 | 1 | Plasma | Intravenous administration |
| G2 | 3 | Compound of the present invention | *5* | 1 | *5* | Plasma | Intragastric administration |

Vehicle for intravenous administration: 5% DMA + 5% Solutol + 90% Saline; Vehicle for intragastric administration: 0.5% MC

(DMA: dimethylacetamide; Solutol: polyethylene glycol-15-hydroxystearate; Saline: physiological saline; MC: Methylcellulose solution)

**Sampling:** Before and after the administration, blood was taken from the jugular veins or limb veins, and placed in an EDTAK2 centrifuge tube. Centrifugation was performed at 5000 rpm at 4°C for 10 min, and plasma was collected.

Time points for blood collection: 0, 5 min, 15 min, 30 min, 1 h, 2 h, 4 h, 6 h, 8 h, 10 h, 12 h, 24 h, 48 h, and 72 h. Before analysis and detection, all the samples were stored at -60°C or lower. The samples were analyzed quantitatively by LC-MS/MS.

**Table 2. Pharmacokinetic parameters of test compounds in plasma of beagle dogs**

| Test compound | Mode of administration | AUC₀₋ₜ (h.ng. mL⁻¹) | F (%) |
|---|---|---|---|
| Compound 2 | i.g. (5 mg/kg) | 6302 | 41.2 |
| Control compound A | i.g. (5 mg/kg) | 994 | 15.8 |

| | | | |
|---|---|---|---|
| * Note: i.g. (intragastrically) administration of compounds. | | | |

Conclusion: the compound synthesized by using the technique of the present invention has a good oral absorption performance in beagle dogs, and the oral performance is superior to that of control compound A, wherein the control compound A is 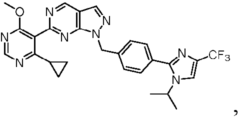 and the preparation method thereof may be found in WO 2020132269.

### Test example 3: Clone formation test of MDA-MB-436 cells

In this experiment, MDA-MB-436 cells were treated by adding a test compound to detect the ability of cell clone formation. At the beginning of the experiment, MDA-MB-436 cells (ATCC, HTB-130) were added to a 96-well plate (Corning, 3599), with 500 cells per well, and they were subjected to adherent culture overnight. The next day, the compound diluted in a gradient was added, and culture was continued in a CO₂-free incubator at 37°C. On the 7th day, the old culture medium was discarded, and the test compound diluted in a gradient was added again. At the 14th day, the 96-well plate was taken out, the culture liquid was discarded, and cell clones were detected by Crystal violet Assay kit. PHERAstar FSX microplate reader (BMG LABTECH) was used for detection of OD at 570 nm. The IC₅₀ value was calculated using GraphPad Prism software.

**Table 3. Results of inhibitory activity of test compounds on MDA-MB-436 cell clones**

| Compound No. | IC₅₀ (nM) |
|---|---|
| Compound 1 | A |
| Compound 2 | A |
| Compound 3 trifluoroacetate | A |
| Compound 4 | A |
| Compound 10 | A |
| Compound 15 | A |
| Compound 16 | A |
| Compound 20 | A |
| Compound 22 | A |

| | |
|---|---|
| Note: A < 200 nM | |

Conclusion: The compounds of the present invention have a good inhibitory effect on MDA-MB-436 cell clone formation.

### Test example 4: Pharmacokinetic test in mice

**Experimental animals:** Male ICR mice, 25-30 g, were purchased from Chengdu Ddossy Experimental Animals Co., Ltd.

**Experimental design:** On the day of the experiment, the ICR mice were randomly grouped according to their body weights. The animals were fasted with water available for 12 to 14 h one day before the administration, and were fed 4 h after the administration.

| Group | Administration information | | | | | | |
|---|---|---|---|---|---|---|---|
| | Test compound | Administration dosage * (mg/kg) | Administration concentration (mg/mL) | Administration volume (mL/kg) | Collected sample | Mode of administration | Vehicle |
| G1 | Compound of the present invention | 2.5 | 0.5 | *5* | Plasma | Intravenous administration | 5% DMA *+* 5% Solutol + 90% Saline |
| G2 | Compound of the present invention | 10 | 1 | 10 | Plasma | Oral (Intragastric administration) | 0.5% MC |

| | | | | | | | |
|---|---|---|---|---|---|---|---|
| (DMA: dimethylacetamide; Solutol: polyethylene glycol-15-hydroxystearate; Saline: physiological saline; MC: methylcellulose) | | | | | | | |

Before and after the administration, blood was taken from the orbit of the animals at specified time points and placed in an EDTAK2 centrifuge tube. The blood was centrifuged at 5000 rpm and 4°C for 10 min to collect plasma. The blood collection time points for the intravenous group and intragastric administration group were: 0, 5 min, 15 min, 30 min, 1 h, 2 h, 4 h, 7 h, and 24 h. Before analysis and detection, all samples were stored below -60°C. The samples were analyzed quantitatively by LC-MS/MS.

**Table 4. Pharmacokinetic parameters of test compounds in plasma of mice**

| Test compound | Mode of administration | Oral absorption |
|---|---|---|
| Compound 1 | i.g. (10 mg/kg) | Yes |
| Compound 2 | i.g. (10 mg/kg) | Yes |
| Compound 8 | i.g. (10 mg/kg) | Yes |
| Compound 12 | i.g. (10 mg/kg) | Yes |
| Compound 14 | i.g. (10 mg/kg) | Yes |
| Compound 16 | i.g. (10 mg/kg) | Yes |
| Compound 21 | i.g. (10 mg/kg) | Yes |
| Compound 22 | i.g. (10 mg/kg) | Yes |

| | | |
|---|---|---|
| * Note: i.g. (intragastrically) administration of compounds. | | |

Conclusion: The compounds synthesized by using the technique of the present invention have a good oral absorption performance in mice.

## Claims

1. A compound or a stereoisomer, tautomer, deuterated substance, solvate, pharmaceutically acceptable salt or eutectic crystal thereof, **characterized in that** the compound is selected from a compound shown in general formula (I), 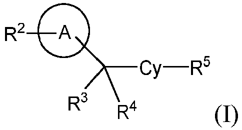
wherein ring A is selected from 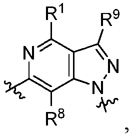
and the left side is connected to R²;
R¹ and R⁹ are each independently selected from H, halogen, cyano, C₁₋₆ alkyl, C₂₋₆ alkenyl, C₂₋₆ alkynyl, and C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, or cyano-substituted C₁₋₆ alkyl;
R⁸ is selected from halogen, cyano, C₁₋₆ alkyl, C₂₋₆ alkenyl, C₂₋₆ alkynyl, and C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, or cyano-substituted C₁₋₆ alkyl;
R² is selected from C₆₋₁₀ carbocycle or 5- to 10-membered heterocycle, wherein the carbocycle or heterocycle is optionally further substituted with 0 to 4 R^{2a};
each R^{2a} is independently selected from H, halogen, OH, =O, cyano, NH₂, NH(C₁₋₆ alkyl), N(C₁₋₆ alkyl)₂, -SO₂-C₁₋₆ alkyl, -SO₂-C₃₋₆ cycloalkyl, C₁₋₆ alkyl, C₁₋₆ alkoxy, C₁₋₆ alkylthio, -Z-C₃₋₆ cycloalkyl, or -Z-3- to 8-membered heterocycle, wherein the alkyl, cycloalkyl, alkoxy, alkylthio, or heterocycle is optionally further substituted with 0 to 4 substituents selected from H, D, halogen, OH, =O, cyano, NH₂, C₁₋₆ alkyl, C₂₋₆ alkynyl, C₁₋₆ alkoxy, or C₃₋₆ cycloalkyl, and the heterocycle contains 1 to 4 heteroatoms selected from O, S, and N;
R³ and R⁴ are each independently selected from H, halogen, OH, cyano, NH₂, C₁₋₆ alkyl, C₂₋₆ alkenyl, C₂₋₆ alkynyl, or C₁₋₆ alkoxy, wherein the alkyl, alkenyl, alkynyl, or alkoxy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, or 3- to 8-membered heterocyclyl, and the heterocyclyl contains 1 to 4 heteroatoms selected from O, S, and N;
Cy is selected from C₃₋₁₂ carbocycle or 4- to 12-membered heterocycle, and the Cy is optionally further substituted with 0 to 4 substituents selected from H, halogen, OH, =O, cyano, NH₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, or 3- to 8-membered heterocyclyl, wherein the heterocycle contains 1 to 4 heteroatoms selected from O, S, and N;
R⁵ is selected from -Z-C₃₋₁₂ carbocycle or -Z-4- to 12-membered heterocycle, and the R⁵ is optionally further substituted with 0 to 4 R^{5a};
each R^{5a} is independently selected from H, halogen, OH, cyano, NH₂, NHC₁₋₆ alkyl, N(C₁₋₆ alkyl)₂, C₁₋₆ alkyl, C₂₋₆ alkynyl, C₁₋₆ alkoxy, -C₁₋₄ alkylene-C₃₋₆ cycloalkyl, -C₁₋₄ alkylene-3- to 6-membered heterocycle, C₃₋₆ cycloalkyl, 3- to 8-membered heterocycle, -CONHC₁₋₆ alkyl, -CONHC₁₋₆ alkoxy, -CONHC₃₋₆ cycloalkyl, -CONH 3- to 8-membered heterocycle, -NHCO-C₁₋₆ alkyl, -NHCO-C₁₋₆ alkoxy, -NHCO-C₃₋₆ cycloalkyl, or -NHCO-3- to 8-membered heterocycle, wherein the alkyl, alkylene, alkynyl, alkoxy, cycloalkyl, or heterocycle is optionally further substituted with 0 to 4 substituents selected from H, D, halogen, OH, =O, cyano, NH₂, -C(=O)NH₂, -C(=O)NHC₁₋₆ alkyl, -C(=O)N(C₁₋₆ alkyl)₂, C₁₋₆ alkyl, C₂₋₆ alkynyl, C₁₋₆ alkoxy, halogen-substituted C₁₋₆ alkyl, hydroxyl-substituted C₁₋₆ alkyl, cyano-substituted C₁₋₆ alkyl, C₃₋₆ cycloalkyl, halogen-substituted C₃₋₆ cycloalkyl, or 3- to 8-membered heterocycle, and the heterocycle contains 1 to 4 heteroatoms selected from O, S, and N;
Z is selected from a bond, O, S, NH, N(C₁₋₄ alkyl), C(=O)NH, NHC(=O), - OCH₂-, -CH₂O-, or C₁₋₃ alkylene.

2. The compound or the stereoisomer, tautomer, deuterated substance, solvate, pharmaceutically acceptable salt or eutectic crystal thereof according to claim 1, **characterized in that** the compound shown in general formula (I) is selected from a compound shown in general formula (Ia), 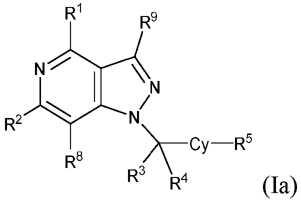
R¹ and R⁹ are each independently selected from H, halogen, cyano, C₁₋₄ alkyl, C₂₋₄ alkenyl, C₂₋₄ alkynyl, and C₁₋₄ alkoxy, wherein the alkyl, alkenyl, alkynyl, or alkoxy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, or cyano-substituted C₁₋₄ alkyl;
R⁸ is selected from halogen, cyano, C₁₋₄ alkyl, C₂₋₄ alkynyl, and C₁₋₄ alkoxy, wherein the alkyl, alkynyl, or alkoxy is optionally further substituted with 0 to 4 substituents selected from H, halogen, OH, cyano, NH₂, C₁₋₄ alkyl, C₂₋₄ alkynyl, and C₁₋₄ alkoxy;
each R^{2a} is independently selected from H, halogen, OH, =O, cyano, NH₂, NH(C₁₋₄ alkyl), N(C₁₋₄ alkyl)₂, -SO₂-C₁₋₄ alkyl, -SO₂-C₃₋₆ cycloalkyl, C₁₋₄ alkyl, C₁₋₄ alkoxy, C₁₋₄ alkylthio, -Z-C₃₋₆ cycloalkyl, or -Z-3- to 6-membered heterocycle, wherein the alkyl, cycloalkyl, alkoxy, alkylthio, or heterocycle is optionally further substituted with 0 to 4 substituents selected from H, D, halogen, OH, =O, cyano, NH₂, C₁₋₄ alkyl, C₂₋₄ alkynyl, C₁₋₄ alkoxy, or C₃₋₆ cycloalkyl, and the heterocycle contains 1 to 4 heteroatoms selected from O, S, and N;
R³ and R⁴ are each independently selected from H, halogen, OH, cyano, and C₁₋₄ alkyl;
Cy is selected from C₃₋₆ monocyclic cycloalkyl, C₅₋₁₁ fused cycloalkyl, C₅₋₁₁ spirocycloalkyl, C₅₋₁₁ bridged cycloalkyl, 4- to 7-membered monocyclic heterocycloalkyl, 6- to 11-membered fused heterocycloalkyl, 6- to 11-membered spiroheterocycloalkyl, 6- to 11-membered bridged heterocycloalkyl, benzene ring, benzo C₄₋₆ carbocycle, benzo 4- to 6-membered heterocycle, 5- to 6-membered heteroaromatic ring, or 8- to 10-membered fused heteroaromatic ring, and the Cy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, or 3- to 8-membered heterocycle, wherein the heterocycle contains 1 to 4 heteroatoms selected from O, S, and N;
R⁵ is selected from -Z-C₆₋₁₂ carbocycle or -Z-5- to 12-membered heterocycle, and the R⁵ is optionally further substituted with 0 to 4 R^{5a};
each R^{5a} is independently selected from H, halogen, OH, cyano, NH₂, C₁₋₄ alkyl, C₂₋₄ alkynyl, C₁₋₄ alkoxy, -C₁₋₂ alkylene-C₃₋₆ cycloalkyl, -C₁₋₂ alkylene-3- to 6-membered heterocycle, C₃₋₆ cycloalkyl, or 3- to 6-membered heterocycle, wherein the alkyl, alkylene, alkynyl, alkoxy, cycloalkyl, or heterocycle is optionally further substituted with 0 to 4 substituents selected from H, D, halogen, OH, =O, cyano, NH₂, -C(=O)NH₂, -C(=O)NHC₁₋₄ alkyl, -C(=O)N(C₁₋₄ alkyl)₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, halogen-substituted C₃₋₆ cycloalkyl, or 3- to 8-membered heterocycle, and the heterocycle contains 1 to 4 heteroatoms selected from O, S, and N.

3. The compound or the stereoisomer, tautomer, deuterated substance, solvate, pharmaceutically acceptable salt or eutectic crystal thereof according to claim 2, **characterized in that**
R¹ and R⁹ are each independently selected from H, F, Cl, Br, I, cyano, methyl, or ethyl;
R⁸ is selected from F, Cl, Br, I, CN, methyl, ethyl, ethynyl, methoxy, or ethoxy;
R² is selected from phenyl, pyridine, pyrimidine, pyrrole, pyrazole, imidazole, furan, thiophene, thiazole, or oxazole, wherein the phenyl, pyridine, pyrimidine, pyrrole, pyrazole, imidazole, furan, thiophene, thiazole, or oxazole is optionally further substituted with 0 to 4 R^{2a};
or R² is selected from 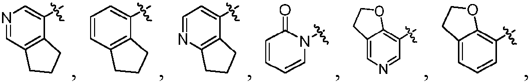 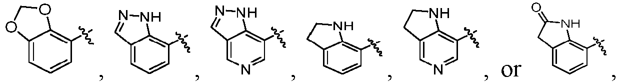 and R² is optionally further substituted with 0 to 4 R^{2a};
each R^{2a} is independently selected from H, F, Cl, Br, I, or cyano, or each R^{2a} is independently selected from one of the following substituted or unsubstituted groups: -SO₂-methyl, -SO₂-ethyl, -SO₂-propyl, -SO₂-isopropyl, -SO₂-cyclopropyl, methyl, ethyl, propyl, isopropyl, methoxy, ethoxy, propoxy, methylthio, ethylthio, propylthio, cyclopropyl, cyclobutyl, cyclopentyl, cyclohexyl, azetidinyl, azacyclopentyl, azacyclohexyl, piperazine, oxetanyl, oxacyclopentyl, oxacyclohexyl, morpholine, -O-azetidinyl, -O-azacyclopentyl, -O-azacyclohexyl, - O-oxetanyl, -O-oxacyclopentyl, -O-oxacyclohexyl, -OCH₂-phenyl, -OCH₂-cyclopropyl, -OCH₂-cyclobutyl, -OCH₂-cyclopentyl, -OCH₂-cyclohexyl, pyrrole, pyrazole, imidazole, thiazole, thiophene, furan, and oxazole, which, when substituted, are optionally further substituted with 0 to 4 substituents selected from H, D, halogen, OH, =O, cyano, NH₂, C₁₋₄ alkyl, C₂₋₄ alkynyl, C₁₋₄ alkoxy, and C₃₋₆ cycloalkyl;
R³ and R⁴ are each independently selected from H, F, Cl, Br, I, methyl, and ethyl;
Cy is selected from cyclopropyl, cyclobutyl, cyclopentyl, cyclohexyl, bicyclo[2.2.2]octyl, bicyclo[1.1.1]pentyl, cyclopentyl-fused cyclopentyl, cyclobutylspirocyclohexyl, cyclobutylspirocyclopentyl, azetidinyl, azacyclopentyl, azacyclohexyl, azetidinylspirocyclobutyl, azetidinylspirocyclopentyl, azetidinylspirocyclohexyl, azetidinylspiroazetidinyl, azetidinylspiroazacyclopentyl, azetidinylspiroazacyclohexyl, 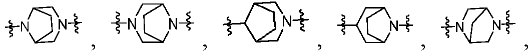 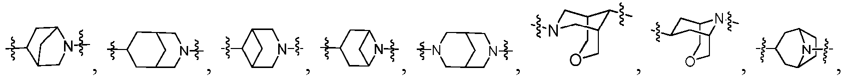 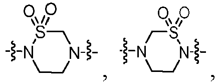 benzene ring, thiophene, furan, pyrrole, thiazole, oxazole, pyrazole, pyrazine, pyrimidine, 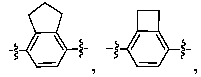 benzothiazole, benzothiophene, benzofuran, benzoxazole, benzopyrrole, benzopyrazole, benzimidazole, indolizine, pyridinopyrrole, pyridinoimidazole, pyridinopyrazole, pyrimidoimidazole, pyrimidopyrazole, pyridinotriazole, or 2-pyridone, and the Cy is optionally further substituted with 0 to 4 substituents selected from H, halogen, OH, =O, cyano, NH₂, C₁₋₄ alkyl, C₂₋₄ alkynyl, C₁₋₄ alkoxy, halogen-substituted C₁₋₄ alkyl, hydroxyl-substituted C₁₋₄ alkyl, cyano-substituted C₁₋₄ alkyl, C₃₋₆ cycloalkyl, or 3- to 8-membered heterocycle, wherein the heterocycle contains 1 to 4 heteroatoms selected from O, S, and N;
R⁵ is selected from benzene ring, pyrazole, pyrrole, imidazole, triazole, pyridine, pyrimidine, pyrazine, or pyridazine, and the R⁵ is optionally further substituted with 0 to 4 R^{5a};
or R⁵ is selected from 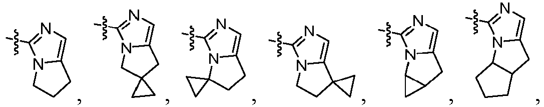 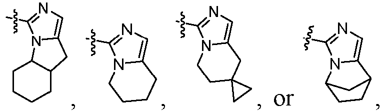 and the R⁵ is optionally further substituted with 0 to 4 R^{5a}
each R^{5a} is independently selected from H, F, Cl, Br, I, cyano, methyl, ethyl, propyl, isopropyl, propargyl, butargyl, methoxy, ethoxy, cyclopropyl, cyclobutyl, cyclopentyl, cyclohexyl, oxetanyl, oxacyclopentyl, oxacyclohexyl, azetidinyl, azacyclopentyl, azacyclohexyl, -CH₂-cyclopropyl, -CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, or -CH₂-azacyclohexyl, wherein the methyl, ethyl, propyl, isopropyl, propargyl, butargyl, methoxy, ethoxy, cyclopropyl, cyclobutyl, cyclopentyl, cyclohexyl, oxetanyl, oxacyclopentyl, oxacyclohexyl, azetidinyl, azacyclopentyl, or azacyclohexyl is optionally further substituted with 0 to 4 substituents selected from H, D, halogen, OH, =O, cyano, NH₂, C₁₋₄ alkyl, C₂₋₄ alkynyl, C₁₋₄ alkoxy, C₃₋₆ cycloalkyl, or halogen-substituted C₃₋₆ cycloalkyl.

4. The compound or the stereoisomer, tautomer, deuterated substance, solvate, pharmaceutically acceptable salt or eutectic crystal thereof according to claim 3, **characterized in that**
R¹ and R⁹ are H;
R⁸ is selected from F, Cl, Br, CN, methyl, ethynyl, or methoxy;
R² is selected from pyrrole, pyrazole, imidazole, pyrimidine, 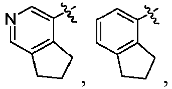 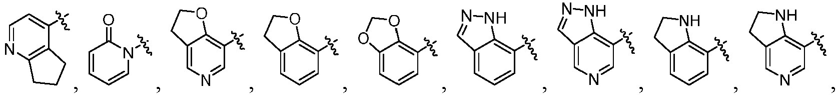 or 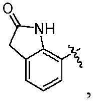 wherein the pyrrole, pyrazole, imidazole, pyrimidine, 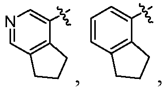 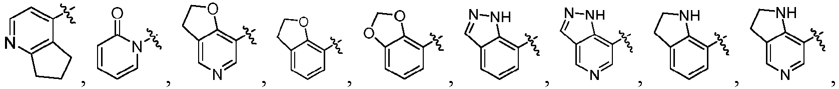 or 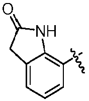 is optionally further substituted with 0 to 4 substituents selected from H, F, Cl, Br, I, cyano, -SO₂-methyl, -SO₂-ethyl, -SO₂-propyl, -SO₂-isopropyl, - SO₂-cyclopropyl, CH₂F, CHF₂, CF₃, -OCH₂F, -OCHF₂, -OCF₃, methyl, ethyl, propyl, isopropyl, methoxy, ethoxy, propoxy, -OCD₃, methylthio, ethylthio, propylthio, cyclopropyl, cyclobutyl, cyclopentyl, cyclohexyl, azetidinyl, azacyclopentyl, azacyclohexyl, piperazine, oxetanyl, oxacyclopentyl, oxacyclohexyl, morpholine, -O-azetidinyl, -O-azacyclopentyl, -O-azacyclohexyl, - O-oxetanyl, -O-oxacyclopentyl, -O-oxacyclohexyl, -OCH₂-phenyl, -OCH₂-cyclopropyl, -OCH₂-cyclobutyl, -OCH₂-cyclopentyl, -OCH₂-cyclohexyl, 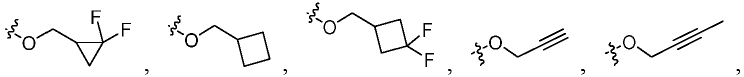 pyrrole, pyrazole, imidazole, thiazole, thiophene, furan, oxazole, 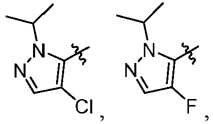 or 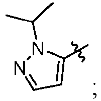
Cy is selected from 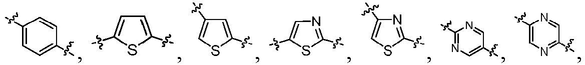 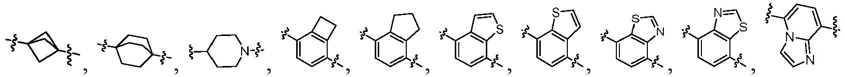 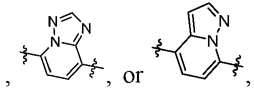 and the Cy is optionally further substituted with 0 to 4 substituents selected from H, F, Cl, Br, I, cyano, CH₂F, CHF₂, CF₃, methyl, ethyl, propyl, isopropyl, methoxy, ethoxy, or cyclopropyl;
alternatively, Cy is selected from 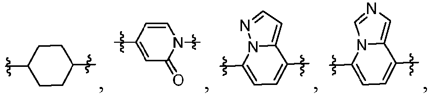 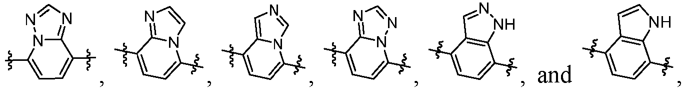 and the Cy is optionally further substituted with 0 to 4 substituents selected from H, F, Cl, Br, I, cyano, CH₂F, CHF₂, CF₃, methyl, ethyl, propyl, isopropyl, methoxy, ethoxy, or cyclopropyl;
R⁵ is selected from pyrrole, pyrazole, or imidazole, and the pyrrole, pyrazole, or imidazole is optionally further substituted with 0 to 4 substituents selected from H, F, Cl, Br, I, cyano, CH₂F, CHF₂, CF₃, CD₃, methyl, ethyl, propyl, isopropyl, - CH₂CH₂F, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, azacyclohexyl, 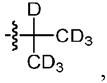 -CH₂-cyclopropyl, -CH₂-cyclobutyl, - CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, -CH₂-azacyclohexyl, 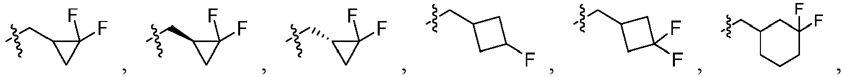 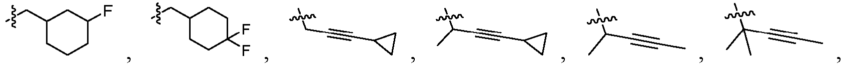 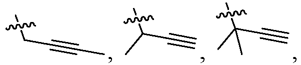 or propargyl;
alternatively, R⁵ is selected from 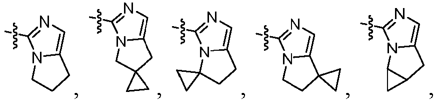 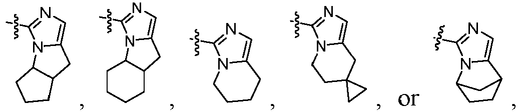 and R⁵ is optionally further substituted with 0 to 4 substituents selected from H, F, Cl, Br, I, cyano, CH₂F, CHF₂, CF₃, CD₃, methyl, ethyl, propyl, isopropyl, -CH₂CH₂F, methoxy, ethoxy, cyclopropyl, cyclobutyl, cyclopentyl, cyclohexyl, oxetanyl, oxacyclopentyl, oxacyclohexyl, azetidinyl, N-methylazetidinyl, azacyclopentyl, azacyclohexyl, 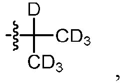 -CH₂-cyclopropyl, -CH₂-cyclobutyl, -CH₂-cyclopentyl, -CH₂-cyclohexyl, -CH₂-oxetanyl, -CH₂-oxacyclopentyl, -CH₂-oxacyclohexyl, -CH₂-azetidinyl, -CH₂-azacyclopentyl, -CH₂-azacyclohexyl, 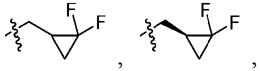 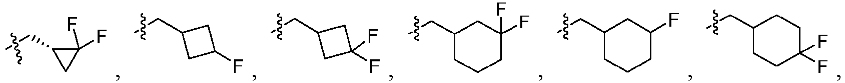 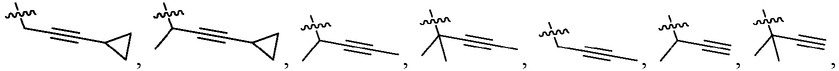 or propargyl.

5. The compound or the stereoisomer, tautomer, deuterated substance, solvate, pharmaceutically acceptable salt or eutectic crystal thereof according to claim 4, **characterized in that**
R² is selected from 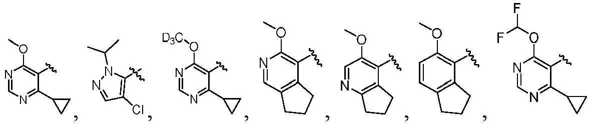 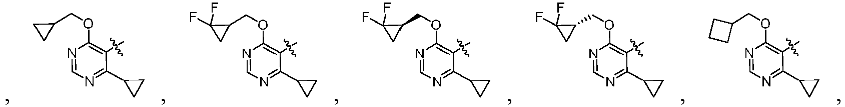 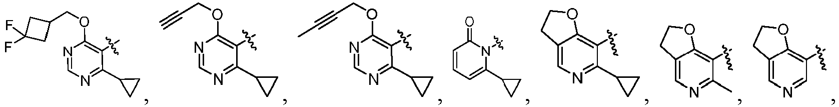 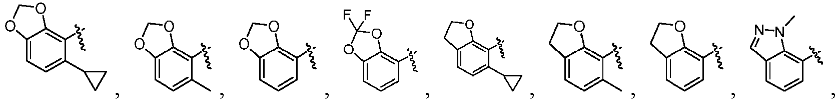 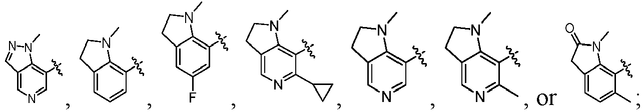
R⁵ is selected from 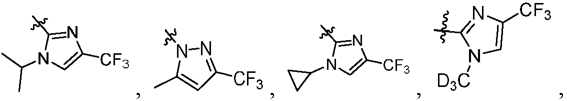 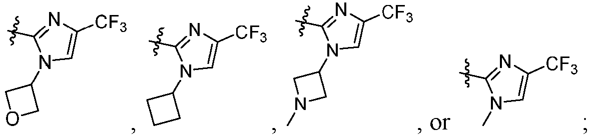
alternatively, R⁵ is selected from 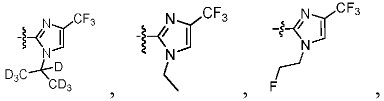 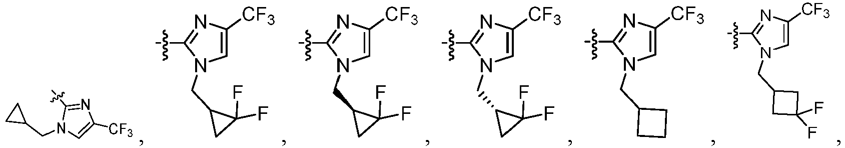 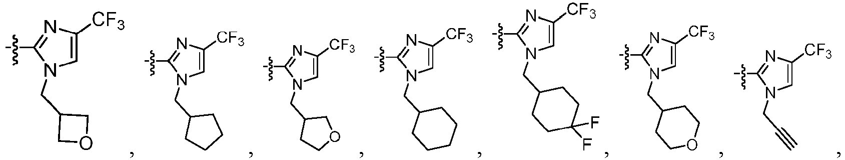 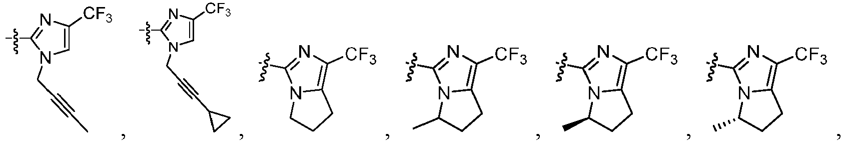 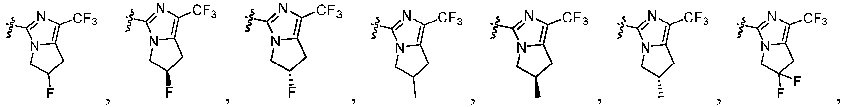 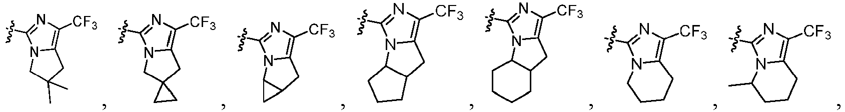 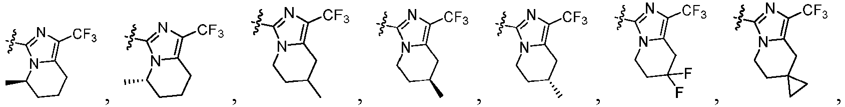 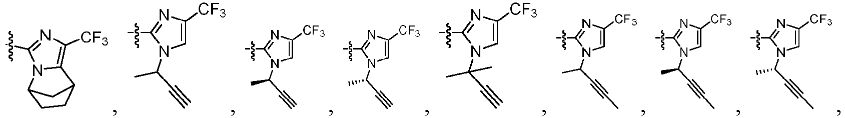 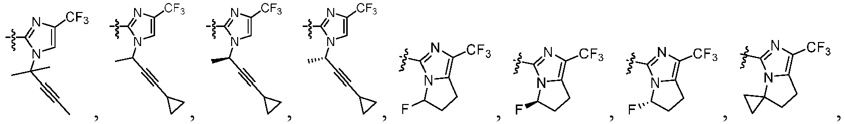 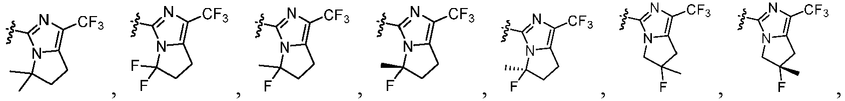 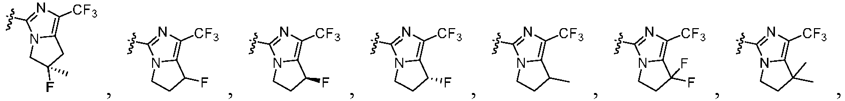 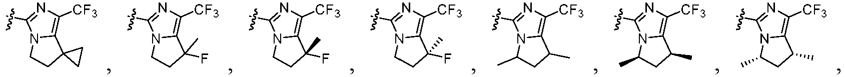 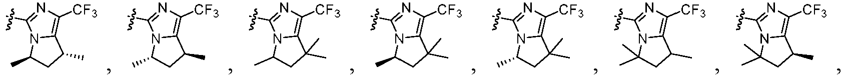 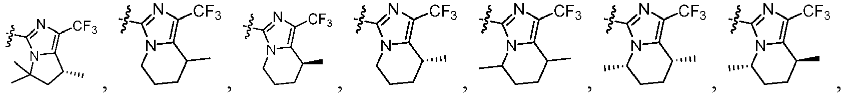 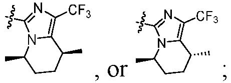
R⁸ is selected from F or Cl.

6. The compound or the stereoisomer, tautomer, deuterated substance, solvate, pharmaceutically acceptable salt or eutectic crystal thereof according to claim 1, **characterized in that** the compound is selected from one of structures shown in following Table:

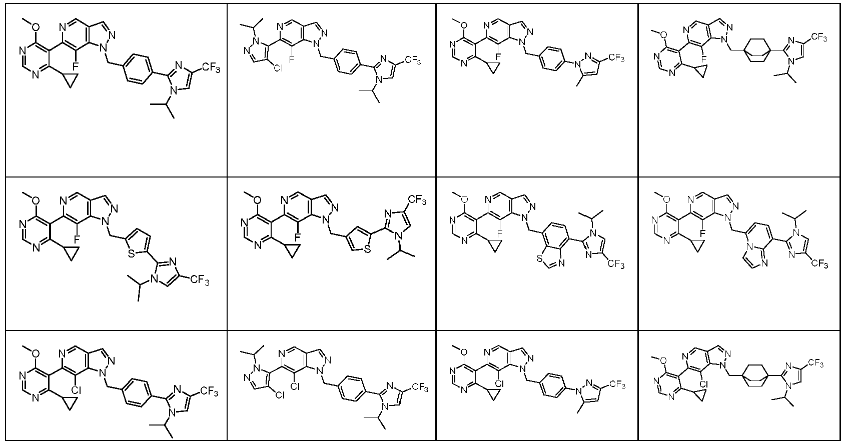

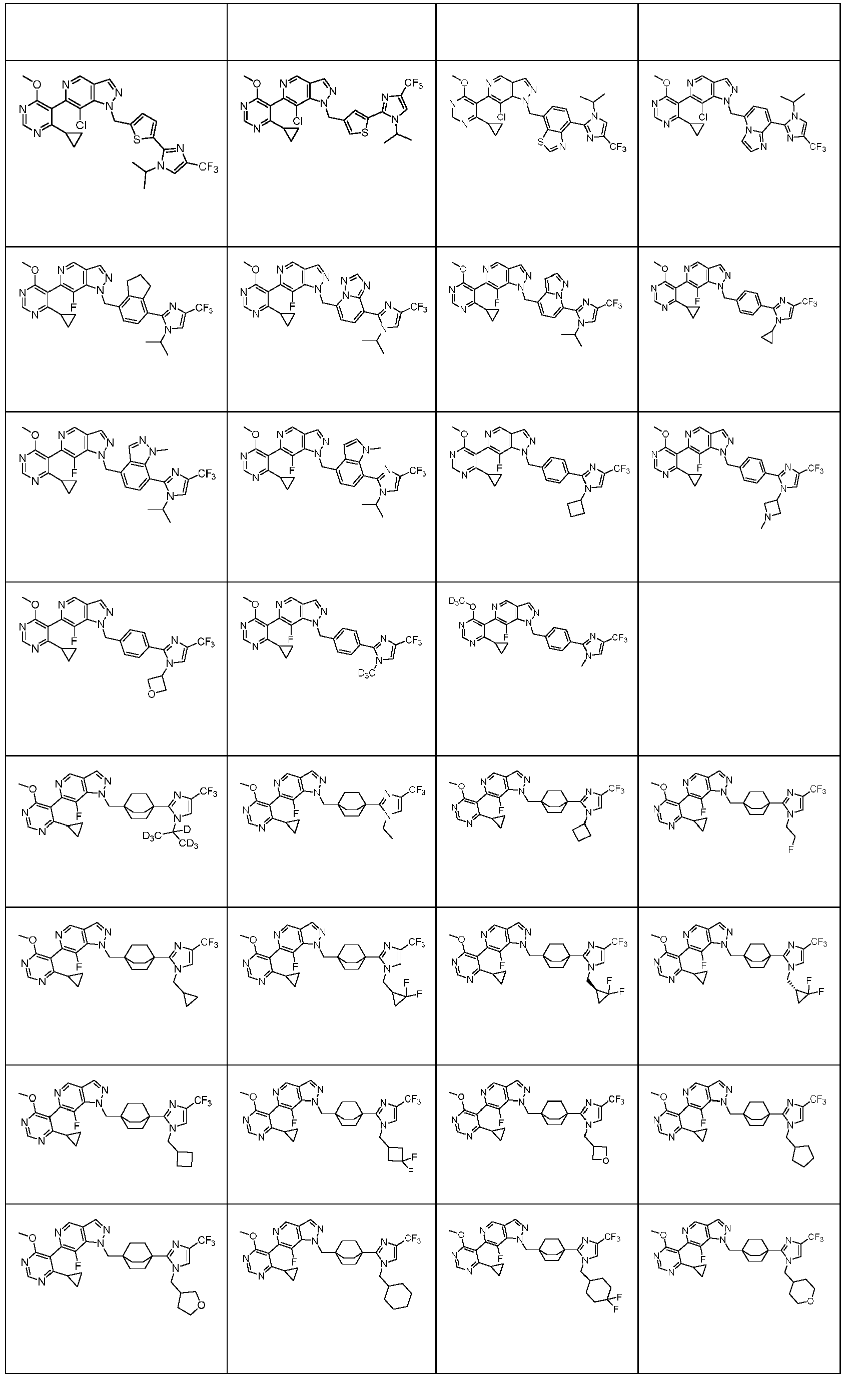

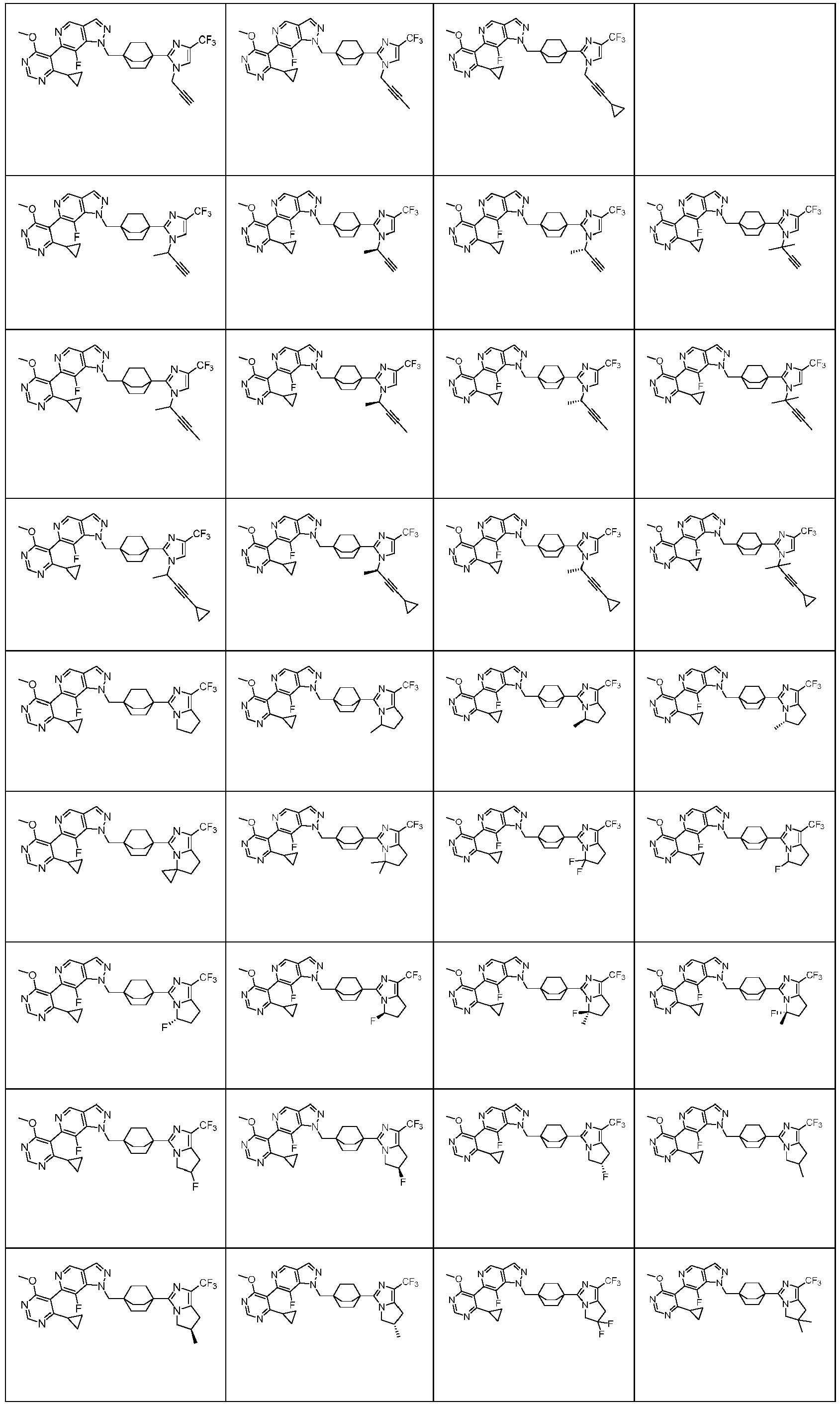

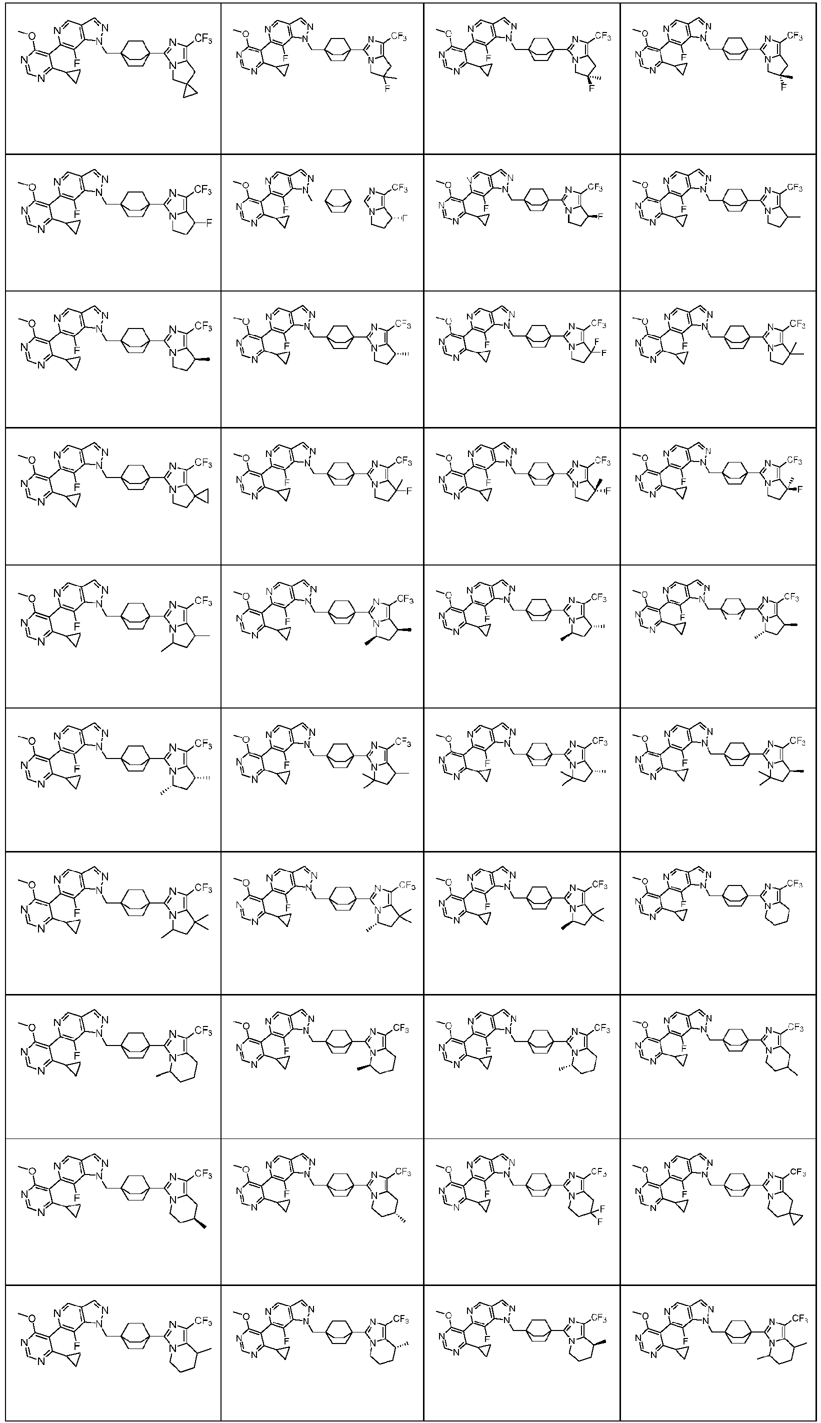

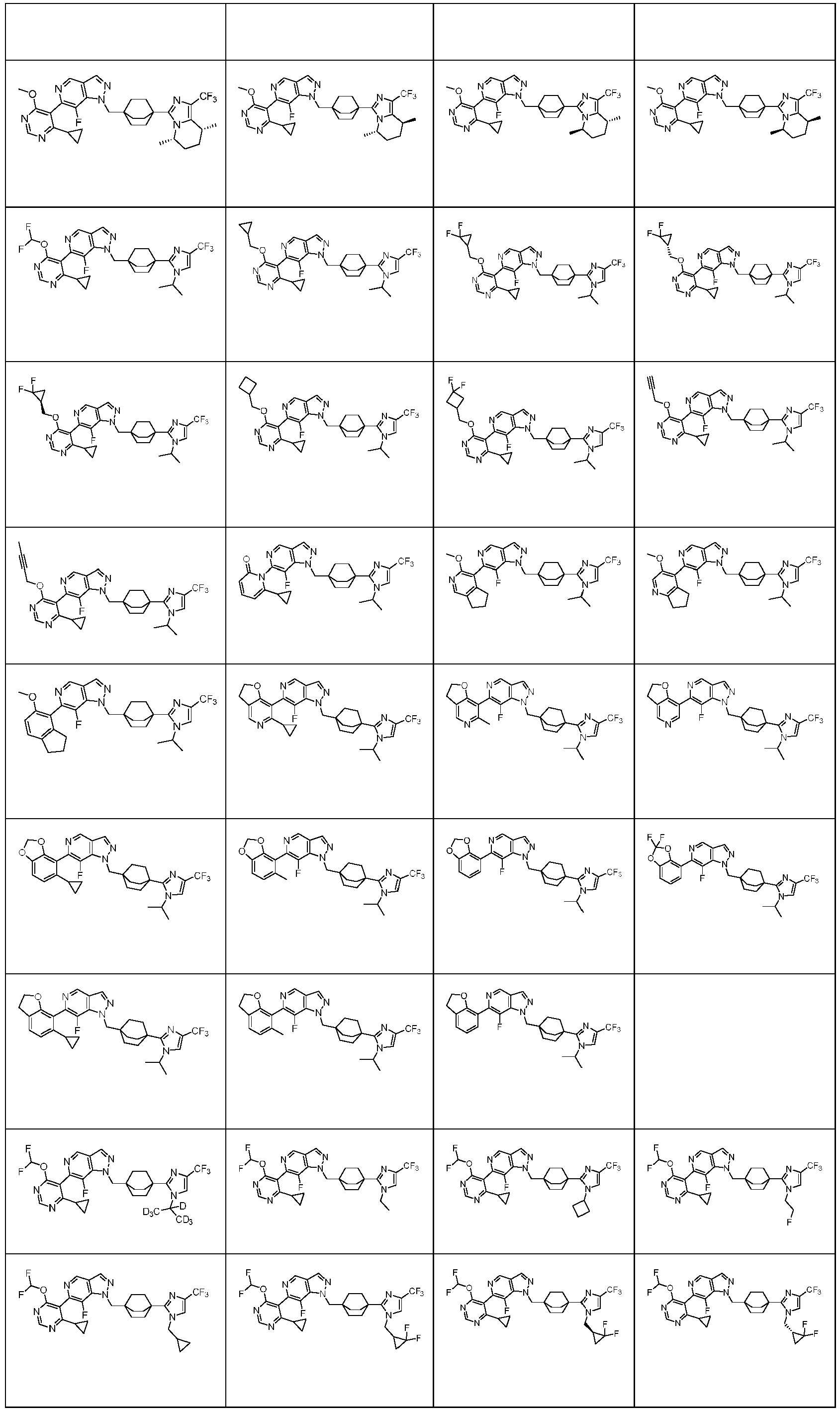

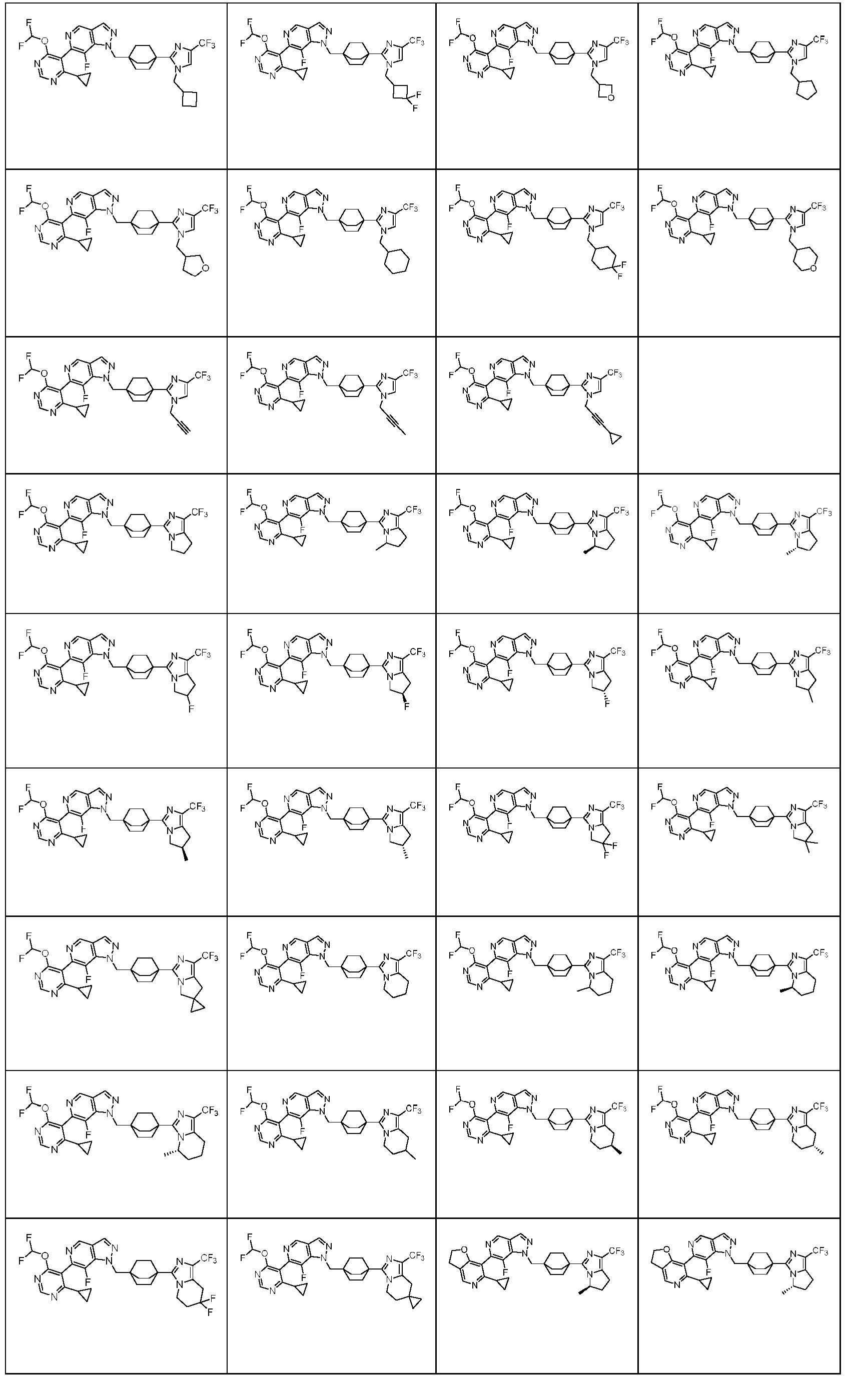

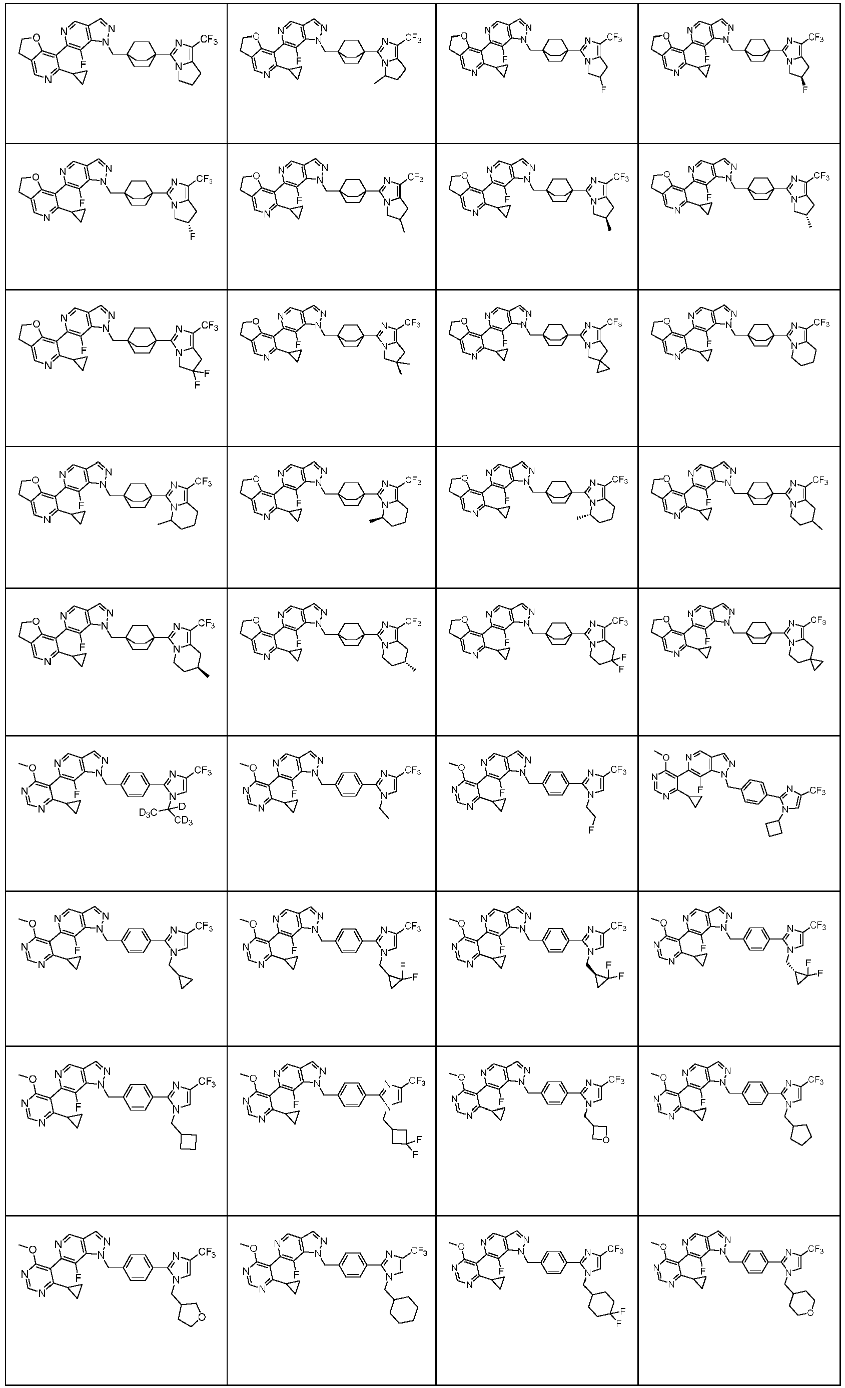

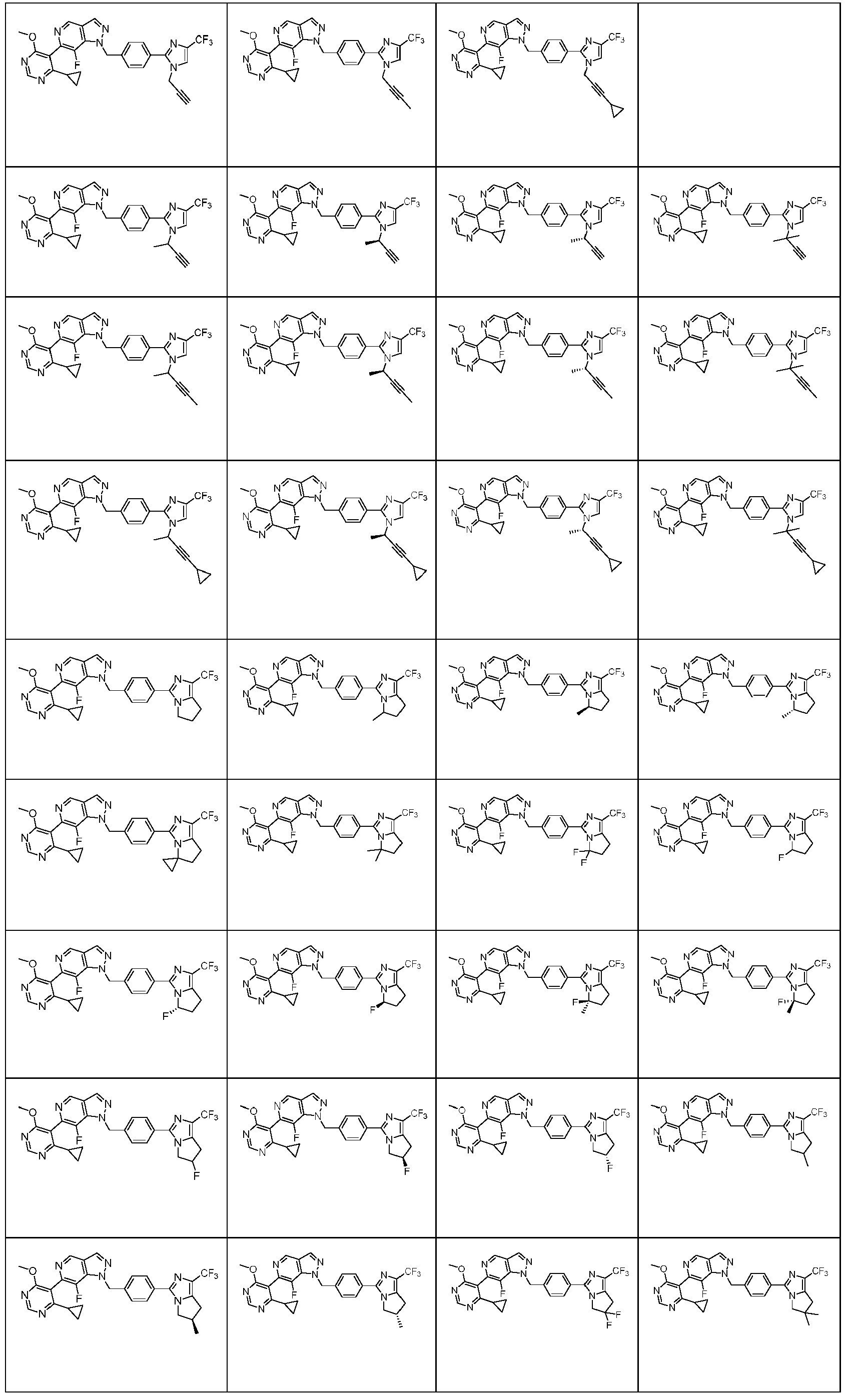

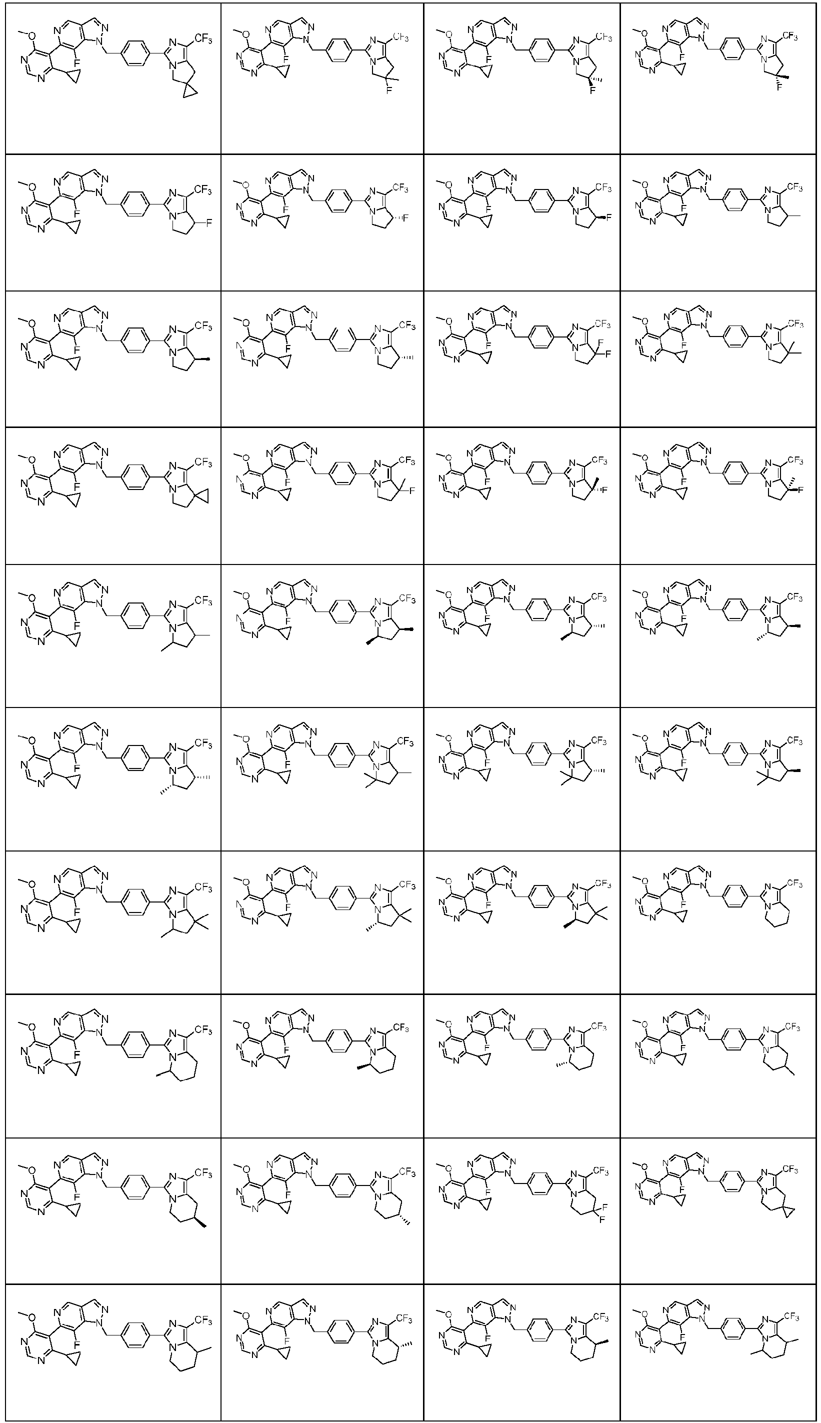

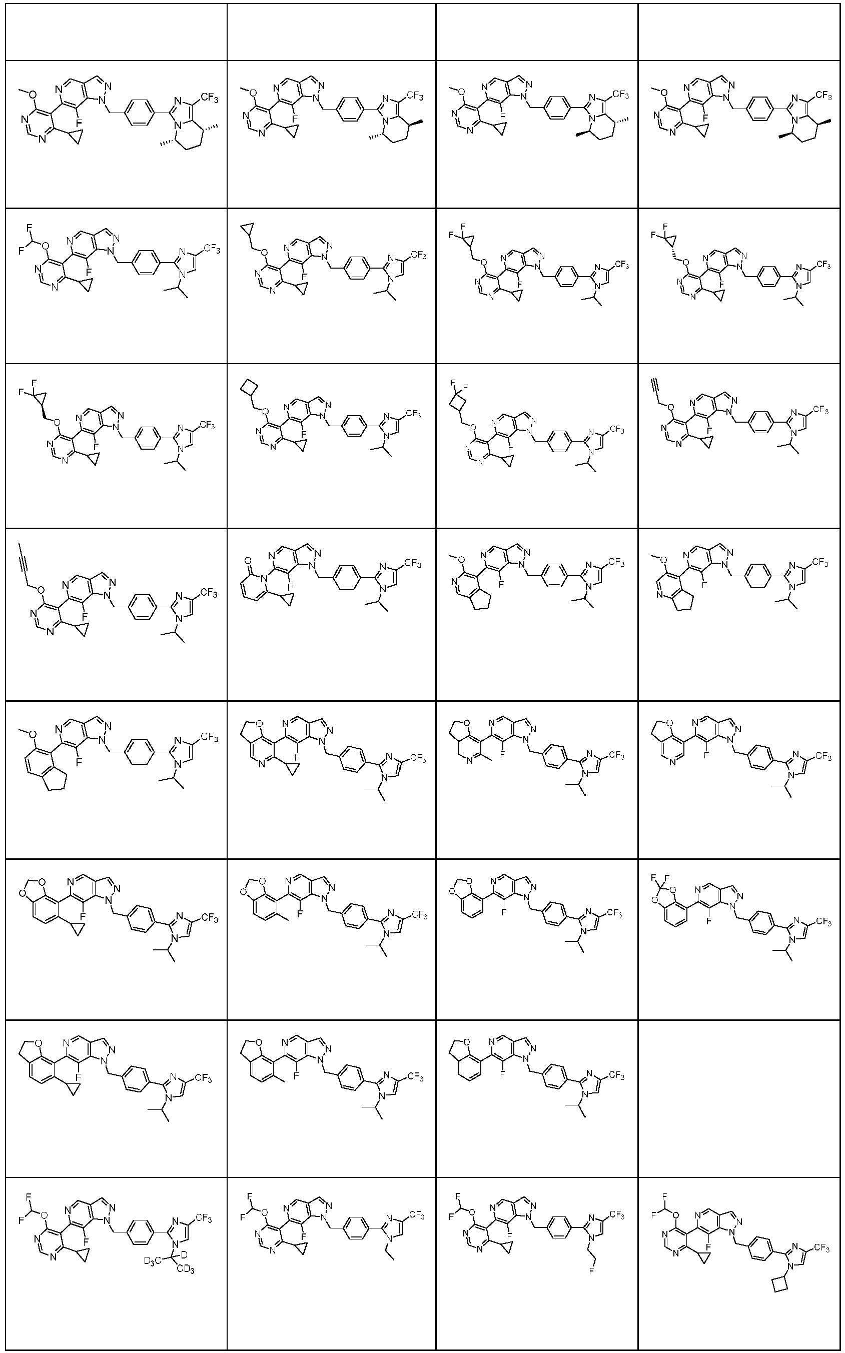

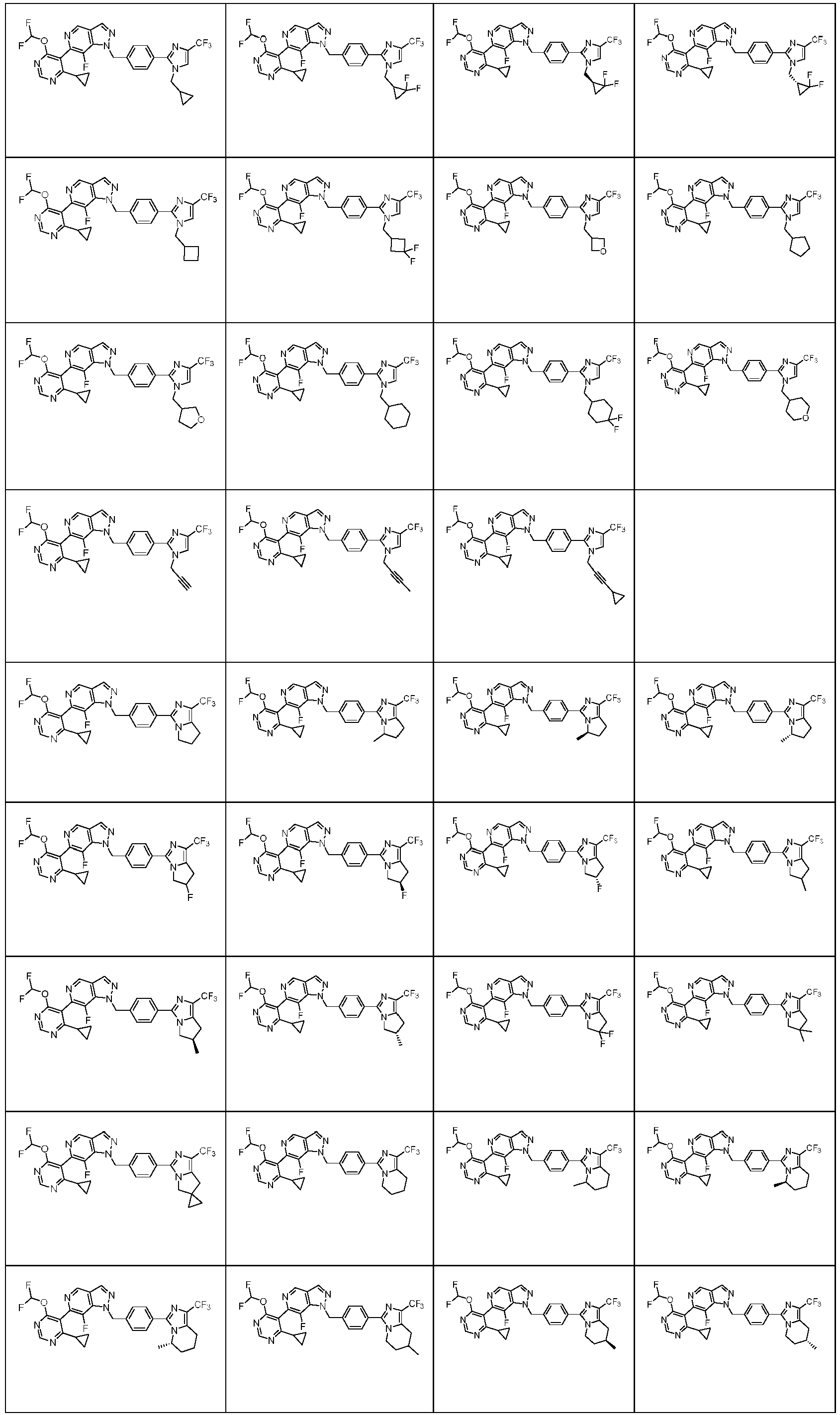

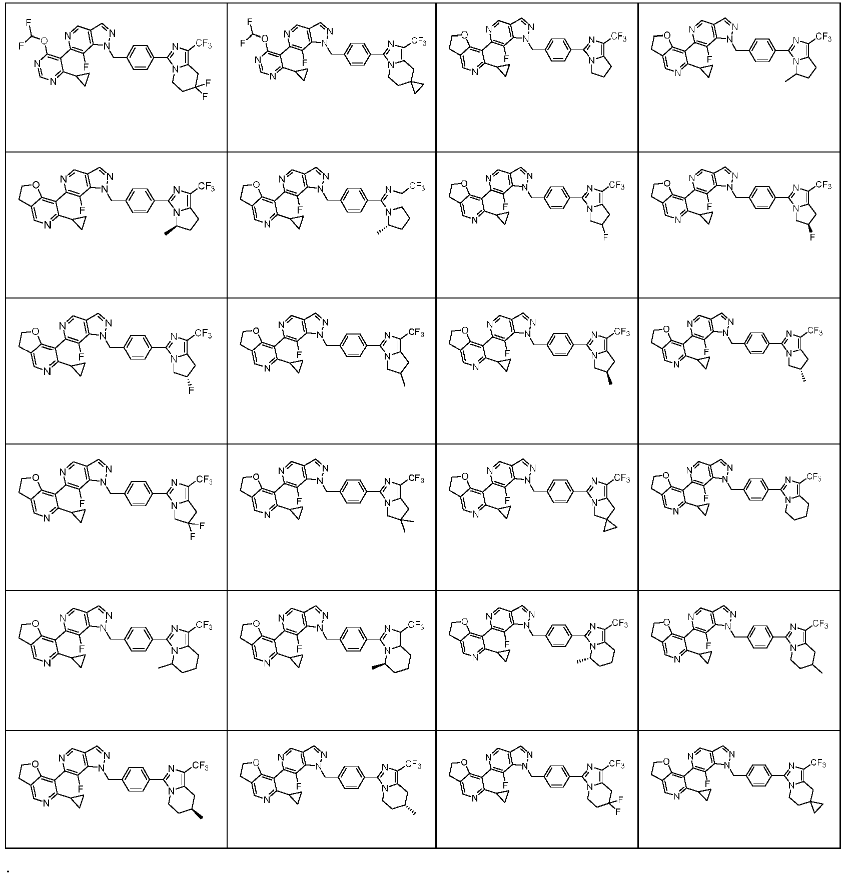

7. A pharmaceutical composition, **characterized by** comprising the compound or the stereoisomer, tautomer, deuterated substance, solvate, pharmaceutically acceptable salt or eutectic crystal thereof according to any one of claims 1-6, and a pharmaceutically acceptable carrier.

8. A pharmaceutical composition or pharmaceutical preparation, **characterized by** comprising 1-1500 mg of the compound or the stereoisomer, deuterated substance, solvate, pharmaceutically acceptable salt or eutectic crystal thereof according to any one of claims 1-6, and a pharmaceutical excipient.

9. Compound or the stereoisomer, tautomer, deuterated substance, solvate, pharmaceutically acceptable salt or eutectic crystal thereof according to any one of claims 1-6 for use in a method for treating a disease related to USP1 activity or expression.

10. The compound or the stereoisomer, deuterated substance, solvate, pharmaceutically acceptable salt or eutectic crystal thereof for use according to claim 9, **characterized in that** the disease is selected from tumors.

11. The compound or the stereoisomer, deuterated substance, solvate, pharmaceutically acceptable salt or eutectic crystal thereof for use according to claim 9, wherein a therapeutically effective amount of the compound is 1-1500 mg.

## Patentansprüche

1. Verbindung oder Stereoisomer, Tautomer, deuterierte Substanz, Solvat, pharmazeutisch verträgliches Salz oder eutektischer Kristall davon, **dadurch gekennzeichnet, dass** die Verbindung aus einer in der allgemeinen Formel (I) gezeigten Verbindung ausgewählt ist, 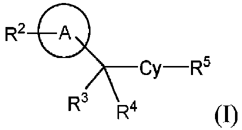
wobei Ring A ausgewählt ist aus 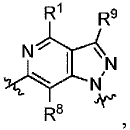 und die linke Seite mit R² verbunden ist;
wobei R¹ und R⁹ jeweils unabhängig voneinander ausgewählt sind aus H, Halogen, Cyano, C₁₋₆-Alkyl, C₂₋₆-Alkenyl, C₂₋₆-Alkinyl und C₁₋₆-Alkoxy, wobei das Alkyl, Alkenyl, Alkinyl oder Alkoxy gegebenenfalls weiter mit 0 bis 4 Substituenten substituiert ist, ausgewählt aus H, Halogen, OH, =O, Cyano, NH₂, C₁₋₆-Alkyl, C₂₋₆-Alkinyl, C₁₋₆-Alkoxy, halogensubstituiertem C₁₋₆-Alkyl, hydroxylsubstituiertem C₁₋₆-Alkyl oder cyanosubstituiertem C₁₋₆-Alkyl;
R⁸ ausgewählt ist aus Halogen, Cyano, C₁₋₆-Alkyl, C₂₋₆-Alkenyl, C₂₋₆-Alkinyl und C₁₋₆-Alkoxy, wobei das Alkyl, Alkenyl, Alkinyl oder Alkoxy gegebenenfalls weiter mit 0 bis 4 Substituenten substituiert ist, ausgewählt aus H, Halogen, OH, =O, Cyano, NH₂, C₁₋₆-Alkyl, C₂₋₆-Alkinyl, C₁₋₆-Alkoxy, halogensubstituiertem C₁₋₆-Alkyl, hydroxylsubstituiertem C₁₋₆-Alkyl oder cyanosubstituiertem C₁₋₆-Alkyl;
R² ausgewählt ist aus C₆₋₁₀-Carbozyklus oder 5- bis 10-gliedrigem Heterozyklus, wobei der Carbozyklus oder Heterozyklus gegebenenfalls weiter mit 0 bis 4 R^{2a} substituiert ist;
jedes R^{2a} unabhängig voneinander ausgewählt ist aus H, Halogen, OH, =O, Cyano, NH₂, NH(C₁₋₆-Alkyl), N(C₁₋₆-Alkyl)2, -SO₂-C₁₋₆-Alkyl, -SO₂-C₃₋₆-Cycloalkyl, C₁₋₆-Alkyl, C₁₋₆-Alkoxy, C₁₋₆- Alkylthio, -Z-C₃₋₆-Cycloalkyl oder -Z-3-bis 8-gliedrigem Heterozyklus, wobei das Alkyl, Cycloalkyl, Alkoxy, Alkylthio oder der Heterozyklus gegebenenfalls weiter mit 0 bis 4 Substituenten substituiert ist, ausgewählt aus H, D, Halogen, OH, =O, Cyano, NH₂, C₁₋₆-Alkyl, C₂₋₆-Alkinyl, C₁₋₆-Alkoxy oder C₃₋₆-Cycloalkyl, und der Heterozyklus 1 bis 4 Heteroatome enthält, ausgewählt aus O, S und N;
R³ und R⁴ jeweils unabhängig voneinander ausgewählt sind aus H, Halogen, OH, Cyano, NH₂, C₁₋₆-Alkyl, C₂₋₆-Alkenyl, C₂₋₆-Alkinyl oder C₁₋₆-Alkoxy, wobei das Alkyl, Alkenyl, Alkinyl oder Alkoxy gegebenenfalls weiter mit 0 bis 4 Substituenten substituiert ist, ausgewählt aus H, Halogen, OH, =O, Cyano, NH₂, C₁₋₆-Alkyl, C₂₋₆-Alkinyl, C₁₋₆-Alkoxy, halogensubstituiertem C₁₋₆-Alkyl, hydroxylsubstituiertem C₁₋₆-Alkyl, cyanosubstituiertem C₁₋₆-Alkyl, C₃₋₆-Cycloalkyl oder 3- bis 8-gliedrigem Heterocyclyl, und das Heterocyclyl 1 bis 4 Heteroatome enthält, ausgewählt aus O, S und N;
Cy ausgewählt ist aus C₃₋₁₂-Carbozyklus oder 4- bis 12-gliedrigem Heterozyklus, und das Cy gegebenenfalls weiter mit 0 bis 4 Substituenten substituiert ist, ausgewählt aus H, Halogen, OH, =O, Cyano, NH₂, C₁₋₆-Alkyl, C₂₋₆-Alkinyl, C₁₋₆-Alkoxy, halogensubstituiertem C₁₋₆-Alkyl, hydroxylsubstituiertem C₁₋₆-Alkyl, cyanosubstituiertem C₁₋₆-Alkyl, C₃₋₆-Cycloalkyl oder 3- bis 8-gliedrigem Heterocyclyl, wobei der Heterozyklus 1 bis 4 Heteroatome enthält, ausgewählt aus O, S und N;
R⁵ ausgewählt ist aus -Z-C₃₋₁₂-Carbozyklus oder -Z-4- bis 12-gliedrigem Heterozyklus, und das R⁵ gegebenenfalls weiter mit 0 bis 4 R^{5a} substituiert ist;
jedes R^{5a} unabhängig voneinander ausgewählt ist aus H, Halogen, OH, Cyano, NH₂, NHC₁₋₆-Alkyl, N(C₁₋₆-Alkyl)₂, C₁₋₆-Alkyl, C₂₋₆-Alkinyl, C₁₋₆-Alkoxy, -C₁₋₄-Alkylen-C₃₋₆-Cycloalkyl, -C₁₋₄-Alkylen-3- bis 6-gliedrigem Heterozyklus, C₃₋₆-Cycloalkyl, 3- bis 8-gliedrigem Heterozyklus, -CONHC₁₋₆-Alkyl, -CONHC₁₋₆-Alkoxy, -CONHC₃₋₆-Cycloalkyl, -CONH 3- bis 8-gliedrigem Heterozyklus, -NHCO-C₁₋₆-Alkyl, -NHCO-C₁₋₆-Alkoxy, -NHCO-C₃₋₆-Cycloalkyl oder -NHCO-3- bis 8-gliedrigem Heterozyklus, wobei das Alkyl, Alkylen, Alkinyl, Alkoxy, Cycloalkyl oder der Heterozyklus gegebenenfalls weiter mit 0 bis 4 Substituenten substituiert ist, ausgewählt aus H, D, Halogen, OH, =O, Cyano, NH₂, -C(=O)NH₂, -C(=O)NHC₁₋₆-Alkyl,-C(=O)N(C₁₋₆-Alkyl)₂, C₁₋₆-Alkyl, C₂₋₆-Alkinyl, C₁₋₆-Alkoxy, halogensubstituiertem C₁₋₆-Alkyl, hydroxylsubstituiertem C₁₋₆-Alkyl, cyanosubstituiertem C₁₋₆-Alkyl, C₃₋₆-Cycloalkyl, halogensubstituiertem C₃₋₆-Cycloalkyl oder 3- bis 8-gliedrigem Heterozyklus, und der Heterozyklus 1 bis 4 Heteroatome enthält, ausgewählt aus O, S und N;
Z ausgewählt ist aus einer Bindung, O, S, NH, N(C₁₋₄-Alkyl), C(=O)NH, NHC(=O), -OCH₂-, -CH₂O- oder C₁₋₃-Alkylen.

2. Verbindung oder Stereoisomer, Tautomer, deuterierte Substanz, Solvat, pharmazeutisch verträgliches Salz oder eutektischer Kristall davon nach Anspruch 1, **dadurch gekennzeichnet, dass** die in der allgemeinen Formel (I) gezeigte Verbindung ausgewählt ist aus einer in der allgemeinen Formel (la) gezeigten Verbindung, 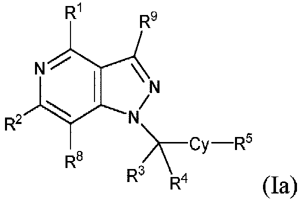
wobei R¹ und R⁹ jeweils unabhängig voneinander ausgewählt sind aus H, Halogen, Cyano, C₁₋₄-Alkyl, C₂₋₄-Alkenyl, C₂₋₄-Alkinyl und C₁₋₄-Alkoxy, wobei das Alkyl, Alkenyl, Alkinyl oder Alkoxy gegebenenfalls weiter mit 0 bis 4 Substituenten substituiert ist, ausgewählt aus H, Halogen, OH, =O, Cyano, NH₂, C₁₋₄-Alkyl, C₂₋₄-Alkinyl, C₁₋₄-Alkoxy, halogensubstituiertem C₁₋₄-Alkyl, hydroxylsubstituiertem C₁₋₄-Alkyl oder cyanosubstituiertem C₁₋₄-Alkyl;
R⁸ ausgewählt ist aus Halogen, Cyano, C₁₋₄-Alkyl, C₂₋₄-Alkinyl und C₁₋₄-Alkoxy, wobei das Alkyl, Alkinyl oder Alkoxy gegebenenfalls weiter mit 0 bis 4 Substituenten substituiert ist, ausgewählt aus H, Halogen, OH, Cyano, NH₂, C₁₋₄-Alkyl, C₂₋₄-Alkinyl und C₁₋₄-Alkoxy;
jedes R^{2a} unabhängig voneinander ausgewählt ist aus H, Halogen, OH, =O, Cyano, NH₂, NH(C₁₋₄-Alkyl),N(C₁₋₄-Alkyl)₂, -SO₂-C₁₋₄-Alkyl, -SO₂-C₃₋₆-Cycloalkyl, C₁₋₄-Alkyl, C₁₋₄-Alkoxy, C₁₋₄- Alkylthio, -Z-C₃₋₆-Cycloalkyl oder -Z-3-bis 6-gliedrigem Heterozyklus, wobei das Alkyl, Cycloalkyl, Alkoxy, Alkylthio oder der Heterozyklus gegebenenfalls weiter mit 0 bis 4 Substituenten substituiert ist, ausgewählt aus H, D, Halogen, OH, =O, Cyano, NH₂, C₁₋₄-Alkyl, C₂₋₄-Alkinyl, C₁₋₄-Alkoxy oder C₃₋₆-Cycloalkyl, und der Heterozyklus 1 bis 4 Heteroatome enthält, ausgewählt aus O, S und N;
R³ und R⁴ jeweils unabhängig voneinander ausgewählt sind aus H, Halogen, OH, Cyano und C₁₋₄-Alkyl;
Cy ausgewählt ist aus C₃₋₆-monocyclischem Cycloalkyl, C₅₋₁₁-kondensiertem Cycloalkyl, C₅₋₁₁-Spirocycloalkyl, C₅₋₁₁-überbrücktem Cycloalkyl, 4-bis 7-gliedrigem monocyclischem Heterocycloalkyl, 6- bis 11-gliedrigem kondensiertem Heterocycloalkyl, 6- bis 11-gliedrigem Spiroheterocycloalkyl, 6- bis 11-gliedrigem überbrücktem Heterocycloalkyl, Benzolring, Benzo-C₄₋₆-Carbozyklus, Benzo-4- bis 6-gliedrigem Heterozyklus, 5- bis 6-gliedrigem heteroaromatischem Ring oder 8- bis 10-gliedrigem kondensiertem heteroaromatischem Ring, und das Cy gegebenenfalls weiter mit 0 bis 4 Substituenten substituiert ist, ausgewählt aus H, Halogen, OH, =O, Cyano, NH₂, C₁₋₄-Alkyl, C₂₋₄-Alkinyl, C₁₋₄-Alkoxy, halogensubstituiertem C₁₋₄-Alkyl, hydroxylsubstituiertem C₁₋₄-Alkyl, cyanosubstituiertem C₁₋₄-Alkyl, C₃₋₆-Cycloalkyl oder 3- bis 8-gliedrigem Heterozyklus, wobei der Heterozyklus 1 bis 4 Heteroatome enthält, ausgewählt aus O, S und N;
R⁵ ausgewählt ist aus -Z-C₆₋₁₂-Carbozyklus oder -Z-5- bis 12-gliedrigem Heterozyklus, und das R⁵ gegebenenfalls weiter mit 0 bis 4 R^{5a} substituiert ist;
jedes R^{5a} unabhängig voneinander ausgewählt ist aus H, Halogen, OH, Cyano, NH₂, C₁₋₄-Alkyl, C₂₋₄-Alkinyl, C₁₋₄-Alkoxy, -C₁₋₂-Alkylen-C₃₋₆-Cycloalkyl, -C₁₋₂-Alkylen-3- bis 6-gliedrigem Heterozyklus, C₃₋₆-Cycloalkyl oder 3- bis 6-gliedrigem Heterozyklus, wobei das Alkyl, Alkylen, Alkinyl, Alkoxy, Cycloalkyl oder der Heterozyklus gegebenenfalls weiter mit 0 bis 4 Substituenten substituiert ist, ausgewählt aus H, D, Halogen, OH, =O, Cyano, NH₂, -C(=O)NH₂, -C(=O)NHC₁₋₄-Alkyl,-C(=O)N(C₁₋₄-Alkyl)₂, C₁₋₄-Alkyl, C₂₋₄-Alkinyl, C₁₋₄-Alkoxy, halogensubstituiertem C₁₋₄-Alkyl, hydroxylsubstituiertem C₁₋₄-Alkyl, cyanosubstituiertem C₁₋₄-Alkyl, C₃₋₆- Cycloalkyl, halogensubstituiertem C₃₋₆-Cycloalkyl oder 3- bis 8-gliedrigem Heterozyklus, und der Heterozyklus 1 bis 4 Heteroatome enthält, ausgewählt aus O, S und N.

3. Verbindung oder Stereoisomer, Tautomer, deuterierte Substanz, Solvat, pharmazeutisch verträgliches Salz oder eutektischer Kristall davon nach Anspruch **2, dadurch gekennzeichnet, dass**
R¹ und R⁹ jeweils unabhängig voneinander ausgewählt sind aus H, F, Cl, Br, I, Cyano, Methyl oder Ethyl;
R⁸ ausgewählt ist aus F, Cl, Br, I, CN, Methyl, Ethyl, Ethinyl, Methoxy oder Ethoxy;
R² ausgewählt ist aus Phenyl, Pyridin, Pyrimidin, Pyrrol, Pyrazol, Imidazol, Furan, Thiophen, Thiazol oder Oxazol, wobei das Phenyl, Pyridin, Pyrimidin, Pyrrol, Pyrazol, Imidazol, Furan, Thiophen, Thiazol oder Oxazol gegebenenfalls weiter mit 0 bis 4 R^{2a} substituiert ist;
oder R² aus 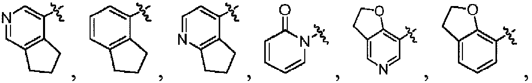 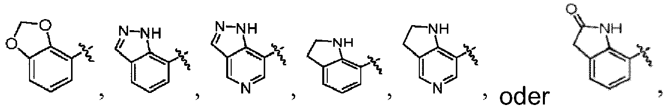 ausgewählt ist und R² gegebenenfalls weiter mit 0 bis 4 R^{2a} substituiert ist;
jedes R^{2a} unabhängig voneinander ausgewählt ist aus H, F, Cl, Br, I oder Cyano, oder jedes R^{2a} unabhängig voneinander ausgewählt ist aus einer der folgenden substituierten oder nicht substituierten Gruppen: -SO₂-Methyl, -SO₂-Ethyl, -SO₂-Propyl, -SO₂-Isopropyl, -SO₂-Cyclopropyl, Methyl, Ethyl, Propyl, Isopropyl, Methoxy, Ethoxy, Propoxy, Methylthio, Ethylthio, Propylthio, Cyclopropyl, Cyclobutyl, Cyclopentyl, Cyclohexyl, Azetidinyl, Azacyclopentyl, Azacyclohexyl, Piperazin, Oxetanyl, Oxacyclopentyl, Oxacyclohexyl, Morpholin, - O-Azetidinyl, -O-Azacyclopentyl, -O-Azacyclohexyl, -O-Oxetanyl, -O-Oxacyclopentyl, -O-Oxacyclohexyl, -OCH₂-Phenyl, -OCH₂-Cyclopropyl, -OCH₂-Cyclobutyl, -OCH₂-Cyclopentyl, -OCH₂-Cyclohexyl, Pyrrol, Pyrazol, Imidazol, Thiazol, Thiophen, Furan und Oxazol, die, wenn sie substituiert sind, gegebenenfalls weiter mit 0 bis 4 Substituenten substituiert sind, ausgewählt aus H, D, Halogen, OH, =O, Cyano, NH₂, C₁₋₄-Alkyl, C₂₋₄-Alkinyl, C₁₋₄-Alkoxy, und C₃₋₆-Cycloalkyl;
R³ und R⁴ jeweils unabhängig voneinander ausgewählt sind aus H, F, Cl, Br, I, Methyl und Ethyl;
Cy ausgewählt ist aus Cyclopropyl, Cyclobutyl, Cyclopentyl, Cyclohexyl, Bicyclo[2.2.2]octyl, Bicyclo[1.1.1]pentyl, cyclopentylanelliertem Cyclopentyl, Cyclobutylspirocyclohexyl, Cyclobutylspirocyclopentyl, Azetidinyl, Azacyclopentyl, Azacyclohexyl, Azetidinylspirocyclobutyl, Azetidinylspirocyclopentyl, Azetidinylspirocyclohexyl, Azetidinylspiroazetidinyl, Azetidinylspiroazacyclopentyl, 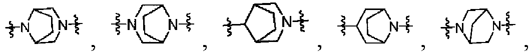 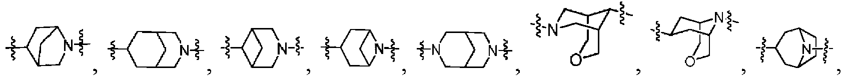 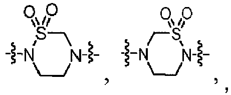 Benzolring, Thiophen, Furan, Pyrrol, Thiazol, Oxazol, Pyrazol, Pyrazin, Pyrimidin, 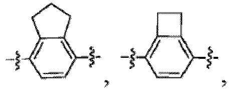 Benzothiazol, Benzothiophen, Benzofuran, Benzoxazol, Benzopyrrol, Benzopyrazol, Benzimidazol, Indolizin, Pyridinopyrrol, Pyridinoimidazol, Pyridinopyrazol, Pyrimidoimidazol, Pyrimidopyrazol, Pyridinotriazol oder 2-Pyridon, und das Cy gegebenenfalls weiter mit 0 bis 4 Substituenten substituiert ist, ausgewählt aus H, Halogen, OH, =O, Cyano, NH₂, C₁₋₄-Alkyl, C₂₋₄-Alkinyl, C₁₋₄-Alkoxy, halogensubstituiertem C₁₋₄-Alkyl, hydroxylsubstituiertem C₁₋₄-Alkyl, cyanosubstituiertem C₁₋₄-Alkyl, C₃₋₆-Cycloalkyl oder 3- bis 8-gliedrigem Heterozyklus, wobei der Heterozyklus 1 bis 4 Heteroatome enthält, ausgewählt aus O, S und N;
R⁵ ausgewählt ist aus Benzolring, Pyrazol, Pyrrol, Imidazol, Triazol, Pyridin, Pyrimidin, Pyrazin oder Pyridazin, und das R⁵ gegebenenfalls weiter mit 0 bis 4 R^{5a} substituiert ist;
oder R⁵ ausgewählt ist aus 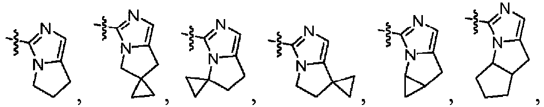 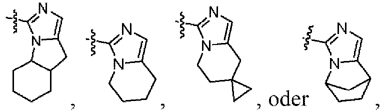 und das R⁵ gegebenenfalls weiter substituiert ist mit 0 bis 4 R^{5a};
jedes R^{5a} unabhängig voneinander ausgewählt ist aus H, F, Cl, Br, I, Cyano, Methyl, Ethyl, Propyl, Isopropyl, Propargyl, Butargyl, Methoxy, Ethoxy, Cyclopropyl, Cyclobutyl, Cyclopentyl, Cyclohexyl, Oxetanyl, Oxacyclopentyl, Oxacyclohexyl, Azetidinyl, Azacyclopentyl, Azacyclohexyl, -CH₂-Cyclopropyl, - CH₂-Cyclobutyl, -CH₂-Cyclopentyl, -CH₂-Cyclohexyl, -CH₂-Oxetanyl, -CH₂-Oxacyclopentyl, -CH₂-Oxacyclohexyl, -CH₂-Azetidinyl, -CH₂-Azacyclopentyl oder - CH₂-Azacyclohexyl, wobei das Methyl, Ethyl, Propyl, Isopropyl, Propargyl, Butargyl, Methoxy, Ethoxy, Cyclopropyl, Cyclobutyl, Cyclopentyl, Cyclohexyl, Oxetanyl, Oxacyclopentyl, Oxacyclohexyl, Azetidinyl, Azacyclopentyl oder Azacyclohexyl gegebenenfalls weiter mit 0 bis 4 Substituenten substituiert ist, ausgewählt aus H, D, Halogen, OH, =O, Cyano, NH₂, C₁₋₄-Alkyl, C₂₋₄-Alkinyl, C₁₋₄-Alkoxy, C₃₋₆-Cycloalkyl, oder halogensubstituiertem C₃₋₆-Cycloalkyl.

4. Verbindung oder Stereoisomer, Tautomer, deuterierte Substanz, Solvat, pharmazeutisch verträgliches Salz oder eutektischer Kristall davon nach Anspruch 3, **dadurch gekennzeichnet, dass**
R¹ und R⁹ H sind;
R⁸ ausgewählt ist aus F, Cl, Br, CN, Methyl, Ethinyl oder Methoxy;
R² ausgewählt ist aus Pyrrol, Pyrazol, Imidazol, Pyrimidin, 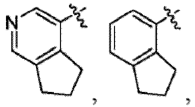 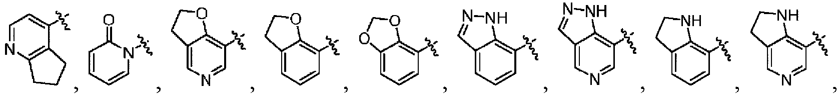 oder 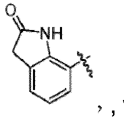 wobei das Pyrrol, Pyrazol, Imidazol, Pyrimidin, 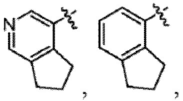 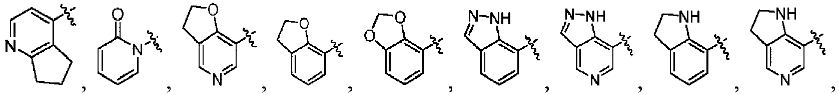 oder 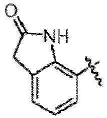 gegebenenfalls weiter substituiert ist mit 0 bis 4 Substituenten, ausgewählt aus H, F, Cl, Br, I, Cyano, -SO₂-Methyl, -SO₂-Ethyl, -SO₂-Propyl, -SO₂-Isopropyl, - SO₂-Cyclopropyl, CH₂F, CHF₂, CF₃, -OCH₂F, -OCHF₂, -OCF₃, Methyl, Ethyl, Propyl, Isopropyl, Methoxy, Ethoxy, Propoxy, -OCD₃, Methylthio, Ethylthio, Propylthio, Cyclopropyl, Cyclobutyl, Cyclopentyl, Cyclohexyl, Azetidinyl, Azacyclopentyl, Azacyclohexyl, Piperazin, Oxetanyl, Oxacyclopentyl, Oxacyclohexyl, Morpholin, -O-Azetidinyl, -O-Azacyclopentyl, -O-Azacyclohexyl, -O-Oxetanyl, -O-Oxacyclopentyl, - O-Oxacyclohexyl, -OCH₂-Phenyl, -OCH₂-Cyclopropyl, -OCH₂-Cyclobutyl, -OCH₂-Cyclopentyl, -OCH₂-Cyclohexyl, 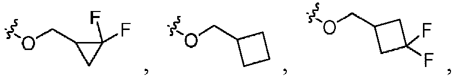 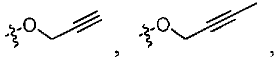 Pyrrol, Pyrazol, Imidazol, Thiazol, Thiophen, Furan, Oxazol, 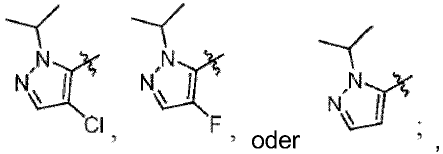
Cy ausgewählt ist aus 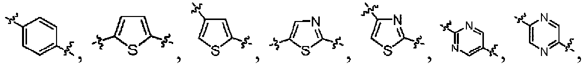 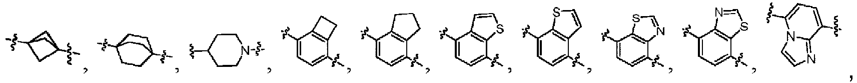 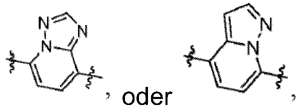 und das Cy gegebenenfalls weiter mit 0 bis 4 Substituenten substituiert ist, ausgewählt aus H, F, Cl, Br, I, Cyano, CH₂F, CHF₂, CF₃, Methyl, Ethyl, Propyl, Isopropyl, Methoxy, Ethoxy oder Cyclopropyl;
wobei alternativ Cy ausgewählt ist aus 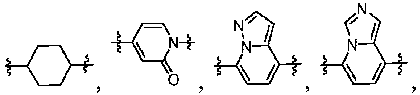 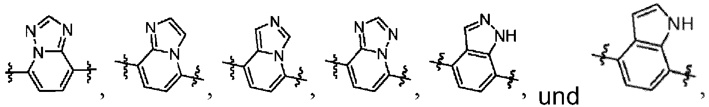 und das Cy gegebenenfalls weiter substituiert ist mit 0 bis 4 Substituenten, ausgewählt aus H, F, Cl, Br, I, Cyano, CH₂F, CHF₂, CF₃, Methyl, Ethyl, Propyl, Isopropyl, Methoxy, Ethoxy oder Cyclopropyl;
R⁵ ausgewählt ist aus Pyrrol, Pyrazol oder Imidazol, und das Pyrrol, Pyrazol oder Imidazol gegebenenfalls weiter substituiert ist mit 0 bis 4 Substituenten, ausgewählt aus H, F, Cl, Br, I, Cyano, CH₂F, CHF₂, CF₃, CD₃, Methyl, Ethyl, Propyl, Isopropyl, -CH₂CH₂F, Methoxy, Ethoxy, Cyclopropyl, Cyclobutyl, Cyclopentyl, Cyclohexyl, Oxetanyl, Oxacyclopentyl, Oxacyclohexyl, Azetidinyl, N-Methylazetidinyl, Azacyclopentyl, Azacyclohexyl, 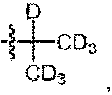 -CH₂-Cyclopropyl, -CH₂-Cyclobutyl, -CH₂-Cyclopentyl, -CH₂-Cyclohexyl, -CH₂-Oxetanyl, -CH₂-Oxacyclopentyl, -CH₂-Oxacyclohexyl, -CH₂-Azetidinyl, -CH₂-Azacyclopentyl, -CH₂-Azacyclohexyl, 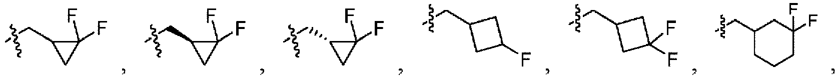 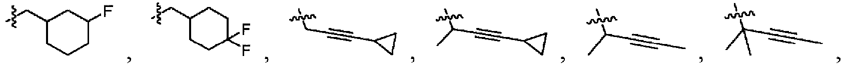 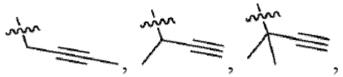 oder Propargyl;
wobei alternativ R⁵ ausgewählt ist aus 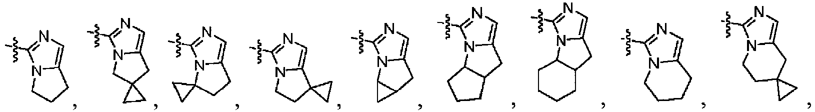 oder 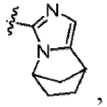 und R⁵ gegebenenfalls weiter substituiert ist mit 0 bis 4 Substituenten, ausgewählt aus H, F, Cl, Br, I, Cyano, CH₂F, CHF₂, CF₃, CD₃, Methyl, Ethyl, Propyl, Isopropyl, -CH2CH2F, Methoxy, Ethoxy, Cyclopropyl, Cyclobutyl, Cyclopentyl, Cyclohexyl, Oxetanyl, Oxacyclopentyl, Oxacyclohexyl, Azetidinyl, N-Methylazetidinyl, Azacyclopentyl, Azacyclohexyl, 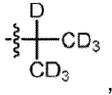 -CH₂-Cyclopropyl, - CH₂-Cyclobutyl, -CH₂-Cyclopentyl, -CH₂-Cyclohexyl, -CH₂-Oxetanyl, -CH₂-Oxacyclopentyl, -CH₂-Oxacyclohexyl, -CH₂-Azetidinyl, -CH₂-Azacyclopentyl, -CH₂-Azacyclohexyl, 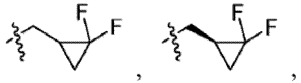 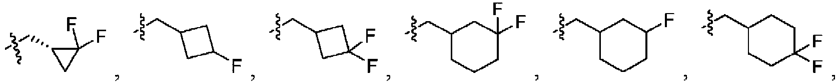 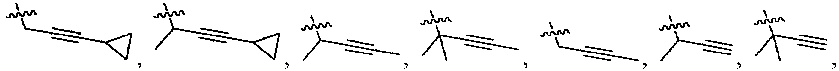 oder Propargyl.

5. Verbindung oder Stereoisomer, Tautomer, deuterierte Substanz, Solvat, pharmazeutisch verträgliches Salz oder eutektischer Kristall davon nach Anspruch 4, **dadurch gekennzeichnet, dass**
R² ausgewählt ist aus 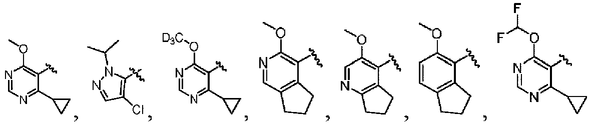 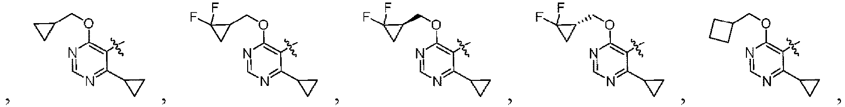 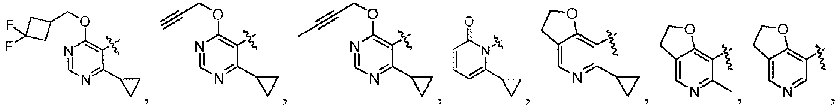 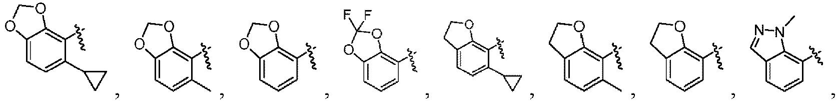 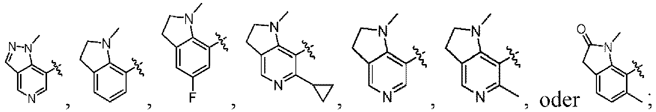
R⁵ ausgewählt ist aus 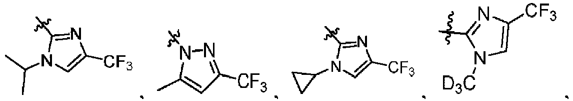 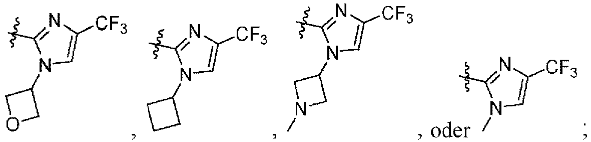 wobei alternativ R⁵ ausgewählt ist aus 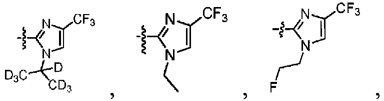 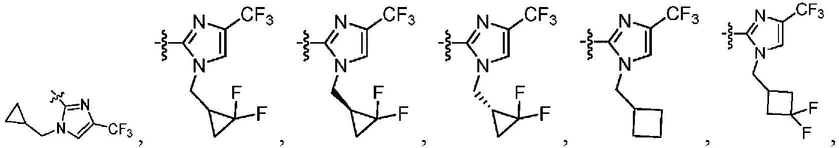 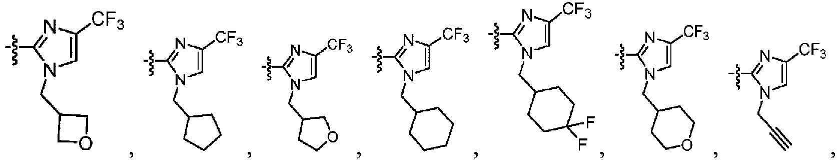 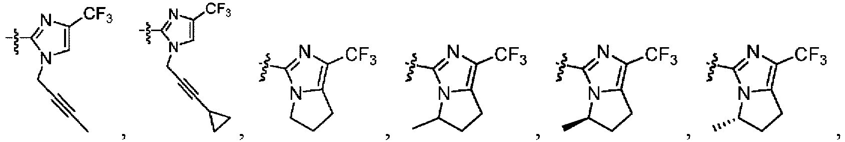 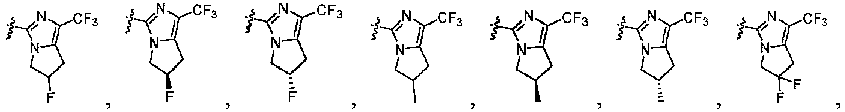 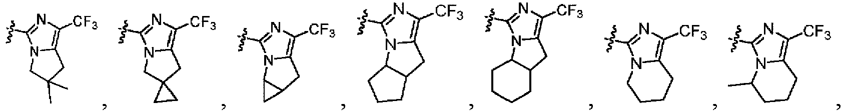 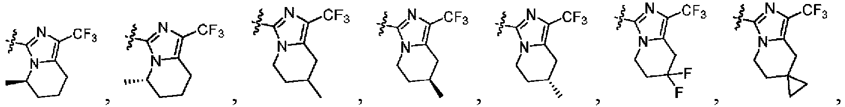 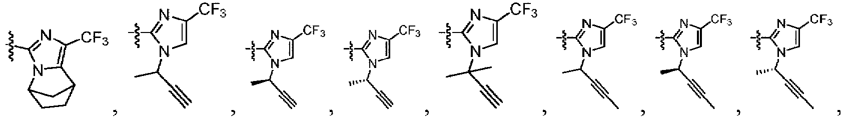 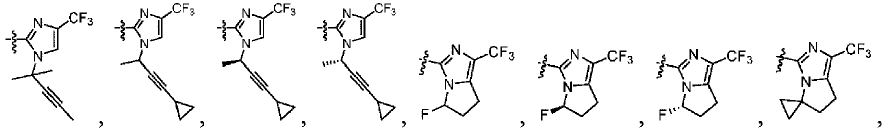 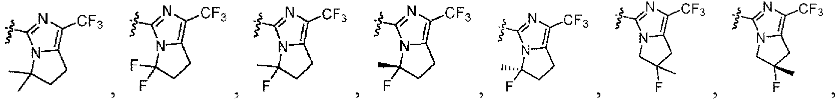 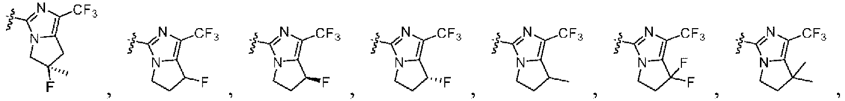 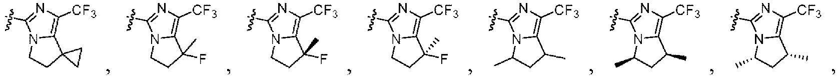 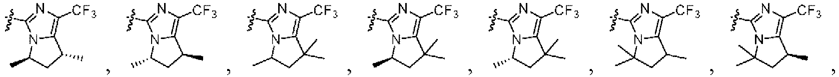 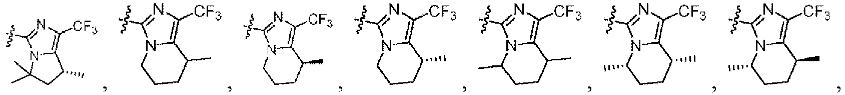 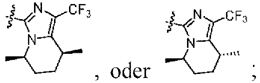
R⁸ ausgewählt ist aus F oder Cl.

6. Verbindung oder Stereoisomer, Tautomer, deuterierte Substanz, Solvat, pharmazeutisch verträgliches Salz oder eutektischer Kristall davon nach Anspruch 1, **dadurch gekennzeichnet, dass** die Verbindung aus einer der in der folgenden Tabelle gezeigten Strukturen ausgewählt ist:

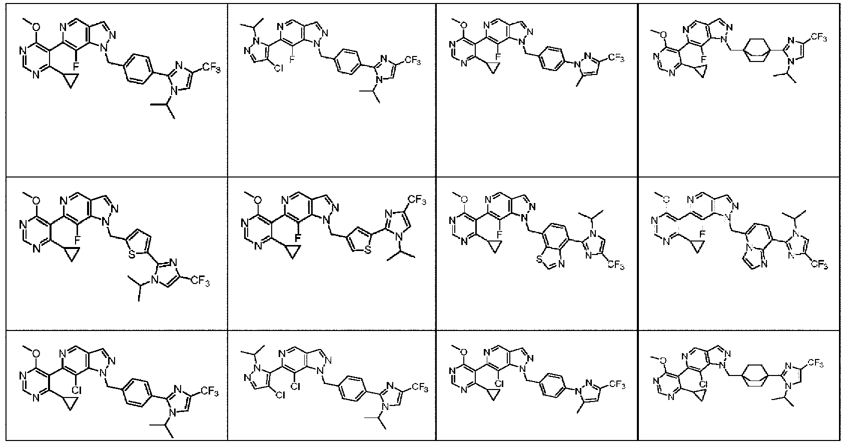

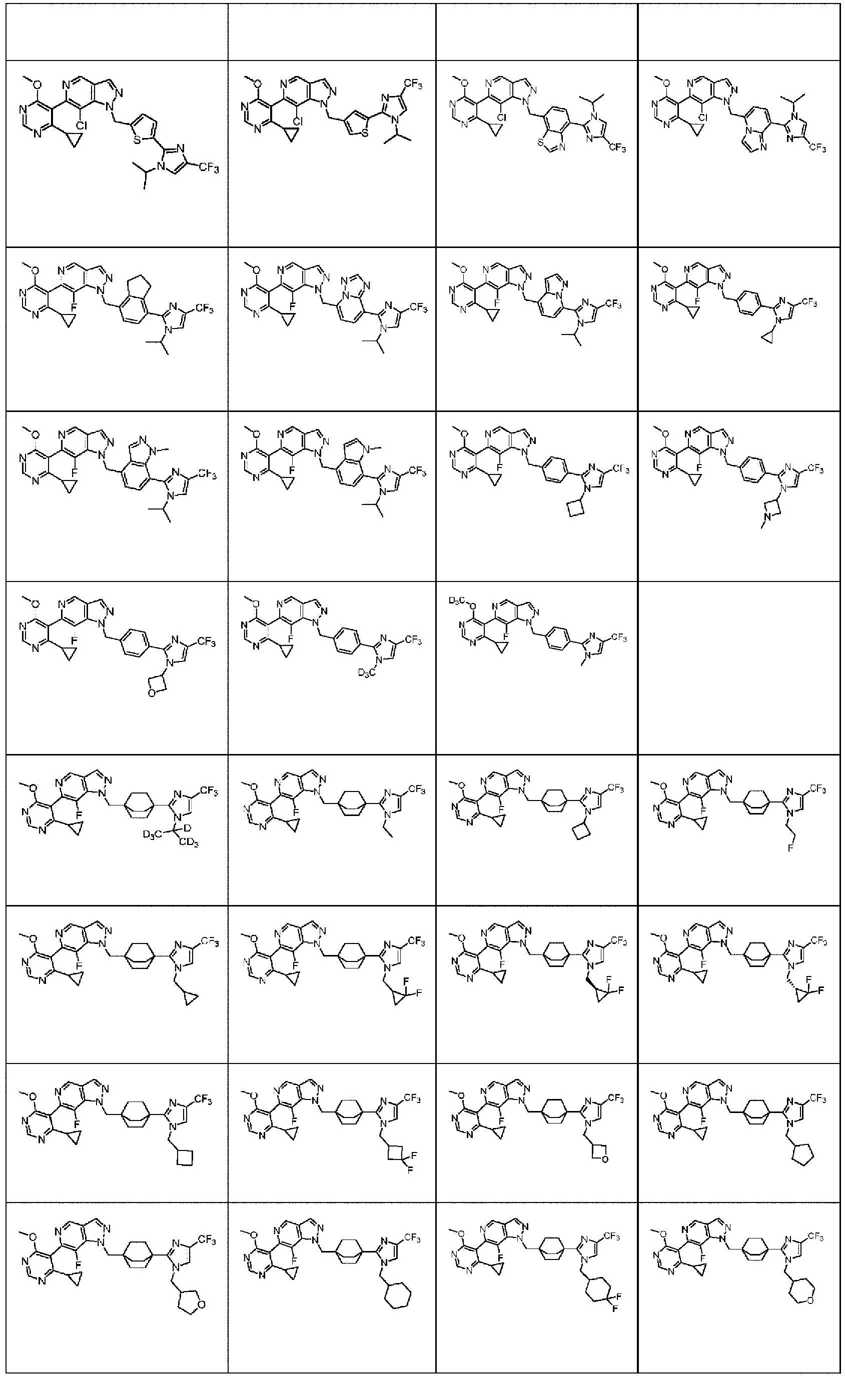

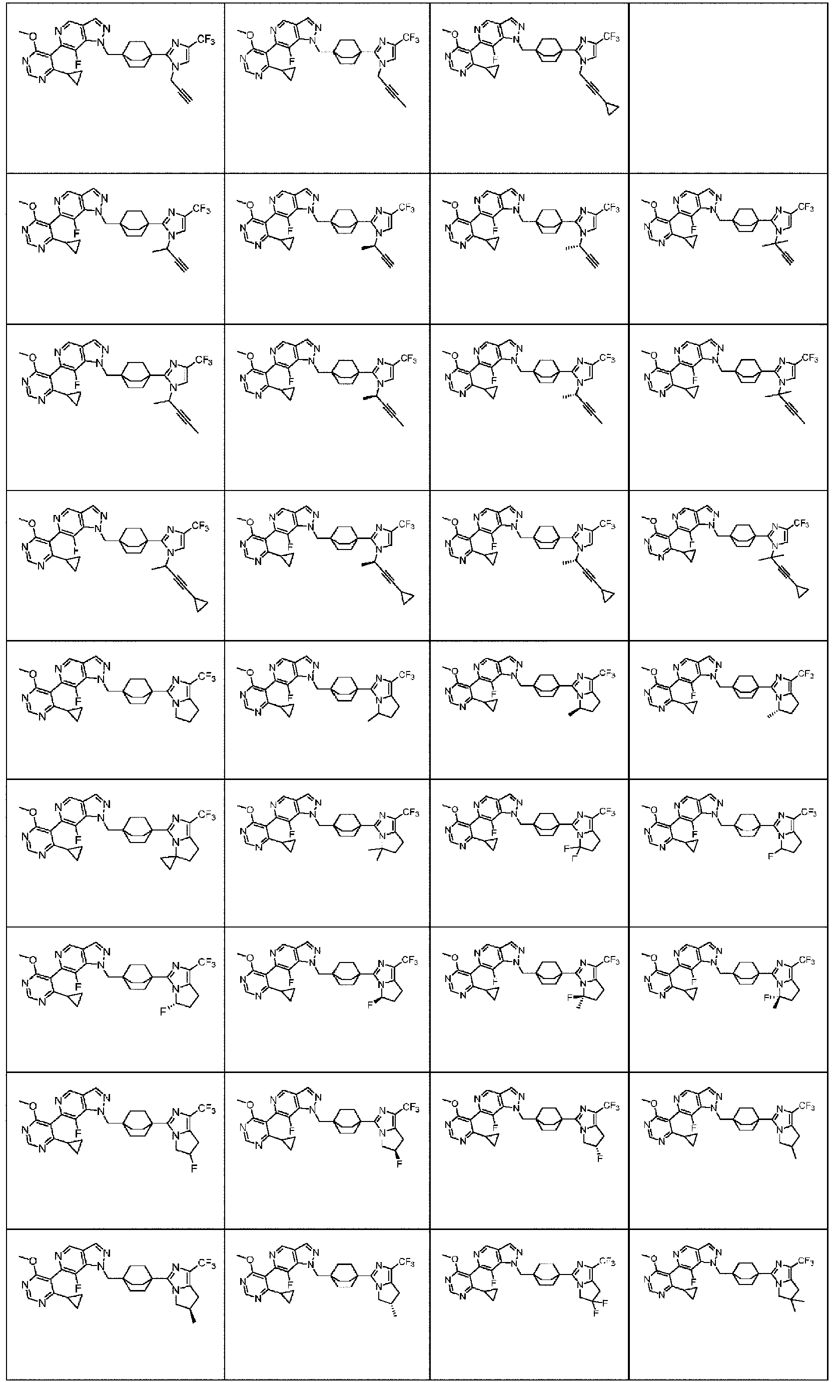

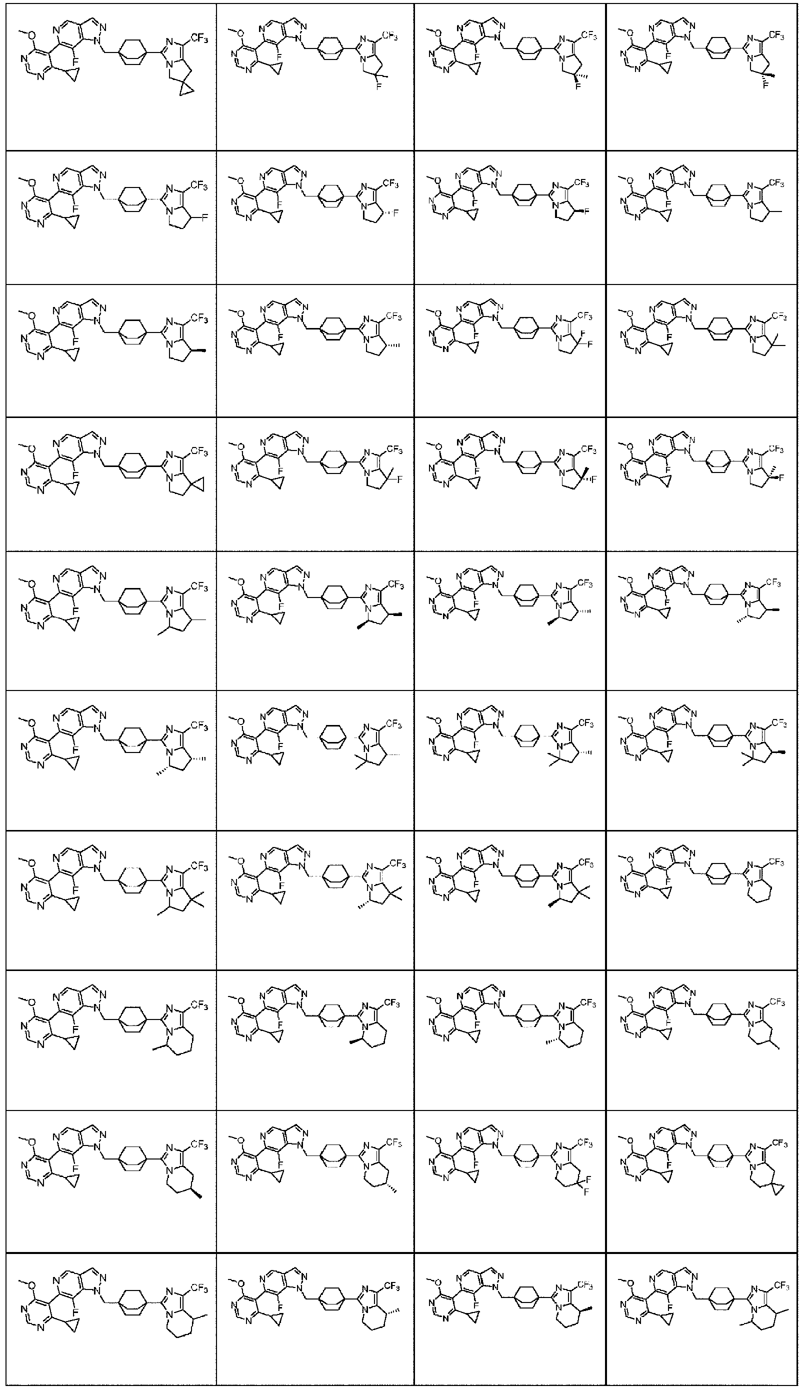

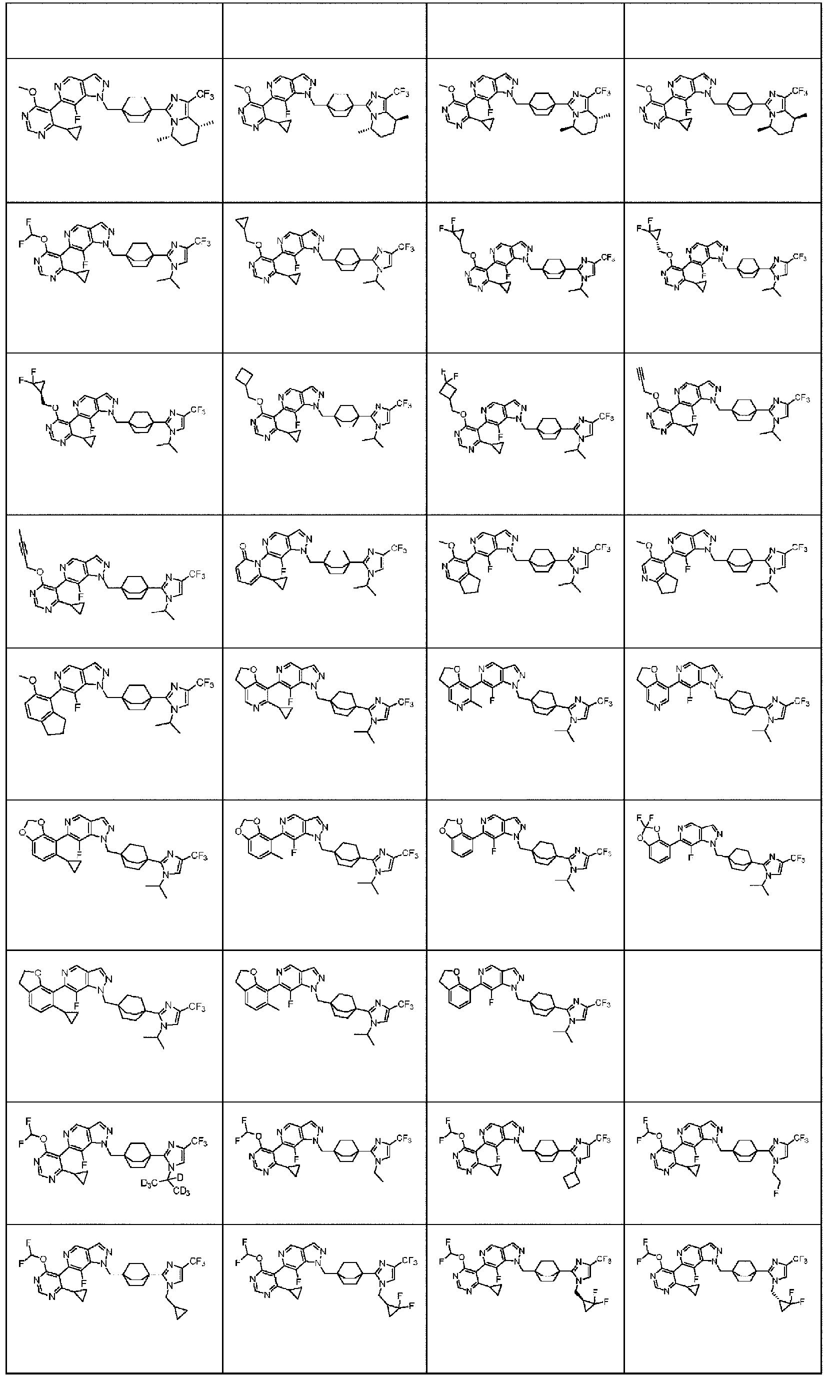

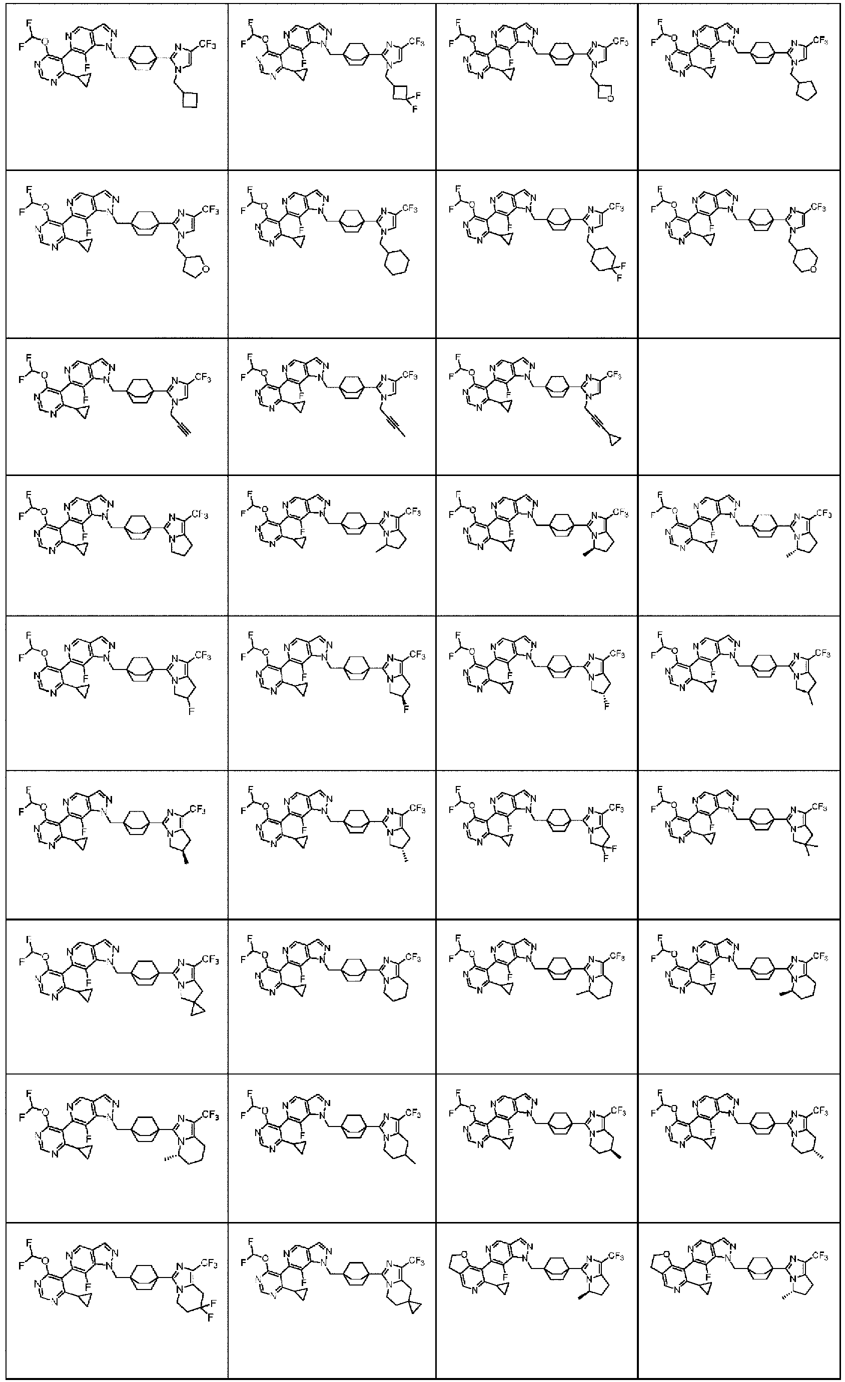

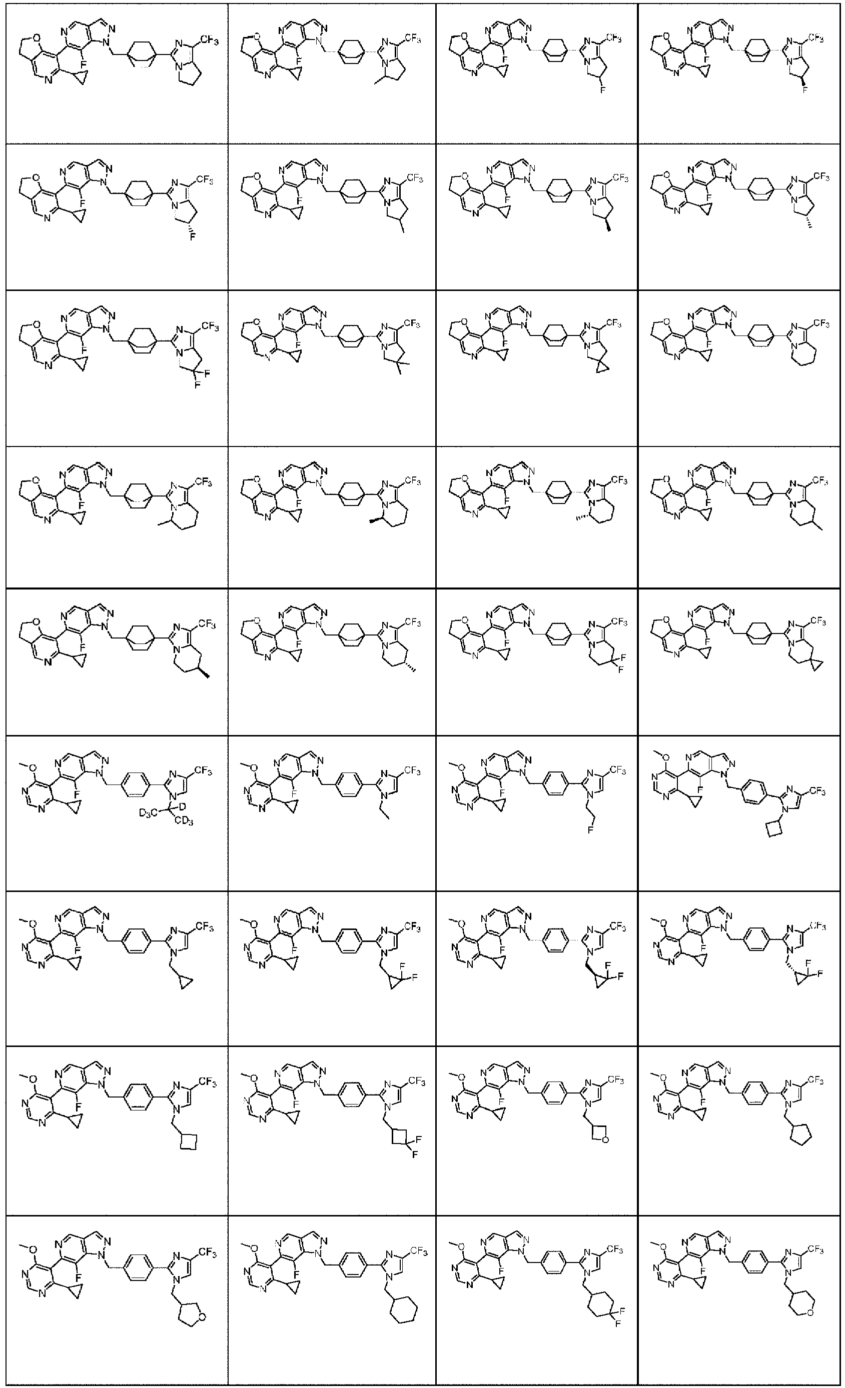

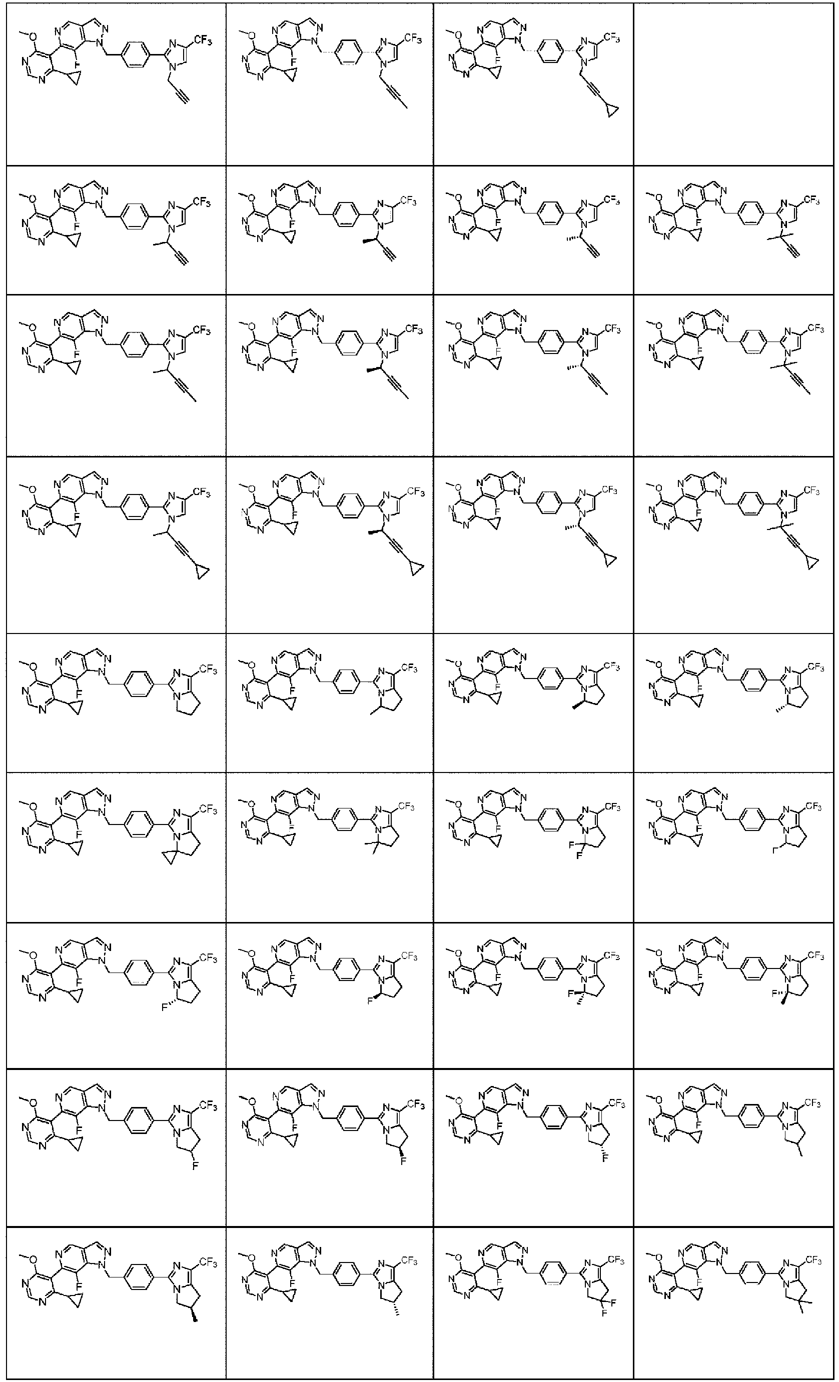

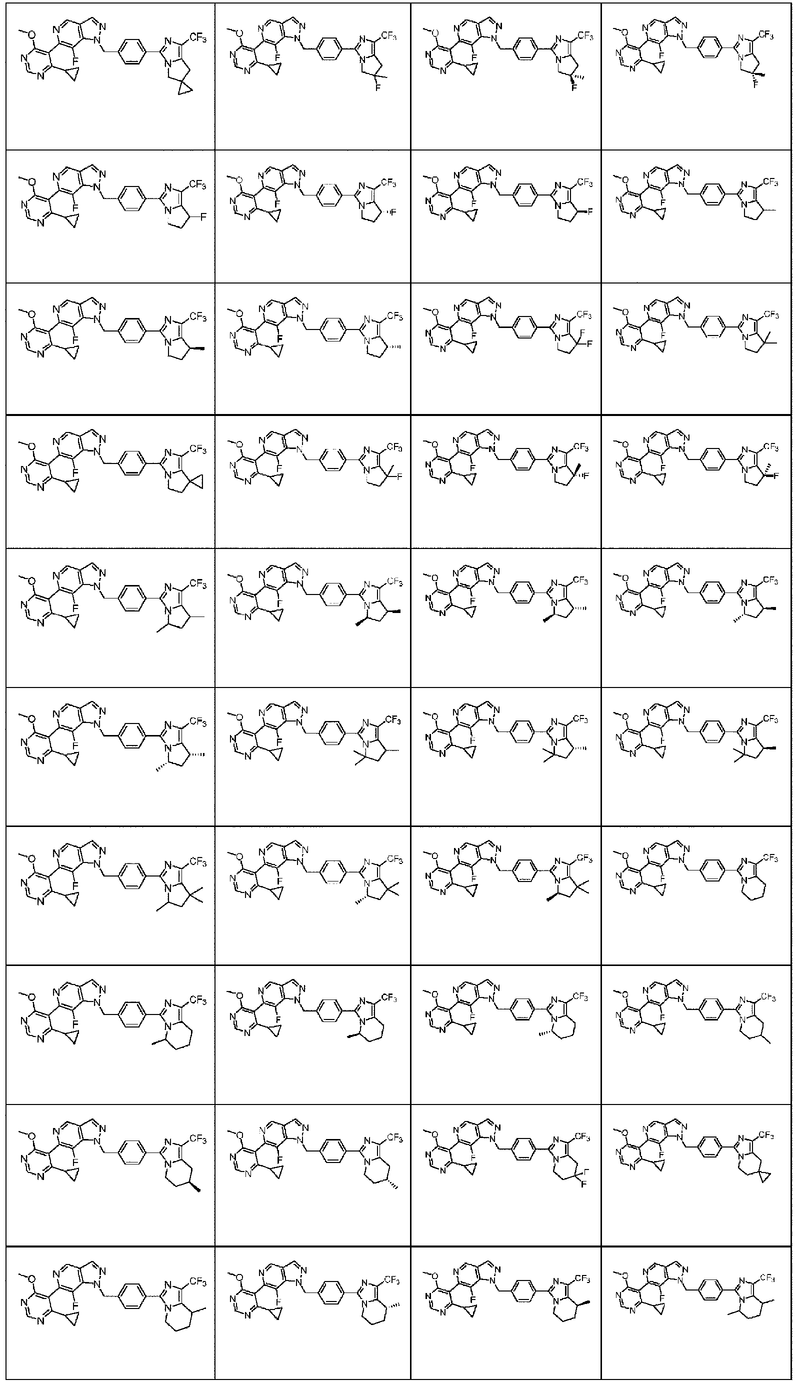

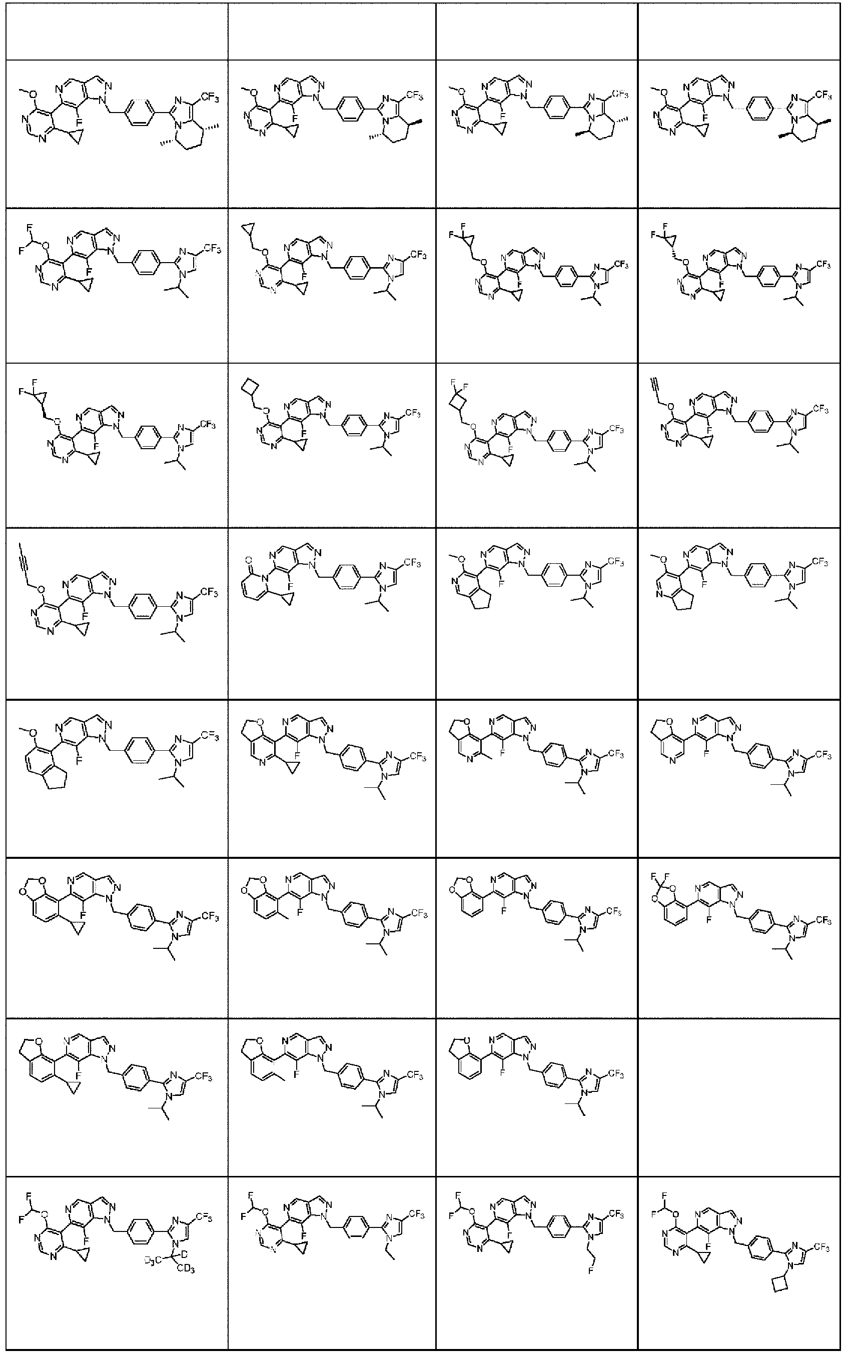

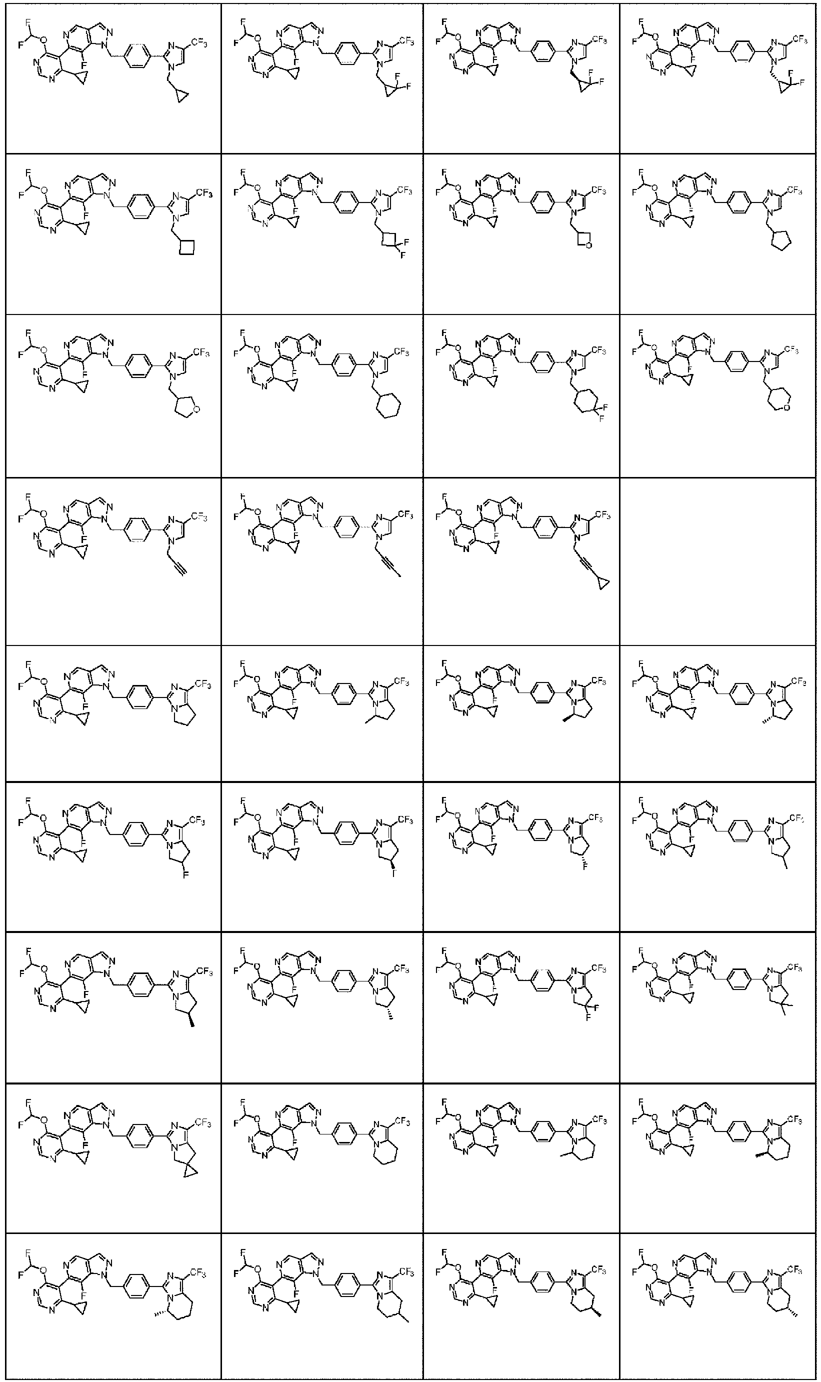

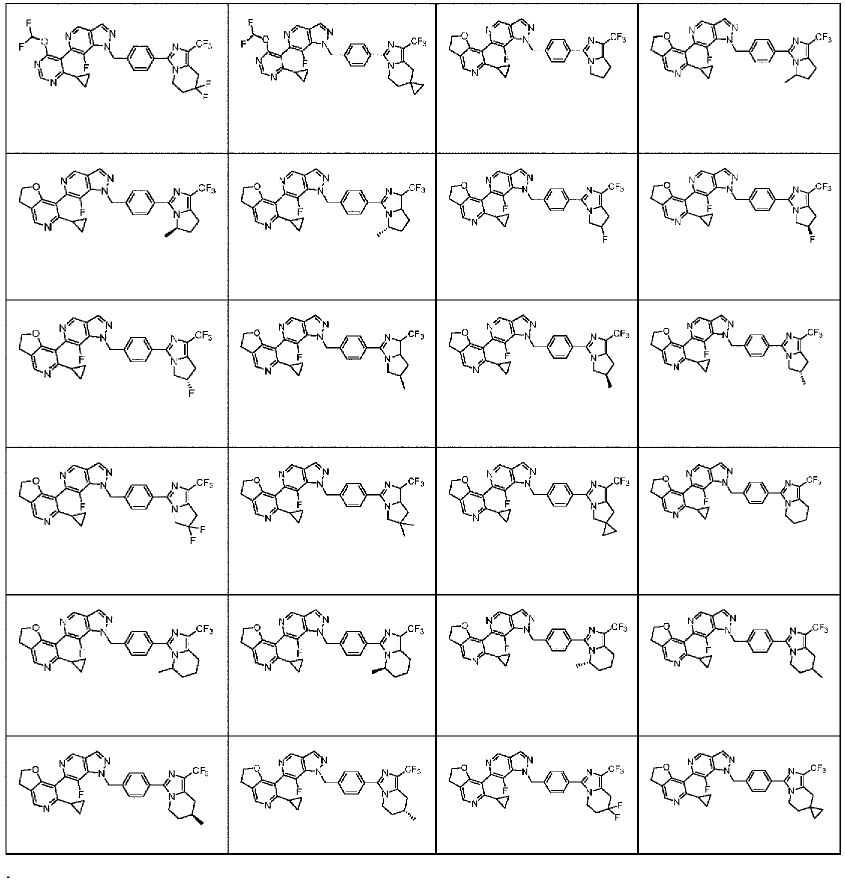

7. Pharmazeutische Zusammensetzung, **gekennzeichnet durch** das Umfassen der Verbindung oder des Stereoisomers, Tautomers, der deuterierten Substanz, des Solvats, des pharmazeutisch verträglichen Salzes oder des eutektischen Kristalls davon nach einem der Ansprüche 1 bis 6 und eines pharmazeutisch verträglichen Trägers.

8. Pharmazeutische Zusammensetzung oder pharmazeutisches Präparat, **gekennzeichnet durch** das Umfassen von 1-1500 mg der Verbindung oder des Stereoisomers, der deuterierten Substanz, des Solvats, des pharmazeutisch verträglichen Salzes oder des eutektischen Kristalls davon nach einem der Ansprüche 1 bis 6 und eines pharmazeutischen Hilfsstoffs.

9. Verbindung oder Stereoisomer, Tautomer, deuterierte Substanz, Solvat, pharmazeutisch verträgliches Salz oder eutektischer Kristall davon nach einem der Ansprüche 1 bis 6 zur Verwendung in einem Verfahren zur Behandlung einer Erkrankung, die mit der Aktivität oder Expression von USP1 in Zusammenhang steht.

10. Verbindung oder Stereoisomer, deuterierte Substanz, Solvat, pharmazeutisch verträgliches Salz oder eutektischer Kristall davon zur Verwendung nach Anspruch 9, **dadurch gekennzeichnet, dass** die Krankheit aus Tumoren ausgewählt ist.

11. Verbindung oder Stereoisomer, deuterierte Substanz, Solvat, pharmazeutisch verträgliches Salz oder eutektischer Kristall davon zur Verwendung nach Anspruch 9, wobei eine therapeutisch wirksame Menge der Verbindung 1-1500 mg beträgt.

## Revendications

1. Composé ou stéréoisomère, tautomère, substance deutérée, solvate, sel pharmaceutiquement acceptable ou cristal eutectique de celui-ci,
**caractérisé en ce que** le composé est choisi parmi un composé représenté par la formule générale (I), 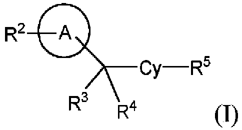
dans lequel le cycle A est choisi parmi 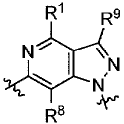 et le côté gauche est relié à R² ;
R¹ et R⁹ sont chacun indépendamment choisis parmi H, halogène, cyano, alkyle en C₁₋₆, alcényle en C₂₋₆, alcynyle en C₂₋₆ et alcoxy en C₁₋₆, dans lequel l'alkyle, l'alcényle, l'alcynyle ou l'alcoxy est éventuellement en outre substitué par 0 à 4 substituants choisis parmi H, halogène, OH, =O, cyano, NH₂, alkyle en C₁₋₆, alcynyle en C₂₋₆, alcoxy en C₁₋₆, alkyle en C₁₋₆ substitué par halogène, alkyle en C₁₋₆ substitué par hydroxyle ou alkyle en C₁₋₆ substitué par cyano ;
R⁸ est choisi parmi halogène, cyano, alkyle en C₁₋₆, alcényle en C₂₋₆, alcynyle en C₂₋₆ et alcoxy en C₁₋₆, dans lequel l'alkyle, l'alcényle, l'alcynyle ou l'alcoxy est éventuellement en outre substitué par 0 à 4 substituants choisis parmi H, halogène, OH, =O, cyano, NH₂, alkyle en C₁₋₆, alcynyle en C₂₋₆, alcoxy en C₁₋₆, alkyle en C₁₋₆ substitué par halogène, alkyle en C₁₋₆ substitué par hydroxyle ou alkyle en C₁₋₆ substitué par cyano ;
R² est choisi parmi carbocycle en C₆₋₁₀ ou hétérocycle à 5 à 10 chaînons, dans lequel le carbocycle ou l'hétérocycle est éventuellement en outre substitué par 0 à 4 R^{2a} ;
chaque R^{2a} est indépendamment choisi parmi H, un halogène, OH, =O, cyano, NH₂, NH(alkyle en C₁₋₆), N(alkyle en C₁₋₆)₂, -SO₂-alkyle en C₁₋₆, -SO₂-cycloalkyle en C₃₋₆, alkyle en C₁₋₆, alcoxy en C₁₋₆, alkylthio en C₁₋₆, -Z-cycloalkyle en C₃₋₆ ou -Z-hétérocycle à 3 à 8 chaînons, dans lequel l'alkyle, le cycloalkyle, l'alcoxy, l'alkylthio ou l'hétérocycle est éventuellement en outre substitué par 0 à 4 substituants choisis parmi H, D, un halogène, OH, =O, cyano, NH₂, alkyle en C₁₋₆, alcynyle en C₂₋₆, alcoxy en C₁₋₆ ou cycloalkyle en C₃₋₆, et l'hétérocycle contient 1 à 4 hétéroatomes choisis parmi O, S et N ;
R³ et R⁴ sont chacun indépendamment choisis parmi H, un halogène, OH, cyano, NH₂, un alkyle en C₁₋₆, un alcényle en C₂₋₆, un alcynyle en C₂₋₆ ou un alcoxy en C₁₋₆, dans lequel l'alkyle, l'alcényle, l'alcynyle ou l'alcoxy est éventuellement en outre substitué par 0 à 4 substituants choisis parmi H, halogène, OH, =O, cyano, NH₂, alkyle en C₁₋₆, alcynyle en C₂₋₆, alcoxy en C₁₋₆, alkyle en C₁₋₆ substitué par halogène, alkyle en C₁₋₆ substitué par hydroxyle, alkyle en C₁₋₆ substitué par cyano, cycloalkyle en C₃₋₆ ou hétérocyclyle à 3 à 8 chaînons, et l'hétérocyclyle contient 1 à 4 hétéroatomes choisis parmi O, S et N ;
Cy est choisi parmi carbocycle en C₃₋₁₂ ou hétérocycle à 4 à 12 chaînons, et le Cy est éventuellement en outre substitué par 0 à 4 substituants choisis parmi H, halogène, OH, =O, cyano, NH₂, alkyle en C₁₋₆, alcynyle en C₂₋₆, alcoxy en C₁₋₆, alkyle en C₁₋₆ substitué par halogène, alkyle en C₁₋₆ substitué par hydroxyle, alkyle en C₁₋₆ substitué par cyano, cycloalkyle en C₃₋₆ ou hétérocyclyle à 3 à 8 chaînons, dans lequel l'hétérocycle contient 1 à 4 hétéroatomes choisis parmi O, S et N ;
R⁵ est choisi parmi -Z-carbocycle en C₃₋₁₂ ou -Z-hétérocycle à 4 à 12 chaînons, et le R⁵ est éventuellement en outre substitué par 0 à 4 R^{5a} ;
chaque R^{5a} est indépendamment choisi parmi H, halogène, OH, cyano, NH₂, NH-(alkyle en C₁₋₆), N-(alkyle en C₁₋₆)₂, alkyle en C₁₋₆, alcynyle en C₂₋₆, alcoxy en C₁₋₆, -alkylène en C₁₋₄-cycloalkyle en C₃₋₆, -alkylène en C₁₋₄-hétérocycle à 3 à 6 chaînons, cycloalkyle en C₃₋₆, hétérocycle à 3 à 8 chaînons, -CONH-(alkyle en C₁₋₆), -CONH-(alcoxy en C₁₋₆), -CONH-(cycloalkyle en C₃₋₆), -CONH-(hétérocycle à 3 à 8 chaînons), -NHCO-(alkyle en C₁₋₆), -NHCO-(alcoxy en C₁₋₆), - NHCO-(cycloalkyle en C₃₋₆) ou -NHCO-(hétérocycle à 3 à 8 chaînons), dans lequel l'alkyle, l'alkylène, l'alcynyle, l'alcoxy, le cycloalkyle ou l'hétérocycle est éventuellement en outre substitué par 0 à 4 substituants choisis parmi H, D, halogène, OH, =O, cyano, NH₂, -C(=O)NH₂, -C(=O)NH-(alkyle en C₁₋₆), -C(=O)N-(alkyle en C₁₋₆)₂, alkyle en C₁₋₆ , alcynyle en C₂₋₆, alcoxy en C₁₋₆, alkyle en C₁₋₆ substitué par halogène, alkyle en C₁₋₆ substitué par hydroxyle, alkyle en C₁₋₆ substitué par cyano, cycloalkyle en C₃₋₆, cycloalkyle en C₃₋₆ substitué par halogène ou un hétérocycle à 3 à 8 chaînons, et l'hétérocycle contient 1 à 4 hétéroatomes choisis parmi O, S et N ;
Z est choisi parmi une liaison, O, S, NH, N(alkyle en C₁₋₄), C(=O)NH, NHC(=O), -OCH₂-, -CH₂O- ou alkylène en C₁₋₃.

2. Composé ou stéréoisomère, tautomère, substance deutérée, solvate, sel pharmaceutiquement acceptable ou cristal eutectique de celui-ci selon la revendication 1, **caractérisé en ce que** le composé représenté par la formule générale (I) est choisi parmi un composé représenté par la formule générale (la), 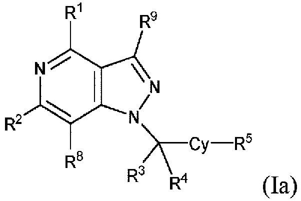
R¹ et R⁹ sont chacun indépendamment choisis parmi H, halogène, cyano, alkyle en C₁₋₄, alcényle en C₂₋₄, alcynyle en C₂₋₄ et alcoxy en C₁₋₄, dans lequel l'alkyle, l'alcényle, l'alcynyle ou l'alcoxy est éventuellement en outre substitué par 0 à 4 substituants choisis parmi H, halogène, OH, =O, cyano, NH₂, alkyle en C₁₋₄, alcynyle en C₂₋₄, alcoxy en C₁₋₄, alkyle en C₁₋₄ substitué par halogène, alkyle en C₁₋₄ substitué par hydroxyle ou alkyle en C₁₋₄ substitué par cyano ;
R⁸ est choisi parmi halogène, cyano, alkyle en C₁₋₄, alcynyle en C₂₋₄ et alcoxy en C₁₋₄, dans lequel l'alkyle, l'alcynyle ou l'alcoxy est éventuellement en outre substitué par 0 à 4 substituants choisis parmi H, halogène, OH, cyano, NH₂, alkyle en C₁₋₄, alcynyle en C₂₋₄ et alcoxy en C₁₋₄ ;
chaque R^{2a} est indépendamment choisi parmi H, halogène, OH, =O, cyano, NH₂, NH(alkyle en C₁₋₄), N(alkyle en C₁₋₄)₂, -SO₂-alkyle en C₁₋₄, -SO₂-cycloalkyle en C₃₋₆, alkyle en C₁₋₄, alcoxy en C₁₋₄, alkylthio en C₁₋₄, -Z-cycloalkyle en C₃₋₆ ou -Z-hétérocycle à 3 à 6 chaînons, dans lequel l'alkyle, le cycloalkyle, l'alcoxy, l'alkylthio ou l'hétérocycle est éventuellement en outre substitué par 0 à 4 substituants choisis parmi H, D, halogène, OH, =O, cyano, NH₂, alkyle en C₁₋₄, alcynyle en C₂₋₄, alcoxy en C₁₋₄ ou cycloalkyle en C₃₋₆, et l'hétérocycle contient 1 à 4 hétéroatomes choisis parmi O, S et N ;
R³ et R⁴ sont chacun indépendamment choisis parmi H, halogène, OH, cyano et alkyle en C₁₋₄ ;
Cy est choisi parmi cycloalkyle monocyclique en C₃₋₆, cycloalkyle condensé en C₅₋₁₁, spirocycloalkyle en C₅₋₁₁, cycloalkyle ponté en C₅₋₁₁, hétérocycloalkyle monocyclique à 4 à 7 chaînons, hétérocycloalkyle condensé à 6 à 11 chaînons, spirohétérocycloalkyle à 6 à 11 chaînons, hétérocycloalkyle ponté à 6 à 11 chaînons, cycle benzénique, benzocarbocycle en C₄₋₆, benzohétérocycle à 4 à 6 chaînons, cycle hétéroaromatique à 5 à 6 chaînons ou cycle hétéroaromatique condensé à 8 à 10 chaînons, et le Cy est éventuellement en outre substitué par 0 à 4 substituants choisis parmi H, halogène, OH, =O, cyano, NH₂, alkyle en C₁₋₄, alcynyle en C₂₋₄, alcoxy en C₁₋₄, alkyle en C₁₋₄ substitué par halogène, alkyle en C₁₋₄ substitué par hydroxyle, alkyle en C₁₋₄ substitué par cyano, cycloalkyle en C₃₋₆ ou hétérocycle à 3 à 8 chaînons, dans lequel l'hétérocycle contient 1 à 4 hétéroatomes choisis parmi O, S et N ;
R⁵ est choisi parmi -Z-carbocycle en C₆₋₁₂ ou -Z-hétérocycle à 5 à 12 chaînons, et le R⁵ est éventuellement en outre substitué par 0 à 4 R^{5a} ;
chaque R^{5a} est indépendamment choisi parmi H, halogène, OH, cyano, NH₂, alkyle en C₁₋₄, alcynyle en C₂₋₄, alcoxy en C₁₋₄, -alkylène en C₁₋₂-cycloalkyle en C₃₋₆, -alkylène en C₁₋₂-hétérocycle à 3 à 6 chaînons, cycloalkyle en C₃₋₆ ou hétérocycle à 3 à 6 chaînons, dans lequel l'alkyle, l'alkylène, l'alcynyle, l'alcoxy, le cycloalkyle ou l'hétérocycle est éventuellement en outre substitué par 0 à 4 substituants choisis parmi H, D, halogène, OH, =O, cyano, NH₂, -C(=O)NH₂, -C(=O)NHalkyle en C₁₋₄, -C(=O)N(alkyle en C₁₋₄)₂, alkyle en C₁₋₄, alcynyle en C₂₋₄, alcoxy en C₁₋₄, alkyle en C₁₋₄ substitué par halogène, alkyle en C₁₋₄ substitué par hydroxyle, alkyle en C₁₋₄ substitué par cyano, cycloalkyle en C₃₋₆, cycloalkyle en C₃₋₆ substitué par halogène ou hétérocycle à 3 à 8 chaînons, et l'hétérocycle contient 1 à 4 hétéroatomes choisis parmi O, S et N.

3. Composé ou stéréoisomère, tautomère, substance deutérée, solvate, sel pharmaceutiquement acceptable ou cristal eutectique de celui-ci selon la revendication 2, **caractérisé en ce que**
R¹ et R⁹ sont chacun indépendamment choisis parmi H, F, CI, Br, I, cyano, méthyle ou éthyle ;
R⁸ est choisi parmi F, CI, Br, I, CN, méthyle, éthyle, éthynyle, méthoxy ou éthoxy ;
R² est choisi parmi phényle, pyridine, pyrimidine, pyrrole, pyrazole, imidazole, furane, thiophène, thiazole ou oxazole, dans lequel le phényle, la pyridine, la pyrimidine, le pyrrole, le pyrazole, l'imidazole, le furane, le thiophène, le thiazole ou l'oxazole est éventuellement en outre substitué par 0 à 4 R^{2a} ;
ou R² est choisi parmi 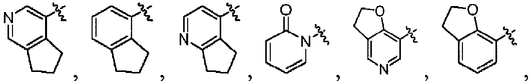 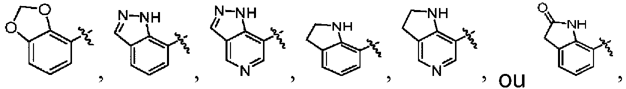 et R² est éventuellement en outre substitué par 0 à 4 R^{2a} ;
chaque R^{2a} est indépendamment choisi parmi H, F, CI, Br, I ou cyano, ou chaque R^{2a} est indépendamment choisi parmi l'un des groupes substitués ou non substitués suivants : -SO₂-méthyle, -SO₂-éthyle, -SO₂-propyle, -SO₂-isopropyle, - SO₂-cyclopropyle, méthyle, éthyle, propyle, isopropyle, méthoxy, éthoxy, propoxy, méthylthio, éthylthio, propylthio, cyclopropyle, cyclobutyle, cyclopentyle, cyclohexyle, azétidinyle, azacyclopentyle, azacyclohexyle, pipérazine, oxétanyle, oxacyclopentyle, oxacyclohexyle, morpholine, -O-azétidinyle, -O-azacyclopentyle, -O-azacyclohexyle, -O-oxétanyle, -O-oxacyclopentyle, -O-oxacyclohexyle, -OCH₂-phényle, -OCH₂-cyclopropyle, -OCH₂-cyclobutyle, -OCH₂-cyclopentyle, -OCH₂-cyclohexyle, pyrrole, pyrazole, imidazole, thiazole, thiophène, furane et oxazole, qui, lorsqu'ils sont substitués, sont éventuellement en outre substitués par 0 à 4 substituants choisis parmi H, D, un halogène, OH, =O, cyano, NH₂, alkyle en C₁₋₄, alcynyle en C₂₋₄, alcoxy en C₁₋₄ et cycloalkyle en C₃₋₆ ;
R³ et R⁴ sont chacun indépendamment choisis parmi H, F, CI, Br, I, méthyle et éthyle ;
Cy est choisi parmi cyclopropyle, cyclobutyle, cyclopentyle, cyclohexyle, bicyclo[2.2.2]octyle, bicyclo[1.1.1]pentyle, cyclopentyle fusionné à un cyclopentyle, cyclobutylspirocyclohexyle, cyclobutylspirocyclopentyle, azétidinyle, azacyclopentyle, azacyclohexyle, azétidinylspirocyclobutyle, azétidinylspirocyclopentyle, azétidinylspirocyclohexyle, azétidinylspiroazétidinyle, azétidinylspiroazacyclopentyle, 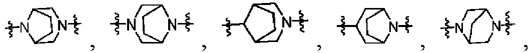 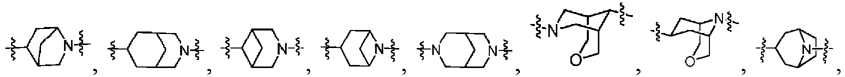 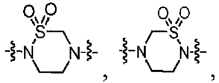 cycle benzénique, thiophène, furane, pyrrole, thiazole, oxazole, pyrazole, pyrazine, pyrimidine, 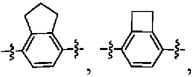 benzothiazole, benzothiophène, benzofurane, benzoxazole, benzopyrrole, benzopyrazole, benzimidazole, indolizine, pyridinopyrrole, pyridinoimidazole, pyridinopyrazole, pyrimidoimidazole, pyrimidopyrazole, pyridinotriazole ou 2-pyridone, et le Cy est éventuellement en outre substitué par 0 à 4 substituants choisis parmi H, halogène, OH, =O, cyano, NH₂, alkyle en C₁₋₄, alcynyle en C₂₋₄, alcoxy en C₁₋₄, alkyle en C₁₋₄ substitué par halogène, alkyle en C₁₋₄ substitué par hydroxyle, alkyle en C₁₋₄ substitué par cyano, cycloalkyle en C₃₋₆ ou hétérocycle à 3 à 8 chaînons, dans lequel l'hétérocycle contient 1 à 4 hétéroatomes choisis parmi O, S et N ;
R⁵ est choisi parmi cycle benzénique, pyrazole, pyrrole, imidazole, triazole, pyridine, pyrimidine, pyrazine ou pyridazine, et le R⁵ est éventuellement en outre substitué par 0 à 4 R^{5a} ;
ou R⁵ est choisi parmi 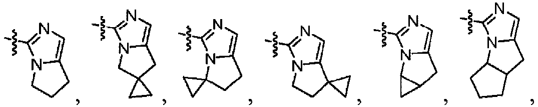 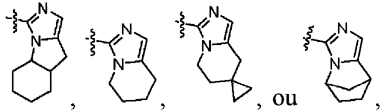 et le R⁵ est éventuellement en outre substitué par 0 à 4 R^{5a} ;
chaque R^{5a} est indépendamment choisi parmi H, F, CI, Br, I, cyano, méthyle, éthyle, propyle, isopropyle, propargyle, butargyle, méthoxy, éthoxy, cyclopropyle, cyclobutyle, cyclopentyle, cyclohexyle, oxétanyle, oxacyclopentyle, oxacyclohexyle, azétidinyle, azacyclopentyle, azacyclohexyle, -CH₂-cyclopropyle, - CH₂-cyclobutyle, -CH₂-cyclopentyle, -CH₂-cyclohexyle, -CH₂-oxétanyle, -CH₂-oxacyclopentyle, -CH₂-oxacyclohexyle, -CH₂-azétidinyle, -CH₂-azacyclopentyle ou -CH₂-azacyclohexyle, dans lequel le méthyle, éthyle, propyle, isopropyle, propargyle, butargyle, méthoxy, éthoxy, cyclopropyle, cyclobutyle, cyclopentyle, cyclohexyle, oxétanyle, oxacyclopentyle, oxacyclohexyle, azétidinyle, azacyclopentyle ou azacyclohexyle est éventuellement en outre substitué par 0 à 4 substituants choisis parmi H, D, halogène, OH, =O, cyano, NH₂, alkyle en C₁₋₄, alcynyle en C₂₋₄, alcoxy en C₁₋₄, cycloalkyle en C₃₋₆ ou cycloalkyle en C₃₋₆ substitué par halogène.

4. Composé ou stéréoisomère, tautomère, substance deutérée, solvate, sel pharmaceutiquement acceptable ou cristal eutectique de celui-ci selon la revendication 3, **caractérisé en ce que**
R¹ et R⁹ sont H ;
R⁸ est choisi parmi F, CI, Br, CN, méthyle, éthynyle ou méthoxy ;
R²est choisi parmi pyrrole, pyrazole, imidazole, pyrimidine, 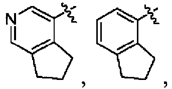 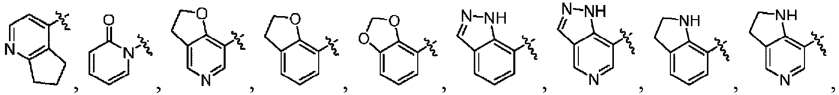 ou 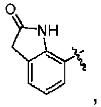 dans lequel le pyrrole, le pyrazole, l'imidazole, la pyrimidine, 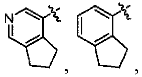 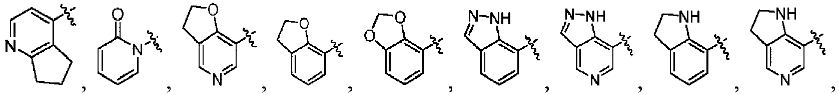 ou 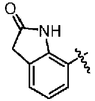 est éventuellement en outre substitué par 0 à 4 substituants choisis parmi H, F, CI, Br, I, cyano, -SO₂-méthyle, -SO₂-éthyle, -SO₂-propyle, -SO₂-isopropyle, - SO₂-cyclopropyle, CH₂F, CHF₂, CF₃, -OCH₂F, -OCHF₂, -OCF₃, méthyle, éthyle, propyle, isopropyle, méthoxy, éthoxy, propoxy, -OCD₃, méthylthio, éthylthio, propylthio, cyclopropyle, cyclobutyle, cyclopentyle, cyclohexyle, azétidinyle, azacyclopentyle, azacyclohexyle, pipérazine, oxétanyle, oxacyclopentyle, oxacyclohexyle, morpholine, -O-azétidinyle, -O-azacyclopentyle, -O-azacyclohexyle, -O-oxétanyle, -O-oxacyclopentyle, -O-oxacyclohexyle, -OCH₂-phényle, -OCH₂-cyclopropyle, -OCH₂-cyclobutyle, -OCH₂-cyclopentyle, -OCH₂-cyclohexyle, 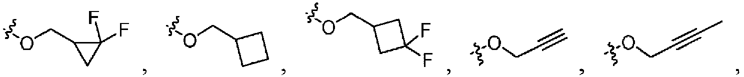 pyrrole, pyrazole, imidazole, thiazole, thiophène, furane, oxazole, 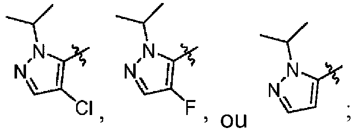
Cy est choisi parmi 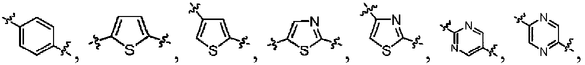 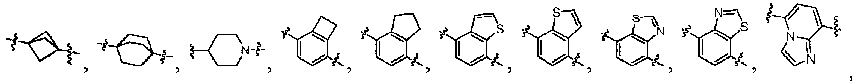 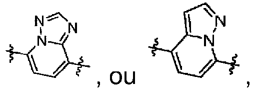 et le Cy est éventuellement en outre substitué par 0 à 4 substituants choisis parmi H, F, CI, Br, I, cyano, CH₂F, CHF₂, CF₃, méthyle, éthyle, propyle, isopropyle, méthoxy, éthoxy ou cyclopropyle ;
alternativement, Cy est choisi parmi 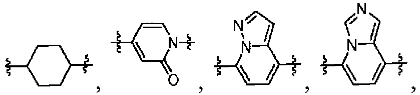 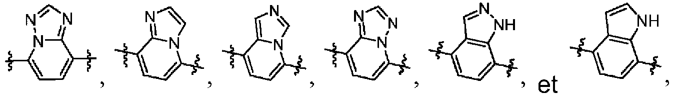 et le Cy est éventuellement en outre substitué par 0 à 4 substituants choisis parmi H, F, CI, Br, I, cyano, CH₂F, CHF₂, CF₃, méthyle, éthyle, propyle, isopropyle, méthoxy, éthoxy ou cyclopropyle ;
R⁵ est choisi parmi pyrrole, pyrazole ou imidazole, et le pyrrole, le pyrazole ou l'imidazole est éventuellement en outre substitué par 0 à 4 substituants choisis parmi H, F, CI, Br, I, cyano, CH₂F, CHF₂, CF₃, CD₃, méthyle, éthyle, propyle, isopropyle, -CH₂CH₂F, méthoxy, éthoxy, cyclopropyle, cyclobutyle, cyclopentyle, cyclohexyle, oxétanyle, oxacyclopentyle, oxacyclohexyle, azétidinyle, N-méthylazétidinyle, azacyclopentyle, azacyclohexyle, 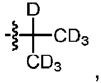 -CH₂-cyclopropyle, - CH₂-cyclobutyle, -CH₂-cyclopentyle, -CH₂-cyclohexyle, -CH₂-oxétanyle, -CH₂-oxacyclopentyle, -CH₂-oxacyclohexyle, -CH₂-azétidinyle, -CH₂-azacyclopentyle, - CH₂-azacyclohexyle, 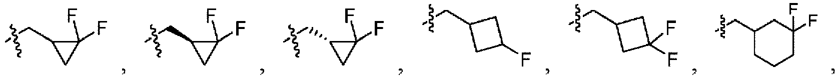 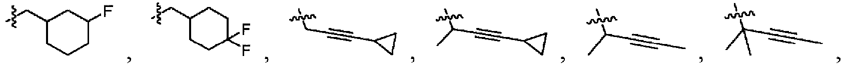 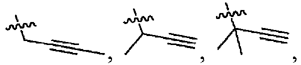 ou propargyle ;
alternativement, R⁵ est choisi parmi 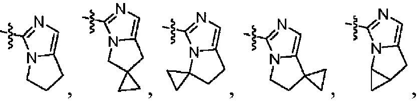 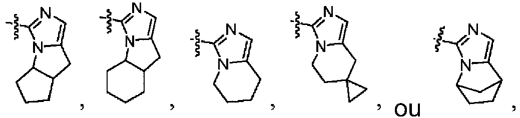 et R⁵ est éventuellement en outre substitué par 0 à 4 substituants choisis parmi H, F, CI, Br, I, cyano, CH₂F, CHF₂, CF₃, CD₃, méthyle, éthyle, propyle, isopropyle, -CH2CH2F, méthoxy, éthoxy, cyclopropyle, cyclobutyle, cyclopentyle, cyclohexyle, oxétanyle, oxacyclopentyle, oxacyclohexyle, azétidinyle, N-méthylazétidinyle, azacyclopentyle, azacyclohexyle, 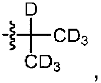 -CH₂-cyclopropyle, -CH₂-cyclobutyle, -CH₂-cyclopentyle, -CH₂-cyclohexyle, -CH₂-oxétanyle, -CH₂-oxacyclopentyle, -CH₂-oxacyclohexyle, - CH₂-azétidinyle, -CH₂-azacyclopentyle, -CH₂-azacyclohexyle, 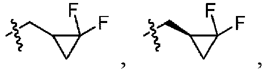 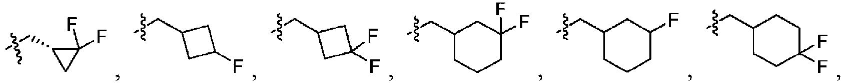 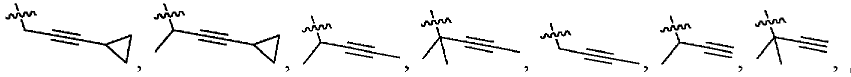 ou propargyle.

5. Composé ou stéréoisomère, tautomère, substance deutérée, solvate, sel pharmaceutiquement acceptable ou cristal eutectique de celui-ci selon la revendication 4, **caractérisé en ce que**
R² est choisi parmi 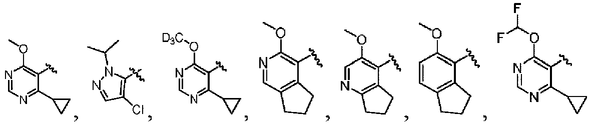 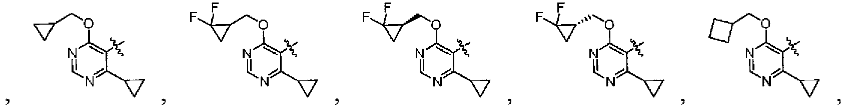 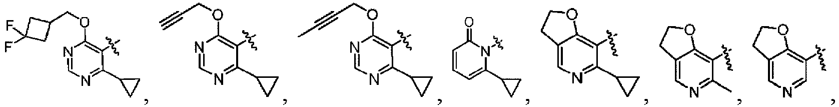 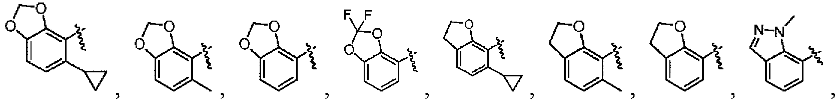 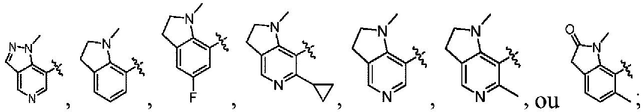
R⁵ est choisi parmi 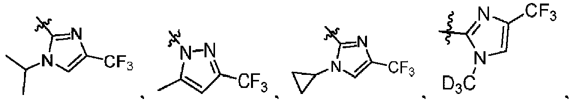 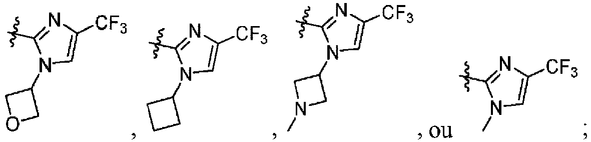
alternativement, R⁵ est choisi parmi 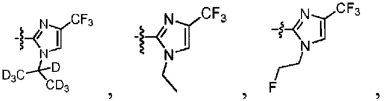 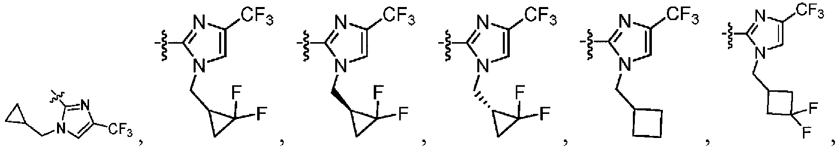 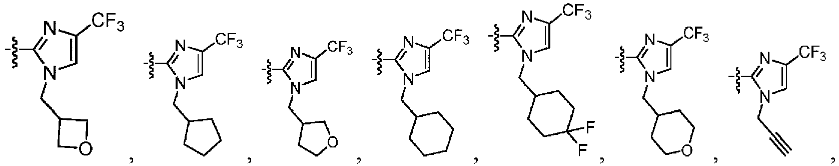 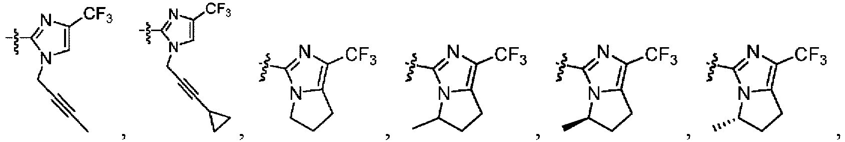 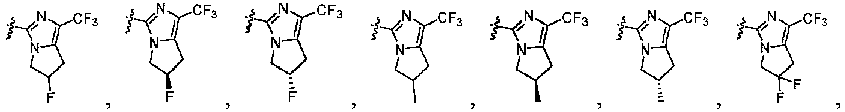 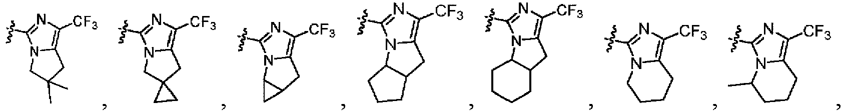 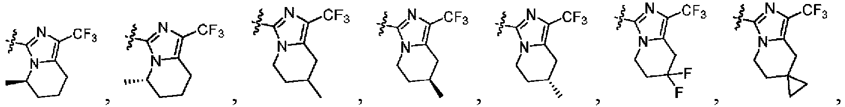 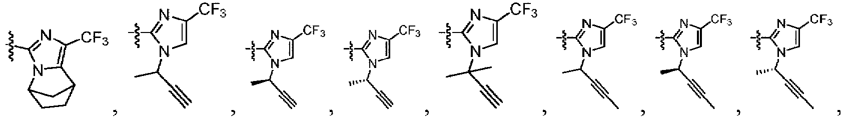 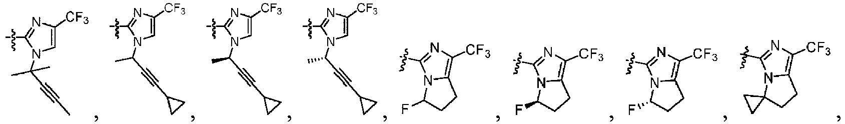 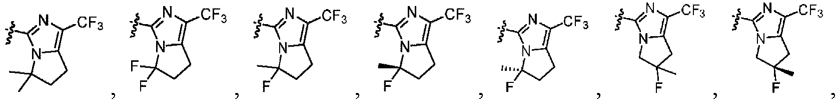 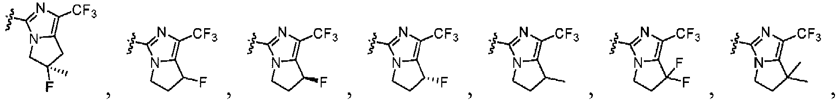 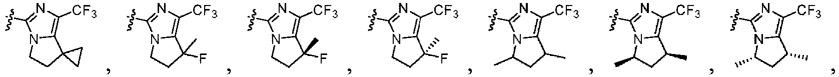 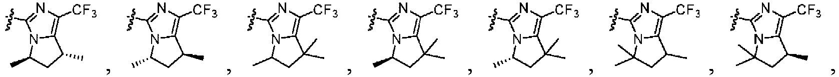 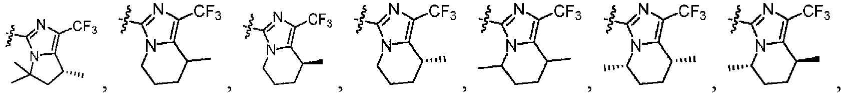 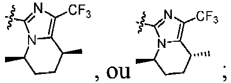
R⁸ est choisi parmi F ou CI.

6. Composé ou stéréoisomère, tautomère, substance deutérée, solvate, sel pharmaceutiquement acceptable ou cristal eutectique de celui-ci selon la revendication 1, **caractérisé en ce que** le composé est choisi parmi l'une des structures représentées dans le tableau suivant :

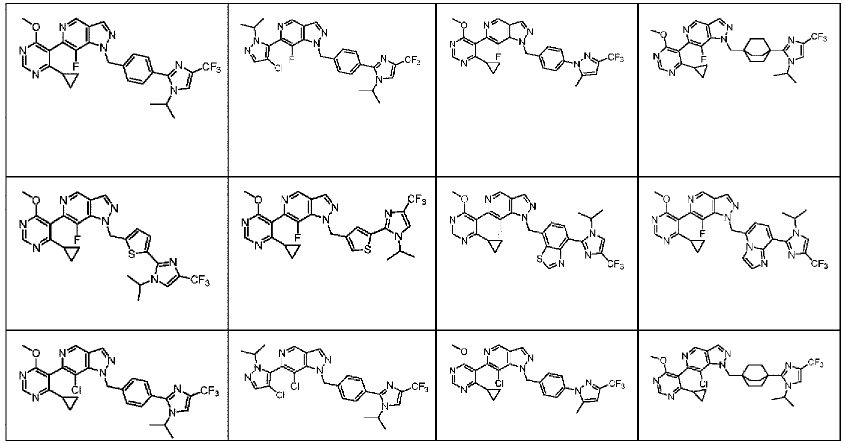

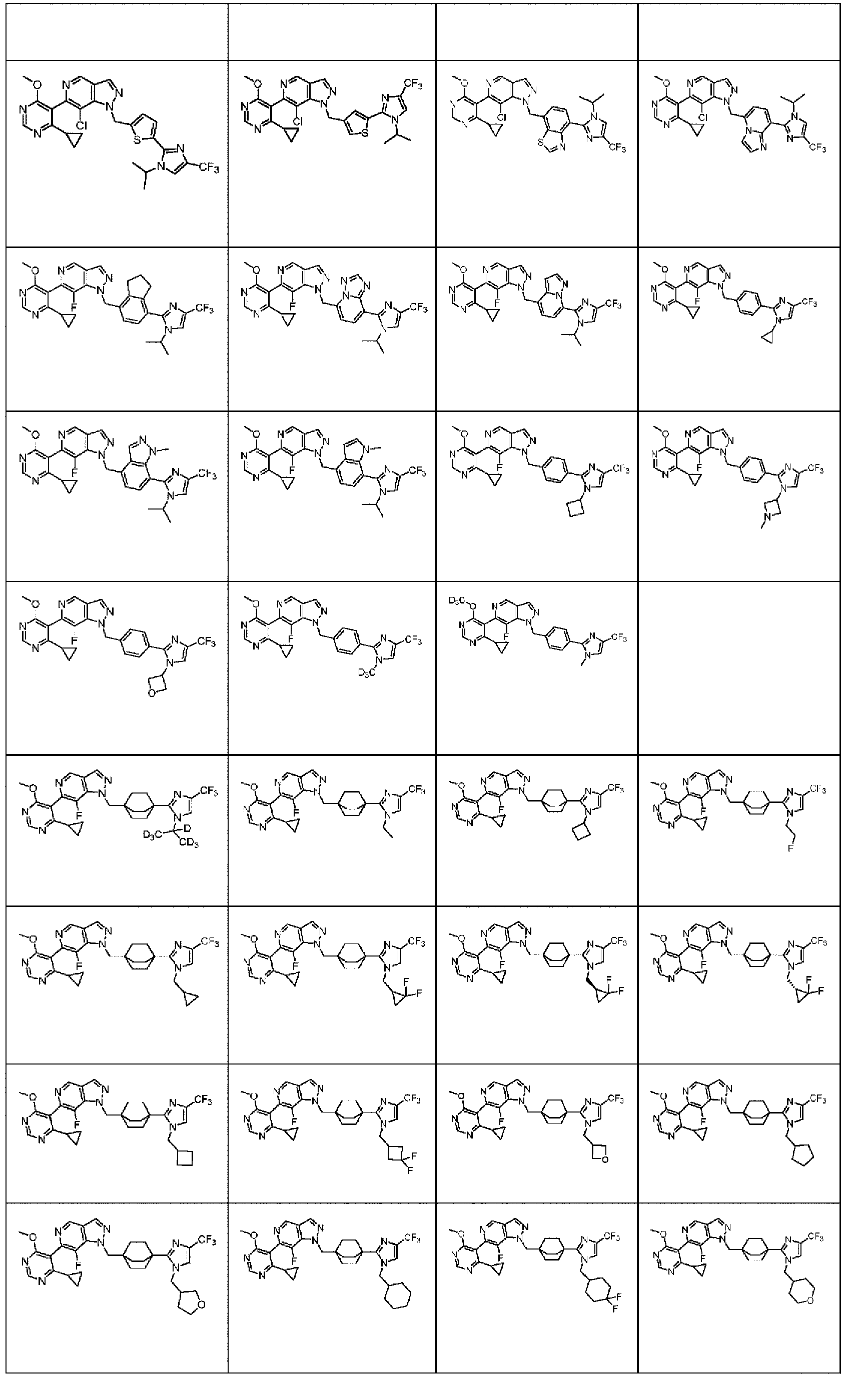

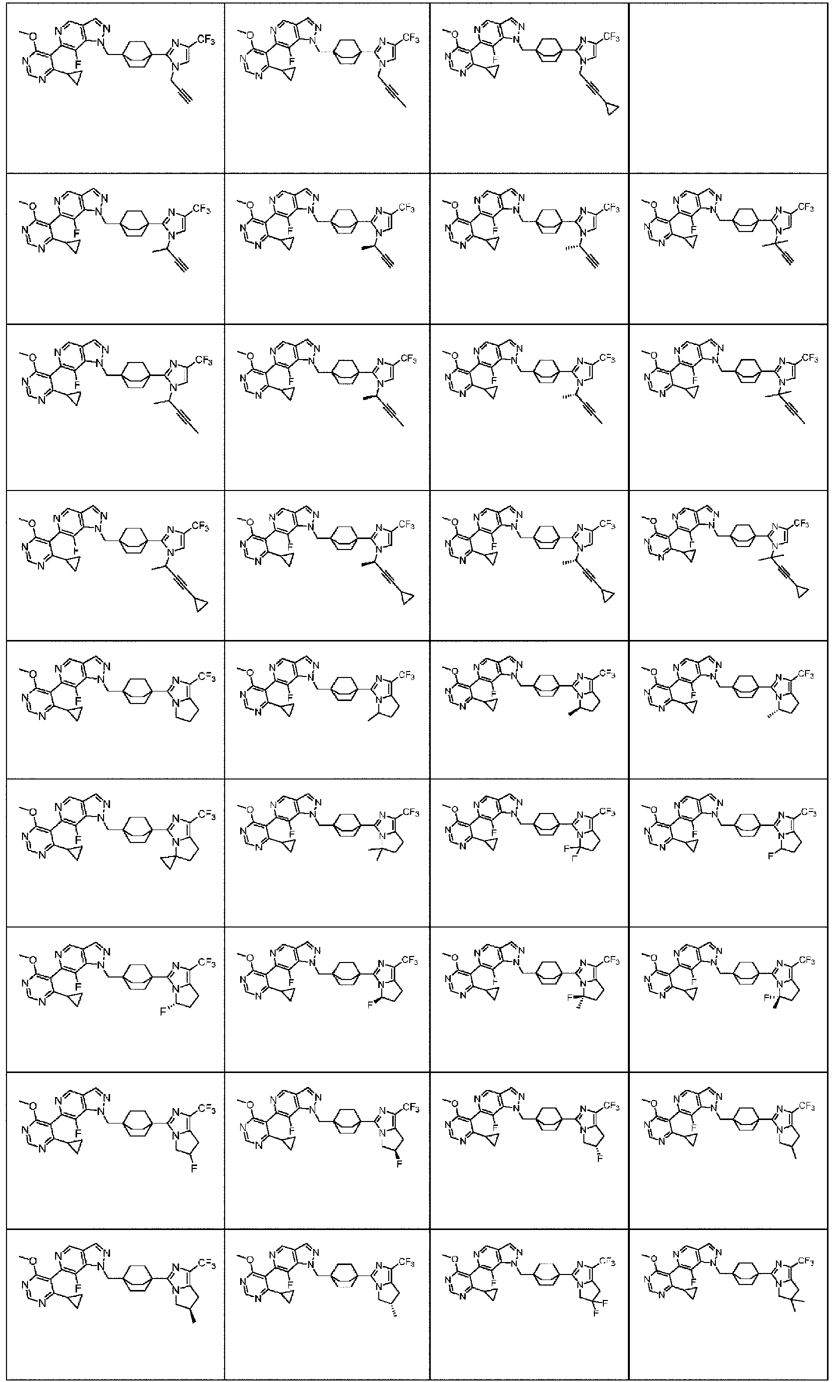

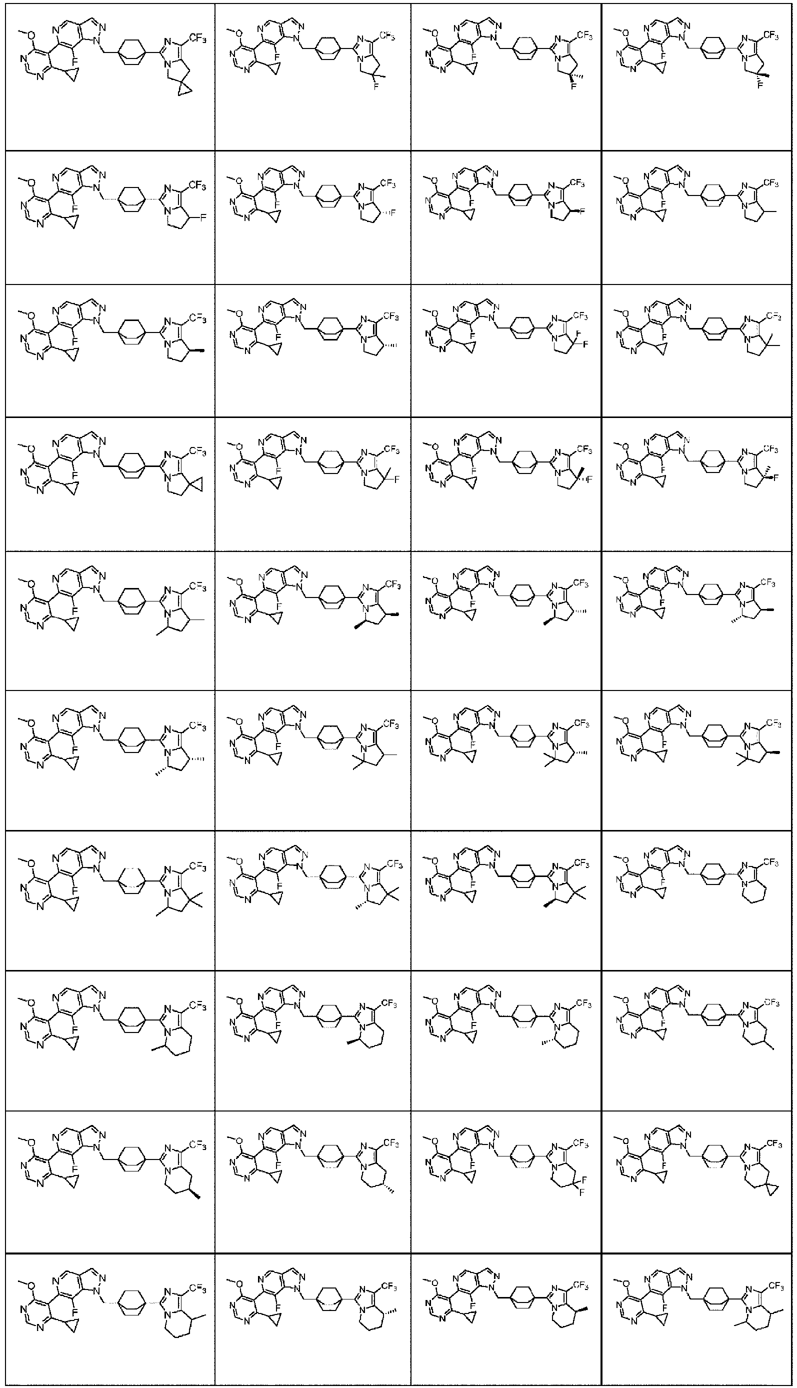

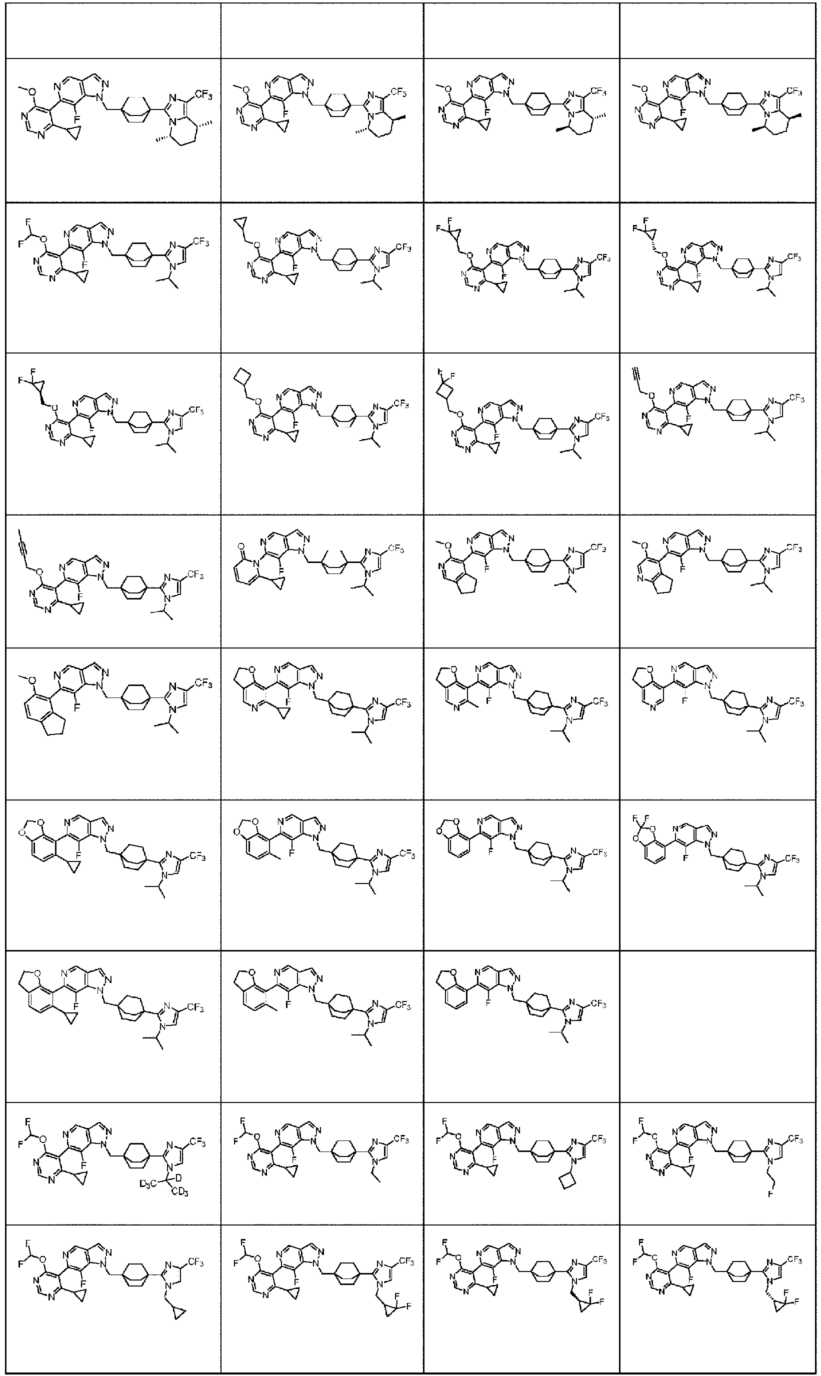

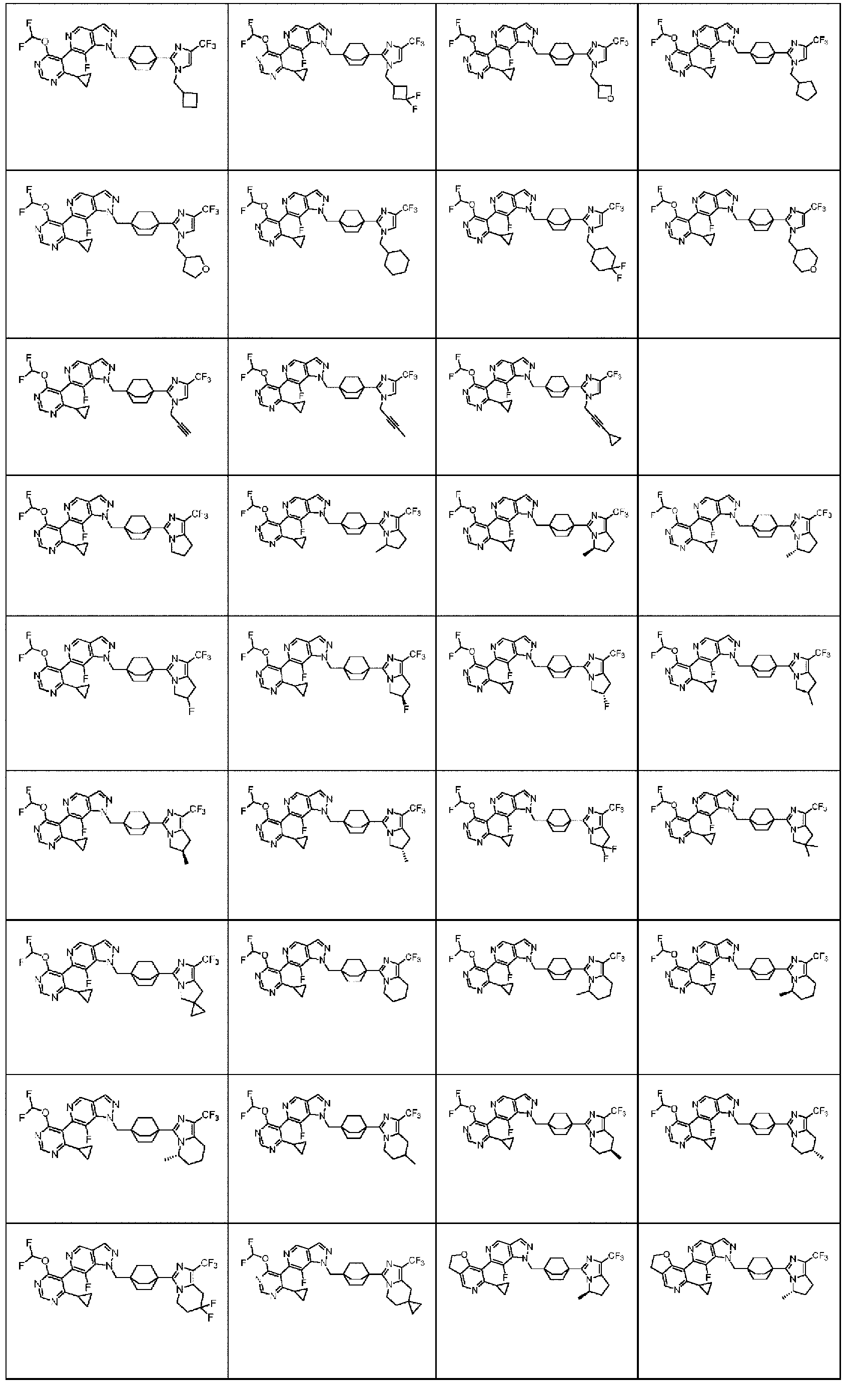

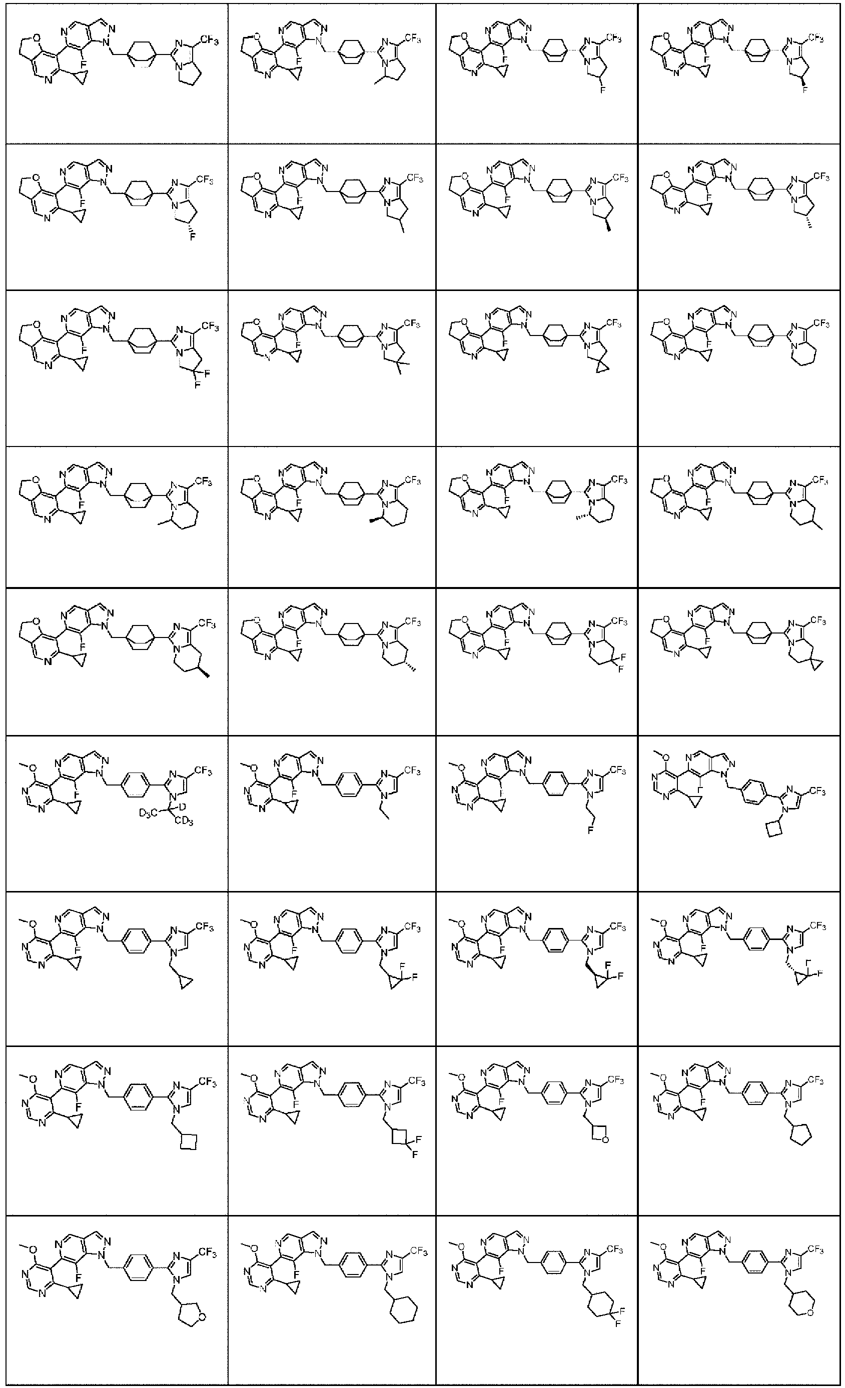

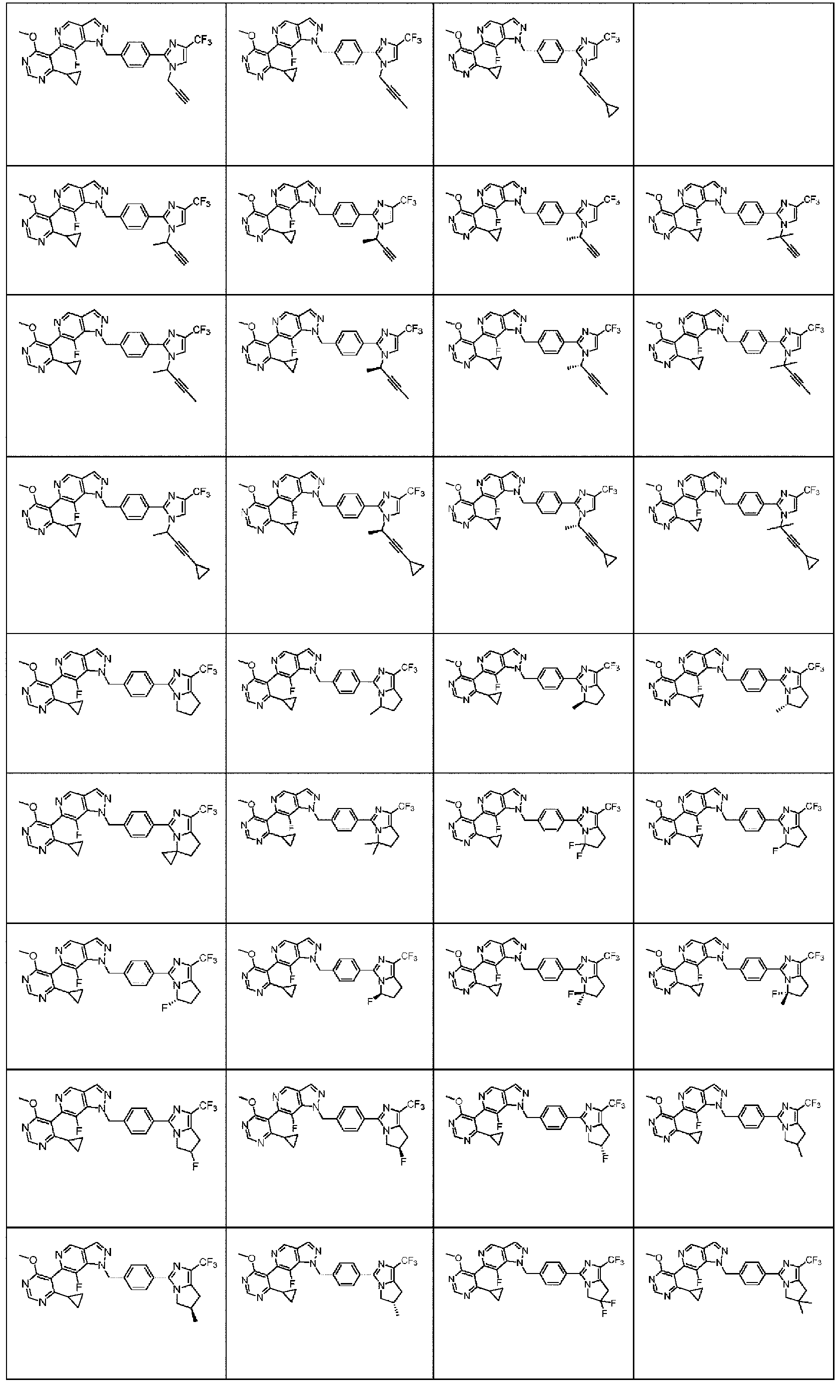

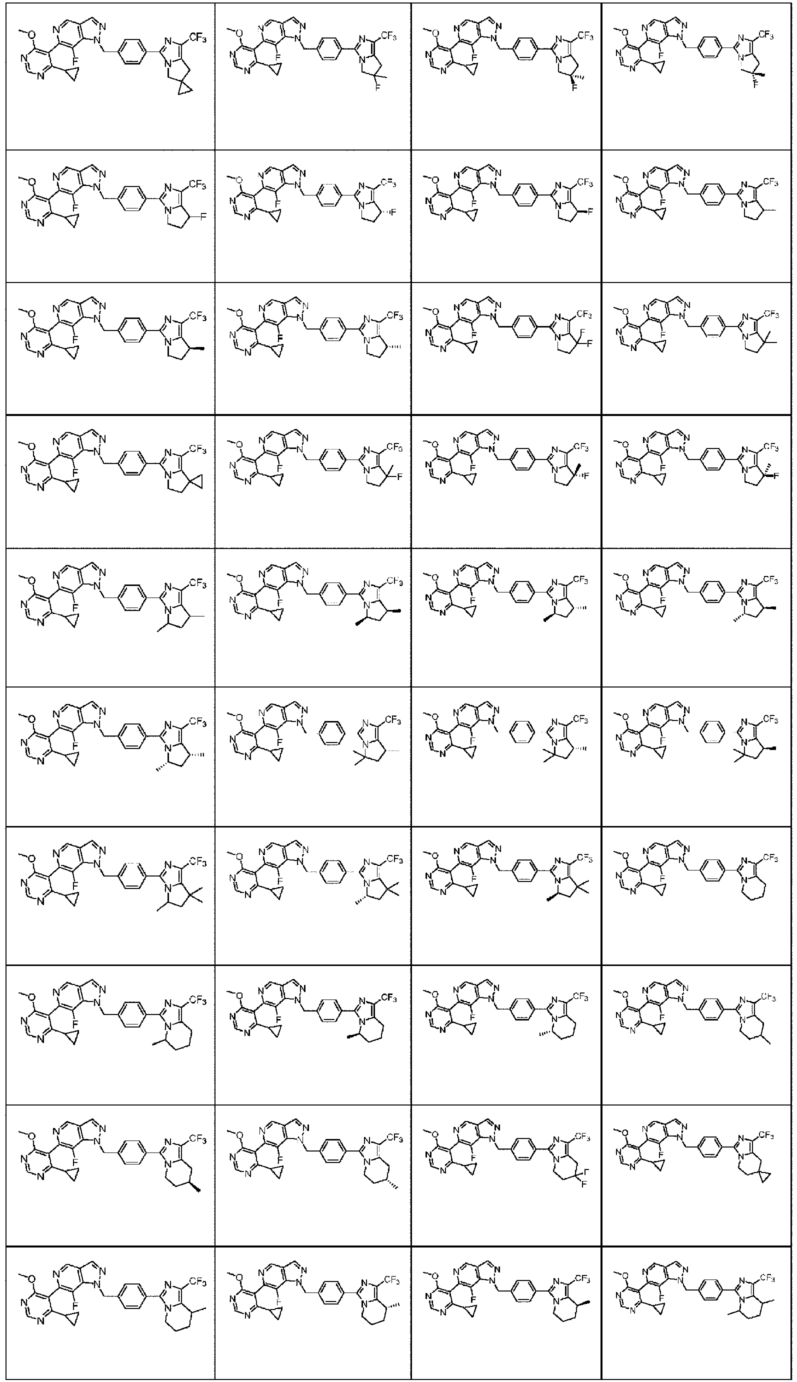

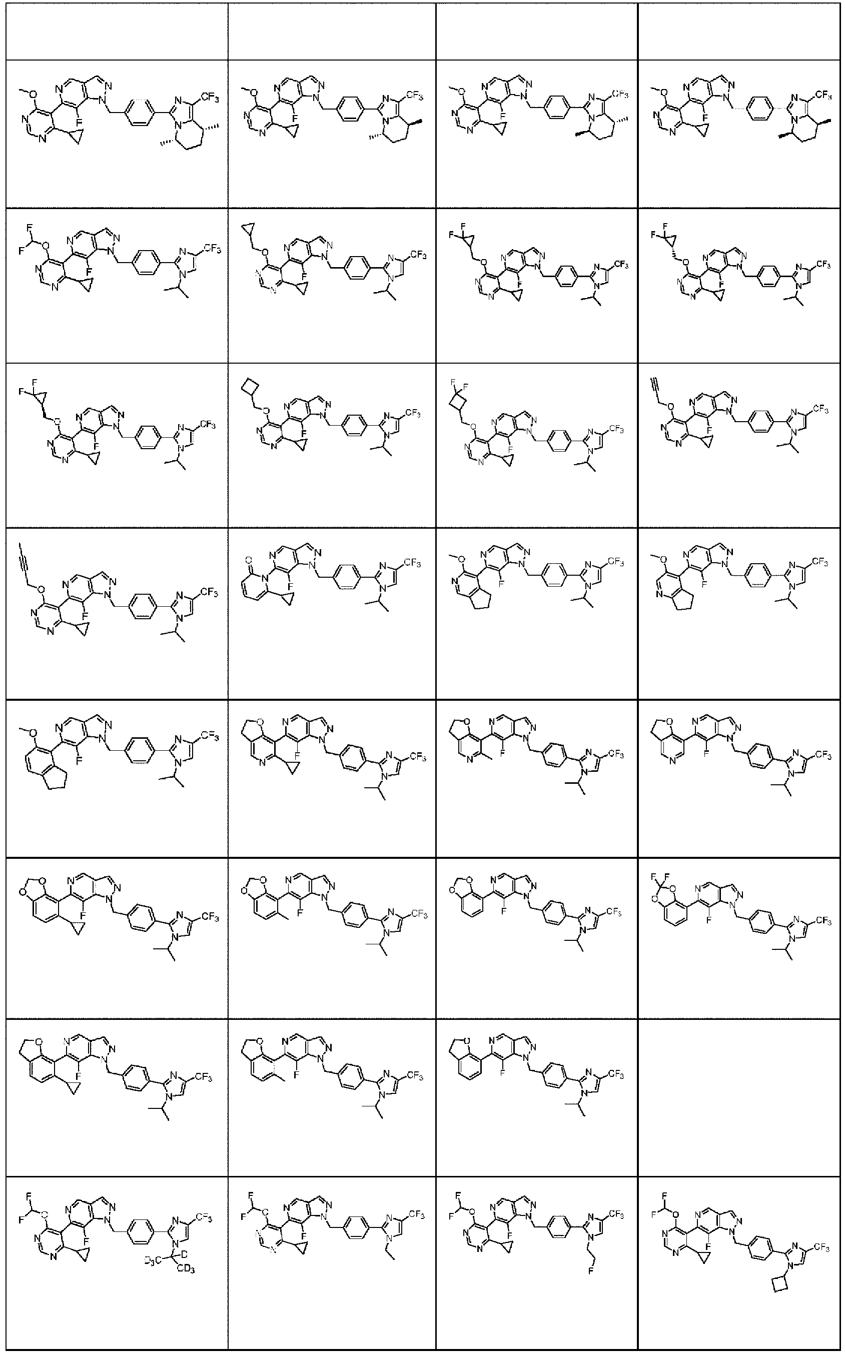

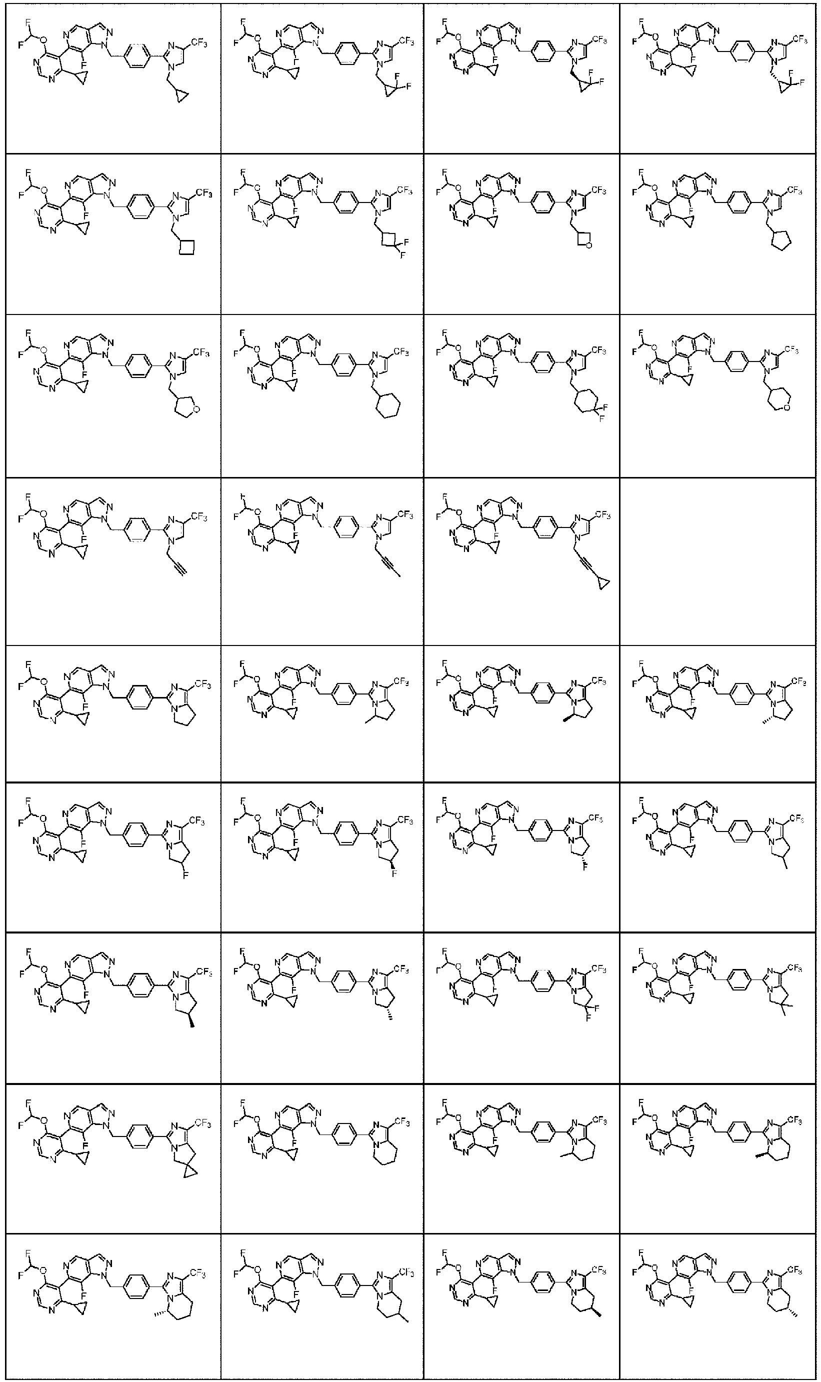

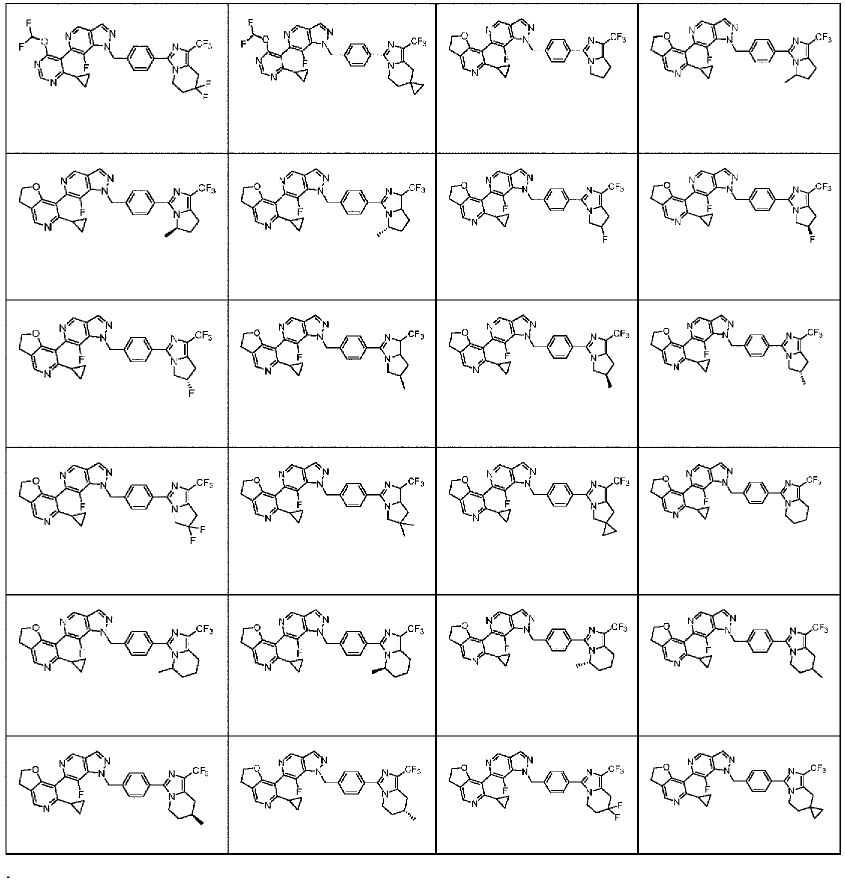

7. Composition pharmaceutique, **caractérisée en ce qu'**elle comprend le composé ou stéréoisomère, tautomère, substance deutérée, solvate, sel pharmaceutiquement acceptable ou cristal eutectique de celui-ci selon l'une quelconque des revendications 1 à 6, et un véhicule pharmaceutiquement acceptable.

8. Composition pharmaceutique ou préparation pharmaceutique, **caractérisée en ce qu'**elle comprend 1 à 1500 mg du composé ou stéréoisomère, substance deutérée, solvate, sel pharmaceutiquement acceptable ou cristal eutectique de celui-ci selon l'une quelconque des revendications 1 à 6, et un excipient pharmaceutique.

9. Composé ou stéréoisomère, tautomère, substance deutérée, solvate, sel pharmaceutiquement acceptable ou cristal eutectique de celui-ci selon l'une quelconque des revendications 1 à 6 destiné à être utilisé dans une méthode de traitement d'une maladie liée à l'activité ou à l'expression de USP1.

10. Composé ou stéréoisomère, tautomère, substance deutérée, solvate, sel pharmaceutiquement acceptable ou cristal eutectique de celui-ci destiné à être utilisé selon la revendication 9, **caractérisé en ce que** la maladie est choisie parmi des tumeurs.

11. Composé ou stéréoisomère, tautomère, substance deutérée, solvate, sel pharmaceutiquement acceptable ou cristal eutectique de celui-ci destiné à être utilisé selon la revendication 9, dans lequel une quantité thérapeutiquement efficace du composé est de 1 à 1500 mg.
